(19) Europäisches Patentamt
European Patent Office
Office européen des brevets

(11) EP 3 929 198 A1

(12) **EUROPEAN PATENT APPLICATION**
published in accordance with Art. 153(4) EPC

(43) Date of publication:
**29.12.2021 Bulletin 2021/52**

(21) Application number: **20760123.8**

(22) Date of filing: **11.02.2020**

(51) Int Cl.:
*C07D 487/08* (2006.01) *C07D 471/08* (2006.01)
*C07D 401/14* (2006.01) *C07D 413/14* (2006.01)
*C07D 417/14* (2006.01) *A61P 35/00* (2006.01)
*A61K 31/506* (2006.01) *A61K 31/504* (2006.01)
*A61K 31/444* (2006.01) *A61K 31/496* (2006.01)

(86) International application number:
**PCT/CN2020/074696**

(87) International publication number:
**WO 2020/168939 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.02.2019 CN 201910124584**
**24.05.2019 CN 201910437878**
**29.09.2019 CN 201910932095**

(71) Applicant: **Sichuan Kelun-Biotech Biopharmaceutical Co., Ltd.**
**Chengdu, Sichuan 611138 (CN)**

(72) Inventors:
- **CHEN, Zhonghui**
  **Chengdu, Sichuan 611138 (CN)**
- **DUAN, Shuangshuang**
  **Chengdu, Sichuan 611138 (CN)**
- **LI, Guiying**
  **Chengdu, Sichuan 611138 (CN)**
- **HAN, Runfeng**
  **Chengdu, Sichuan 611138 (CN)**
- **SUN, Qizheng**
  **Chengdu, Sichuan 611138 (CN)**
- **JING, Liandong**
  **Chengdu, Sichuan 611138 (CN)**
- **HAN, Xiaojun**
  **Chengdu, Sichuan 611138 (CN)**
- **TIAN, Qiang**
  **Chengdu, Sichuan 611138 (CN)**
- **SONG, Hongmei**
  **Chengdu, Sichuan 611138 (CN)**
- **XUE, Tongtong**
  **Chengdu, Sichuan 611138 (CN)**
- **WANG, Jingyi**
  **Chengdu, Sichuan 611138 (CN)**

(74) Representative: **Carpmaels & Ransford LLP**
**One Southampton Row**
**London WC1B 5HA (GB)**

(54) **HETEROCYCLIC COMPOUND, PHARMACEUTICAL COMPOSITION COMPRISING SAME, PREPARATION METHOD THEREFOR, AND USE THEREOF**

(57) The present invention relates to a heterocyclic compound, a pharmaceutical composition comprising same, a preparation method therefor, and a use thereof. Specifically, the compound of the present invention is represented by formula (I), and used for preventing or treating a disease or condition related to RET activity.

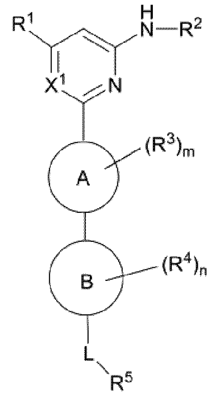

(I)

EP 3 929 198 A1

## Description

### Technical Field

The present disclosure relates to a novel heterocyclic compound, a pharmaceutical composition containing the same, a method for preparing the same, and use thereof in the prevention or treatment of a disease or condition associated with RET (Rearranged during transfection) activity.

### Background

Protein kinases are a class of enzymes catalyzing protein phosphorylation reactions. By mediating the process of cell signal transduction, protein phosphorylation regulates the physiological activities of cells, such as cell survival, proliferation, differentiation, apoptosis, and metabolism. The dysfunction of the protein kinases is closely associated with many diseases, including tumors, autoimmune diseases, inflammatory reactions, central nervous system diseases, cardiovascular diseases, diabetes, and the like.

As a protooncogene, RET encodes a RET protein that is a transmembrane receptor tyrosine protein kinase, and that consists of a cysteine-rich cadherin-like extracellular domain (binding to ligands), a transmembrane domain, and an intracellular domain with tyrosine kinase activity. The activated RET protein can activate multiple downstream signal pathways, including RAS/RAF/ERK pathway, PI3K/Akt pathway, and JNK pathway, thereby resulting in cell proliferation, migration, and differentiation. The alteration (mutation or fusion) of the RET gene and the abnormal expression of wild-type RET gene lead to abnormal activation of RET proteins, such that the signal pathways are overactive, which is one of the main mechanisms of carcinogenesis. Abnormally activated RET proteins are involved in the proliferation and invasion of different tumor cells through a variety of signal pathways, thereby affecting the occurrence and development of tumors. The alteration of the RET gene has a more significant effect on downstream cascade reactions, where the mutation of the RET gene is mainly associated with medullary thyroid cancer and papillary thyroid cancer, and the fusion of the RET gene is mainly associated with non-small cell lung cancer and chronic myeloid leukemia. Therefore, inhibiting the RET activity has great medical value (Nature Reviews Cancer, 2014, 14 (3): 173-86).

RET inhibitors have great potentials to treat and prevent a variety of diseases (such as a tumor, and irritable bowel syndrome). At present, five compounds are in a clinical trial stage, and compounds from many companies are in a preclinical research stage. However, at present, no inhibitors on the market are mainly targeted for RET. Therefore, it is necessary to develop novel RET inhibitors with high efficacy and low toxicity to meet clinical needs.

### Summary

The present disclosure provides a novel heterocyclic compound, which has a desirable inhibitory effect on RET, and has desirable pharmacokinetic properties, safety, and the like.

In one aspect, the present disclosure provides a compound of formula I, a stereoisomer, tautomer, or mixture thereof, a N-oxide thereof, a pharmaceutically acceptable salt, eutecticum, polymorph, or solvate thereof, or a stable isotope derivative, metabolite, or prodrug thereof:

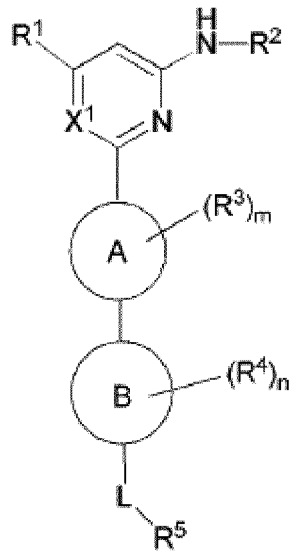

Formula I

where:

ring A is selected from $C_{6-10}$ aromatic ring and 5-6-membered heteroaromatic ring;
ring B is selected from $C_{3-8}$ cycloalkyl and 4-11-membered heterocyclyl;
$X^1$ is selected from CH and N;
$R^1$ is selected from the group consisting of H, halogen, hydroxy, cyano, $C_{1-6}$ alkyl, $C_{1-6}$ heteroalkyl (e.g., $C_{1-6}$ alkoxy), $C_{3-8}$ cycloalkyl, 4-10-membered heterocyclyl, and $-NR^{20a}R^{20b}$, and the alkyl, heteroalkyl (for example, alkoxy), cycloalkyl, and heterocyclyl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, and $C_{1-4}$ heteroalkyl (e.g., $C_{1-4}$ alkoxy);
$R^2$ is selected from the group consisting of $C_{1-6}$ alkyl, $C_{1-6}$ heteroalkyl, $C_{3-8}$ cycloalkyl, 4-10-membered heterocyclyl, 5-10-membered heteroaryl, and $-C(=O)R^{21}$, and the alkyl, heteroalkyl, cycloalkyl, heterocyclyl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;
$R^3$ and $R^4$ are absent or are, at each occurrence, each independently selected from the group consisting of hydroxy, halogen, CN, $C_{1-6}$ alkyl, $C_{1-6}$ heteroalkyl (e.g., $C_{1-6}$ alkoxy), and $C_{3-6}$ cycloalkyl, the alkyl, heteroalkyl (for example, alkoxy), and cycloalkyl are each optionally substituted with one or more substituents selected from the group consisting of: halogen, CN, $C_{1-4}$ alkyl, $C_{1-4}$ haloalkyl, $C_{1-4}$ alkoxy, and $C_{1-4}$ haloalkoxy; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl;
L is selected from the group consisting of -O-, -S-, -S(O)-, $-S(O)_2-$, $-N=CR^{21}-$, $-N(R^{23a})-C(O)-$, $C_{1-6}$ alkylene, $C_{1-6}$ heteroalkylene, $C_{2-6}$ alkenylene, $C_{2-6}$ alkynylene,

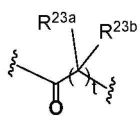 , 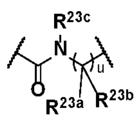 ,

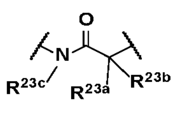 , 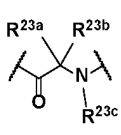 , 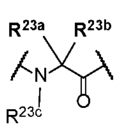 , 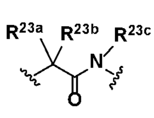 , 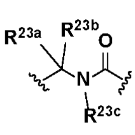 , 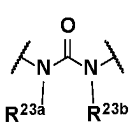 , 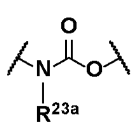 , 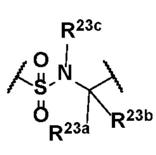 ,

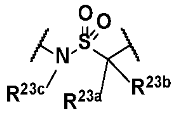, 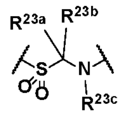, 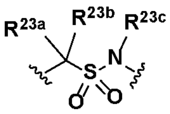, 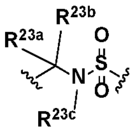, 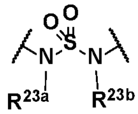, 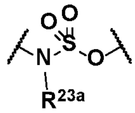 and 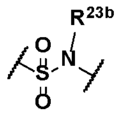, the alkylene, heteroalkylene, alkenylene, and alkynylene are each optionally substituted with one or more substituents selected from the group consisting of: hydroxy, halogen, CN, $NO_2$, $C_{1-6}$ alkyl, $C_{1-6}$ haloalkyl, $C_{1-6}$ hydroxyalkyl, $C_{1-6}$ haloalkoxy, $C_{1-6}$ heteroalkyl (e.g., $C_{1-6}$ alkoxy), and $C_{3-8}$ cycloalkyl; or L is $-N(R^{23a})-$;

$R^5$ is selected from the group consisting of hydroxy, halogen, CN, $NO_2$, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-OR^{21}$, $-SR^{21}$,$-S(=O)R^{22}$, $-S(=O)_2R^{22}$, $-S(=O)NR^{20a}R^{20b}$, $-S(=O)_2NR^{20a}R^{20b}$, $-NR^{20a}S(=O)R^{20b}$, $-NR^{20a}S(=O)_2R^{20b}$, $-C(=O)R^{21}$, $-C(=O)NR^{23a}R^{23b}$, $-NR^{23a}C(=O)R^{23b}$, $-OC(=O)NR^{23a}R^{23b}$, and $-NR^{24a}C(=O)NR^{25a}R^{25b}$, and the alkyl, heteroalkyl (e.g., alkoxy), alkenyl, alkynyl, cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$,$-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl;

$R^{20a}$, $R^{20b}$, $R^{23a}$, $R^{23b}$, $R^{23c}$, $R^{24a}$, $R^{25a}$, and $R^{25b}$ are each independently selected from the group consisting of H, OH, $C_{1-6}$ alkyl, $C_{1-6}$ alkoxy, and $C_{3-8}$ cycloalkyl; or $R^{20a}$ and $R^{20b}$, $R^{23a}$ and $R^{23b}$, or $R^{25a}$ and $R^{25b}$ form, together with an atom to which they are attached, a 3-8-membered cycloalkyl or heterocyclyl, and the alkyl, alkoxy, cycloalkyl, and heterocyclyl are each optionally substituted with one or more substituents selected from the group consisting of: OH, CN, halogen, $NO_2$, $C_{1-4}$ alkyl, $C_{1-4}$ alkoxy, $C_{1-4}$ hydroxyalkyl, $C_{1-4}$ haloalkyl, and $C_{1-4}$ haloalkoxy;

$R^{30a}$, $R^{30b}$, $R^{33a}$, $R^{33b}$, $R^{34a}$, $R^{35a}$, and $R^{35b}$ are each independently selected from the group consisting of H, $C_{1-6}$ alkyl,$C_{1-6}$ haloalkyl, $C_{1-6}$ hydroxyalkyl, $C_{1-6}$ alkoxy, and $C_{1-6}$ haloalkoxy;

$R^{21}$, $R^{22}$, $R^{31}$, and $R^{32}$ are each independently selected from the group consisting of $C_{1-6}$ alkyl, $C_{1-6}$ alkoxy, $C_{3-8}$ cycloalkyl, 4-10-membered heterocyclyl, $C_{6-12}$ aryl, and 5-10-membered heteroaryl, and the alkyl, alkoxy, cycloalkyl, heterocyclyl, aryl, and heteroaryl are each optionally substituted with one or more substituents selected from the group consisting of: OH, halogen, CN, $C_{1-4}$ alkyl, $C_{1-4}$ alkoxy, $C_{1-4}$ haloalkyl, $C_{1-4}$ haloalkoxy, $C_{3-6}$ cycloalkyl, and 4-10-membered heterocyclyl;

m is 0, 1, 2, 3, or 4;

n is 0, 1, 2, 3, or 4;

t is 0, 1, 2, 3, or 4; and

u is 0, 1, 2, 3, or 4;

provided that when ring B is a piperazine ring and $X^1$ is CH, $R^2$ is not 4-$CF_3$-pyridin-2-yl or 4-CN -pyridin-2-yl.

In another aspect, the present disclosure provides a pharmaceutical composition, comprising a prophylactically or therapeutically effective amount of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof. Optionally, the pharmaceutical composition further comprises one or more pharmaceutically acceptable carriers.

In another aspect, the present disclosure provides use of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above in the preparation of a drug for preventing or treating a disease or condition associated with RET activity.

In another aspect, the present disclosure provides the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above, for use in the prevention or treatment of a disease or condition associated with RET activity.

In another aspect, the present disclosure provides a method for preventing or treating a disease or condition associated with RET activity, including administering to an individual in need thereof an effective amount of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof,

or the pharmaceutical composition as described above.

In another aspect, the present disclosure provides a method for preparing the compound of the present disclosure.

## Brief Description of the Drawings

FIG. 1 shows in vivo efficacy test results of Compound 17 and control compound BLU-667 in a subcutaneous xenograft model of medullary thyroid carcinoma TT cells.

## Detailed Description of the Invention

### Definitions

Unless otherwise defined in the context, all technical terms and scientific terms used herein are intended to have the same meaning as commonly understood by those skilled in the art. The reference to a technology used herein is intended to refer to a technology generally understood in the art, including those technological alterations or equivalent technological replacements that are obvious to those skilled in the art. While it is believed that the following terms are well understood by those skilled in the art, the following definitions are still set forth to better explain the present disclosure.

The term "including," "comprising," "having," "containing," or "relating to" and additional variations thereof herein are inclusive or open-ended, and do not exclude additional unlisted elements or method steps, although additional unlisted elements or method steps do not necessarily exist (i.e., these terms also encompass the terms "substantially consisting of" and "consisting of").

As used herein, the term "alkyl" is defined as a linear or branched saturated aliphatic hydrocarbon. In some embodiments, an aryl group has from 1 to 12, e.g., from 1 to 6, carbon atoms. For example, as used herein, the terms "$C_{1-6}$ alkyl" and "$C_{1-4}$ alkyl" refer to a linear or branched radical group having from 1 to 6 carbon atoms and a linear or branched radical group having from 1 to 4 carbon atoms respectively (e.g., methyl, ethyl, n-propyl, isopropyl, n-butyl, isobutyl, sec-butyl, tert-butyl, n-pentyl, isopentyl, neopentyl, or n-hexyl), which are optionally substituted with one or more (e.g., from 1 to 3) suitable substituents, e.g., halogen (in this case, the radical group is termed "haloalkyl") (e.g., $CH_2F$, $CHF_2$, $CF_3$, $CCl_3$, $C_2F_5$, $C_2Cl_5$, $CH_2CF_3$, $CH_2Cl$, or $-CH_2CH_2CF_3$). The term "$C_{1-4}$ alkyl" refers to a linear or branched aliphatic hydrocarbon chain having from 1 to 4 carbon atoms (i.e., methyl, ethyl, n-propyl, isopropyl, n-butyl, isobutyl, sec-butyl, or tert-butyl). The term "alkylene" represents a corresponding divalent radical group, including, e.g., "$C_{1-8}$ alkylene," "$C_{1-6}$ alkylene," "$C_{1-4}$ alkylene," and the like, and its specific examples include, but are not limited to: methylene ($-CH_2-$), ethylidene ($-CH_2CH_2-$ or $-CH(CH_3)-$), propylidene ($-CH_2CH_2CH_2-$), isopropylidene ($-CH(CH_3)CH_2-$), butylidene, pentylidene, hexylidene, and the like. The alkylene is optionally substituted with one or more (e.g., from 1 to 3) same or different substituents.

As used herein, the term "heteroalkyl" refers to an optionally substituted alkyl radical that has one or more backbone chain atoms selected from atoms other than carbon, such as oxygen, nitrogen, sulfur, phosphorus, or combinations thereof. A numerical range (e.g., $C_{1-6}$ heteroalkyl) that may be given refers to the number of carbons in a chain, including from 1 to 6 carbon atoms in this example. For example, $-CH_2OCH_2CH_3$ group is termed $C_3$ heteroalkyl. The points of attachment to the rest of the molecule may be through a heteroatom or carbon atom in the heteroalkyl chain. The term "heteroalkylene" represents a corresponding divalent radical group, including, for example, "$C_{1-6}$ heteroalkylene," "$C_{1-4}$ heteroalkylene," and the like.

As used herein, the term "haloalkyl" refers to an alkyl radical substituted with one or more (e.g., from 1 to 3) same or different halogen atoms, and the term "$C_{1-8}$ haloalkyl," "$C_{1-6}$ haloalkyl," and "$C_{1-4}$ haloalkyl" refer to a haloalkyl radical having from 1 to 8 carbon atoms, a haloalkyl radical having from 1 to 6 carbon atoms, and a haloalkyl radical having from 1 to 4 carbon atoms respectively, such as $-CF_3$, $-C_2F_5$, $-CHF_2$, $-CH_2F$, $-CH_2CF_3$, $-CH_2Cl$, or $-CH_2CH_2CF_3$.

As used herein, the term "hydroxyalkyl" refers to a radical group formed by substituting hydrogen atom(s) in an alkyl radical with one or more hydroxy, e.g., $C_{1-4}$ hydroxyalkyl or $C_{1-3}$ hydroxyalkyl, and its examples include, but are not limited to, hydroxymethyl, hydroxyethyl, hydroxypropyl, hydroxybutyl, $-CH(OH)CH_3$, $-C(CH_3)_2OH$, and the like.

As used herein, the term "alkoxy" refers to a radical group formed by inserting an oxygen atom into any reasonable position of an alkyl radical (as defined above), and is preferably $C_{1-8}$ alkoxy, $C_{1-6}$ alkoxy, $C_{1-4}$ alkoxy, or $C_{1-3}$alkoxy. Representative examples of $C_{1-6}$ alkoxy include, but are not limited to, methoxy, ethoxy, propoxy, isopropoxy, n-propoxy, isopropoxy, n-butoxy, isobutoxy, tert-butoxy, pentyloxy, hexyloxy, $-CH_2-OCH_3$, and the like, and the alkoxy is optionally substituted with one or more (e.g., from 1 to 3) same or different substituents.

As used herein, the term "alkoxylene" refers to a divalent alkoxy group, such as $-OCH_2-$,$-OCH(CH_3)CH_2-$, $-OCH_2CH_2O-$, and $-CH_2CH_2O-$, and the alkoxylene is optionally substituted with one or more (e.g., from 1 to 3) same

or different substituents.

As used herein, the term "alkenyl" refers to a linear or branched monovalent hydrocarbyl containing one or more double bonds and having from 2 to 6 carbon atoms ("$C_{2-6}$ alkenyl"). The alkenyl is, for example, $-CH=CH_2$, $-CH_2CH=CH_2$, $-C(CH_3)=CH_2$, $-CH_2-CH=CH-CH_3$, 2-pentenyl, 3-pentenyl, 4-pentenyl, 2-hexenyl, 3-hexenyl, 4-hexenyl, 5-hexenyl, 2-methyl-2-propenyl, and 4-methyl-3-pentenyl. When the compound of the present disclosure contains an alkenyl radical, the compound may exist in a pure E (entgegen) form, a pure Z (zusammen) form, or a form of any mixture thereof. The term "alkenylene" is a corresponding divalent radical group, including, for example, "$C_{2-6}$alkenylene," and "$C_{2-4}$ alkenylene", and its specific examples include, but are not limited to: $-CH=CH-$, $-CH_2CH=CH-$, $-C(CH_3)=CH-$, butenylene, pentenylene, hexenylene, cyclopentenylene, cyclohexenylene, and the like.

As used herein, the term "alkynyl" represents a monovalent hydrocarbyl containing one or more triple bonds, and preferably has 2, 3, 4, 5, or 6 carbon atoms, for example, ethynyl, 2-propynyl, 2-butynyl, 1,3-butadiynyl. The alkynyl is optionally substituted with one or more (e.g., from 1 to 3) same or different substituents. The term "alkynylene" is a corresponding divalent radical group, including, e.g., "$C_{2-8}$ alkynylene," "$C_{2-6}$ alkynylene," and "$C_{2-4}$ alkynylene." Its examples include, but are not limited to,

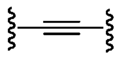 , 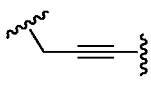 , 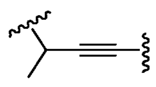 , 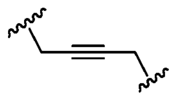 ,

and the like. The alkynylene is optionally substituted with one or more (e.g., from 1 to 3) same or different substituents.

As used herein, the term "condensed ring" or "fused ring" refers to a ring system formed by two or more than two ring structures sharing two adjacent atoms with each other.

As used herein, the term "spiro ring" refers to a ring system formed by two or more than two ring structures sharing one ring atom with each other.

As used herein, the term "bridged ring" refers to a ring system formed by two or more than two ring structures sharing two atoms (that are not directly connected) with each other.

As used herein, the term "cycloalkyl" refers to a saturated or unsaturated non-aromatic monocyclic or multicyclic (such as bicyclic) hydrocarbon ring radical, including but not limited to monocycloalkyl (such as cyclopropyl, cyclobutyl, cyclopentyl, cyclohexyl, cycloheptyl, cyclooctyl, and cyclononyl) and bicycloalkyl, including a spiro ring, fused ring, or bridged ring system (i.e., spirocycloalkyl, condensed (fused) cycloalkyl, and bridged cycloalkyl, such as bicyclo[1.1.1]pentyl, and bicyclo[2.2.1]heptyl). In the present disclosure, a cycloalkyl radical is optionally substituted with one or more (e.g., from 1 to 3) same or different substituents. A carbon atom on a cycloalkyl radical is optionally substituted with an oxo group (i.e., forming C=O). The term "$C_{3-8}$ cycloalkyl" refers to a cycloalkyl radical having from 3 to 8 ring-forming carbon atoms, such as $C_{3-6}$ cycloalkyl, which may be a monocycloalkyl radical, such as cyclopropyl, cyclobutyl, cyclopentyl, cyclohexyl, cycloheptyl, or cyclooctyl, and may also be a bicycloalkyl radical, such as $C_{5-8}$ spirocycloalkyl, $C_{5-8}$ bridged cycloalkyl, $C_{5-8}$ fused cycloalkyl, $C_{5-6}$ spirocycloalkyl, $C_{5-6}$ bridged cycloalkyl, or $C_{5-6}$ condensed cycloalkyl.

As used herein, the term "cycloalkoxy" means -O-cycloalkyl, where the cycloalkyl is as defined above. Representative examples of cycloalkoxy groups include, but are not limited to, cyclopropoxy, cyclobutoxy, cyclopentoxy, cyclohexoxy, and the like.

As used herein, the term "heterocyclyl" or "heterocyclic ring" refers to a monocyclic or multicyclic (for example, condensed, spiro, or bridged cyclic) radical group having 2 or more than 2 (e.g., 3, 4, 5, 6, 7, 8, 9, 10, 11, 12, 13, or 14) carbon atoms, and one or more (for example, 1, 2, 3, or 4) heteroatoms, where the heteroatoms include, but are not limited to, an oxygen atom, a nitrogen atom, and a sulfur atom, and the carbon atom and heteroatom on a heterocyclyl are optionally substituted with an oxo group (for example, forming C=O, S(=O), or $S(=O)_2$).

As used herein, the term "4-11-membered heterocyclyl" means a heterocyclyl containing from 4 to 11 ring atoms, including but not limited to 4-10-membered heterocyclyl, 4-9-membered heterocyclyl, 4-8-membered heterocyclyl, 4-7-membered heterocyclyl, 5-6-membered heterocyclyl, 3-8-membered heterocyclyl, 3-7-membered heterocyclyl, 4-7-membered nitrogen-containing heterocyclyl, 4-7-membered oxygen-containing heterocyclyl, 4-7-membered sulfur-containing heterocyclyl, 5-6-membered nitrogen-containing heterocyclyl, 5-6-membered oxygen-containing heterocyclyl, 5-6-membered sulfur-containing heterocyclyl, and the like. The "nitrogen-containing heterocyclyl," "oxygen-containing heterocyclyl," and "sulfur-containing heterocyclyl" each optionally further includes one or more additional heteroatoms selected from oxygen, nitrogen, and sulfur. Examples of 4-11-membered heterocyclyl include, but are not limited to, oxiranyl, aziridinyl, azacyclobutyl, oxacyclobutyl, tetrahydrofuryl, pyrrolidinyl, pyrrolidonyl (e.g.,

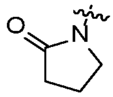),

imidazolidinyl, pyrazolidinyl, tetrahydropyranyl, piperidinyl, morpholinyl, dithianyl, thiomorpholinyl, piperazinyl, and trithianyl.

As used herein, the term "heterocyclyl" encompasses a condensed ring structure, and the points of attachment of the condensed ring structure to additional radical groups may be on any ring in the condensed ring structure. Accordingly, the heterocyclyl of the present disclosure further includes, but is not limited to, heterocyclyl-heterocyclyl, heterocyclyl-cycloalkyl, monoheterocyclyl-monoheterocyclyl, monoheterocyclyl-monocycloalkyl, for example, 3-7-membered (mono)heterocyclyl-3-7-membered (mono)heterocyclyl, 3-7-membered (mono)heterocyclyl-(mono)cycloalkyl, and 3-7-membered (mono)heterocyclyl-$C_{4-6}$ (mono)cycloalkyl. Its examples include, but are not limited to, pyrrolidinyl-cyclopropyl, cyclopentyl-azacyclopropyl, pyrrolidinyl-cyclobutyl, pyrrolidinyl-pyrrolidinyl, pyrrolidinyl-piperidinyl, pyrrolidinyl-piperazinyl, piperidinyl-morpholinyl,

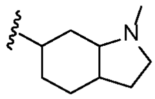, 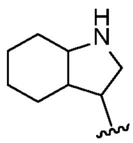,

or

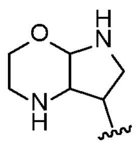.

As used herein, the term "heterocyclyl" encompasses bridged heterocyclyl and spiroheterocyclyl.

As used herein, the term "bridged heterocyclic ring" refers to a cyclic structure that is formed by two saturated rings sharing two ring atoms which are not directly connected and that comprises one or more (for example, 1, 2, 3, or 4) heteroatoms (such as an oxygen atom, a nitrogen atom, and/or a sulfur atom), including but not limited to 7-10-membered bridged heterocyclic ring, 8-10-membered bridged heterocyclic ring, 7-10-membered nitrogen-containing bridged heterocyclic ring, 7-10-membered oxygen-containing bridged heterocyclic ring, 7-10-membered sulfur-containing bridged heterocyclic ring, and the like, such as

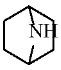, 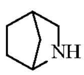, 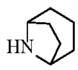, 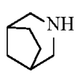, 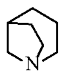, 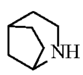, 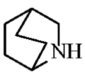, 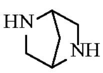, 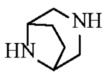, 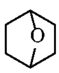, 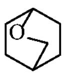, 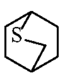, 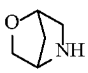, 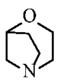, 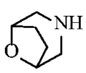, 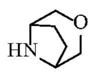, 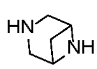, 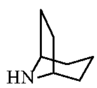,

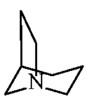, 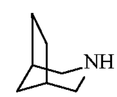, and 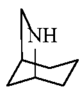.

The "nitrogen-containing bridged heterocyclic ring," "oxygen-containing bridged heterocyclic ring," and "sulfur-containing bridged heterocyclic ring" optionally further comprise one or more additional heteroatoms selected from oxygen, nitrogen, and sulfur.

As used herein, the term "spiroheterocyclic ring" refers to a cyclic structure that is formed by two or more than two saturated rings sharing one ring atom and that comprises one or more (for example, 1, 2, 3, or 4) heteroatoms (such as an oxygen atom, a nitrogen atom, and/or a sulfur atom), including but not limited to 5-10-membered spiroheterocyclic

ring, 6-10-membered spiroheterocyclic ring, 6-10-membered nitrogen-containing spiroheterocyclic ring, 6-10-membered oxygen-containing spiroheterocyclic ring, 6-10-membered sulfur-containing spiroheterocyclic ring, and the like, such as

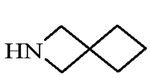, 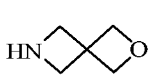, 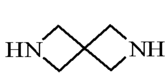, 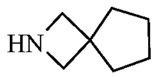,

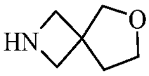, 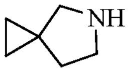, 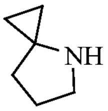, 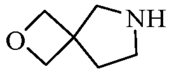, 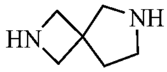, 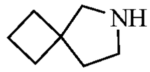, 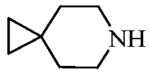,

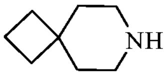, 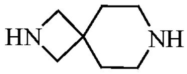, 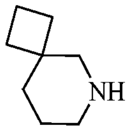, 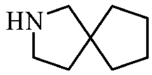, 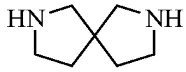, 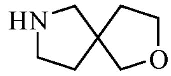,

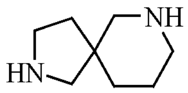, and 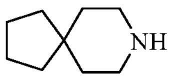.

The "nitrogen-containing spiroheterocyclic ring", "oxygen-containing spiroheterocyclic ring", and "sulfur-containing spiroheterocyclic ring" optionally further comprise one or more additional heteroatoms selected from oxygen, nitrogen, and sulfur. The term "6-10-membered nitrogen-containing spiroheterocyclyl" refers to a spiroheterocyclyl that totally comprises from 6-10 ring atoms and where at least one of the ring atoms is a nitrogen atom.

In the present disclosure, a heterocyclyl may be fused with an aryl group to form a fused ring structure. Examples of fused ring structures include, but are not limited to:

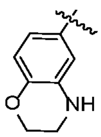, 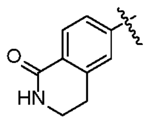,

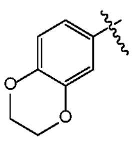, 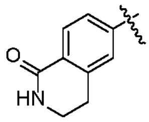, and 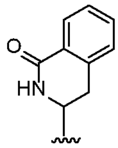.

As used herein, the term "aryl" or "aromatic ring" refers to an all-carbon monocyclic or fused multicyclic aromatic group having a conjugated π-electron system. As used herein, the term "$C_{6-12}$ aryl (aromatic ring)" means an aryl group (aromatic ring) comprising from 6 to 12 carbon atoms, and is preferably $C_{6-10}$ aryl group (aromatic ring), preferably phenyl or naphthyl. An aryl group is optionally substituted with one or more (e.g., from 1 to 3) same or different substituents (such as halogen, OH, CN, $NO_2$, or $C_1$-$C_6$ alkyl).

As used herein, the term "heteroaryl" or "heteroaromatic ring" refers to a monocyclic or multicyclic aromatic group comprising one or more same or different heteroatoms, including a monocyclic heteroaryl group and a bicyclic or multicyclic ring system comprising at least one heteroaromatic ring (an aromatic ring system comprising at least one heteroatom), which may have 5, 6, 7, 8, 9, 10, 11, 12, 13, or 14 ring atoms, for example, 5, 6, 7, 8, 9, or 10 ring atoms. The heteroatom may be oxygen, nitrogen, or sulfur. A carbon atom and a heteroatom on the heteroaryl are optionally substituted with an oxo group (for example, forming C=O, S(=O), or $S(=O)_2$).

As used herein, the term "5- 10-membered heteroaryl" or "5- 10-membered heteroaromatic ring" means a heteroaryl group (heteroaromatic ring) comprising from 5 to 10 (e.g., from 5 to 6) ring atoms, including 5- 10-membered nitrogen-containing heteroaryl group, 5- 10-member oxygen-containing heteroaryl group, 5- 10-membered sulfur-containing heteroaryl group, 5-6-membered nitrogen-containing heteroaryl group, 5-6-membered oxygen-containing heteroaryl group, 5-6-membered sulfur-containing heteroaryl group, and the like. The "nitrogen-containing heteroaryl," "oxygen-containing heteroaryl," and "sulfur-containing heteroaryl" each optionally comprise one or more additional heteroatoms selected from oxygen, nitrogen, and sulfur. Examples thereof include, but are not limited to, thienyl, furyl, pyrrolyl, oxazolyl, thiazolyl, imidazolyl, pyrazolyl, isoxazolyl, isothiazolyl, triazolyl, tetrazolyl, oxadiazolyl , thiadiazolyl, etc., or pyridyl, pyridazinyl, pyrimidinyl, pyrazinyl, triazinyl, etc., and 5-10-membered condensed ring groups comprising these groups.

As used herein, the term "heteroaryl" encompasses a condensed ring structure, and the points of attachment of the condensed ring structure to additional radical groups may be on any ring of the condensed ring structure. Therefore, heteroaryl groups of the present disclosure further include, but are not limited to, (mono)heteroaryl-(mono)heteroaryl, (mono)heteroaryl-(monocyclo)aryl, (mono)heteroaryl-(mono)heterocyclyl, and (mono)heteroaryl-(mono)cycloalkyl, such as 5-6-membered (mono)heteroaryl-5-6-membered (mono)heteroaryl, 5-6 membered (mono)heteroaryl-phenyl, 5-6-membered (mono)heteroaryl-5-6-membered (mono)heterocyclyl, or 5-6-membered (mono)heteroaryl-$C_{4-6}$(mono)cycloalkyl (e.g., 5-6-membered heteroaryl-cyclobutyl, 5-6-membered heteroaryl-cyclopentyl, or 5-6-membered heteroaryl-cyclohexyl). Examples of heteroaryl groups include, but are not limited to, indolyl, isoindolyl, indazolyl, benzimidazolyl, quinolinyl, isoquinolinyl,

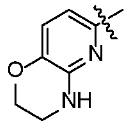 , 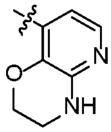 , 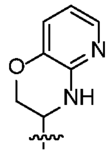 , 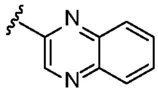 , 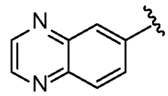 , 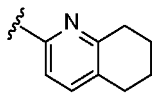 ,

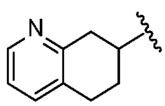 , 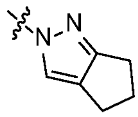 ,

and the like.

As used herein, the term "halo" or "halogen" group is defined to encompass F, Cl, Br, or I.

The term "substitution" means that one or more (for example, one, two, three, or four) hydrogens on a specified atom are replaced by a selection from the indicated group, provided that the normal valence of the specified atom in the current case is not exceeded and the substitution forms a stable compound. A combination of substituents and/or variables is permissible only when such a combination forms a stable compound.

If a substituent is described as "optionally...substituted," the substituent may be (1) unsubstituted, or (2) substituted. If a carbon of a substituent is described as being optionally substituted with one or more substituents in a list of substituents, one or more hydrogens on the carbon (to the extent of any present hydrogens) may be independently and/or together replaced with independently selected optional substituents. If a nitrogen of a substituent is described as being optionally substituted with one or more substituents in a list of substituents, one or more hydrogens on the nitrogen (to the extent of any present hydrogens) may each be replaced with independently selected optional substituents.

If a substituent is described as being "independently selected from" a group, each substituent is selected independently of the other. Therefore, each substituent may be the same as or different from another (other) substituent(s).

As used herein, the term "one or more" means 1 or more than 1, such as 2, 3, 4, 5, or 10, under reasonable conditions.

Unless otherwise specified, as used herein, the points of attachment of a substituent may be from any suitable positions of the substituent.

When a bond of a substituent is shown as a bond connecting two atoms through a ring, such a substituent may bond to any ring-forming atom in the substitutable ring.

The present disclosure further includes all pharmaceutically acceptable isotopically-labelled compounds, which are the same as the compounds of the present disclosure, except that one or more atoms are replaced with atoms which have the same atomic number, but an atomic mass or mass number different from the atomic mass or mass number predominantly found in nature. Examples of isotopes suitable for inclusion in the compounds of the present disclosure include, but are not limited to, isotopes of hydrogen (e.g., deuterium ($^{2}H$), tritium ($^{3}H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulfur (e.g., $^{35}S$). Certain isotopically labeled compounds (e.g., those incorporated into a radioisotope)

of the present disclosure are useful in drug and/or substrate tissue distribution study (e.g., analysis). Tritiated (i.e., $^3H$) and carbon-14 (i.e., $^{14}C$) isotopes are particularly preferred for their ease of incorporation and detectability. Substitutions with positron emission isotopes (such as $^{11}C$, $^{18}F$, $^{15}O$, and $^{13}N$) may be used to test substrate receptor occupancy in positron emission tomography (PET) studies. The isotopically-labeled compounds of the present disclosure may be prepared by methods analogous to those described in the accompanying Routes and/or Examples and preparations by replacing a non-isotopically labeled reagent with an appropriate isotopically labeled reagent. Pharmaceutically acceptable solvates of the present disclosure include those in which the crystallization solvent may be replaced with an isotope, for example, $D_2O$, acetone-$d_6$, or DMSO-$d_6$.

The term "stereoisomer" means an isomer formed due to at least one asymmetric center. In a compound with one or more (for example, one, two, three, or four) asymmetric centers, its exo/meso mixtures, single enantiomers, and diastereomer mixtures and individual diastereomers may be produced. Specific individual molecules may also exist as geometric isomers (cis/trans). Similarly, the compound of the present disclosure may exist in a mixture of two or more structurally different forms (commonly referred to as tautomers) in rapid equilibrium. Representative examples of tautomers include keto-enol tautomers, phenol-keto tautomers, nitroso-oxime tautomers, imine-enamine tautomers, and the like. For example, nitroso-oximes may exist in equilibrium in the following tautomeric forms in solution:

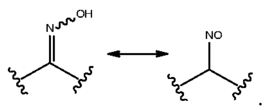

It should be understood that the scope of the present disclosure encompasses all such isomers or mixtures thereof in any proportions (for example, 60%, 65%, 70%, 75%, 80%, 85%, 90%, 95%, 96%, 97%, 98%, 99%).

A solid line

(),

a solid wedge

(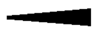),

or a dashed wedge

(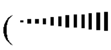)

may be used herein to depict chemical bonds of the compounds of the present disclosure. The use of solid lines to depict bonds to asymmetric carbon atoms is intended to indicate that all possible stereoisomers at that carbon atom (e.g., specific enantiomers or racemic mixtures) are included. The use of solid or dashed wedges to depict bonds to asymmetric carbon atoms is intended to indicate that the stereoisomers shown exist. When present in a racemic mixture, solid and dashed wedges are used to define relative stereochemistry, rather than absolute stereochemistry. Unless otherwise specified, the compound of the present disclosure is intended to exist in the form of stereoisomers (which include cis and trans isomers, optical isomers (such as R and S enantiomers), diastereomers, geometric isomers, rotamers, conformational isomers, atropisomers, and mixtures thereof). The compound of the present disclosure may exhibit more than one type of isomerism, and are composed of mixtures thereof (for example, racemic mixtures and diastereomeric pairs).

The present disclosure encompasses all possible crystalline forms or polymorphs of the compound of the present disclosure, which may be a single polymorph or a mixture of more than one polymorph at any ratio.

Eutectic crystallization refers to the fact that the active molecules of a drug and additional physiologically acceptable molecules of acids, bases, salts, and non-ionic compounds are connected by hydrogen bonds, π-π stacking, van der Waals forces, and additional non-covalent bonds to be combined in the same crystal lattice.

It should also be understood that some compounds of the present disclosure may exist in free form for treatment, or, where appropriate, in the form of pharmaceutically acceptable derivatives thereof. In the present disclosure, pharmaceutically acceptable derivatives include, but are not limited to, pharmaceutically acceptable salts, esters, solvates, *N*-oxides, metabolites, or prodrugs, which, after being administered to patients in need thereof, can directly or indirectly provide the compound of the present disclosure or metabolites or residues thereof. Therefore, when the "compound of

the present disclosure" is referred to herein, it is also intended to encompass the above various derivative forms of the compound.

Pharmaceutically acceptable salts of the compound of the present disclosure includes both acid addition salts and base addition salts thereof. for example, hexafluorophosphate, and meglumine salt. For a review of suitable salts, see Stahl and Wermuth, "Handbook of Pharmaceutical Salts: Properties, Selection, and Use" (Wiley-VCH, 2002).

As used herein, the term "ester" means an ester derived from the compound of each general formula in the present disclosure, which includes physiologically hydrolyzable esters (hydrolyzable under physiological conditions to free the compound of the present disclosure in the form of free acid or alcohol). The compound of the present disclosure itself may also be an ester.

The compound of the present disclosure may exist in the form of solvate (preferably hydrate), where the compound of the present disclosure comprises a polar solvent as a structural element of the crystal lattice of said compound, especially, for example, water, methanol, or ethanol. The amount of a polar solvent, especially water, may be present at a stoichiometric or non-stoichiometric ratio.

Those skilled in the art will understand that, since available lone pairs of electrons are required to oxidize nitrogen to form oxides, not all nitrogen-containing heterocyclic rings can form *N*-oxides. Those skilled in the art will recognize nitrogen-containing heterocyclic rings that can form *N*-oxides. Those skilled in the art will also recognize that tertiary amines can form *N*-oxides. The synthesis methods for the preparation of *N*-oxides of heterocyclic rings and tertiary amines are well known to those skilled in the art, including but not limited to the use of peroxyacids such as peroxyacetic acid and m-chloroperoxybenzoic acid (MCPBA), hydrogen peroxide, alkyl hydroperoxides such as tert-butyl hydroperoxide and sodium perborate, and dioxirane such as dimethyl dioxirane to oxidize heterocyclic rings and tertiary amines. These methods for the preparation of *N*-oxides have been widely described and summarized in literature (see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.)

The present disclosure further includes, within its scope, metabolites of the compound of the present disclosure, i.e., substances formed in vivo when the compound of the present disclosure is administered. Such products may be produced by, for example, oxidation, reduction, hydrolysis, amidation, deamidation, esterification, enzymolysis, and the like of the administered compound. Therefore, the present disclosure includes metabolites of the compound of the present disclosure, including compounds prepared by contacting the compound of the present disclosure with a mammal for a time sufficient to produce its metabolites.

The present disclosure further includes, within its scope, the prodrugs of the compound of the present disclosure, which are certain derivatives of the compound of the present disclosure that may themselves have less pharmacological activity or no pharmacological activity when administered into or onto a human body, and that may be converted into the compound of the present disclosure having the desired activity by, for example, hydrolytic cleavage. Generally, such prodrugs will be functional group derivatives of the compound, which are easily converted into the desired therapeutically active compound in vivo. Additional information on the use of prodrugs may be found in "Pro-drugs as Novel Delivery Systems", Volume 14, ACS Symposium Series (T. Higuchi and V. Stella). The prodrugs of the present disclosure may be prepared by, for example, substituting appropriate functional groups present in the compound of the present disclosure with certain moieties known to those skilled in the art as "pro-moiety (for example, as described in "Design of Prodrugs," H. Bundgaard (Elsevier, 1985))".

The present disclosure further includes the compound of the present disclosure comprising protective groups. In any process of preparing the compound of the present disclosure, protection of sensitive groups or reactive groups on any related molecules may be necessary and/or desirable, thereby forming a chemically protected form of the compound of the present disclosure. This may be achieved by conventional protective groups, for example, those protective groups as described in T. W. Greene & P. G. M. Wuts, Protective Groups in Organic Synthesis, John Wiley & Sons, 1991. These references are incorporated herein by reference. The protective groups may be removed at an appropriate subsequent stage using methods known in the art.

The term "about" means within $\pm$10%, preferably within $\pm$5%, more preferably within $\pm$2%, of said value.

## Compounds

In one aspect, the present disclosure provides a compound of formula I, a stereoisomer, tautomer, or mixture thereof, a N-oxide thereof, a pharmaceutically acceptable salt, eutecticum, polymorph, or solvate thereof, or a stable isotope derivative, metabolite, or prodrug thereof:

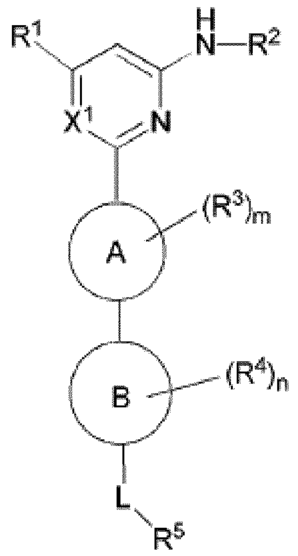

Formula I

where:

ring A is selected from $C_{6-10}$ aromatic ring and 5-6-membered heteroaromatic ring;
ring B is selected from $C_{3-8}$ cycloalkyl and 4-11-membered heterocyclyl;
$X^1$ is selected from CH and N;
$R^1$ is selected from the group consisting of H, halogen, hydroxy, cyano, $C_{1-6}$ alkyl, $C_{1-6}$ heteroalkyl (e.g., $C_{1-6}$ alkoxy), $C_{3-8}$ cycloalkyl, 4-10-membered heterocyclyl, and $-NR^{20a}R^{20b}$, and the alkyl, heteroalkyl (for example, alkoxy), cycloalkyl, and heterocyclyl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, and $C_{1-4}$ heteroalkyl (e.g., $C_{1-4}$ alkoxy);
$R^2$ is selected from the group consisting of $C_{1-6}$ alkyl, $C_{1-6}$ heteroalkyl, $C_{3-8}$ cycloalkyl, 4-10-membered heterocyclyl, 5-10-membered heteroaryl, and $-C(=O)R^{21}$, and the alkyl, heteroalkyl, cycloalkyl, heterocyclyl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;
$R^3$ and $R^4$ are absent or are, at each occurrence, each independently selected from the group consisting of hydroxy, halogen, CN, $C_{1-6}$ alkyl, $C_{1-6}$ heteroalkyl (e.g., $C_{1-6}$ alkoxy), and $C_{3-6}$ cycloalkyl, the alkyl, heteroalkyl (for example, alkoxy), and cycloalkyl are each optionally substituted with one or more substituents selected from the group consisting of: halogen, CN, $C_{1-4}$ alkyl, $C_{1-4}$ haloalkyl, $C_{1-4}$ alkoxy, and $C_{1-4}$ haloalkoxy; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl;
L is selected from the group consisting of -O-, -S-, -S(O)-, $-S(O)_2-$, $-N=CR^{21}-$, $-N(R^{23a})-C(O)-$, $C_{1-6}$ alkylene, $C_{1-6}$ heteroalkylene, $C_{2-6}$ alkenylene, $C_{2-6}$ alkynylene,

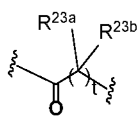 , 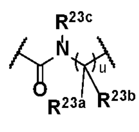 ,

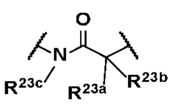 , 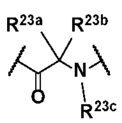 , 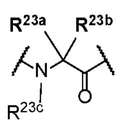 , 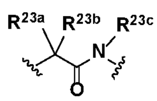 , 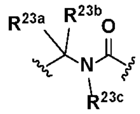 , 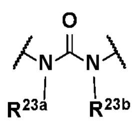 , 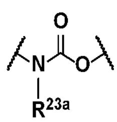 , 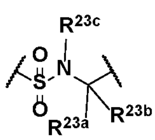 ,

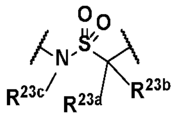, 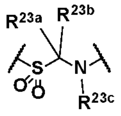, 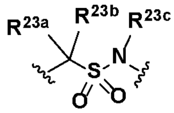, 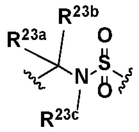, 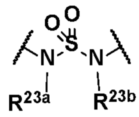, 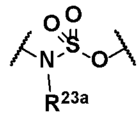 and 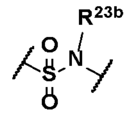,

the alkylene, heteroalkylene, alkenylene, and alkynylene are each optionally substituted with one or more substituents selected from the group consisting of: hydroxy, halogen, CN, $NO_2$, $C_{1-6}$ alkyl, $C_{1-6}$ haloalkyl, $C_{1-6}$ hydroxyalkyl, $C_{1-6}$ haloalkoxy, $C_{1-6}$ heteroalkyl (e.g., $C_{1-6}$ alkoxy), and $C_{3-8}$ cycloalkyl; or L is $-N(R^{23a})-$;

$R^5$ is selected from the group consisting of hydroxy, halogen, CN, $NO_2$, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-OR^{21}$, $-SR^{21}$,$-S(=O)R^{22}$, $-S(=O)_2R^{22}$, $-S(=O)NR^{20a}R^{20b}$, $-S(=O)_2NR^{20a}R^{20b}$, $-NR^{20a}S(=O)R^{20b}$, $-NR^{20a}S(=O)_2R^{20b}$, $-C(=O)R^{21}$, $-C(=O)NR^{23a}R^{23b}$, $-NR^{23a}C(=O)R^{23b}$, $-OC(=O)NR^{23a}R^{23b}$, and $-NR^{24a}C(=O)NR^{25a}R^{25b}$, and the alkyl, heteroalkyl (e.g., alkoxy), alkenyl, alkynyl, cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$,$-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$,$-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$, and$-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl;

$R^{20a}$, $R^{20b}$, $R^{23a}$, $R^{23b}$, $R^{23c}$, $R^{24a}$, $R^{25a}$, and $R^{25b}$ are each independently selected from the group consisting of H, OH, $C_{1-6}$ alkyl, $C_{1-6}$ alkoxy, and $C_{3-8}$ cycloalkyl; or $R^{20a}$ and $R^{20b}$, $R^{23a}$ and $R^{23b}$, or $R^{25a}$ and $R^{25b}$ form, together with an atom to which they are attached, a 3-8-membered cycloalkyl or heterocyclyl, and the alkyl, alkoxy, cycloalkyl, and heterocyclyl are each optionally substituted with one or more substituents selected from the group consisting of: OH, CN, halogen, $NO_2$, $C_{1-4}$ alkyl, $C_{1-4}$ alkoxy, $C_{1-4}$ hydroxyalkyl, $C_{1-4}$ haloalkyl, and $C_{1-4}$ haloalkoxy;

$R^{30a}$, $R^{30b}$, $R^{33a}$, $R^{33b}$, $R^{34a}$, $R^{35a}$, and $R^{35b}$ are each independently selected from the group consisting of H, $C_{1-6}$ alkyl,$C_{1-6}$ haloalkyl, $C_{1-6}$ hydroxyalkyl, $C_{1-6}$ alkoxy, and $C_{1-6}$ haloalkoxy;

$R^{21}$, $R^{22}$, $R^{31}$, and $R^{32}$ are each independently selected from the group consisting of $C_{1-6}$ alkyl, $C_{1-6}$ alkoxy, $C_{3-8}$ cycloalkyl, 4-10-membered heterocyclyl, $C_{6-12}$ aryl, and 5-10-membered heteroaryl, and the alkyl, alkoxy, cycloalkyl, heterocyclyl, aryl, and heteroaryl are each optionally substituted with one or more substituents selected from the group consisting of: OH, halogen, CN, $C_{1-4}$ alkyl, $C_{1-4}$ alkoxy, $C_{1-4}$ haloalkyl, $C_{1-4}$ haloalkoxy, $C_{3-6}$ cycloalkyl, and 4-10-membered heterocyclyl;

m is 0, 1, 2, 3, or 4;

n is 0, 1, 2, 3, or 4;

t is 0, 1, 2, 3, or 4; and

u is 0, 1, 2, 3, or 4;

provided that when ring B is a piperazine ring and $X^1$ is CH, $R^2$ is not 4-$CF_3$-pyridin-2-yl or 4-CN-pyridin-2-yl.

In some embodiments, the ring A is a benzene ring or a 5-6-membered heteroaromatic ring; preferably, the ring A is a benzene ring, a thiazole ring, a pyridine ring, a pyrazine ring, or a pyrimidine ring; and more preferably, the ring A is

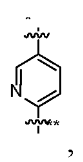, 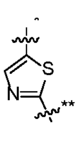, or 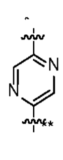,

is linked to the ring where $X^1$ is located through a position marked with *, and is linked to the ring B through a position marked with **.

In some embodiments, the ring B is a $C_{3-6}$ cycloalkyl or a 5-7-membered heterocyclyl; preferably, the ring B is a piperidine ring, a piperazine ring, an azacycloheptane bridged ring, or a diazacycloheptane bridged ring; and more preferably, the ring B is

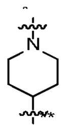, 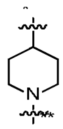, 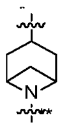, 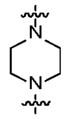, 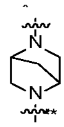,

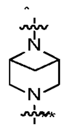, or 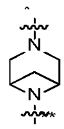,

which is linked to the ring A through a position marked with *, and is linked to L through a position marked with **.

In some embodiments, $X^1$ is CH or N, and preferably $X^1$ is N.

In some embodiments, $R^1$ is selected from the group consisting of H, halogen, hydroxy, cyano, $C_{1-4}$ alkyl, $C_{1-4}$ heteroalkyl (e.g., $C_{1-4}$ alkoxy), $C_{3-6}$ cycloalkyl, and 4-10-membered heterocyclyl, and the alkyl, heteroalkyl (e.g., alkoxy), cycloalkyl, and heterocyclyl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, and $C_{1-4}$ heteroalkyl (e.g., $C_{1-4}$ alkoxy).

In some embodiments, $R^1$ is selected from the group consisting of $C_{1-4}$ alkyl, 5-membered nitrogen-containing heterocyclyl, and $C_{1-4}$ heteroalkyl (e.g., $C_{1-4}$ alkoxy), and the alkyl, heterocyclyl, and heteroalkyl (e.g., alkoxy) are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, and $C_{1-3}$ heteroalkyl (e.g., $C_{1-4}$ alkoxy).

In some embodiments, $R^1$ is selected from the group consisting of $C_{1-3}$ alkyl (e.g., methyl), pyrrolidinyl (e.g., pyrrolidin-1-yl), and $C_{1-3}$ alkoxy (e.g., ethoxy).

In some embodiments, $R^2$ is selected from the group consisting of $C_{1-4}$ alkyl, $C_{1-4}$ heteroalkyl, $C_{3-6}$ cycloalkyl, 4-6-membered heterocyclyl, 5-6-membered heteroaryl, and $-C(=O)R^{21}$, and the alkyl, heteroalkyl, cycloalkyl, heterocyclyl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl.

In some embodiments, $R^2$ is selected from the group consisting of $C_{1-3}$ alkyl, 5-6-membered heteroaryl, and $-C(=O)CH_3$, and the alkyl and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl, and $C_{3-6}$ cycloalkyl.

In some embodiments, $R^2$ is selected from the group consisting of $C_{1-3}$ alkyl (e.g., methyl), - $C(=O)CH_3$, thienyl, pyrrolyl, pyrazolyl, imidazolyl, thiazolyl, thiadiazolyl, isothiazolyl , oxazolyl, oxadiazolyl, isoxazolyl, and pyridyl, and the alkyl, thienyl, pyrrolyl, pyrazolyl, imidazolyl, thiazolyl, thiadiazolyl, isothiazolyl, oxazolyl, oxadiazolyl, isoxazolyl, and pyridyl are each optionally substituted with one or more substituents selected from the group consisting of: hydroxy, halogen, CN, $C_{1-3}$ alkyl (e.g., methyl), $C_{1-3}$ haloalkyl, $C_{1-3}$ haloalkoxy, $C_{1-3}$ heteroalkyl (e.g., $C_{1-3}$ alkoxy), and $C_{3-6}$ cycloalkyl; and preferably, $R^2$ is methyl-substituted pyrazolyl (e.g., 5-methyl-1H-pyrazol-3-yl, or 1-methyl-1H-pyrazol-4-yl), cyclopropyl-substituted pyrazolyl (e.g., 5-cyclopropyl-1H-pyrazol-3-yl), or $-C(O)CH_3$.

In some embodiments, $R^3$ and $R^4$ are absent or are, at each occurrence, independently selected from the group consisting of hydroxy, halogen, CN, $C_{1-4}$ alkyl, and $C_{1-4}$ alkoxy, the alkyl and alkoxy are each optionally substituted with one or more substituents selected from the group consisting of: halogen, CN, $C_{1-4}$ alkyl, $C_{1-4}$ haloalkyl, $C_{1-4}$ alkoxy, and $C_{1-4}$ haloalkoxy; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl.

In some embodiments, $R^3$ and $R^4$ are absent or are, at each occurrence, independently selected from the group consisting of hydroxy, halogen, CN, $C_{1-3}$ alkyl, $C_{1-3}$ alkoxy, the alkyl and alkoxy are each optionally substituted with one or more substituents selected from the group consisting of: halogen, CN, and $C_{1-3}$ alkyl; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl.

In some embodiments, $R^3$ and $R^4$ are absent or are, at each occurrence, independently selected from the group consisting of: F, Cl, CN, OH, $C_{1-3}$ alkyl, and $C_{1-3}$ alkoxy; and preferably, $R^3$ and $R^4$ are absent.

In some embodiments, L is selected from the group consisting of -O-, -S-, -C(O)-, $-N(R^{23a})-C(O)-$, $-C(O)-N(R^{23c})-$, $C_{1-4}$ alkylene, $C_{1-4}$ heteroalkylene,

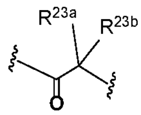, 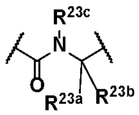, 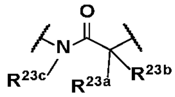,

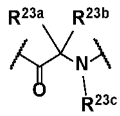, 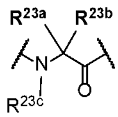, 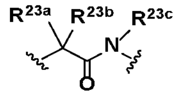, and 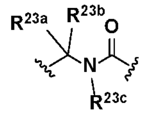,

and the alkylene and heteroalkylene are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), and $C_{3-6}$ cycloalkyl.

In some embodiments, L is selected from the group consisting of -O-, -C(O)-, -NHC(O)-, - C(O)NH-, $C_{1-3}$ alkylene, $C_{1-3}$ heteroalkylene,

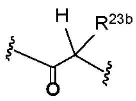, 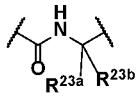, 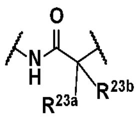, 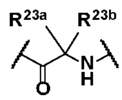,

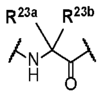, 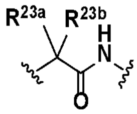, and 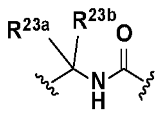,

and the alkylene and heteroalkylene are each optionally substituted with one or more substituents selected from the group of: hydroxy, halogen, CN, $NO_2$, $C_{1-3}$ alkyl, $C_{1-3}$ haloalkyl, $C_{1-3}$ hydroxyalkyl, $C_{1-3}$ haloalkoxy, $C_{1-3}$ heteroalkyl(e.g., $C_{1-3}$ alkoxy), and $C_{3-6}$ cycloalkyl, where $R^{23a}$ and $R^{23b}$ are preferably H or $C_{1-3}$ alkyl.

In some embodiments, L is selected from the group consisting of -O-, -C(O)-, -NHC(O)-, - C(O)NH-, $C_{1-3}$ alkylene,

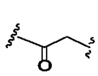, 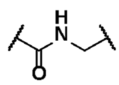, 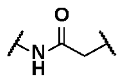, 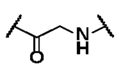, 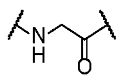, 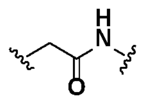,

and

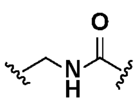,

and the alkylene is optionally substituted with one or more substituents selected from the group consisting of: hydroxy, halogen, CN, $C_{1-3}$ alkyl, and $C_{1-3}$ haloalkyl. Preferably, L is - $CH_2$-, -$CH(CH_3)$-, -O-, -C(O)-,

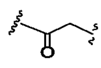,

-C(O)NH-, or

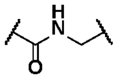 .

In some embodiments, $R^5$ is selected from the group consisting of hydroxy, halogen, CN, $NO_2$, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{20a}R^{20b}$,$-OR^{21}$, $-SR^{21}$, $-S(=O)R^{22}$, $-S(=O)_2R^{22}$, $-S(=O)NR^{20a}R^{20b}$, $-S(=O)_2NR^{20a}R^{20b}$, $-NR^{20a}S(=O)R^{20b}$,$-NR^{20a}S(=O)_2R^{20b}$, $-C(=O)R^{21}$, $-C(=O)NR^{23a}R^{23b}$, $-NR^{23a}C(=O)R^{23b}$, $-OC(=O)NR^{23a}R^{23b}$, and$-NR^{24a}C(=O)NR^{25a}R^{25b}$, and the alkyl, heteroalkyl (e.g., alkoxy), alkenyl, alkynyl, cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$ $-NR^{30a}S(=O)_2R^{30b}$, $-C(=O)R^{31}$, $-C(=O)NR^{33a}R^{33b}$,$-NR^{33a}C(=O)R^{33b}$, $-OC(=O)NR^{33a}R^{33b}$, and $-NR^{34a}C(=O)NR^{35a}R^{35b}$ where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl.

In some embodiments, $R^5$ is selected from the group consisting of $C_{3-6}$ cycloalkyl, 4-10-membered heterocyclyl, $C_{6-12}$ aryl, and 5-10-membered heteroaryl, and the cycloalkyl, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$,$-NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl.

In some embodiments, $R^5$ is selected from the group consisting of $C_{6-10}$ aryl and 5-6-membered heteroaryl, and the aryl and heretoaryl are each optionally substituted with one or more substituents selected from the group consisting of: hydroxy, halogen, CN, $NO_2$, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, - $OR^{31}$, $-C(=O)R^{31}$, $-C(=O)NR^{33a}R^{33b}$, and $-NR^{33a}C(=O)R^{33b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4- 6-membered heterocyclyl.

In some embodiments, $R^5$ is selected from phenyl and 5-6-membered heteroaryl (e.g., pyridyl, pyrimidyl, pyrazinyl, pyridazinyl, pyrazolyl, oxazolyl, imidazolyl, or thiazolyl), and the phenyl and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$alkoxy), $C_{3-6}$ cycloalkyl, $C_{3-6}$ cycloalkoxy, 4- 6-membered heterocyclyl, 5- 8-membered heteroaryl (e.g., pyridyl, pyrrolyl, pyrazolyl, furyl, oxazolyl, imidazolyl, thiazolyl, or cyclopentyl-pyrazolyl), $-NR^{30a}R^{30b}$, $-OR^{31}$, $-C(=O)R^{31}$, $-C(=O)NR^{33a}R^{33b}$, and $-NR^{33a}C(=O)R^{33b}$, where the cycloalkyl, cycloalkoxy, heterocyclyl, and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$alkoxy), $C_{3-6}$ cycloalkyl, $C_{3-6}$ cycloalkoxy, and 4- 6-membered heterocyclyl.

In some embodiments, $R^5$ is selected from the group consisting of phenyl, pyridyl, pyrazolyl, and thiazolyl, and the phenyl, pyridyl, pyrazolyl, and thiazolyl are each optionally substituted with one or more substituents selected from the group consisting of: halogen, CN, $C_{1-3}$ alkyl, $C_{1-3}$ haloalkyl, $C_{1-3}$ hydroxyalkyl, $C_{1-3}$ haloalkoxy, $C_{1-3}$ alkoxy, $C_{3-6}$ cycloalkyl, $C_{3-6}$ cycloalkoxy, 4-6-membered heterocyclyl, 5-8-membered heteroaryl (e.g., pyridyl, pyrrolyl, pyrazolyl, furyl, oxazolyl, imidazolyl, thiazolyl, or cyclopentyl-pyrazolyl), $-NR^{30a}R^{30b}$, and $-OR^{31}$, where the heterocyclyl and heteroaryl are each optionally substituted with one or more substituents selected from the group consisting of: halogen, CN, $C_{1-3}$ alkyl, $C_{1-3}$ haloalkyl, $C_{1-3}$ hydroxyalkyl, $C_{1-3}$ haloalkoxy, $C_{1-3}$ alkoxy, $C_{3-6}$ cycloalkyl, $C_{3-6}$ cycloalkoxy, and 4-6-membered heterocyclyl. Preferably, $R^5$ is phenyl, pyridyl, pyrazolyl, or thiazolyl that is optionally substituted with one or more substituents selected from the group consisting of halogen (e.g., fluoro or chloro), CN, $C_{1-3}$ alkyl (e.g., methyl or ethyl), $C_{1-3}$ haloalkyl (e.g., trifluoromethyl), $C_{1-3}$ alkoxy (e.g., methoxy or ethoxy), $C_{3-6}$ cycloalkyl (e.g., cyclopropyl), $C_{3-6}$ cycloalkoxy (e.g., cyclopropoxy), and 5-6-membered heteroaryl (e.g., pyridyl, pyrrolyl, pyrazolyl, furyl, oxazolyl, imidazolyl, or thiazolyl), where the 5-6-membered heteroaryl is optionally further substituted with one or more substituents selected from the group consisting of halogen (e.g., fluoro or chloro), $C_{1-3}$ alkyl (e.g., methyl, ethyl, or isopropyl), $C_{1-3}$

haloalkyl (e.g., fluoromethyl), $C_{1-3}$ hydroxyalkyl (e.g., hydroxymethyl or hydroxypropyl), $C_{1-3}$ alkoxy (e.g., methoxy), $C_{3-6}$ cycloalkyl (e.g., cyclopropyl), and $C_{3-6}$ cycloalkoxy (e.g., cyclopropoxy or cyclobutoxy).

In some embodiments, $R^5$ is selected from the group consisting of phenyl, pyridyl, pyrazolyl, and thiazolyl, and the phenyl, pyridyl, pyrazolyl, and thiazolyl are each optionally substituted with one or more substituents selected from the group consisting of: halogen, CN, $C_{1-3}$ alkyl, $C_{1-3}$ haloalkyl, $C_{1-3}$ hydroxyalkyl, $C_{1-3}$ haloalkoxy, $C_{1-3}$ alkoxy, $C_{3-6}$ cycloalkyl, $C_{3-6}$ cycloalkoxy, 4-6-membered heterocyclyl, 5-6-membered heteroaryl (e.g., pyridyl, pyrrolyl, furyl, pyrazolyl, oxazolyl, imidazolyl, or thiazolyl), $-NR^{30a}R^{30b}$, and $-OR^{31}$, where the heterocyclyl and heteroaryl are each optionally substituted with one or more substituents selected from the group consisting of: halogen, CN, $C_{1-3}$ alkyl, $C_{1-3}$ haloalkyl, $C_{1-3}$ haloalkoxy, $C_{1-3}$ alkoxy, $C_{3-6}$ cycloalkyl, and 4-6-membered heterocyclyl. Preferably, $R^5$ is phenyl, pyridyl, pyrazolyl, or thiazolyl that is optionally substituted with one or more substituents selected from the group consisting of halogen (e.g., fluoro or chloro), CN, $C_{1-3}$ alkyl (e.g., methyl or ethyl), $C_{1-3}$ haloalkyl (e.g., trifluoromethyl), $C_{1-3}$ alkoxy (e.g., methoxy or ethoxy), $C_{3-6}$ cycloalkyl (e.g., cyclopropyl), $C_{3-6}$ cycloalkoxy (e.g., cyclopropoxy), and five-membered heteroaryl (e.g., pyrazolyl, imidazolyl, or thiazolyl), where the five-membered heteroaryl is optionally further substituted with one or more substituents selected from the group consisting of halogen (e.g., fluoro or chloro), $C_{1-3}$ alkyl (e.g., methyl), and $C_{1-3}$ hydroxyalkyl (e.g., hydroxymethyl or hydroxypropyl).

In some embodiments, $R^{20a}$, $R^{20b}$, $R^{23a}$, $R^{23b}$, $R^{23c}$, $R^{24a}$, $R^{25a}$, and $R^{25b}$ are each independently selected from the group consisting of H, $C_{1-4}$ alkyl, $C_{1-4}$ alkoxy, and $C_{3-8}$ cycloalkyl; or $R^{20a}$ and $R^{20b}$, $R^{23a}$ and $R^{23b}$, or $R^{25a}$ and $R^{25b}$ form, together with an atom to which they are attached, a 3-8-membered cycloalkyl or heterocyclyl, and the alkyl, alkoxy, cycloalkyl, and heterocyclyl are each optionally substituted with one or more substituents selected from the group consisting of: OH, CN, halogen, $NO_2$, $C_{1-4}$ alkyl, $C_{1-4}$ alkoxy, $C_{1-4}$ hydroxyalkyl, $C_{1-4}$ haloalkyl, and $C_{1-4}$ haloalkoxy.

In some embodiments, $R^{20a}$, $R^{20b}$, $R^{23a}$, $R^{23b}$, $R^{23c}$, $R^{24a}$, $R^{25a}$, and $R^{25b}$ are each independently H, $C_{1-4}$ alkyl, or $C_{1-4}$ alkoxy.

In some embodiments, $R^{23a}$ and $R^{23b}$ are each independently selected from the group consisting of H, $C_{1-3}$ alkyl, $C_{1-3}$ alkoxy, and $C_{3-6}$ cycloalkyl; or $R^{23a}$ and $R^{23b}$ form, together with a C atom to which they are attached, a $C_{3-6}$ cycloalkyl or heterocyclyl, and the alkyl, alkoxy, cycloalkyl, and heterocyclyl are each optionally substituted with one or more substituents selected from the group consisting of: halogen, $C_{1-3}$ alkyl, $C_{1-3}$ alkoxy, $C_{1-3}$ hydroxyalkyl, $C_{1-3}$ haloalkyl, and $C_{1-3}$ haloalkoxy.

In some embodiments, $R^{21}$, $R^{22}$, $R^{31}$, and $R^{32}$ are each independently selected from the group consisting of $C_{1-4}$ alkyl, $C_{1-4}$ alkoxy, $C_{3-8}$ cycloalkyl, and 4-10-membered heterocyclyl, and the alkyl, alkoxy, cycloalkyl, and heterocyclyl are each optionally substituted with one or more substituents selected from the group consisting of: OH, halogen, CN, $C_{1-4}$ alkyl, $C_{1-4}$ alkoxy, $C_{1-4}$ haloalkyl, $C_{1-4}$ haloalkoxy, $C_{3-6}$ cycloalkyl, and 4-10-membered heterocyclyl.

In some embodiments, $R^{21}$, $R^{22}$, $R^{31}$, and $R^{32}$ are each independently selected from $C_{1-4}$ alkyl.

In some embodiments, $R^{30a}$, $R^{30b}$, $R^{33a}$, $R^{33b}$, $R^{34a}$, $R^{35a}$, and $R^{35b}$ are each independently selected from the group consisting of H, $C_{1-4}$ alkyl,$C_{1-4}$ haloalkyl, $C_{1-4}$ hydroxyalkyl, $C_{1-4}$ alkoxy, and $C_{1-4}$ haloalkoxy.

In some embodiments, $R^{30a}$, $R^{30b}$, $R^{33a}$, $R^{33b}$, $R^{34a}$, $R^{35a}$, and $R^{35b}$ are each independently selected from H and $C_{1-4}$ alkyl.

In some embodiments, m is 0.

In some embodiments, n is 0, 1, or 2.

In some embodiments, t is 0 or 1.

In some embodiments, u is 0 or 1.

In some embodiments, the compound of the present disclosure has a structure shown in formula I-A:

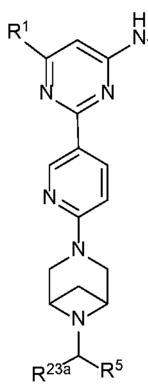

I-A ;

where:

$R^1$ and $R^2$ are as defined in the above formula I; and

$R^5$ is selected from the group consisting of $C_{6-12}$ aryl and 5-10-membered heteroaryl, where (1) the $C_{6-12}$ aryl is optionally substituted with one or more substituents selected from the group consisting of: $C_{3-6}$ cycloalkoxy, $C_{6-12}$ aryl, 5-10-membered heteroaryl, $-S(=O)R^{32}$, $-S(=O)_2R^{32}$,$-S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$,$-C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, where the cycloalkoxy, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl, and (2) the 5-10-membered heteroaryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$,$-S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$,$-NR^{33a}C(=O)R^{33b}$, $-OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl; and

$R^{23a}$, $R^{30a}$, $R^{30b}$, $R^{31}$, $R^{32}$, $R^{33a}$, $R^{33b}$, $R^{34a}$, $R^{35a}$, and $R^{35b}$ are as defined in the above formula I, and $R^{23a}$ is preferably H or $C_{1-3}$ alkyl.

In some embodiments, $R^5$ is selected from $C_{6-12}$ aryl and 5-10-membered heteroaryl, where (1) the $C_{6-12}$ aryl is optionally substituted with one or more substituents selected from the group consisting of: $C_{3-6}$ cycloalkoxy, $C_{6-12}$ aryl, 5-10-membered heteroaryl, $-S(=O)R^{32}$, $-S(=O)_2R^{32}$, $- S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, $- C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, where the cycloalkoxy, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl, and (2) the 5-10-membered heteroaryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $- S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $- NR^{33a}C(=O)R^{33b}$, $-OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and

$R^{23a}$, $R^{30a}$, $R^{30b}$, $R^{31}$, $R^{32}$, $R^{33a}$, $R^{33b}$, $R^{34a}$, $R^{35a}$, and $R^{35b}$ are as defined in the above formula I, and $R^{23a}$ is preferably H or $C_{1-3}$ alkyl.

In some embodiments, the compound of the present disclosure has a structure shown in formula I-B:

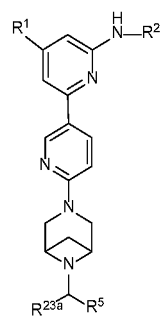

I-B

where:

$R^1$, $R^2$, $R^5$, and $R^{23a}$ are as defined in the above formula I, and $R^{23a}$ is preferably H or $C_{1-3}$ alkyl.

In some embodiments, the compound of the present disclosure has a structure shown in formula I-C:

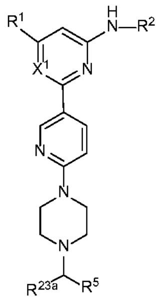

I-C

where:

when $X^1$ is CH, $R^1$, $R^2$, $R^5$, and $R^{23a}$ are as defined in the above formula I, $R^{23a}$ is preferably H or $C_{1-3}$ alkyl; and when $X^1$ is N, $R^1$, $R^2$, $R^5$, and $R^{23a}$ are as defined in the above formula I-A.

In some embodiments, the compound of the present disclosure has a structure shown in formula I-D:

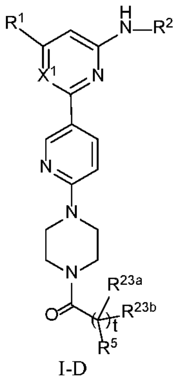

I-D

where:

$R^1$, $R^2$, $R^{23a}$, $R^{23b}$, and t are as defined in the above formula I;
when $X^1$ is CH, $R^5$ is as defined in the above formula I; and
when $X^1$ is N, $R^5$ is $C_{6-12}$ aryl or 5-10-membered heteroaryl, wherein

(i) when t is 0, the $C_{6-12}$ aryl and 5-10-membered heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $- S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $- NR^{33a}C(=O)R^{33b}$, $-OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$cycloalkyl, and 4-10-membered heterocyclyl,

(ii) when t is 1, (1) the $C_{6-12}$ aryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $- NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $- OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl, and (2) the 5-10-membered heteroaryl is optionally substituted with one or more substituents selected from the group consisting of: $NO_2$,

$C_{2-6}$ alkenyl, $C_{2-6}$ alkynyl, $C_{3-6}$ cycloalkoxy, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, - $S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, - $NR^{33a}C(=O)R^{33b}$, $-OC(=O)NR^{33a}R^{33b}$, and $-NR^{34a}C(=O)NR^{35a}R^{35b}$, where the aryl and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and

$R^{30a}$, $R^{30b}$, $R^{31}$, $R^{32}$, $R^{33a}$, $R^{33b}$, $R^{34a}$, $R^{35a}$, and $R^{35b}$ are as defined in the above formula I.

In some embodiments, the compound of the present disclosure has a structure shown in formula I-E:

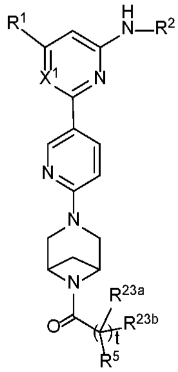

I-E

where:
$R^1$, $R^2$, $R^5$, $R^{23a}$, $R^{23b}$, $X^1$, and t are as defined in the above formula I-D.
In some embodiments, the compound of the present disclosure has a structure shown in formula I-F:

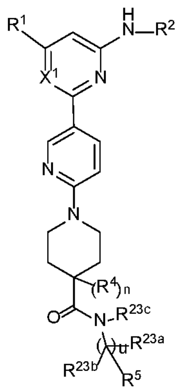

I-F

where:

$R^1$, $R^2$, $R^5$, $R^{23a}$, $R^{23b}$, and $X^1$ are as defined in the above formula I-D;
$R^4$ is as defined in the above formula I, and is preferably $C_{1-3}$ alkyl or $C_{1-3}$ alkoxy;
$R^{23c}$ is H, $C_{1-3}$ alkyl, or $C_{1-3}$ alkoxy, and the alkyl and alkoxy are each optionally substituted with one or more substituents selected from the group consisting of: OH, CN, halogen, $C_{1-4}$ alkoxy, and $C_{1-4}$ hydroxyalkyl;
u is 0 or 1; and
n is 0 or 1.

In some embodiments, the compound of the present disclosure has a structure shown in formula I-G:

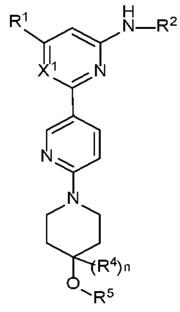

I-G

where:

$X^1$ is CH orN;

$R^1$, $R^2$, and $R^4$ are as defined in the above formula I, and $R^4$ is preferably $C_{1-3}$ alkyl or $C_{1-3}$ alkoxy;

n is 0 or 1;

$R^5$ is selected from $C_{6-12}$ aryl and 5-10-membered heteroaryl, where (1) the $C_{6-12}$ aryl is optionally substituted with one or more substituents selected from the group consisting of: $C_{3-6}$ cycloalkoxy, $C_{6-12}$ aryl, 5-10-membered heteroaryl, $-S(=O)R^{32}$, $-S(=O)_2R^{32}$, $-S(=O)NR^{30a}R^{30b}$, - $S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, - $NR^{33a}C(=O)R^{33b}$, $-OC(=O)NR^{33a}R^{33b}$, and $-NR^{34a}C(=O)NR^{35a}R^{35b}$, where the cycloalkoxy, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and (2) the 5-10-membered heteroaryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, - $NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, - $OC(=O)NR^{33a}R^{33b}$, and $-NR^{34a}C(=O)NR^{35a}R^{35b}$, where the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and

$R^{30a}$, $R^{30b}$, $R^{31}$, $R^{32}$, $R^{33a}$, $R^{33b}$, $R^{34a}$, $R^{35a}$, and $R^{35b}$ are as defined in the above formula I.

In some embodiments, the compound of the present disclosure has a structure shown in formula I, where:

the ring A is

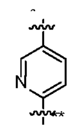 , 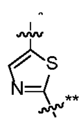 , or 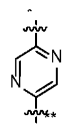 ,

is linked to the ring where $X^1$ is located through a position marked with *, and is linked to the ring B through a position marked with **;

the ring B is

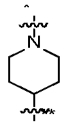 , 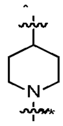 , 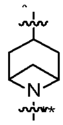 , 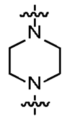 , 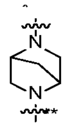 , 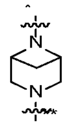 , or 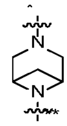 ,

is linked to the ring A through the position marked with *, and is linked to L through the position marked with **;

$X^1$ is N;

$R^1$ is selected from the group consisting of $C_{1-3}$ alkyl (e.g., methyl), pyrrolidinyl (e.g., pyrrolidin-1-yl), and $C_{1-3}$ alkoxy

(e.g., ethoxy);
$R^2$ is a methyl-substituted pyrazolyl (e.g., 5-methyl-1H-pyrazol-3-yl or 1-methyl-1H-pyrazol-4-yl), a cyclopropyl-substituted pyrazolyl (e.g., 5-cyclopropyl-1H-pyrazol-3-yl), or $-C(O)CH_3$;
$R^3$ and $R^4$ are absent;
L is $-CH_2-$, $-CH(CH_3)-$, -O-, -C(O)-,

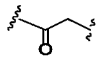,

-C(O)NH-, or

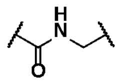;

and
$R^5$ is phenyl, pyridyl, pyrazolyl, or thiazolyl that is optionally substituted with one or more substituents selected from the group consisting of halogen (e.g., fluoro or chloro), CN, $C_{1-3}$ alkyl (e.g., methyl or ethyl), $C_{1-3}$ haloalkyl (e.g., trifluoromethyl), $C_{1-3}$ alkoxy (e.g., methoxy or ethoxy), $C_{3-6}$ cycloalkyl (e.g., cyclopropyl), $C_{3-6}$ cycloalkoxy (e.g., cyclopropoxy), and 5-6-membered heteroaryl (e.g., pyridyl, pyrrolyl, pyrazolyl, furyl, oxazolyl, imidazolyl, or thiazolyl), where the 5-6-membered heteroaryl is optionally further substituted with one or more substituents selected from the group consisting of halogen (e.g., fluoro or chloro), $C_{1-3}$ alkyl (e.g., methyl, ethyl, or isopropyl), $C_{1-3}$ haloalkyl (e.g., fluoromethyl), $C_{1-3}$ hydroxyalkyl (e.g., hydroxymethyl or hydroxypropyl), $C_{1-3}$ alkoxy (e.g., methoxy), $C_{3-6}$ cycloalkyl (e.g., cyclopropyl), and $C_{3-6}$ cycloalkoxy (e.g., cyclopropoxy **or** cyclobutoxy).

The present disclosure encompasses any combination of the above embodiments.

In some embodiments, the compound of the present disclosure includes, but is not limited to:

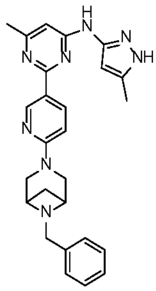
1
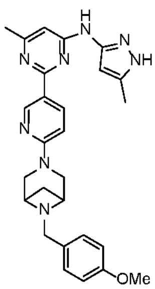
2
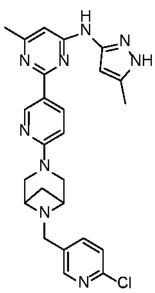
3
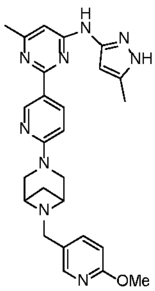
4
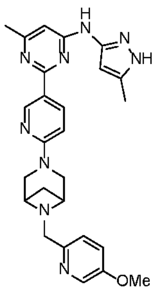
5
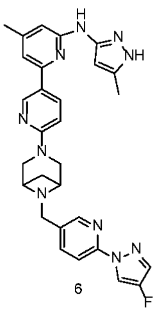
6
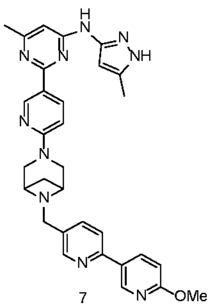
7

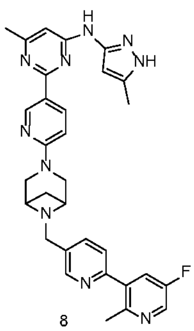
8
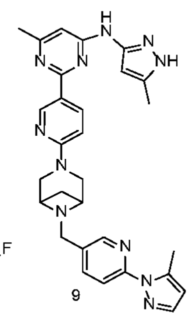
9
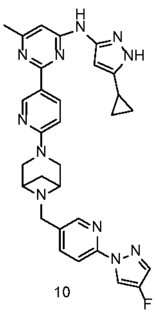
10
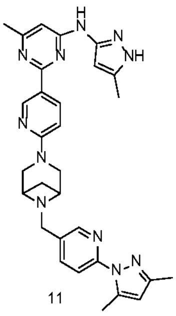
11
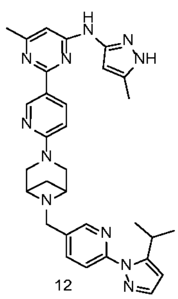
12
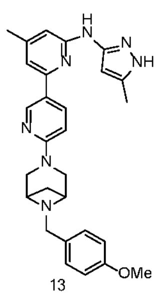
13

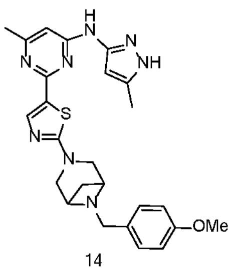
14

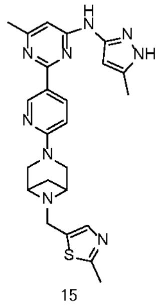
15

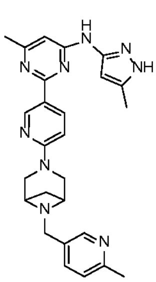
16

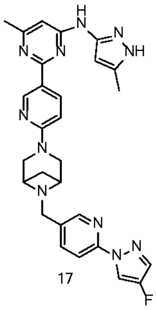
17

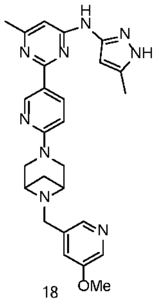
18

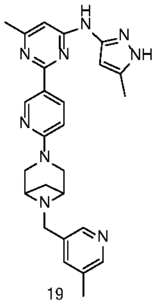
19

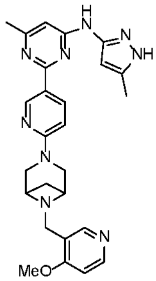
20

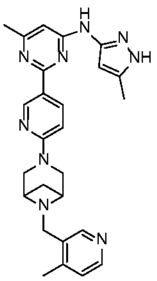
21

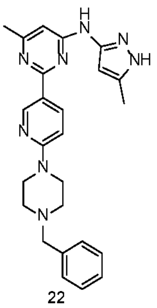
22

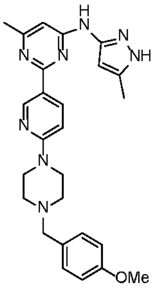
23

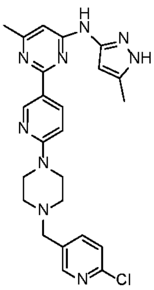
24

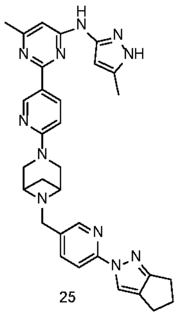
25

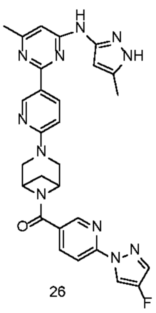
26

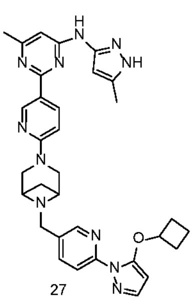
27

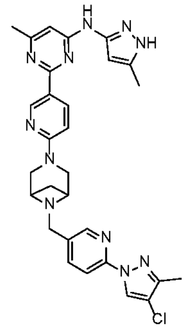

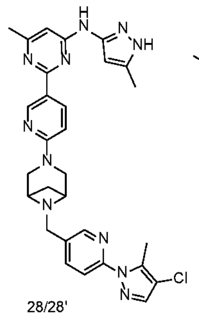
28/28'

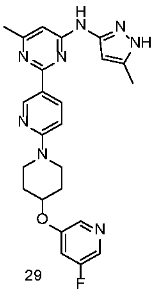
29

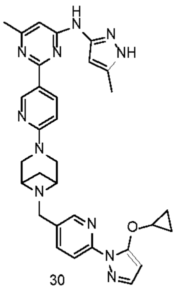
30

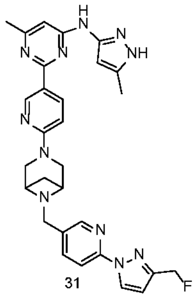
31

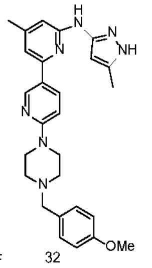
32

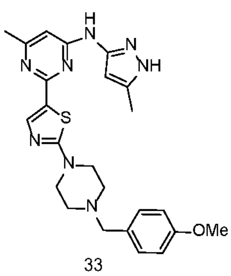
33

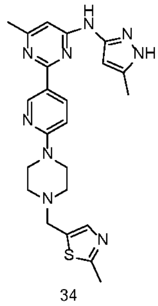
34

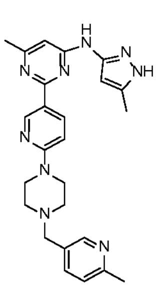
35

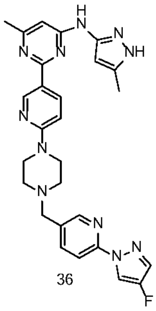
36

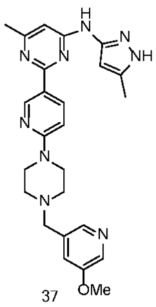
37

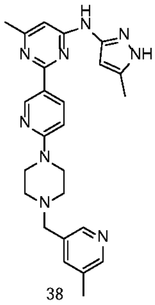
38

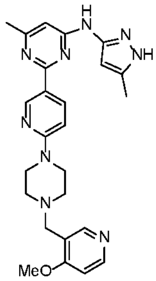
39

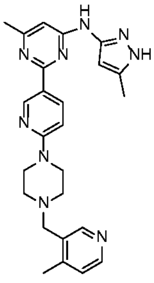
40

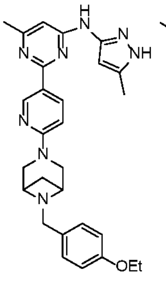
41

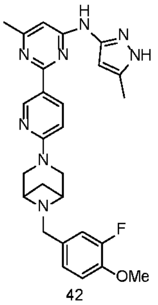
42

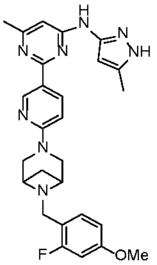
43

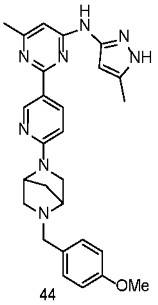
44

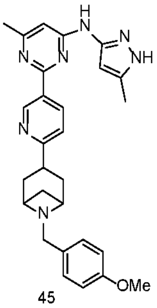
45

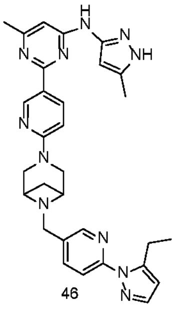
46

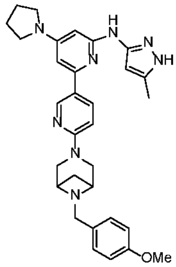
47

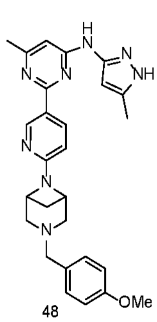
48

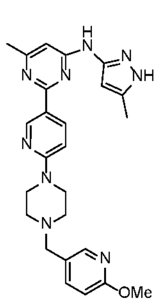
49

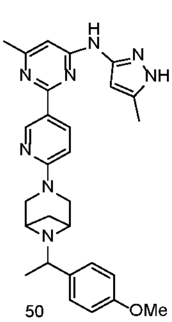
50

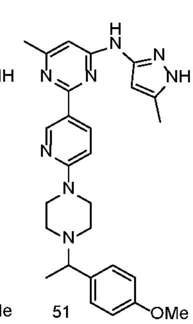
51

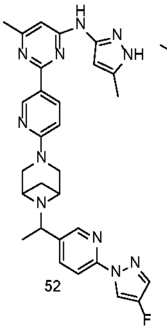
52

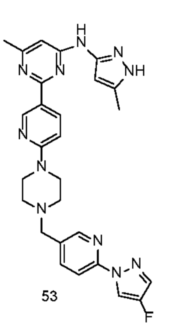
53

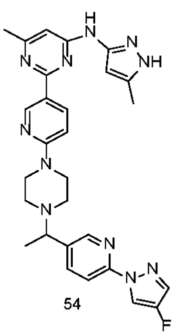
54

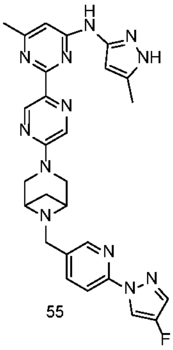
55

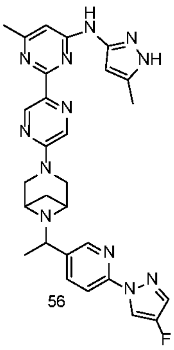
56

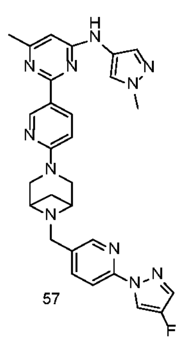
57

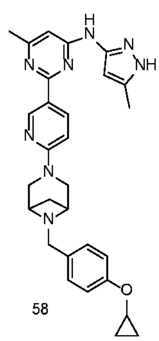
58

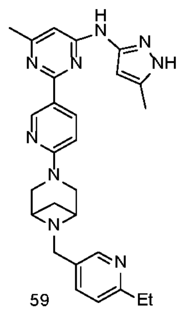
59

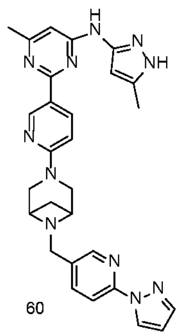
60

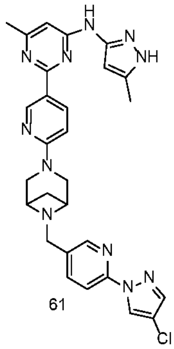
61

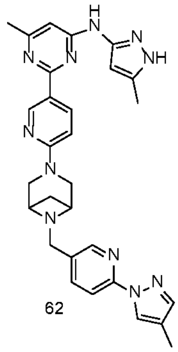
62

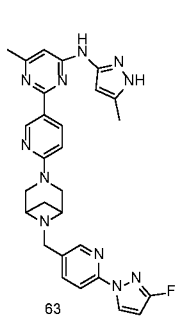
63

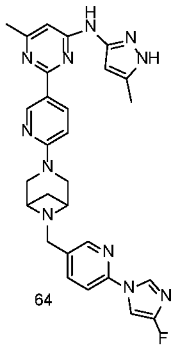
64

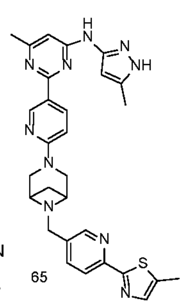
65

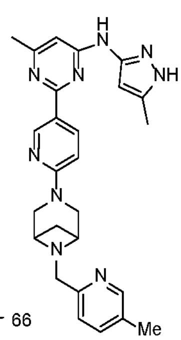
66

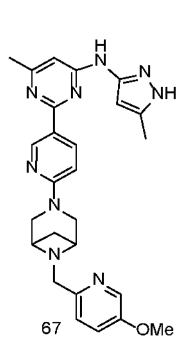
67

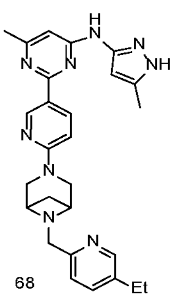
68

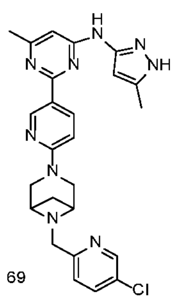
69

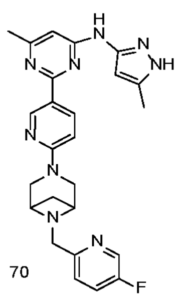
70

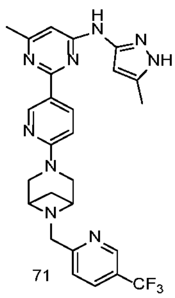
71

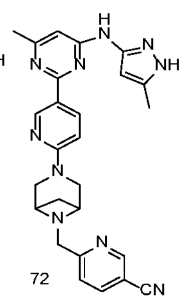
72

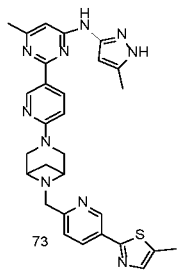
73

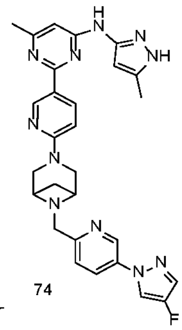
74

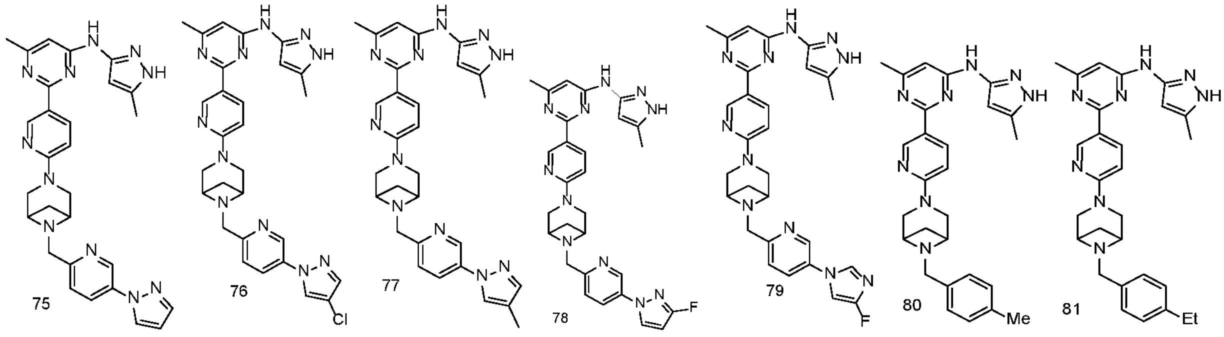

75 76 77 78 79 80 81

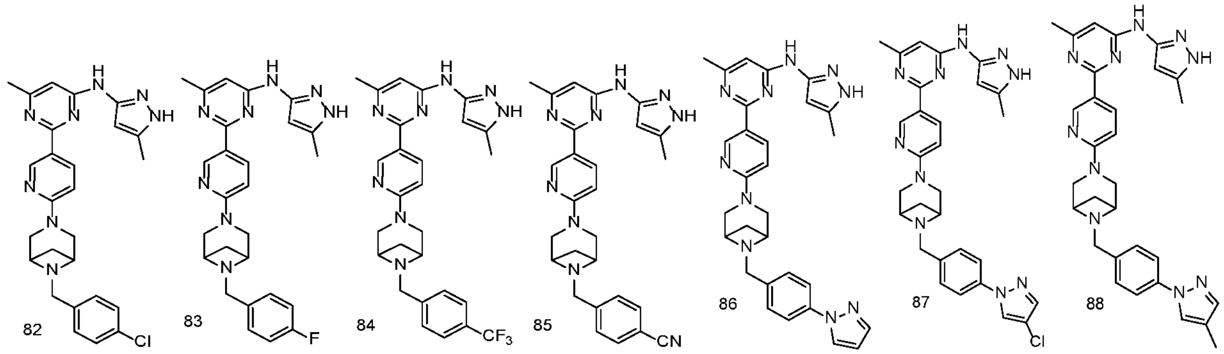

82 83 84 85 86 87 88

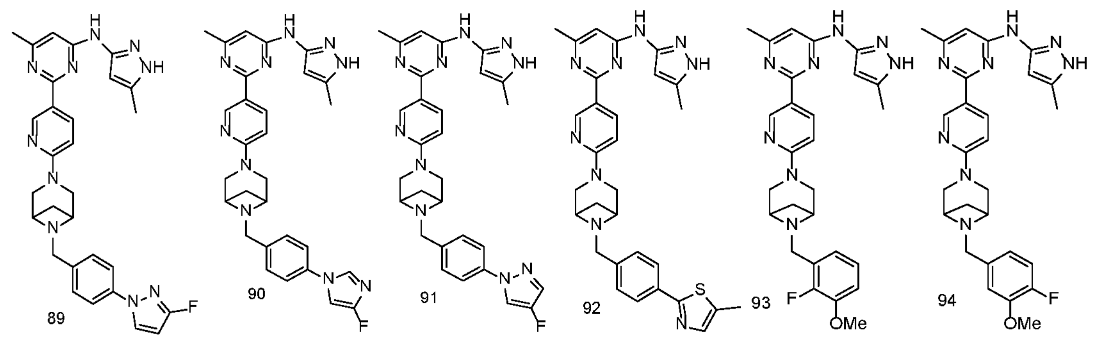

89 90 91 92 93 94

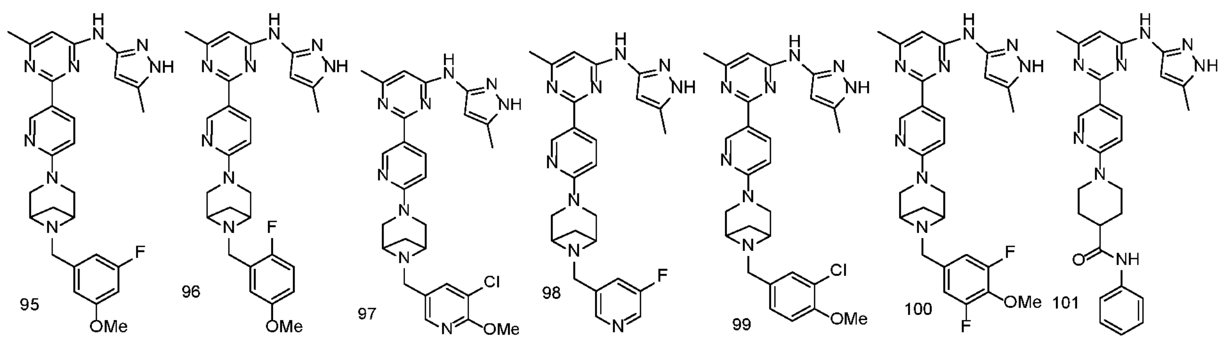

95 96 97 98 99 100 101

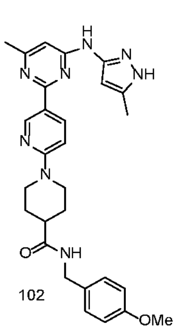
102

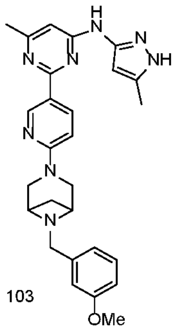
103

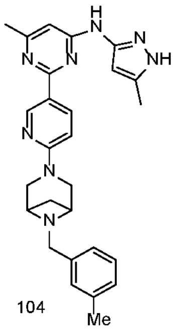
104

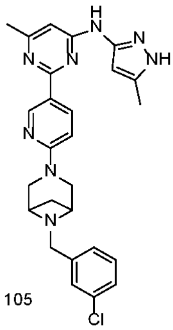
105

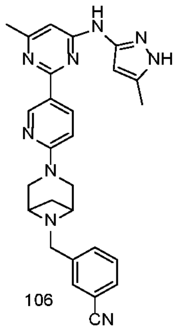
106

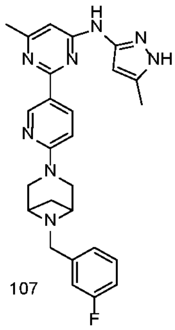
107

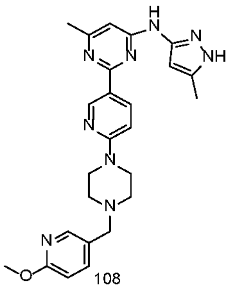
108

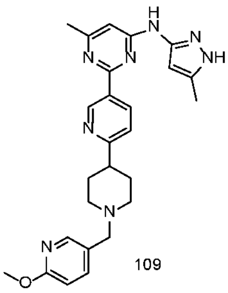
109

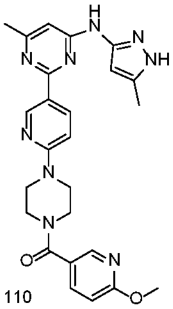
110

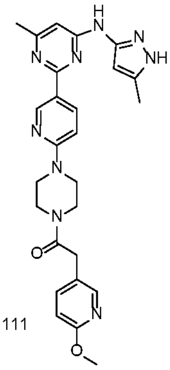
111

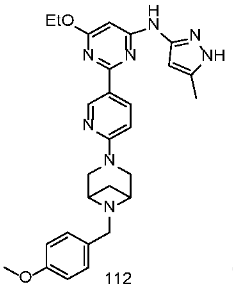
112

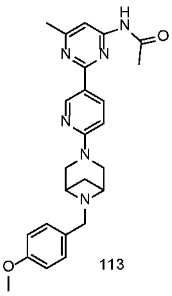
113

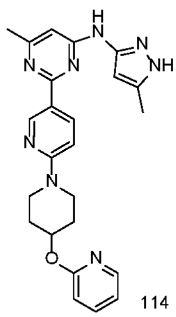
114

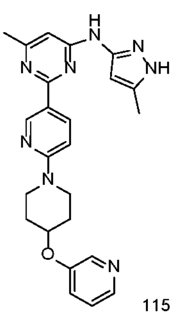
115

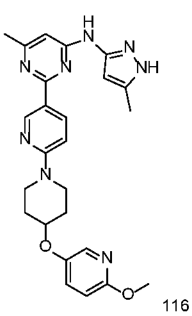
116

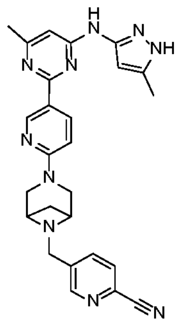
117

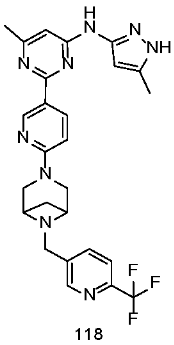
118

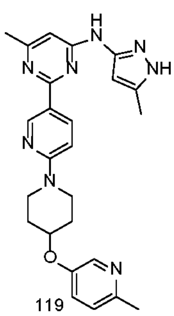
119

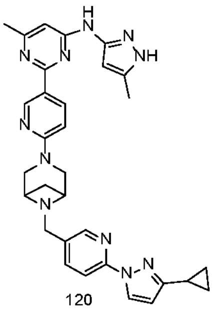
120

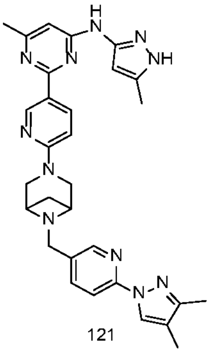
121

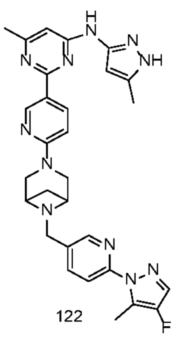
122

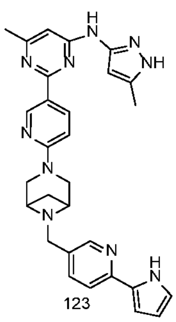
123

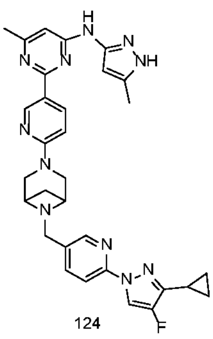
124

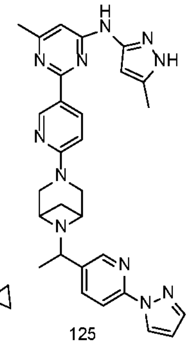
125

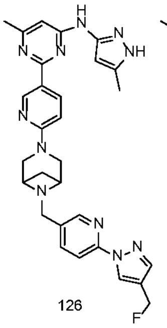
126

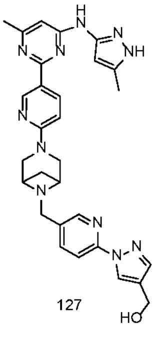
127

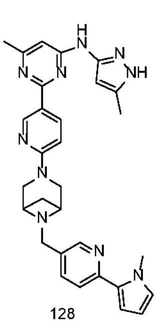
128

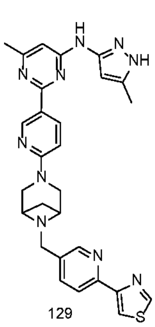
129

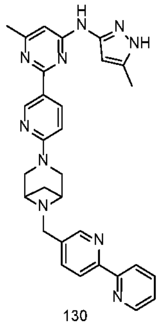
130

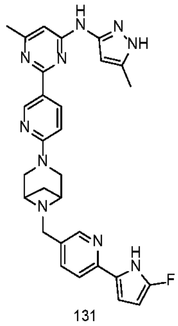
131

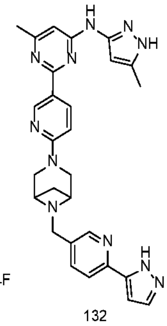
132

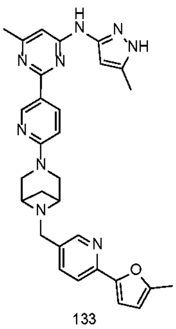
133

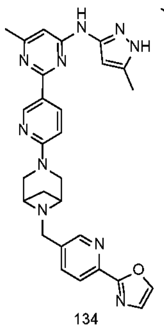
134

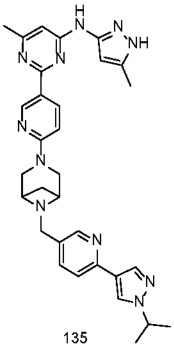
135

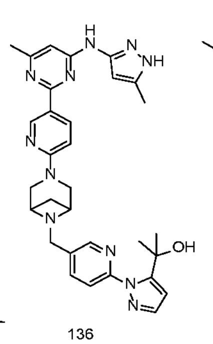
136

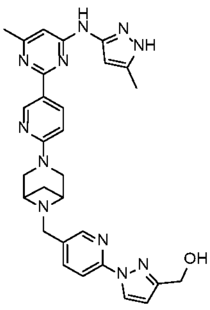
137

and

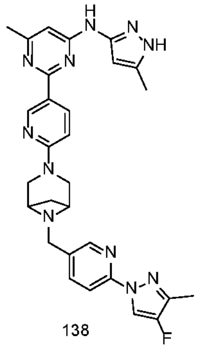
138 .

## Preparation Methods

In some embodiments, the compound of formula I-A may be synthesized using the method shown in Route A as follows:

Route A

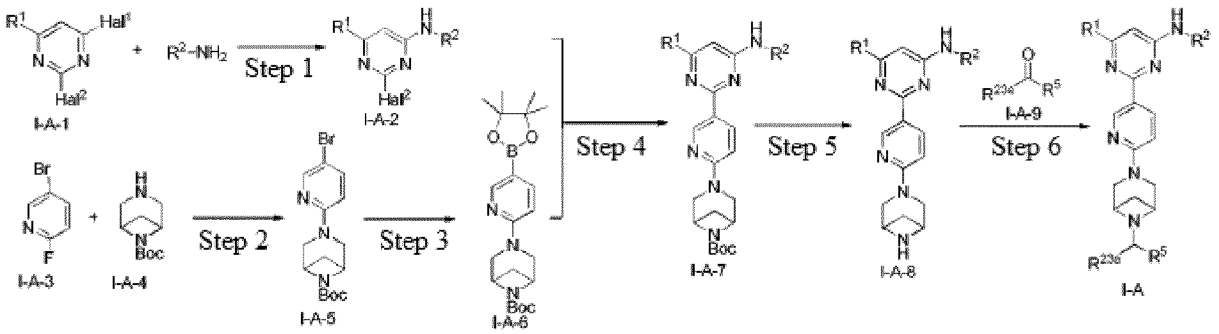


where:

Hal[1] and Hal[2] are each independently F, Cl, Br, or I; and preferably, Hal[1] is F, Cl, Br, or I, and Hal[2] is Cl, Br, or I;

$R^1$ is selected from the group consisting of H, cyano, $C_{1-6}$ alkyl, $C_{1-6}$ heteroalkyl (e.g., $C_{1-6}$ alkoxy), $C_{3-8}$ cycloalkyl, 4-6-membered heterocyclyl, and $-NR^{20a}R^{20b}$, and the alkyl, heteroalkyl (e.g., alkoxy), cycloalkyl, and heterocyclyl are each optionally substituted with one or more substituents selected from the group consisting of: halogen, CN, $C_{1-4}$ alkyl, $C_{1-4}$ haloalkyl, $C_{1-4}$ haloalkoxy, and $C_{1-4}$ heteroalkyl (e.g., $C_{1-4}$ alkoxy);

$R^2$ is selected from the group consisting of $C_{1-6}$ alkyl, $C_{1-6}$ heteroalkyl, $C_{3-8}$ cycloalkyl, 4- 6-membered heterocyclyl, and 5-6-membered heteroaryl, and the alkyl, heteroalkyl, cycloalkyl, heterocyclyl, and heteroaryl are each optionally substituted with one or more substituents selected from the group consisting of: hydroxy, halogen, CN, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;

$R^{23a}$ is selected from the group consisting of H, $C_{1-6}$ alkyl, $C_{1-6}$ alkoxy, and $C_{3-8}$ cycloalkyl, and the alkyl, alkoxy, and cycloalkyl are each optionally substituted with one or more substituents selected from the group consisting of:

OH, CN, halogen, $C_{1-4}$ alkyl, $C_{1-4}$ alkoxy, $C_{1-4}$ hydroxyalkyl, $C_{1-4}$ haloalkyl, and $C_{1-4}$haloalkoxy;
$R^{20a}$ and $R^{20b}$ are as defined in the above formula I; and
$R^5$ is as defined in the above formula I-A.

Step 1: reacting compound I-A-1 with $R^2$-$NH_2$ through a substitution or coupling reaction (e.g., a Buchwald reaction, a Suzuki reaction, or an Ullmann reaction) in the presence of a base to generate compound I-A-2.

For a substitution reaction, usable bases are, for example, $^tBuONa$, $^tBuOK$, $^tBuOLi$, $CS_2CO_3$, DIPEA, LiHMDS, LDA, NaHMDS, KHMDS, $K_3PO_4$, $Na_2CO_3$, KOAc, $NaHCO_3$, or $K_2CO_3$; usable solvents are, for example, tertiary butanol, toluene, xylene, THF, DME, 1,4-dioxane, DMF, DMSO, or NMP; and the reaction temperature is from 40 °C to 140 °C.

For a Buchwald reaction, usable catalysts are, for example, $Pd(OAc)_2$, $Pd_2(dba)_3$, $Pd(dba)_2$, $PdCl_2$, $Pd(PPh_3)_4$, $Pd(dppf)Cl_2$, $Pd(acac)_2$, or $Pd(allyl)_2$; usable ligands are, for example, $PPh_3$, XPhos, SPhos, RuPhos, XantPhos, Dppf, BINOL, BINAP, or $Pcy_3$; usable bases are, for example, $^tBuONa$, $^tBuOK$, $^tBuOLi$, $CS_2CO_3$, LiHMDS, LDA, NaHMDS, KHMDS, $K_3PO_4$, $Na_2CO_3$, KOAc, $NaHCO_3$, or $K_2CO_3$,; usable solvents are, for example, toluene, xylene, THF, DME, 1,4-dioxane, DMF, DMSO, or NMP; and the reaction temperature is from 40 °C to 140 °C.

For a Suzuki reaction, usable catalysts are, for example, $Pd(PPh_3)_4$ or $Pd(dppf)Cl_2$; usable bases are, for example, $CS_2CO_3$, $K_3PO_4$, $Na_2CO_3$, AcOK, $NaHCO_3$, or $K_2CO_3$; usable solvents are, for example, 1,4-dioxane/$H_2O$, DMF/$H_2O$, DMSO/$H_2O$, or $CH_3CN$/$H_2O$; and the reaction temperature is from 60 °C to 120 °C;

For an Ullmann reaction, usable catalysts are, for example, CuCl, CuBr, CuI, or $Cu_2O$; usable ligands are, for example, salicylaldoxime, cyclohexanediamine, N,N'-dimethylethylenediamine, TMEDA, or ethylenediamine; usable bases are, for example, $^tBuONa$, $^tBuOK$, $^tBuOLi$, $Cs_2CO_3$, LiHMDS, LDA, NaHMDS, KHMDS, $K_3PO_4$, $Na_2CO_3$, KOAc, $NaHCO_3$, or $K_2CO_3$; usable solvents are, for example, toluene, xylene, THF, DME, 1,4-dioxane, DMF, DMSO, or NMP; and the reaction temperature is from 40 °C to 140 °C.

Step 2: reacting compound I-A-3 with compound I-A-4 in the presence of a base to generate compound I-A-5.

Usable bases are, for example, $^tBuONa$, $^tBuOK$, $^tBuOLi$, $Cs_2CO_3$, DIPEA, LiHMDS, LDA, NaHMDS, KHMDS, $K_3PO_4$, $Na_2CO_3$, KOAc, $NaHCO_3$, or $K_2CO_3$. Usable solvents are, for example, tertiary butanol, toluene, xylene, THF, DME, 1,4-dioxane, DMF, DMSO, or NMP. The reaction temperature is from 40 °C to 140 °C.

Step 3: reacting the compound I-A-5 with a boron-containing reagent to generate compound I-A-6.

Usable boron-containing reagents are, for example, $B_2(pin)_2$. Usable catalysts are, for example, $Pd(PPh_3)_4$, $Pd(dppf)Cl_2$, or $Pd(dppf)_2Cl_2\cdot DCM$. Usable bases are, for example, $Cs_2CO_3$, $K_3PO_4$, $Na_2CO_3$, KOAc, $NaHCO_3$, or $K_2CO_3$. Usable solvents are, for example, 1,4-dioxane, DMF, DMSO, or $CH_3CN$. The reaction temperature is from 50 °C to 120 °C.

Step 4: reacting the compound I-A-2 with the compound I-A-6 through a coupling reaction (e.g., a Suzuki reaction) to generate compound I-A-7.

Usable catalysts are, for example, $Pd(PPh_3)_4$, $Pd(dppf)Cl_2$, or $Pd(dppf)_2Cl_2\cdot DCM$. Usable bases are, for example, $Cs_2CO_3$, $K_3PO_4$, $Na_2CO_3$, KOAc, $NaHCO_3$, or $K_2CO_3$. Usable solvents are, for example, 1,4-dioxane, DMF, DMSO, or $CH_3CN$, or a mixture of any of the above solvents and $H_2O$. The reaction temperature is from 50 °C to 120 °C.

Step 5: deprotecting the compound I-A-7 under an acidic condition to generate compound I-A-8.

Usable acids are, for example, a solution of HCl in 1,4-dioxane, a solution of HCl in EA, or a solution of TFA in DCM. The reaction temperature is from 0 °C to 80 °C.

Step 6: reacting the compound I-A-8 with compound I-A-9 through a reductive amination reaction to generate compound I-A.

Usable solvents are, for example, methanol, ethanol, THF, DCM, DCE, DMA, or a mixture of them and acetic acid at any ratio. Usable reducing agents are, for example, NaBH4, NaBH3CN, or $NaBH(OAc)_3$. The reaction temperature is from 0 °C to 80 °C. In some embodiments, the reaction may be carried out in the presence of a base or an acid, the base is, for example, TEA or DIPEA, and the acid is, for example, AcOH, HCl, or $Ti(O^iPr)_4$.

In some embodiments, the compound of formula I-A may be synthesized using the method shown in Route B as follows:

Route B

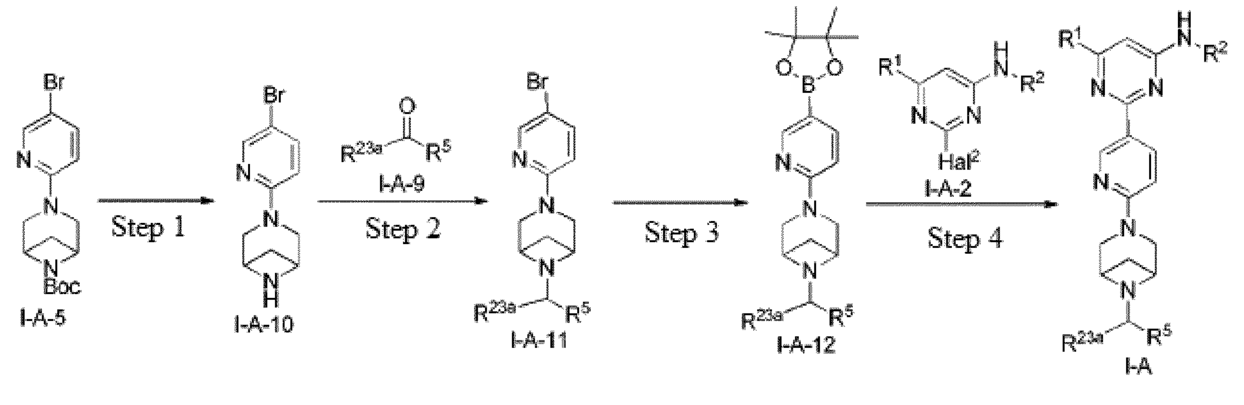


where:

$Hal^2$, $R^1$, $R^2$, $R^5$, and $R^{23a}$ are as defined in the above Route A.

Step 1: deprotecting the compound I-A-5 under an acidic condition to generate compound I-A-10.

Usable acids are, for example, a solution of HCl in 1,4-dioxane, a solution of HCl in EA, or a solution of TFA in DCM. The reaction temperature is from 0 °C to 80 °C.

Step 2: reacting the compound I-A-10 with the compound I-A-9 through a reductive amination reaction to generate compound I-A-11.

Usable bases are, for example, DIPEA or TEA. Usable reducing agents are, for example, $NaBH_3CN$ or $NaBH(OAc)_3$. Usable solvents are, for example, MeOH, EtOH, or DCE. The reaction temperature is from 0 °C to 80 °C.

Usable solvents are, for example, methanol, ethanol, THF, DCM, DCE, DMA, or a mixture of them and acetic acid at any ratio. Usable reducing agents are, for example, NaBH4, $NaBH_3CN$, or $NaBH(OAc)_3$. The reaction temperature is from 0 °C to 80 °C. In some embodiments, the reaction may be carried out in the presence of a base or an acid, the base is, for example, TEA or DIPEA, and the acid is, for example, AcOH, HCl, or $Ti(O^iPr)_4$.

Step 3: reacting the compound I-A-11 with a boron-containing reagent to generate compound I-A-12.

Usable boron-containing reagents are, for example, $B_2(pin)_2$. Usable catalysts are, for example, $Pd(PPh_3)_4$, $Pd(dppf)Cl_2$, or $Pd(dppf)_2Cl_2{\cdot}DCM$. Usable bases are, for example, $Cs_2CO_3$, $K_3PO_4$, $Na_2CO_3$, KOAc, $NaHCO_3$, or $K_2CO_3$. Usable solvents are, for example, 1,4-dioxane, DMF, DMSO, or *$CH_3CN$. The reaction temperature is from 50 °C to 120 °C.

Step 4: reacting the compound I-A-12 with the compound I-A-2 through a coupling reaction (e.g., a Suzuki reaction) to generate the compound I-A.

Usable catalysts are, for example, $Pd(PPh_3)_4$, $Pd(dppf)Cl_2$, or $Pd(dppf)_2Cl_2{\cdot}DCM$. Usable bases are, for example, $Cs_2CO_3$, $K_3PO_4$, $Na_2CO_3$, KOAc, $NaHCO_3$, or $K_2CO_3$. Usable solvents are, for example, 1,4-dioxane, DMF, DMSO, or $CH_3CN$, or a mixture of any of the above solvents and $H_2O$. The reaction temperature is from 50 °C to 120 °C.

In some embodiments, the compound of formula I-B may be synthesized using the method shown in Route C as follows:

Route C

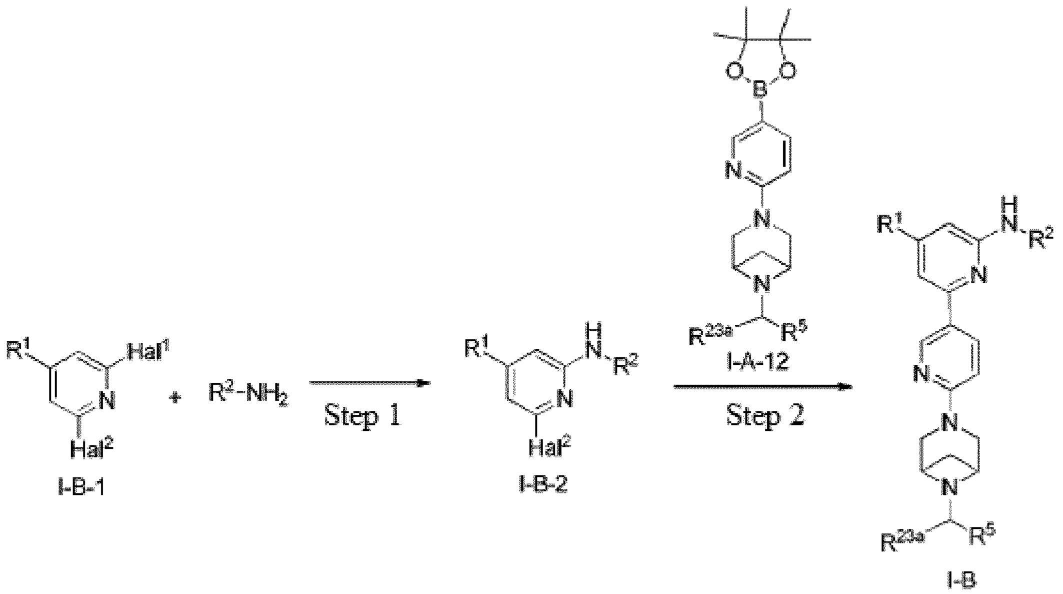


where:
Hal[1], Hal[2], $R^1$, $R^2$, $R^5$, $R^{23a}$ are as defined in the above Route A.

Step 1: reacting compound I-B-1 with $R^2$-$NH_2$ through a substitution or coupling reaction (e.g., a Buchwald reaction, a Suzuki reaction, or an Ullmann reaction) in the presence of a base to generate compound I-B-2.

The reaction conditions are as described in the Step 1 of Route A for the preparation of the compound of formula I-A.

Step 2: reacting the compound I-B-2 with the compound I-A-12 through a coupling reaction (e.g., a Suzuki reaction) to generate compound I-B.

The reaction conditions are as described in the Step 4 of Route A for the preparation of the compound of formula I-A.

In some embodiments, the compound of formula I-C may be synthesized using the method shown in Route D as follows:

Route D

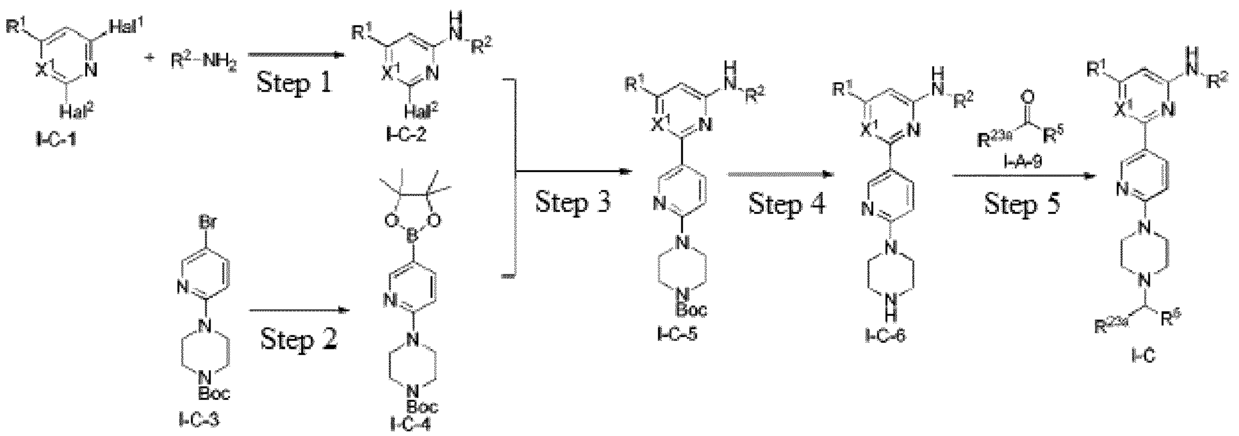


where:

Hal[1], Hal[2], $R^1$, $R^2$, $R^5$, and $R^{23a}$ are as defined in the above Route A; and
$X^1$ is selected from CH and N.

Step 1: reacting compound I-C-1 with $R^2$-$NH_2$ through a substitution or coupling reaction (e.g., a Buchwald reaction, a Suzuki reaction, or an Ullmann reaction) in the presence of a base to generate compound I-C-2.

The reaction conditions are as described in the Step 1 of Route A for the preparation of the compound of formula I-A.

Step 2: reacting compound I-C-3 with a boron-containing reagent to generate compound I-C-4.

The reaction conditions are as described in the Step 3 of Route A for the preparation of the compound of formula I-A.

Step 3: reacting the compound I-C-2 with the compound I-C-4 through a coupling reaction (e.g., a Suzuki reaction) to generate compound I-C-5.

The reaction conditions are as described in the Step 4 of Route A for the preparation of the compound of formula I-A.

Step 4: deprotecting the compound I-C-5 under an acidic condition to generate compound I-C-6.

The reaction conditions are as described in the Step 5 of Route A for the preparation of the compound of formula I-A.

Step 5: reacting the compound I-C-6 with the compound I-A-9 through a reductive amination reaction to generate compound I-C.

The reaction conditions are as described in the Step 6 of Route A for the preparation of the compound of formula I-A.

In some embodiments, the compound of formula I-D may be synthesized using the method shown in Route E as follows:

Route E

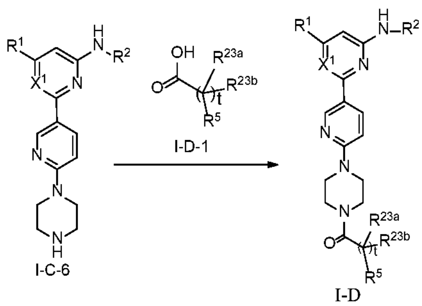


where:

$R^1$ and $R^2$ are as defined in the above Route A;
$R^5$ is as defined in the above formula I-D;
$R^{23a}$ and $R^{23b}$ are each independently selected from the group consisting of H, $C_{1-6}$ alkyl, $C_{1-6}$ alkoxy, and $C_{3-8}$ cycloalkyl; or $R^{23a}$ and $R^{23b}$ form, together with a C atom to which they are attached, a 3-8-membered cycloalkyl or heterocyclyl, and the alkyl, alkoxy, cycloalkyl, and heterocyclyl are each optionally substituted with one or more substituents selected from the group consisting of: CN, halogen, $C_{1-4}$ alkyl, $C_{1-4}$ alkoxy, $C_{1-4}$ hydroxyalkyl, $C_{1-4}$ haloalkyl, and $C_{1-4}$ haloalkoxy;
$X^1$ is selected from CH and N; and
t is 0 or 1.

The compound I-C-6 reacts with the compound I-D-1 through a condensation reaction to generate compound I-D.

Usable condensing agents are, for example, HATU, CDI, HOBt, DMAP, DCC, DIC, EDC, HBTU, HCTU, or PyBOP. Usable bases are, for example, TEA, DIPEA, $^tBuOK$, $^tBuONa$, $^tBuOLi$, NaH, NaOH, $CS_2CO_3$, $K_3PO_4$, or $Na_2CO_3$. Usable solvents are, for example, THF, DCM, DCE, MeOH, EtOH, DMF, DMSO, acetone, $CH_3CN$, 1,4-dioxane, or toluene. The reaction temperature is from 0 °C to 120 °C, such as room temperature.

Alternatively, the compound I-D-1 first reacts with an acylating reagent to form an acyl halide which then reacts with the compound I-C-6 optionally in the presence of a base to form the compound of formula I-D. Usable acylating agents are, for example, thionyl chloride or oxalyl chloride. The reaction may also be carried out under the catalysis of a small amount of DMF. Usable bases are, for example, TEA or DIPEA. Usable solvents are, for example, THF, DCM, DCE, $CH_3CN$, 1,4-dioxane, or toluene. The reaction temperature is from 0 °C to 100 °C.

In some embodiments, the compound of formula I-E may be synthesized using the method shown in Route F as follows:

Route F

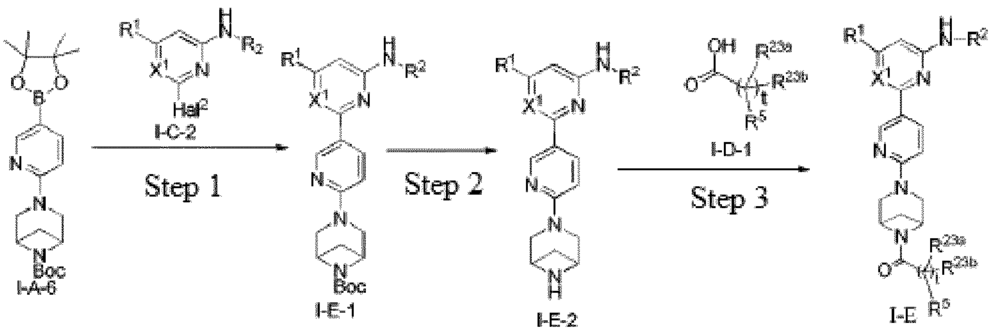


where:

$R^1$, $R^2$, $R^5$, $R^{23a}$, $R^{23b}$, and t are as defined in the above Route E;
$X^1$ is selected from CH and N; and
$Hal^2$ is F, Cl, Br, or I; and preferably, $Hal^2$ is Cl, Br, or I.

Step 1: reacting the compound I-C-2 with the compound I-A-6 through a coupling reaction (e.g., a Suzuki reaction) to generate compound I-E-1.

The reaction conditions are as described in the Step 4 of Route A for the preparation of the compound of formula I-A.

Step 2: deprotecting the compound I-E-1 under an acidic condition to generate compound I-E-2.

The reaction conditions are as described in the Step 5 of Route A for the preparation of the compound of formula I-A.

Step 3: reacting the compound I-E-2 with the compound I-D-1 through a condensation reaction to generate compound I-E.

The reaction conditions are as described in Route E for the preparation of the compound of formula I-D.

In some embodiments, the compound of formula I-F may be synthesized using the method shown in Route G as follows:

Route G

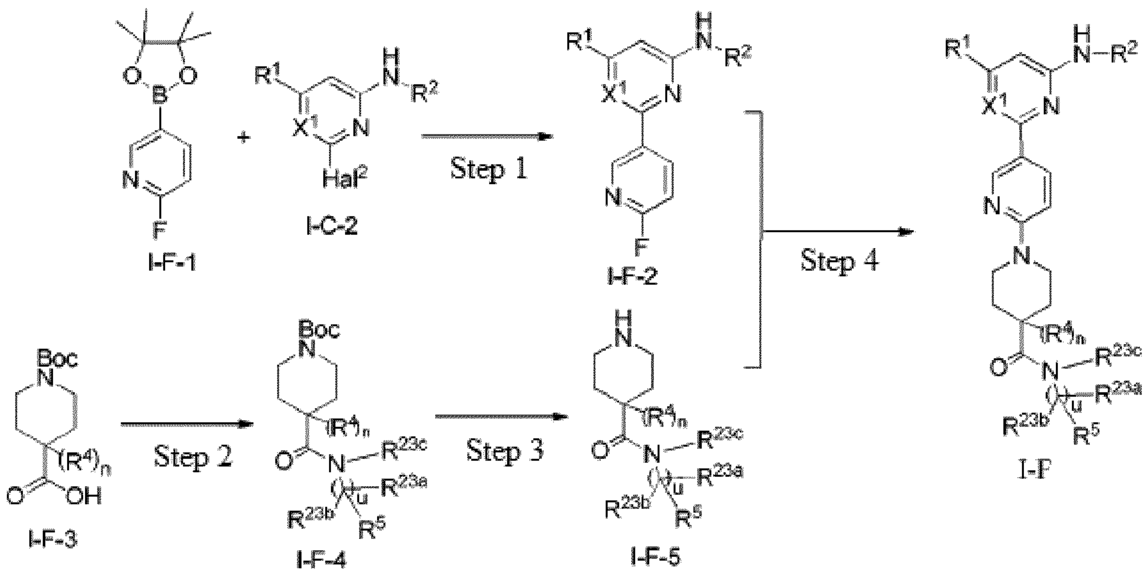


where:

$R^1$, $R^2$, $R^{23a}$, $R^{23b}$, and $R^5$ are as defined in the above Route E;
$X^1$ is selected from CH and N;
$R^4$ is absent or is selected from the group consisting of hydroxy, CN, $C_{1-6}$ alkyl, $C_{1-6}$ haloalkyl, and $C_{1-6}$ heteroalkyl (e.g., $C_{1-6}$ alkoxy);
$R^{23c}$ is H, $C_{1-3}$ alkyl, or $C_{1-3}$ alkoxy, and the alkyl and alkoxy are each optionally substituted with one or more substituents selected from the group consisting of: OH, CN, halogen, $C_{1-4}$ alkoxy, and $C_{1-4}$ hydroxyalkyl;
$Hal^2$ is F, Cl, Br, or I; and preferably, $Hal^2$ is Cl, Br, or I;
u is 0 or 1; and
n is 0 or 1.

Step 1: reacting the compound I-C-2 with the compound I-F-1 through a coupling reaction (e.g., a Suzuki reaction) to generate compound I-F-2.

The reaction conditions are as described in the Step 4 of Route A for the preparation of the compound of formula I-A.

Step 2: reacting compound I-F-3 with an amine through a condensation reaction to generate compound I-F-4.

The reaction conditions are as described in Route E for the preparation of the compound of formula I-D.

Step 3: deprotecting the compound I-F-4 under an acidic condition to generate compound I-F-5.

The reaction conditions are as described in the Step 5 of Route A for the preparation of the compound of formula I-A.

Step 4: reacting the compound I-F-2 with the compound I-F-5 through a nucleophilic substitution reaction in the presence of a base to generate compound I-F.

The reaction conditions are as described in the Step 2 of Route A for the preparation of the compound of formula I-A.

In some embodiments, the compound of formula I-G may be synthesized using the method shown in Route H as follows:

<u>Route H</u>

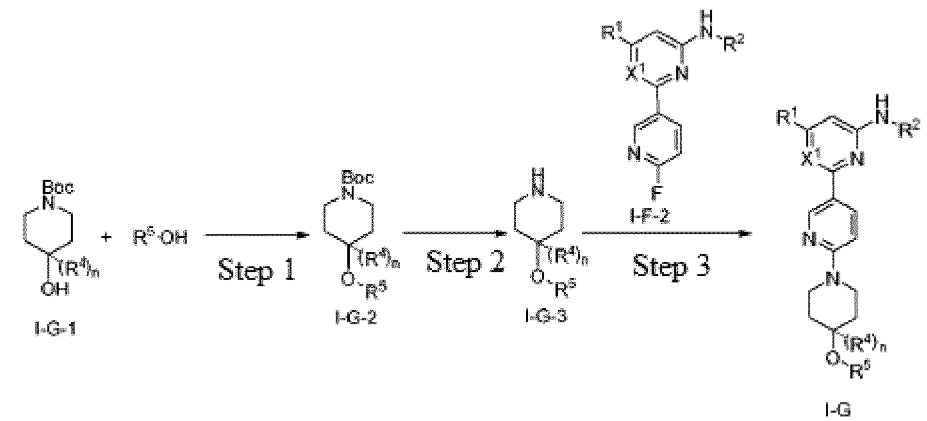


where:

$R^1$ and $R^2$ are as defined in the above Route A;
$X^1$ is selected from CH and N;
$R^4$ is selected from the group consisting of H, $C_{1-6}$ alkyl, $C_{1-6}$ haloalkyl, and $C_{1-6}$ heteroalkyl;
$R^5$ is as defined in the above formula I-G; and
n is 0 or 1.

Step 1: reacting compound I-G-1 with $R^5$-OH through a Mitsunobu reaction to generate compound I-G-2.

Usable reaction reagents are, for example, $PPh_3$, $PMe_3$, DIAD, DEAD, or DBAD. Usable solvents are aprotic solvents, such as THF, diethyl ether, DCM, DMF or toluene. The reaction temperature is from -20 °C to 100 °C, such as room temperature.

Step 2: deprotecting the compound I-G-2 under an acidic condition to generate compound I-G-3.

Usable acids are, for example, a solution of HCl in 1,4-dioxane, a solution of HCl in EA, or a solution of TFA in DCM, or the reaction is carried out in a mixture of the above acid solution and, e.g., any one solvent selected from

THF, MeOH, and EtOH. The reaction temperature is from 0 °C to 80 °C.

Step 3: reacting the compound I-F-2 with the compound I-G-3 through a nucleophilic substitution reaction in the presence of a base to generate compound I-G.

The reaction conditions are as described in the Step 2 of Route A for the preparation of the compound of formula I-A.

**Pharmaceutical Compositions, Formulations, and Therapeutic Methods**

In some embodiments, the present disclosure provides a pharmaceutical composition, comprising a prophylactically or therapeutically effective amount of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof. Optionally, the pharmaceutical composition further comprises one or more pharmaceutically acceptable carriers.

In some embodiments, the present disclosure provides a pharmaceutical formulation, which is preferably a solid formulation, a semi-solid formulation, a liquid formulation, or a gas formulation. In some embodiments, the pharmaceutical composition may further comprise one or more additional therapeutic agents.

In some embodiments, the pharmaceutical composition or pharmaceutical formulation is preferably administered through an oral, intravenous, intraarterial, subcutaneous, intraperitoneal, intramuscular, or transdermal route.

In some embodiments, the present disclosure provides use of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above, or the pharmaceutical formulation of the present disclosure in the preparation of a drug for preventing or treating a disease or condition associated with RET activity.

In some embodiments, the present disclosure provides use of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above, or the pharmaceutical formulation of the present disclosure in the preparation of a drug for adjusting (e.g., reducing or inhibiting) RET activity.

In some embodiments, the present disclosure provides the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above, or the pharmaceutical formulation of the present disclosure, for use in the prevention or treatment of a disease or condition associated with RET activity.

In some embodiments, the present disclosure provides a method for preventing or treating a disease or condition associated with RET activity, including administering to an individual in need thereof an effective amount of the compound of the present disclosure, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, or the pharmaceutical composition as described above, or the pharmaceutical formulation of the present disclosure.

In some embodiments, the disease or condition associated with RET activity is preferably cancer or tumor, or irritable bowel syndrome.

In some embodiments, the cancer or tumor is further preferably lung cancer (such as non-small cell lung cancer), breast cancer, head and neck cancer, rectal cancer, liver cancer, lymphoma, thyroid cancer (such as medullary thyroid carcinoma or papillary thyroid carcinoma), colon cancer, multiple myeloma, melanoma, glioma, brain tumor, or sarcoma.

"Pharmaceutically acceptable carriers" in the present disclosure refer to diluents, adjuvants, excipients, or vehicles which are administered together with a therapeutic agent, and are, within the scope of sound medical judgment, suitable for contact with the tissues of human beings and/or other animals without excessive toxicity, irritation, allergic response, or other problems or complications commensurate with a reasonable benefit/risk ratio.

Pharmaceutically acceptable carriers usable in the pharmaceutical composition of the present disclosure include, but are not limited to, sterile liquid. Examples of suitable pharmaceutically acceptable carriers are as described in Remington's Pharmaceutical Sciences (1990).

Pharmaceutical compositions of the present disclosure may act systemically and/or locally. For this purpose, they may be administered through a suitable route.

For these administration routes, the pharmaceutical composition of the present disclosure may be administered in a suitable dosage form.

The term "effective amount" as used herein refers to an amount of a compound that, after being administered, will relieve one or more symptoms of the condition being treated to a certain extent.

This dosage regimen may be adjusted to provide the optimum desired response. For example, a single bolus may be administered, several divided doses may be administered over time, or the dose may be proportionally reduced

or increased as indicated by the exigencies of the therapeutic situation. It should be noted that the dose value may vary with the type and severity of the condition to be alleviated, and may include single or multiple doses. It should be further understood that for any particular individual, the specific dosage regimen should be adjusted over time according to the needs of the individual and the professional judgment of the person administering the composition or supervising the administration of the composition.

The amount of the administered compound of the present disclosure will depend on the individual being treated, the severity of the disorder or condition, the rate of administration, the disposal of the compound, and the judgment of the prescribing physician. In general, the effective dosage ranges from about 0.0001 to about 50 mg per kg body weight per day. In some instances, dosage levels not higher than the lower limit of the aforesaid range may be adequate, while in other cases still larger doses may be employed without causing any harmful side effect, provided that such larger doses are first divided into several small doses for administration throughout the day.

The content or amount of the compound of the present disclosure in the pharmaceutical composition may be from about 0.01 mg to about 1000 mg.

Unless otherwise specified, the term "treating" as used herein means reversing, alleviating, inhibiting the progress of, or preventing the disorder or condition to which such term applies, or one or more symptoms of such disorder or condition.

The "Individual" as used herein includes human or non-human animals. Exemplary human individuals include human individuals (referred to as patients) suffering from diseases (such as the diseases described herein) or normal individuals. In the present disclosure, "non-human animals" include all vertebrates, for example, non-mammals (such as birds, amphibians, reptiles) and mammals, for example, non-human primates, livestock, and/or domesticated animals (such as sheep, dogs, cats, cows, and pigs).

In some embodiments, the pharmaceutical composition of the present disclosure may further comprise one or more additional therapeutic agents or prophylactic agents (for example, additional drugs for treating a cancer or neoplastic disease). In some embodiments, the method of the present disclosure may also include the administration of one or more additional therapeutic agents or prophylactic agents (e.g., additional drugs for treating a cancer or a neoplastic disease).

## Examples

The present disclosure will be further described below in combination with examples, but the provision of these examples is not intended to limit the scope of the present disclosure.

The abbreviations as used herein have the following meanings:

| Abbreviation | Meaning | Abbreviation | Meaning |
|---|---|---|---|
| NMR | Nuclear magnetic resonance | MS | Mass spectrometry |
| TLC | Thin layer chromatography | LC-MS | Liquid chromatography-mass spectrometry |
| HPLC | High performance liquid chromatography | MPLC | Medium pressure liquid chromatography |
| TFA | Trifluoroacetic acid | h | hour |
| min | minute | Dppf | 1,1-bis(diphenylphosphino) ferrocene |
| Prep-HPLC | Preparative high performance liquid chromatography | $CD_3OD$ | Deuterated methanol |
| DMSO-$d_6$ | Deuterated dimethyl sulfoxide | DCM/$CH_2Cl_2$ | Dichloromethane |
| DMSO | Dimethyl sulfoxide | DCE | 1,2-dichloroethane |
| PE | Petroleum ether | DMF | *N,N*-dimethylformamide |
| RET | Rearrangement during transfection | EA/EtOAc | Ethyl acetate |
| DIEA/DIPEA | N,N-diisopropylethylamine | THF | Tetrahydrofuran |
| TEA | Triethylamine | NMP | N -methylpyrrolidone |

| | | | |
|---|---|---|---|
| LiHMDS | Lithium bis(trimethylsilyl)amide | LDA | Lithium diisopropylamide |
| NaHMDS | Sodium bis(trimethylsilyl)amide | KHMDS | Potassium bis(trimethylsilyl)amide |
| Pd $(PPh_3)_4$ | Tetrakis(triphenylphosphine)palladium | Pd | Palladium |
| $Pd(OAc)_2$ | Palladium acetate | Pd(dppf) $Cl_2$ | 1,1'-bis(diphenylphosphino)ferrocene dichloropalladium |
| $Pd(dba)_2$ | Bis(dibenzylideneacetone)palladium | $Pd_2(dba)_3$ | Tris(dibenzylideneacetone)dipalladium |
| $Pd(acac)_2$ | Bis(acetylacetone)palladium | XPhos | 2-dicyclohexylphosphino-2',4',6'-triisopropylbiphenyl |
| RuPhos | 2-dicyclohexylphosphino-2',6'-diisopropoxy-1,1'-biphenyl | SPhos | 2-dicyclohexylphosphino-2',6'-dimethoxy -biphenyl |
| BINOL | 1,1'-binaphthol | XantPhos | 4,5-bis(diphenylphosphino)-9,9-dimethylxanthene |
| $PCy_3$ | Tricyclohexylphosphine | BINAP | 2,2'-bis(diphenylphosphino)-1,1'-binaphthyl |
| Boc | Tert-butoxycarbonyl | $B_2(pin)_2$ | Bis(pinacolato)diboron |
| Et | Ethyl | Allyl | Allyl |
| Me | Methyl | Ms | Methanesulfonyl |
| HATU | 0-(7-azabenzotriazolyl)-N,N,N',N'-tetramethyluronium hexafluorophosphate | EDC | 1-ethyl-(3-dimethylaminopropyl)carbodiimide |
| HCTU | O-(6-chloro-1-benzotriazol-1-yl)-N,N,N',N'-tetramethylurea hexafluorophosphate | HBTU | Benzotriazolyl-N,N,N',N'- tetramethylurea hexafluorophosphate |
| CDI | Carbonyldiimidazole | PyBOP | 1H-benzotriazol-1 -yloxytripyrrolidinyl hexafluorophosphate |
| DMAP | 4-dimethylaminopyridine | HOBt | 1-hydroxybenzotriazole |
| DIC | N,N'-diisopropylcarbodiimide | DCC | Dicyclohexylcarbodiimide |
| DME | Dimethoxyethane | $^tBuOK$ | Potassium tert-butoxide |
| $^tBuONa$ | Sodium tert-butoxide | $^tBuOLi$ | Lithium tert-butoxide |
| MeOH | Methanol | - | - |

The compound of the present disclosure is separated and purified by preparative TLC, silica gel column chromatography, Prep-HPLC, and/or flash column chromatography, and its structure is validated by $^1H$ NMR and/or MS. The reaction is monitored by TLC or LC-MS.

A Bruker superconducting nuclear magnetic resonance spectrometer (model AVACE III HD 400 MHz) is employed for $^1H$ NMR spectroscopy.

Aglient 1260 Infinity/Aglient 6120 Quadrupole is employed for LC/MS.

Silica gel GF 254 is used as the stationary phase for TLC.

200-300 mesh silica gel (Qingdao Haiyang) is generally used as the stationary phase for column chromatography.

A Biotage flash column chromatograph is used for flash column chromatography.

Agilent 1260, Waters 2489, and GeLai 3500 chromatographic insturments are used for Prep-HPLC.

A BiotageInitiator microwave reactor is used for microwave reaction.

In the following examples, unless otherwise specified, the reaction temperature is room temperature (from 15 to 30 °C).

Reagents used in the present disclosure are purchased from companies such as Acros Organics, Aldrich Chemical Company, or Terbo Chemical.

**Example 1: 2-(6-(6-benzyl-3,6-diazabicyclo[3.1.1]heptan-3-yl)pyridin-3-yl)-6-methyl-N-(5-methyl-1H-pyrazol-3-yl)pyrimidin-4-amine (Compound 1)**

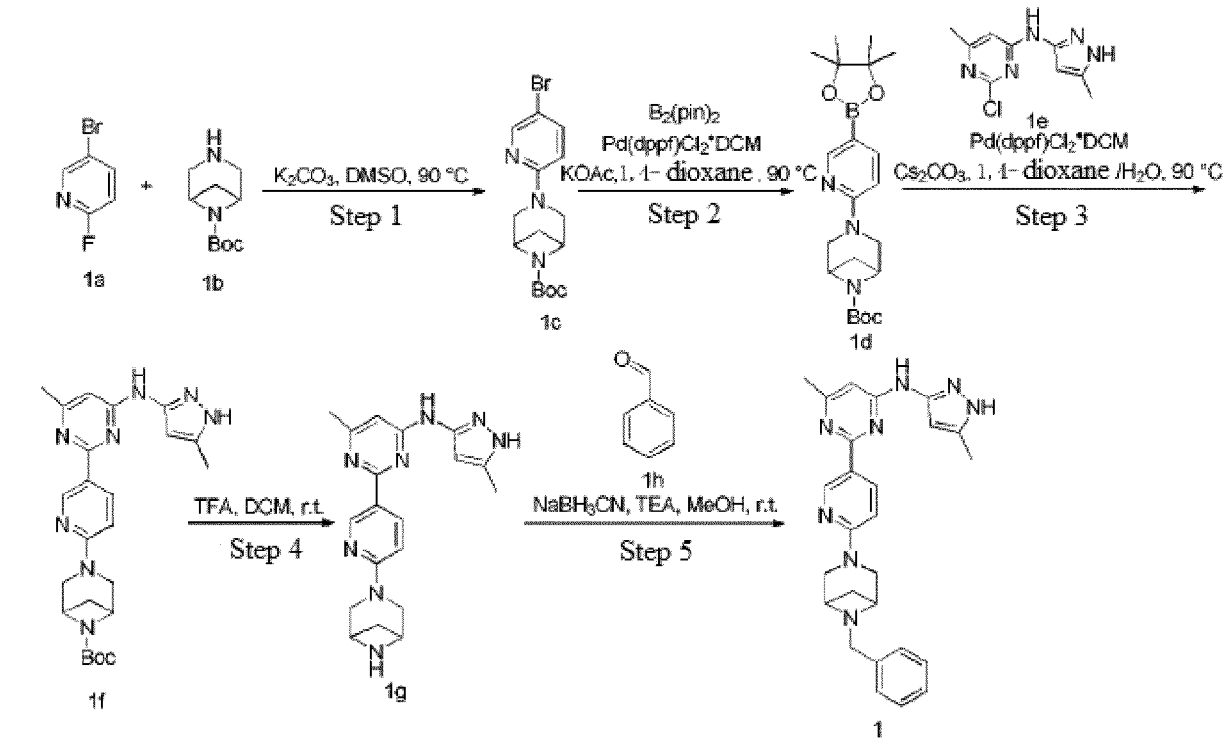


1a 1b 1c 1d 1e 1f 1g 1h 1

**Step 1: Preparation of tert-butyl 3-(5-bromopyridin-2-yl)-3,6-diazabicyclo[3.1.1]heptane-6-carboxylate (Compound 1c)**

Compound 1a (1.50 g) and Compound 1b (1.77 g) were successively added into a 100 mL single-necked flask, and then DMSO (20.0 mL) and $K_2CO_3$ (5.83 g) were successively added. The mixture was heated to 90 °C, and stirred under the protection of nitrogen at this temperature for 20 h. After completion of the reaction, the reaction mixture was cooled to room temperature, diluted with water (100 mL), and extracted with EA. The organic phases were combined, washed with saturated brine, dried over anhydrous sodium sulfate, filtered, concentrated under reduced pressure, and separated and purified by flash silica gel column chromatography (PE:EA=5:1), to provide Compound 1c (2.03 g). MS m/z (ESI): 354.1 $[M+H]^+$.

**Step 2: Preparation of tert-butyl 3-(5-(4,4,5,5-tetramethyl-1,3,2-dioxaborolan-2-yl)pyridin-2-yl)-3,6-diazabicyclo[3.1.1]heptane-6-carboxylate (Compound Id)**

Compound 1c (2.03 g), $B_2(pin)_2$ (4.01 g), KOAc (1.55 g), 1,4-dioxane (15.0 mL), and $Pd(dppf)Cl_2 \cdot DCM$ (644.67 mg) were successively added into a 100 mL single-necked flask, and were heated to 90 °C for reaction under the protection of nitrogen. After completion of the reaction, the reaction mixture was cooled to room temperature, diluted with water (30 mL), and extracted with EA (40 mL×3). The organic phases were combined, washed with saturated brine, dried over anhydrous sodium sulfate, filtered, concentrated to dryness under reduced pressure, and separated and purified by flash silica gel column chromatography (DCM:MeOH=15:1), to provide Compound Id (2.11 g). MS m/z (ESI): 402.3 $[M+H]^+$.

**Step 3: Preparation of tert-butyl 3-(5-(4-methyl-6-((5-methyl-1H-pyrazol-3-yl)-amino)pyrimidin-2-yl)pyridin-2-yl)-3,6-diazabicyclo [3.1.1] heptane-6-carboxylate (Compound If)**

Compound 1e (950 mg) was dissolved in 1,4-dioxane (50.0 mL), Compound Id (2.11 g), $Cs_2CO_3$ (3.15 g), and water (5.0 mL) were successively added, and then $Pd(dppf)Cl_2 \cdot DCM$ (477.83 mg) was added. The mixture was heated to 90°C, and kept for reaction under the protection of nitrogen at this temperature for 14 h. After completion of the

reaction, the reaction mixture was cooled to room temperature, diluted with water (100 mL), and extracted with EA (60 mL×3). The organic phases were combined, washed with saturated brine, dried over anhydrous sodium sulfate, filtered, and then concentrated to dryness under reduced pressure, to provide Compound If (587.0 mg). MS m/z (ESI): 463.3 $[M+H]^+$.

**Step 4: Preparation of 2-(6-(3,6-diazabicyclo[3.1.1]heptan-3-yl)pyridin-3-yl)-6-methyl-N-(5-methyl-1H-pyrazol-3-yl)pyrimidin-4-amine (Compound 1g)**

Compound If (1.36 g) was dissolved in DCM (20.0 mL), TFA (20.0 mL) was then added, and the mixture was kept for reaction under the protection of nitrogen at room temperature. After completion of the reaction, the reaction mixture was concentrated to dryness under reduced pressure, and separated and purified by Prep-HPLC to provide trifluoroacetate of Compound 1g (587.0 mg). MS m/z (ESI): 363.3 $[M+H]^+$.

**Step 5: Preparation of 2-(6-(6-benzyl-3,6-diazabicyclo[3.1.1]heptan-3-yl)pyridin-3-yl)-6-methyl-N-(5-methyl-1H-pyrazol-3-yl)pyrimidin-4-amine (Compound 1)**

Trifluoroacetate of Compound 1g (31.58 mg) and Compound 1h (27.74 mg) were dissolved in MeOH (0.5 mL), TEA (8.46 mg) and sodium cyanoborohydride (26.27 mg) were successively added, and the mixture was kept for reaction at room temperature for 16 h. After completion of the reaction, the reaction mixture was concentrated to dryness under reduced pressure, and separated and purified by Prep-HPLC to provide Compound 1 (11.0 mg). MS m/z (ESI): 453.2 $[M+H]^+$.

$^1H$ NMR (400 MHz, DMSO-$d_6$) δ 12.15 (br, 1H), 9.67 (s, 1H), 9.12 (d, *J*= 2.4 Hz, 1H), 8.44 (dd, *J*= 8.8, 2.4 Hz, 1H),7.43-7.21 (m, 5H), 6.78 (d, *J* = 8.8 Hz, 2H), 6.30 (br, 1H), 3.98-3.57 (m, 8H), 2.72-2.61 (m, 1H), 2.33 (s, 3H), 2.24 (s, 3H), 1.72-1.64 (m, 1H).

**Example 2: 2-(6-(6-(4-methoxybenzyl)-3,6-diazabicyclo[3.1.1]heptan-3-yl)pyridin-3-yl)-6-methyl-N-(5-methyl-1H-pyrazol-3-yl)pyrimidin-4-amine (Compound 2)**

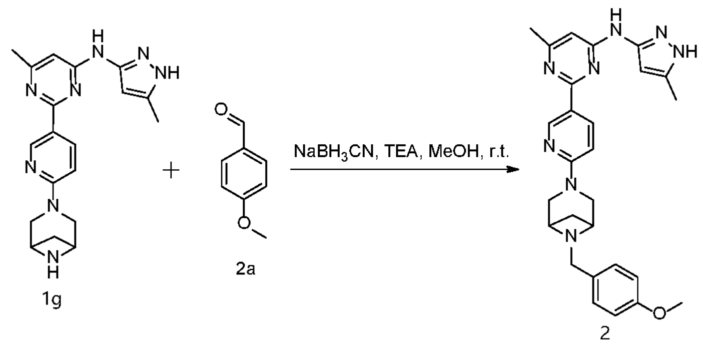


Trifluoroacetate of Compound 1g (30.0 mg) and Compound 2a (33.81 mg) were dissolved in MeOH (0.5 mL), TEA (8.12 mg) and sodium cyanoborohydride (25.21 mg) were successively added, and the mixture was kept for reaction at room temperature for 16 h. After completion of the reaction, the reaction mixture was concentrated to dryness under reduced pressure, and separated and purified by Prep-HPLC to provide Compound 2 (15.0 mg). MS m/z (ESI): 483.3 $[M+H]^+$.

$^1H$ NMR (400 MHz, DMSO-$d_6$) δ 12.14 (br, 1H), 9.66 (s, 1H), 9.12 (d, *J*= 2.4 Hz, 1H), 8.43 (dd, *J* = 8.8, 2.4 Hz, 1H), 7.26 (d, *J* = 8.8 Hz, 2H), 6.88-6.76 (m, 4H), 6.30 (br, 1H), 3.79-3.72 (m, 7H), 3.58-3.53 (m, 4H), 2.59-2.55 (m, 1H), 2.33 (s, 3H), 2.26 (s, 3H), 1.60 (d, *J*= 8.4 Hz, 1H).

**Example 3: 2-(6-(6-((6-chloropyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 3)**

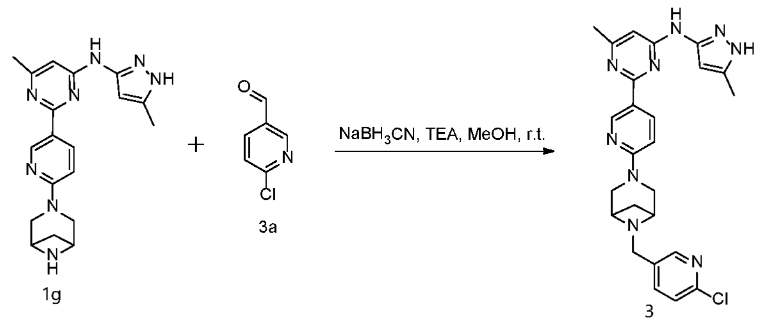


Trifluoroacetate of Compound 1g (30.0 mg) and Compound 3a (35.15 mg) were dissolved in MeOH (0.5 mL), TEA (8.12 mg) and sodium cyanoborohydride (25.21 mg) were successively added, and the mixture was kept for reaction at room temperature for 16 h. After completion of the reaction, the reaction mixture was concentrated to dryness under reduced pressure, and separated and purified by Prep-HPLC to provide Compound 3 (20.0 mg). MS m/z (ESI): 488.2 $[M+H]^+$.

$^1$H NMR (400 MHz, DMSO-$d_6$) δ 12.12 (br, 1H), 9.67 (s, 1H), 9.11 (d, *J*= 2.0 Hz, 1H), 8.46-8.42 (m, 2H), 7.87-7.80 (m, 1H), 7.50-7.47 (m, 1H), 6.80-6.77 (m, 2H), 6.31 (br, 1H), 4.01-3.52 (m, 8H), 2.66-2.57 (m, 1H), 2.33 (s, 3H), 2.26 (s, 3H), 1.69-1.62 (m, 1H).

**Example 4: 2-(6-(6-((6-methoxypyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 4)**

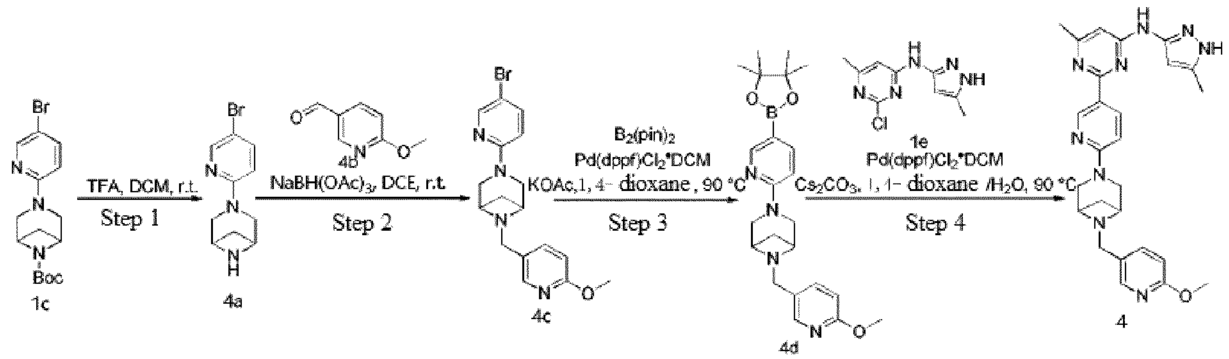


**Step 1: Preparation of 3-(5-bromopyridin-2-yl)-3,6-diazabicyclo[3.1.1]heptane (Compound 4a)**

Compound 1c (460.0 mg) was dissolved in DCM (5.0 mL) under the protection of nitrogen, TFA (5.0 mL) was added, and the mixture was kept for reaction at room temperature for 2 h until the raw materials were fully converted. After completion of the reaction, the reaction mixture was concentrated to dryness under reduced pressure, washed with saturated sodium carbonate solution, extracted with DCM (30 mL x 3), dried over anhydrous sodium sulfate, filtered, and then concentrated under reduced pressure to provide Compound 4a (250.0 mg). MS m/z (ESI): 254.0 $[M+H]^+$.

**Step 2: Preparation of 3-(5-bromopyridin-2-yl)-6-((6-methoxypyridin-3-yl)methyl)-3,6-diazabicyclo[3.1.1]heptane (Compound 4c)**

Compound 4a (250.0 mg) and Compound 4b (275.0 mg) were dissolved in DCE (5.0 mL), NaBH(OAc)$_3$ (1.06 g) was added, and the mixture was kept for reaction at room temperature for 10 h. After completion of the reaction, the reaction mixture was diluted with water (100 mL), and extracted with EA (50 mL×3). The organic phases were combined, washed with saturated brine, dried over anhydrous sodium sulfate, filtered, concentrated to dryness under reduced pressure, and separated and purified by flash silica gel column chromatography (PE:EA=10:1-1:3), to provide Compound 4c (320.0 mg). MS m/z (ESI): 375.1 $[M+H]^+$.

**Step 3: Preparation of 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane (Compound 4d)**

Compound 4c (332.0 mg) was dissolved in 1,4-dioxane (5.0 mL), $B_2(pin)_2$ (619.76 mg) and KOAc (237.11 mg) were successively added, and $Pd(dppf)Cl_2{\cdot}DCM$ (99.65 mg) was added under the protection of nitrogen. The mixture was heated to 90 °C, and kept for reaction at this temperature for 4 h. After completion of the reaction, the reaction mixture was cooled to room temperature, diluted with water (100 mL), and extracted with EA (60 mL×3). The organic phases were combined, washed with saturated brine, dried over anhydrous sodium sulfate, filtered, concentrated to dryness under reduced pressure, and separated and purified by flash silica gel column chromatography (DCM:MeOH=10:1), to provide Compound 4d (320.0 mg). MS m/z (ESI): 423.3 $[M+H]^+$.

**Step 4: Preparation of 2-(6-(6-((6-methoxypyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 4)**

Compound 4d (129.8 mg) was dissolved in 1,4-dioxane (5.0 mL), Compound 1e (55 mg), $Cs_2CO_3$ (149 mg), and water (5.0 mL) were successively added, and $Pd(dppf)Cl_2{\cdot}DCM$ (27.93 mg) was added under the protection of nitrogen. The mixture was heated to 90°C, and kept for reaction at this temperature for 5 h. After completion of the reaction, the reaction mixture was cooled to room temperature, diluted with water (100 mL), and extracted with EA (60 mL×3). The organic phases were combined, washed with saturated brine, dried over anhydrous sodium sulfate, filtered, concentrated to dryness under reduced pressure, and separated and purified by Prep-HPLC, to provide Compound 4 (18.0 mg). MS m/z (ESI): 484.3 $[M+H]^+$.

$^1H$ NMR (400 MHz, DMSO-$d_6$) $\delta$ 11.97 (s, 1H), 9.65 (s, 1H), 9.12 (d, *J*= 2.4 Hz, 1H), 8.43 (dd, *J*= 8.8, 2.4 Hz, 1H), 8.07 (d, *J* = 2.0 Hz, 1H), 7.68 (dd, *J*= 8.4, 2.4 Hz, 1H), 6.78-6.75 (m, 3H), 6.30 (br, 1H), 3.82 (s, 3H), 3.74 (d, *J*= 11.6 Hz, 2H), 3.66 (d, *J*=6.0 Hz, 2H), 3.61-3.45 (m, 4H), 2.53-2.51 (m, 1H), 2.33 (s, 3H), 2.26 (s, 3H), 1.57 (d, *J*=8.0 Hz, 1H).

**Example 5: 2-(6-(6-((5-methoxypyridin-3-yl)methyl)-3,6-diazabicyclo [3.1.1]heptan-3-yl)pyridin-3-yl)-6-methyl-N-(5-methyl-1H-pyrazol-3-yl)pyrimidin-4-amine (Compound 18)**

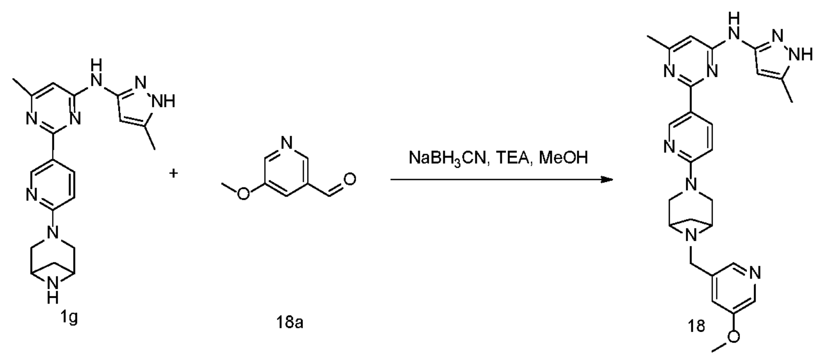


1g 18a 18

Trifluoroacetate of Compound 1g (35.00 mg) and Compound 18a (27.75 mg) were added into methanol (1.0 mL), and then triethylamine (6.83 mg) and sodium cyanoborohydride (17.00 mg) were successively added. The mixture was kept for reaction at room temperature for 16 h. After completion of the reaction, the reaction mixture was concentrated to dryness under reduced pressure, and separated and purified by Prep-HPLC to provide Compound 18 (6.0 mg). MS m/z (ESI): 484.3 $[M+H]^+$.

$^1H$ NMR (400 MHz, DMSO-$d_6$) $\delta$ 12.13 (br, 1H), 9.65 (s, 1H), 9.12 (d, *J*= 2.2 Hz, 1H), 8.43 (dd, *J* = 8.92, 2.32 Hz, 1H), 8.21 (s, 1H), 8.16-8.12 (m, 2H), 7.35-7.31 (m, 1H), 6.77 (d, *J* = 9.0 Hz, 1H), 6.31 (br, 1H), 3.81 (s, 3H), 3.78-3.69 (m, 4H), 3.59 (br, 4H), 2.59-2.52 (m, 1H), 2.33 (s, 3H), 2.25 (s, 3H), 1.59 (d, *J* = 8.36 Hz, 1H).

**Example 6: 2-(6-(6-((6-(4-fluoro-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 17)**

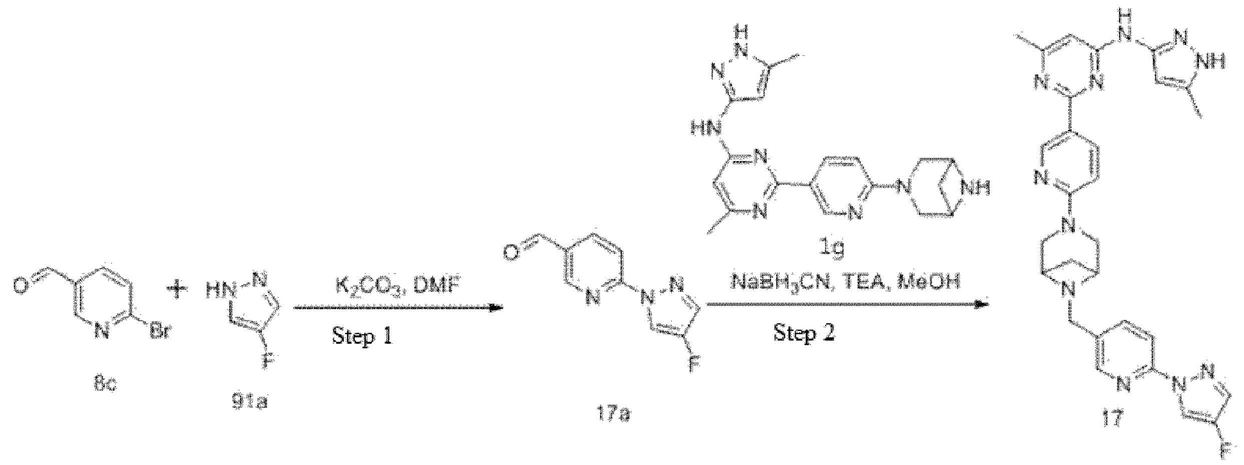


**Step 1: Preparation of 6-(4-fluoro-1H-pyrazol-1-yl)nicotinaldehyde (Compound 17a)**

Compound 8c (2.0 g), hydrochloride of Compound 91a (1.58 g), and potassium carbonate (4.45 g) were successively added into DMF (15 mL), heated to 80 °C, and stirred at this temperature for 14 h. The reaction mixture was cooled to room temperature, diluted with water (100 mL), and extracted with DCM (50 mL×2). The organic phases were combined, washed with water and saturated brine, dried over anhydrous sodium sulfate, filtered, concentrated under reduced pressure, and separated and purified by flash silica gel column chromatography (PE:EA=10:1), to provide Compound 17a (0.81 g). MS m/z (ESI): 192.1 $[M+H]^+$.

**Step 2: Preparation of 2-(6-(6-((6-(4-fluoro-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 17)**

Trifluoroacetate of Compound 1g (22.82 mg) and Compound 17a (27.47 mg) were added into methanol (1.0 mL), and then triethylamine (4.45 mg) and sodium cyanoborohydride (13.86 mg) were successively added. The mixture was kept for reaction at room temperature for 14 h. After completion of the reaction, the reaction mixture was concentrated to dryness under reduced pressure, and separated and purified by Prep-HPLC to provide Compound 17 (7.0 mg). MS m/z (ESI): 538.3 $[M+H]^+$.

$^1$H NMR (400 MHz, DMSO-$d_6$) δ 11.98 (s, 1H), 9.66 (s, 1H), 9.12 (d, *J* = 2.16 Hz, 1H), 8.67 (dd, *J*= 4.54, 0.64 Hz, 1H), 8.43 (dd, *J* = 8.94, 2.28 Hz, 1H), 8.41 (d, *J* = 1.68, 1H), 7.98 (dd, *J* = 8.48 Hz, 2.12 1H), 7.92 (d, *J* = 4.28, 1H), 7.87 (d, *J* = 8.4, 1H), 6.78 (d, *J* = 9.0 Hz, 2H), 6.31 (br, 1H), 3.78-3.71 (m, 4H), 3.68-3.52 (m, 4H), 2.59-2.52 (m, 1H), 2.33 (s, 3H), 2.25 (s, 3H), 1.60 (d, *J*= 8.36 Hz, 1H).

**Example 7: 6-methyl-N-(5-methyl-1H-pyrazol-3-yl)-2-(6-(6-((6-methylpyridin-3-yl)methyl)-3,6-diazabicyclo[3.1.1]heptan-3-yl)pyridin-3-yl)pyrimidin-4-amine (Compound 16)**

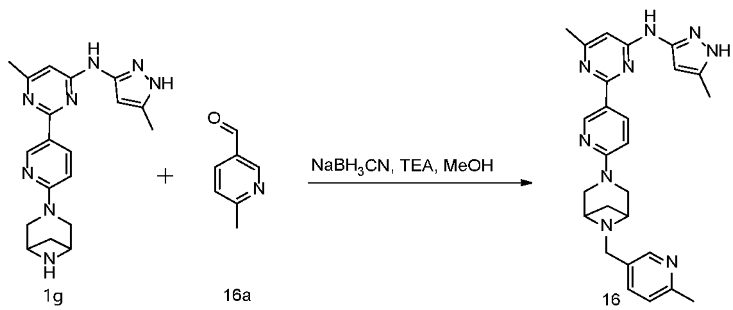


Trifluoroacetate of Compound 1g (35.0 mg) and Compound 16a (35.1 mg) were added into methanol (0.5 mL), and then triethylamine (9.5 mg) and sodium cyanoborohydride (29.4 mg) were successively added. The mixture was kept for reaction at room temperature for 16 h. After completion of the reaction, the reaction mixture was concentrated to dryness under reduced pressure, and separated and purified by Prep-HPLC to provide Compound 16 (15.0 mg). MS

m/z (ESI): 468.2 [M+H]+.

$^1$H NMR (400 MHz, DMSO-$d_6$) δ 11.97 (s, 1H), 9.66 (s, 1H), 9.12 (d, *J* = 2.4 Hz, 1H), 8.43 (dd, *J* = 8.8, 2.4 Hz, 1H), 8.38 (s, 1H), 7.63 (dd, *J* = 7.6, 1.6 Hz, 1H), 7.18 (d, *J* = 8.0 Hz, 1H), 6.77 (d, *J* = 8.8 Hz, 2H), 6.29 (br, 1H), 3.83-3.64 (m, 4H), 3.63-3.45 (m, 4H), 2.57-2.52 (m, 1H), 2.43 (s, 3H), 2.33 (s, 3H), 2.26 (s, 3H), 1.58 (d, *J* = 8.0 Hz, 1H).

**Example 8: 6-methyl-N-(5-methyl-1H-pyrazol-3-yl)-2-(6-(6-((2-methylthiazol-5-yl)methyl)-3,6-diazabicyclo[3.1.1]heptan-3-yl)pyridin-3-yl)pyrimidin-4-amine (Compound 15)**

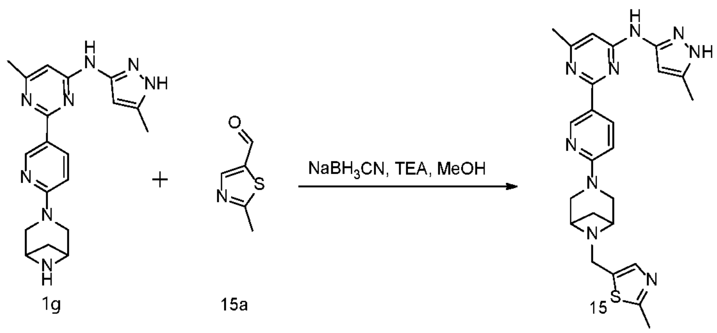


1g 15a 15

Trifluoroacetate of Compound 1g (35.0 mg) and Compound 15a (36.8 mg) were added into methanol (0.5 mL), and then triethylamine (9.5 mg) and sodium cyanoborohydride (29.4 mg) were successively added. The mixture was kept for reaction at room temperature for 16 h. After completion of the reaction, the reaction mixture was concentrated to dryness under reduced pressure, and separated and purified by Prep-HPLC to provide Compound 15 (16.0 mg). MS m/z (ESI): 474.2 [M+H]+.

$^1$H NMR (400 MHz, DMSO-$d_6$) δ 12.19 (br, 1H), 9.65 (s, 1H), 9.11 (d, *J* = 2.0 Hz, 1H), 8.43 (dd, *J* = 8.8, 2.4 Hz, 1H), 8.17 (s, 1H), 7.46 (s, 1H), 6.76 (d, *J* = 8.8 Hz, 1H), 6.29 (br, 1H), 3.78-3.66 (m, 6H), 3.64-3.51 (m, 2H), 2.59 (s, 3H), 2.49-2.44 (m, 1H), 2.33 (s, 3H), 2.25 (s, 3H), 1.57 (d, *J* = 8.4 Hz, 1H).

**Example 9: 2-(6-(4-((6-methoxypyridin-3-yl)methyl)piperazin-1-yl)pyridin-3-yl)-6-methyl-N-(5-methyl-1H-pyrazol-3-yl)pyrimidin-4-amine (Compound 49)**

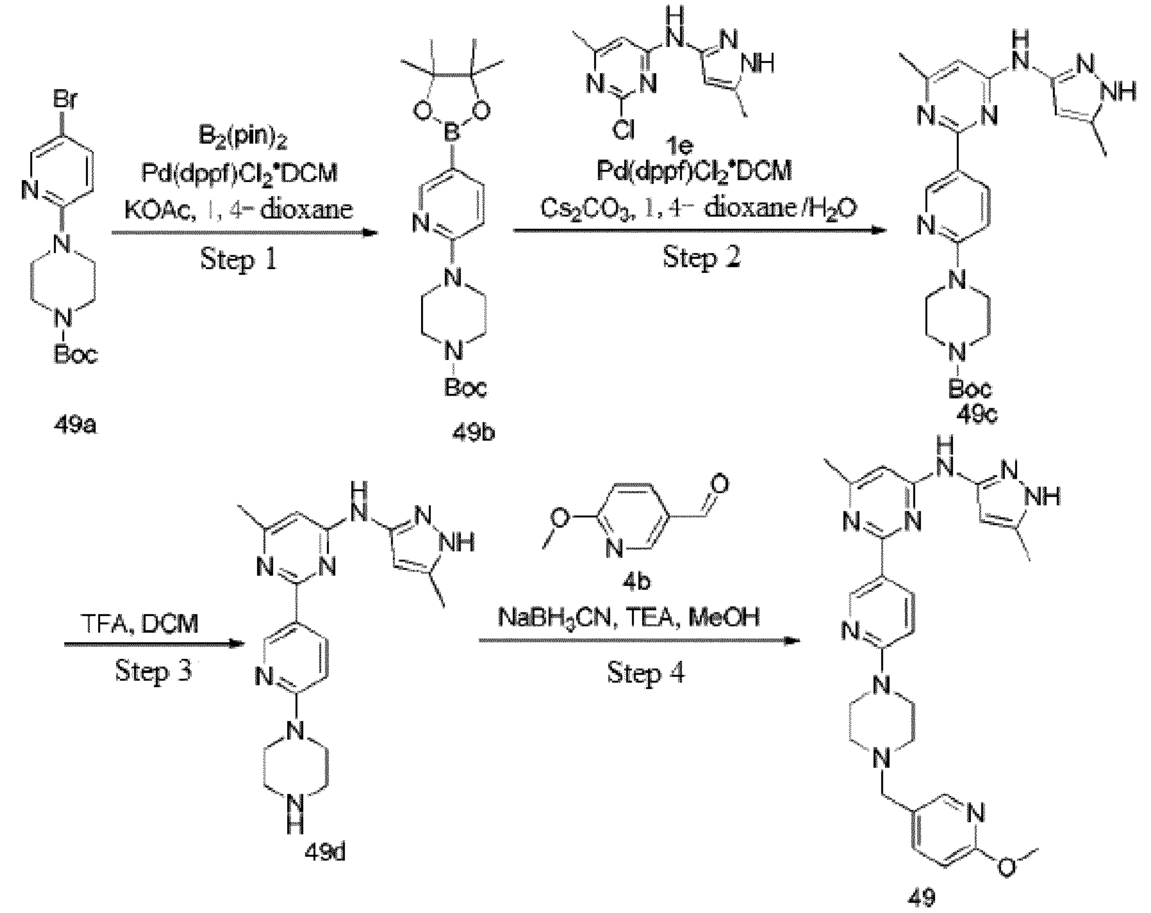


49a 49b 49c 49d 49

**Step 1: Preparation of tert-butyl 4-(5-(4,4,5,5-tetramethyl-1,3,2-dioxaborolan-2-yl)pyridin-2-yl)-piperazine-1-carboxylate (Compound 49b)**

Compound 49a (2.00 g), $B_2(pin)_2$ (4.09 g), KOAc (1.58 g), 1,4-dioxane (15.0 mL), and $Pd(dppf)Cl_2 \cdot DCM$ (657.43 mg) were successively added into a reaction flask. The mixture was heated to 90 °C, and kept for reaction under the protection of nitrogen at this temperature for 3 h. After completion of the reaction, the reaction mixture was cooled to room temperature, diluted with water (30 mL), and extracted with EA (40 mL×3). The organic phases were combined, washed with saturated brine, dried over anhydrous sodium sulfate, filtered, concentrated to dryness under reduced pressure, and separated and purified by flash silica gel column chromatography (DCM:MeOH=15:1), to provide Compound 49b (2.77 g). MS m/z (ESI): 390.3 $[M+H]^+$.

**Step 2: Preparation of tert-butyl 4-(5-(4-methyl-6-((5-methyl-1H-pyrazol-3-yl)-amino)pyrimidin-2-yl)pyridin-2-yl)piperazine-1-carboxylate (Compound 49c)**

Compound 1e (1.30 g) was dissolved in 1,4-dioxane (50.0 mL), Compound 49b (2.80 g), $CS_2CO_3$ (3.44 g), and water (2.0 mL) were successively added, and then $Pd(dppf)Cl_2 \cdot DCM$ (647.3 mg) was added. The mixture was heated to 90°C, and kept for reaction under the protection of nitrogen at this temperature for 4 h. After completion of the reaction, the reaction mixture was cooled to room temperature, diluted with water (100 mL), and extracted with EA (60 mL×3). The organic phases were combined, washed with saturated brine, dried over anhydrous sodium sulfate, filtered, and then concentrated to dryness under reduced pressure, to provide Compound 49c (587.0 mg). MS m/z (ESI): 451.3 $[M+H]^+$.

**Step 3: Preparation of 6-methyl-N-(5-methyl-1H-pyrazol-3-yl)-2-(6-(piperazin-1-yl)pyridin-3-yl)pyrimidin-4-amine (Compound 49d)**

Compound 49c (2.7 g) was dissolved in DCM (20.0 mL), and then TFA (20.0 mL) was added. The mixture was kept for reaction under the protection of nitrogen at room temperature for 4 h. After completion of the reaction, the reaction mixture was concentrated to dryness under reduced pressure, and separated and purified by Prep-HPLC to

provide trifluoroacetate of Compound 49d (761.0 mg). MS m/z (ESI): 351.2 $[M+H]^+$.

**Step 4: Preparation of 2-(6-(4-((6-methoxypyridin-3-yl)methyl)piperazin-1-yl)pyridin-3-yl)-6-methyl-N-(5-methyl-1H-pyrazol-3-yl)pyrimidin-4-amine (Compound 49)**

Trifluoroacetate of Compound 49d (35.0 mg) and Compound 4b (38.9 mg) were added into methanol (0.5 mL), and then triethylamine (9.3 mg) and sodium cyanoborohydride (28.8 mg) were successively added. The mixture was kept for reaction at room temperature for 16 h. After completion of the reaction, the reaction mixture was concentrated to dryness under reduced pressure, and separated and purified by Prep-HPLC to provide Compound 49 (25.0 mg). MS m/z (ESI): 472.3 $[M+H]^+$.

$^1H$ NMR (400 MHz, DMSO-$d_6$) δ 12.20 (br, 1H), 9.65 (s, 1H), 9.03 (d, *J* = 2.4 Hz, 1H), 8.36 (dd, *J* = 8.8, 2.0 Hz, 1H), 8.09 (d, *J* = 2.0 Hz, 1H), 7.68 (dd, *J* = 8.4, 2.4 Hz, 1H), 6.94-6.78 (m, 3H), 6.29 (br, 1H), 3.84 (s, 3H), 3.64-3.56 (m, 4H), 3.49 (s, 2H), 2.49-2.44 (m, 4H), 2.31 (s, 3H), 2.24 (s, 3H).

**Example 10: 2-(6-(4-(4-methoxybenzyl)piperazin-1-yl)pyridin-3-yl)-6-methyl-N-(5-methyl-1H-pyrazol-3-yl)pyrimidin-4-amine (Compound 23)**

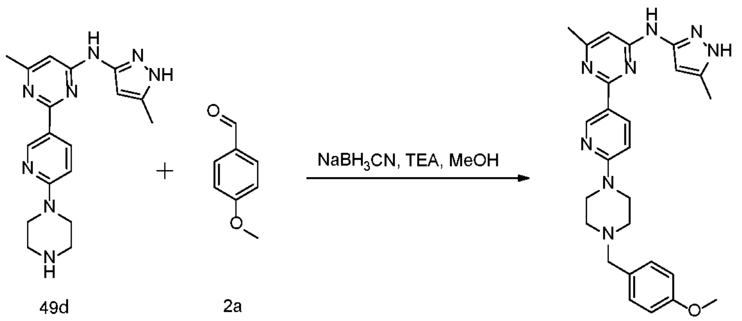


49d 2a 23

Trifluoroacetate of Compound 49d (35.0 mg) and Compound 2a (38.9 mg) were added into methanol (0.5 mL), and then triethylamine (9.3 mg) and sodium cyanoborohydride (28.8 mg) were successively added. The mixture was kept for reaction at room temperature for 16 h. After completion of the reaction, the reaction mixture was concentrated to dryness under reduced pressure, and separated and purified by Prep-HPLC to provide Compound 23 (18.0 mg). MS m/z (ESI): 471.3 $[M+H]^+$.

$^1H$ NMR ($CD_3OD$, 400 MHz) δ 9.07 (d, *J*= 2.4 Hz, 1H), 8.45 (dd, *J* = 8.8, 2.4 Hz, 1H), 8.28 (s, 1H), 7.42-7.35 (m, 2H), 7.02-6.92 (m, 3H), 6.76 (s, 1H), 6.26 (s, 1H), 3.99 (s, 2H), 3.82 (s, 7H), 3.08-3.00 (m, 4H), 2.40 (s, 3H), 2.31 (s, 3H).

**Example 11: 6-methyl-N-(5-methyl-1H-pyrazol-3-yl)-2-(6-(6-((4-methylpyridin-3-yl)methyl)-3,6-diazabicyclo[3.1.1]heptan-3-yl)pyridin-3-yl)pyrimidin-4-amine (Compound 21)**

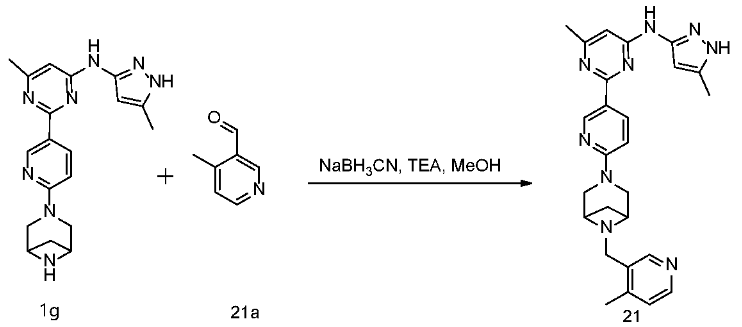


1g 21a 21

Trifluoroacetate of Compound 1g (35.0 mg) and Compound 21a (27.52 mg) were added into methanol (0.5

mL), and then triethylamine (7.4 mg) and sodium cyanoborohydride (23.08 mg) were successively added. The mixture was kept for reaction at room temperature for 16 h. After completion of the reaction, the reaction mixture was concentrated to dryness under reduced pressure, and separated and purified by Prep-HPLC to provide Compound 21 (10.0 mg). MS m/z (ESI): 468.3 $[M+H]^+$.

$^1H$ NMR (400 MHz, DMSO-$d_6$) δ 11.93 (s, 1H), 9.61 (s, 1H), 9.07 (d, *J* = 1.2 Hz, 1H), 8.39 (dd, *J*= 8.8, 2.2 Hz, 1H), 8.35 (s, 1H), 8.25 (d, *J* = 4.8 Hz, 1H), 7.11 (d, *J* = 4.8 Hz, 1H), 7.01-6.63 (m, 2H), 6.27 (br, 1H), 3.82-3.69 (m, 2H), 3.63 (d, *J*= 6.4 Hz, 2H), 3.59-3.42 (m, 4H), 2.52-2.46 (m, 1H), 2.28 (s, 3H), 2.25 (s, 3H), 2.21 (s, 3H), 1.53 (d, *J* = 8.4 Hz, 1H).

**Example 12: 2-(6-(6-(2-fluoro-5-methoxybenzyl)-3,6-diazabicyclo[3.1.1]heptan-3-yl)pyridin-3-yl)-6-methyl-N-(5-methyl-1H-pyrazol-3-yl)pyrimidin-4-amine (Compound 96)**

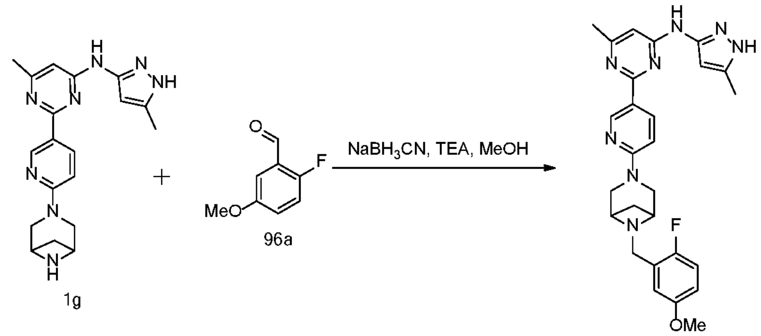


1g 96a 96

Trifluoroacetate of Compound 1g (30.0 mg) and Compound 96a (19.41 mg) were dissolved in MeOH (0.5 mL), TEA (6.37 mg) and sodium cyanoborohydride (19.78 mg) were successively added, and the mixture was kept for reaction at room temperature for 16 h. After completion of the reaction, the reaction mixture was concentrated to dryness under reduced pressure, and separated and purified by Prep-HPLC to provide Compound 96 (10.0 mg). MS m/z (ESI): 501.2 $[M+H]^+$.

$^1H$ NMR (400 MHz, DMSO-$d_6$) δ 11.97 (br, 1H), 9.65 (s, 1H), 9.11 (d, *J* = 2.0 Hz, 1H), 8.43 (dd, *J*= 9.2, 2.4 Hz, 1H), 7.06 (t, *J*= 9.2 Hz, 1H), 7.03-7.00 (m, 1H), 6.83-6.78 (m, 1H), 7.03-6.64 (m, 2H), 6.32 (br, 1H), 3.77-3.71 (m, 7H), 3.58-3.53 (m, 4H), 2.58-2.53 (m, 1H), 2.33 (s, 3H), 2.25 (s, 3H), 1.58 (d, *J* = 8.4 Hz, 1H).

**Example 13: (6-methoxypyridin-3-yl)(4-(5-(4-methyl-6-((5-methyl-1H-pyrazol-3-yl)amino)pyrimidin-2-yl)pyridin-2-yl)piperazin-1-yl)methanone (Compound 110)**

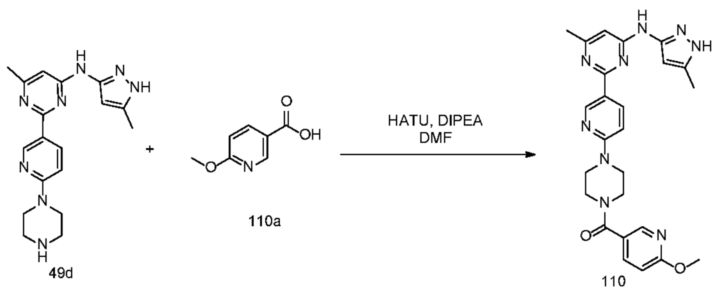


49d 110a 110

Compound 110a (16.5 mg), HATU (53.2 mg), and DIPEA (41.7 mg) were added into DMF (3.0 mL), and the mixture was kept for reaction at room temperature for 5 min. Then, trifluoroacetate of Compound 49d (50.0 mg) was added, and the mixture was reacted at room temperature for 0.5 h. After completion of the reaction, the reaction mixture was diluted with EA, washed with saturated sodium chloride solution for 3 times, dried over anhydrous sodium sulfate, filtered, concentrated, and separated and purified by Prep-HPLC, to provide Compound 110 (19.0 mg). MS m/z (ESI): 486.3 $[M+H]^+$.

$^1H$ NMR (400 MHz, DMSO-$d_6$) δ 12.01 (br, 1H), 9.69 (s, 1H), 9.08 (d, *J* = 2.2 Hz, 1H), 8.41 (dd, *J* = 9.0, 2.3

Hz, 1H), 8.34 (d, *J* = 2.1 Hz, 1H), 7.84 (dd, *J* = 8.5, 2.3 Hz, 1H), 7.12-6.63 (m, 3H), 6.30 (s, 1H), 3.92 (s, 3H), 3.72 (s, 8H), 2.34 (s, 3H), 2.26 (s, 3H).

**Example 14: 2-(6-methoxypyridin-3-yl)-1-(4-(5-(4-methyl-6-((5-methyl-1H-pyrazol-3-yl)amino)pyrimidin-2-yl)pyridin-2-yl)piperazin-1-yl)ethan-1-one (Compound 111)**

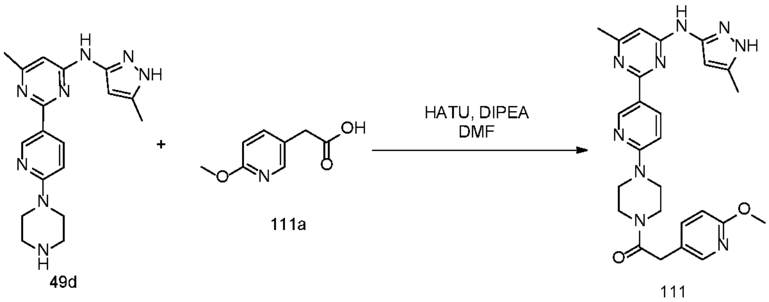


49d 111a 111

Compound 111a (18.0 mg), HATU (53.2 mg), and DIPEA (41.7 mg) were added into DMF (3.0 mL), and the mixture was kept for reaction at room temperature for 5 min. Then, trifluoroacetate of Compound 49d (50.0 mg) was added, and the mixture was reacted at room temperature for 0.5 h. After completion of the reaction, the reaction mixture was diluted with EA, washed with saturated sodium chloride solution for 3 times, dried over anhydrous sodium sulfate, filtered, concentrated, and separated and purified by Prep-HPLC, to provide Compound 111 (6.0 mg). MS m/z (ESI): 500.3 $[M+H]^+$.

$^1H$ NMR (400 MHz, DMSO-$d_6$) δ 12.02 (br, 1H), 9.68 (s, 1H), 9.06 (d, *J* = 2.3 Hz, 1H), 8.39 (dd, *J* = 9.0, 2.3 Hz, 1H), 8.02 (d, *J* = 2.2 Hz, 1H), 7.56 (dd, *J* = 8.5, 2.4 Hz, 1H), 6.94 (d, *J* = 9.0 Hz, 1H), 6.90-6.69 (m, 2H), 6.27 (s, 1H), 3.83 (s, 3H), 3.74 (s, 2H), 3.70-3.59 (m, 8H), 2.32 (s, 3H), 2.25 (s, 3H).

**Example 15: 6-methyl-N-(5-methyl-1H-pyrazol-3-yl)-2-(6-(6-(4-methylbenzyl)-3,6-diazabicyclo[3.1.1]heptan-3-yl)pyridin-3-yl)pyrimidin-4-amine (Compound 80)**

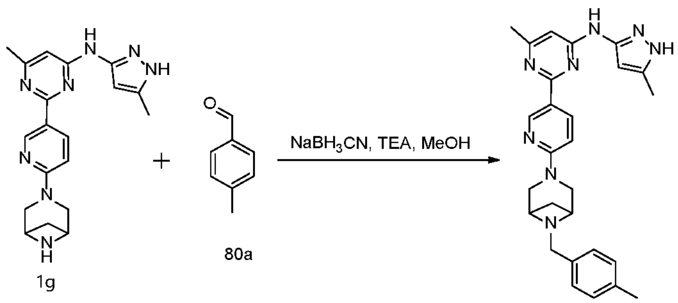


1g 80a 80

Trifluoroacetate of Compound 1g (30 mg) and Compound 80a (22.70 mg) were added into methanol (0.5 mL), and then triethylamine (6.37 mg) and sodium cyanoborohydride (19.78 mg) were successively added. The mixture was kept for reaction at room temperature for 16 h. After completion of the reaction, the reaction mixture was concentrated to dryness under reduced pressure, and separated and purified by Prep-HPLC to provide Compound 80 (10.0 mg). MS m/z (ESI): 467.3 $[M+H]^+$.

$^1H$ NMR (400 MHz, DMSO-$d_6$) δ 12.16 (s, 1H), 9.66 (s, 1H), 9.11 (d, *J* = 2.2 Hz, 1H), 8.43 (dd, *J* = 8.9, 2.3 Hz, 1H), 7.22 (d, *J* = 7.9 Hz, 2H), 7.11 (d, *J* = 7.9 Hz, 2H), 6.76 (d, *J* = 9.0 Hz, 2H), 6.31 (br, 1H), 3.78-3.65 (m, 4H), 3.64-3.49 (m, 4H), 2.60-2.49 (m, 1H), 2.33 (s, 3H), 2.27 (s, 3H), 2.25 (s, 3H),1.59 (d, *J*= 8.4 Hz, 1H).

**Example 16: 5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)-2-cyanopyridine (Compound 117)**

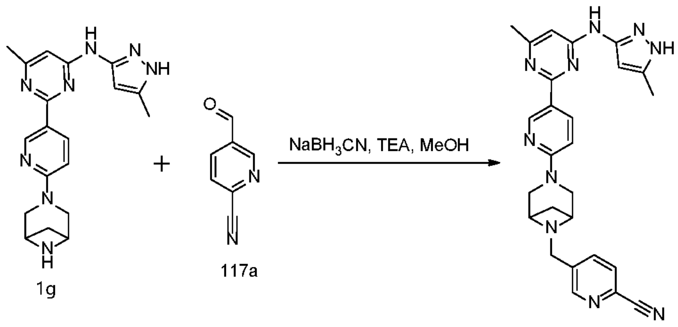


1g 117a 117

Trifluoroacetate of Compound 1g (30 mg) and Compound 117a (24.96 mg) were added into methanol (0.5 mL), and then triethylamine (6.37 mg) and sodium cyanoborohydride (19.78 mg) were successively added. The mixture was kept for reaction at room temperature for 16 h. After completion of the reaction, the reaction mixture was concentrated to dryness under reduced pressure, and separated and purified by Prep-HPLC to provide Compound 117 (2.0 mg). MS m/z (ESI): 479.2 $[M+H]^+$.

$^1$H NMR (400 MHz, DMSO-*d$_6$*) δ 11.97 (s, 1H), 9.65 (s, 1H), 9.11 (d, *J*= 2.2 Hz, 1H), 8.74 (d, *J* = 1.2 Hz, 1H), 8.43 (dd, *J* = 8.9, 2.3 Hz, 1H), 8.01 (dt, *J* = 17.2, 5.0 Hz, 2H), 6.77 (d, *J* = 9.0 Hz, 2H), 6.32 (br, 1H), 3.81-3.66 (m, 6H), 3.65-3.52 (m, 2H), 2.61-2.54 (m, 1H), 2.33 (s, 3H), 2.25 (s, 3H), 1.60 (d, *J* = 8.4 Hz, 1H).

**Example 17: 6-methyl-N-(5-methyl-1H-pyrazol-3-yl)-2-(6-(6-((6-(trifluoromethyl) pyridin-3-yl)methyl)-3,6-diazabicyclo [3.1.1] heptan-3-yl)pyridin-3-yl)pyrimidin-4-amine (Compound 118)**

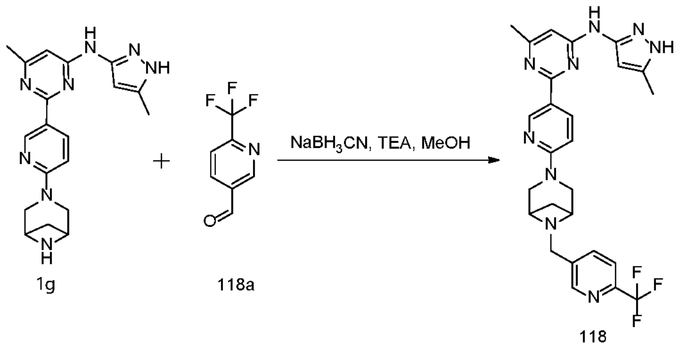


1g 118a 118

Trifluoroacetate of Compound 1g (30 mg) and Compound 118a (43.48 mg) were added into methanol (0.5 mL), and then triethylamine (8.38 mg) and sodium cyanoborohydride (26.01 mg) were successively added. The mixture was kept for reaction at room temperature for 16 h. After completion of the reaction, the reaction mixture was concentrated to dryness under reduced pressure, and separated and purified by Prep-HPLC to provide Compound 118 (6.0 mg). MS m/z (ESI): 522.3 $[M+H]^+$.

$^1$H NMR (400 MHz, DMSO-*d$_6$*) δ 11.97 (s, 1H), 9.65 (s, 1H), 9.12 (d, *J* = 2.1 Hz, 1H), 8.74 (s, 1H), 8.43 (dd, *J* = 8.9, 2.3 Hz, 1H), 8.06 (d, *J* = 7.1 Hz, 1H), 7.84 (d, *J* = 8.1 Hz, 1H), 6.77 (d, *J* = 9.0 Hz, 2H), 6.30 (br, 1H), 3.84-3.65(m, 6H), 3.60-3.48 (m, 2H), 2.61-2.54 (m, 1H), 2.33 (s, 3H), 2.25 (s, 3H), 1.61 (d, *J* = 8.4 Hz, 1H).

**Example 18: 6-methyl-2-(6-(6-((6-(4-methyl-1H-pyrazol-1-yl)pyridin-3-yl)methyl)-3,6-diazabicyclo[3.1.1]heptan-3-yl)pyridin-3-yl)-N-(5-methyl-1H-pyrazol-3-yl)pyrimidin-4-amine (Compound 62)**

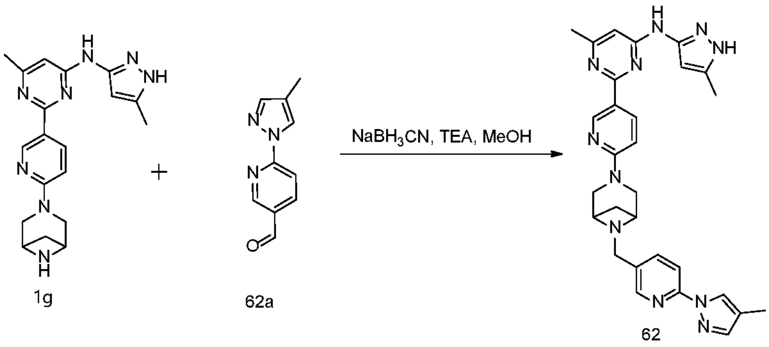


1g 62a 62

Trifluoroacetate of Compound 1g (30 mg) and Compound 62a (46.49 mg, prepared by referring to the method of preparing Compound 17a in Example 6 except that 4-fluoropyrazole was replaced with 4-methylpyrazole) were added into methanol (0.5 mL), and then triethylamine (8.38 mg) and sodium cyanoborohydride (26.01 mg) were successively added. The mixture was kept for reaction at room temperature for 16 h. After completion of the reaction, the reaction mixture was concentrated to dryness under reduced pressure, and separated and purified by Prep-HPLC to provide Compound 62 (6.0 mg). MS m/z (ESI): 534.2 $[M+H]^+$.

$^1H$ NMR (400 MHz, DMSO-$d_6$) δ 11.97 (s, 1H), 9.65 (s, 1H), 9.12 (d, *J* = 2.2 Hz, 1H), 8.44 (dd, *J* = 8.9, 2.3 Hz, 1H), 8.37 (m, 2H), 7.93 (dd, *J* = 8.5, 2.2 Hz, 1H), 7.82 (d, *J* = 8.4 Hz, 1H), 7.62 (s, 1H), 6.78 (d, *J* = 9.0 Hz, 2H), 6.30 (br, 1H), 3.83-3.67 (m, 4H), 3.66-3.50 (m, 4H), 2.60-2.52 (m, 1H), 2.33 (s, 3H), 2.26 (s, 3H), 2.11 (s, 3H), 1.59 (d, *J* = 8.4 Hz, 1H).

**Example 19: 2-(6-(6-((6-(1H-pyrazol-1-yl)pyridin-3-yl)methyl)-3,6-diazabicyclo [3.1.1] heptan-3-yl)pyridin-3-yl)-6-methyl-N-(5-methyl-1H-pyrazol-3-yl)pyrimidin-4-amine (Compound 60)**

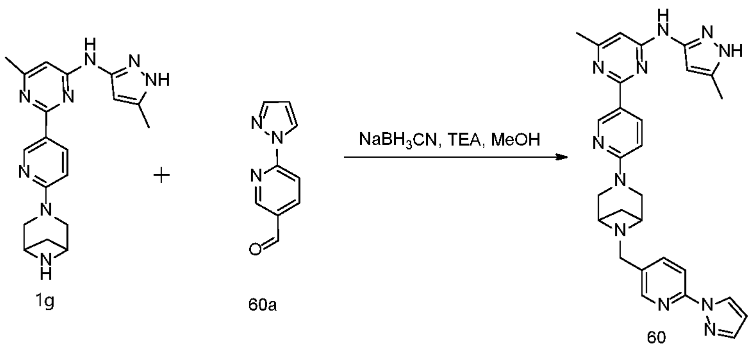


1g 60a 60

Trifluoroacetate of Compound 1g (30 mg) and Compound 60a (43.00 mg) were added into methanol (0.5 mL), and then triethylamine (8.38 mg) and sodium cyanoborohydride (26.01 mg) were successively added. The mixture was kept for reaction at room temperature for 16 h. After completion of the reaction, the reaction mixture was concentrated to dryness under reduced pressure, and separated and purified by Prep-HPLC to provide Compound 60 (11.0 mg). MS m/z (ESI): 520.2 $[M+H]^+$.

$^1H$ NMR (400 MHz, DMSO-$d_6$) δ 11.98 (s, 1H), 9.65 (s, 1H), 9.13 (d, *J* = 2.2 Hz, 1H), 8.59 (dd, *J* = 2.6, 0.5 Hz, 1H),8.46-8.42 (dd, *J* = 8.8, 2.4 Hz, 1H), 8.41-8.39 (d, *J* = 2.0 Hz, 1H), 7.97 (dd, *J* = 8.4, 2.2 Hz, 1H), 7.88 (d, *J* = 8.4 Hz, 1H), 7.81 (d, *J* = 1.0 Hz, 1H), 6.78 (d, *J* = 9.0 Hz, 2H), 6.56 (dd, *J* = 2.5, 1.7 Hz, 1H), 6.29 (br, 1H), 3.82-3.68 (m, 4H), 3.67-3.52 (m, 4H), 2.59-2.52 (m, 1H), 2.33 (s, 3H), 2.26 (s, 3H), 1.60 (d, *J* = 8.4 Hz, 1H).

**Example 20: 2-(6-(6-((5-fluoropyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 98)**

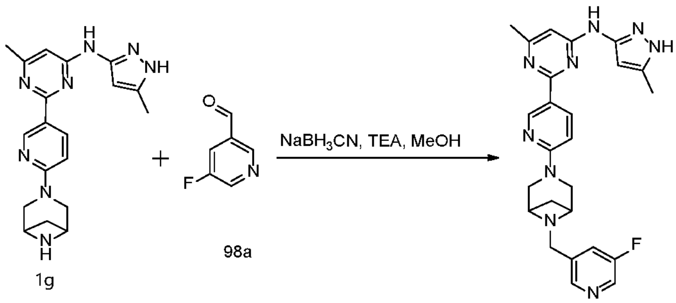


1g 98a 98

Trifluoroacetate of Compound 1g (35.0 mg) and Compound 98a (36.24 mg) were added into methanol (0.5 mL), and then triethylamine (9.77 mg) and sodium cyanoborohydride (30.34 mg) were successively added. The mixture was kept for reaction at room temperature for 16 h. After completion of the reaction, the reaction mixture was concentrated to dryness under reduced pressure, and separated and purified by Prep-HPLC to provide Compound 98 (15.0 mg). MS m/z (ESI): 472.3 $[M+H]^+$.

$^1H$ NMR (400 MHz, DMSO-$d_6$) δ 11.98 (br, 1H), 9.66 (s, 1H), 9.12 (d, *J*= 2.2 Hz, 1H), 8.45-8.41 (m, 3H), 7.73-7.65 (m, 1H), 6.77 (d, *J* = 9.0 Hz, 2H), 6.29 (br, 1H), 3.74 (t, *J* = 8.7 Hz, 4H), 3.64 (s, 2H), 3.58 (d, *J* = 6.5 Hz, 2H), 2.55-2.51 (m, 1H), 2.33 (s, 3H), 2.26 (s, 3H), 1.59 (d, *J*= 8.4 Hz, 1H).

**Example 21: 2-(6-(6-((5-chloropyridin-2-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 69)**

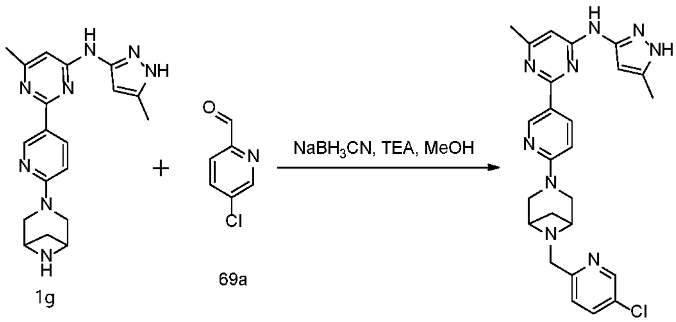


1g 69a 69

Trifluoroacetate of Compound 1g (35.0 mg) and Compound 69a (41.0 mg) were added into methanol (0.5 mL), and then triethylamine (9.77 mg) and sodium cyanoborohydride (30.34 mg) were successively added. The mixture was kept for reaction at room temperature for 16 h. After completion of the reaction, the reaction mixture was concentrated to dryness under reduced pressure, and separated and purified by Prep-HPLC to provide Compound 69 (16.0 mg). MS m/z (ESI): 488.2 $[M+H]^+$.

$^1H$ NMR (400 MHz, DMSO-$d_6$) δ 12.01 (br, 1H), 9.66 (s, 1H), 9.11 (d, *J* = 2.2 Hz, 1H), 8.50 (d, *J* = 2.3 Hz, 1H), 8.43 (dd, *J* = 8.9, 2.3 Hz, 1H), 7.89 (dd, *J* = 8.4, 2.5 Hz, 1H), 7.52 (d, *J* = 8.4 Hz, 1H), 6.76 (d, *J* = 9.0 Hz, 2H), 6.30 (s, 1H), 3.79 (d, *J* = 11.7 Hz, 2H), 3.73 (d, *J* = 5.9 Hz, 2H), 3.65 (s, 2H), 3.57 (d, *J*= 10.0 Hz, 3H), 2.55-2.51 (m, 1H), 2.33 (s, 3H), 2.26 (s, 3H), 1.60 (d, *J* = 8.4 Hz, 1H).

**Example 22: 2-(6-(6-((5-methoxypyridin-2-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 67)**

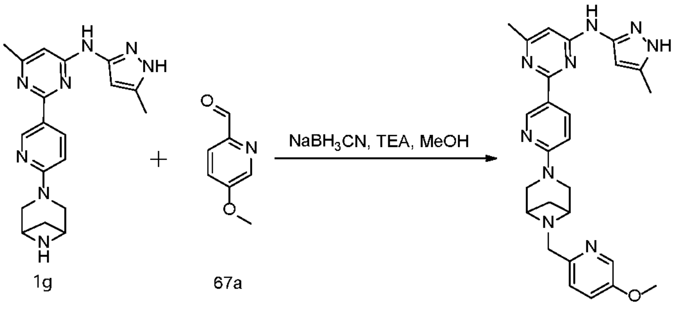


1g 67a 67

Trifluoroacetate of Compound 1g (30 mg) and Compound 67a (25.90 mg) were added into methanol (0.5 mL), and then triethylamine (6.37 mg) and sodium cyanoborohydride (19.78 mg) were successively added. The mixture was kept for reaction at room temperature for 16 h. After completion of the reaction, the reaction mixture was concentrated to dryness under reduced pressure, and separated and purified by Prep-HPLC to provide Compound 67 (10.0 mg). MS m/z (ESI): 484.3 $[M+H]^+$.

$^1H$ NMR (400 MHz, DMSO-$d_6$)) δ 11.97 (br, 1H), 9.65 (s, 1H), 9.11 (d, *J* = 2.1 Hz, 1H), 8.43 (dd, *J* = 8.9, 2.3 Hz, 1H), 8.16 (dd, *J* = 2.8, 0.6 Hz, 1H), 7.39 (d, *J* = 8.3 Hz, 1H), 7.35 (dd, *J* = 8.6, 2.9 Hz, 1H), 6.76 (d, *J* = 9.0 Hz, 2H), 6.30 (s, 1H), 3.85-3.77 (m, 5H), 3.69 (d, *J* = 5.9 Hz, 2H), 3.60-3.47 (m, 4H), 2.55-2.51 (m, 1H), 2.33 (s, 3H), 2.26 (s, 3H), 1.58 (d, *J*= 8.4 Hz, 1H).

**Example 23: 2-(6-(6-(4-ethoxybenzyl)-3,6-diazabicyclo[3.1.1]heptan-3-yl)pyridin-3-yl)-6-methyl-N-(5-methyl-1H-pyrazol-3-yl)pyrimidin-4-amine (Compound 41)**

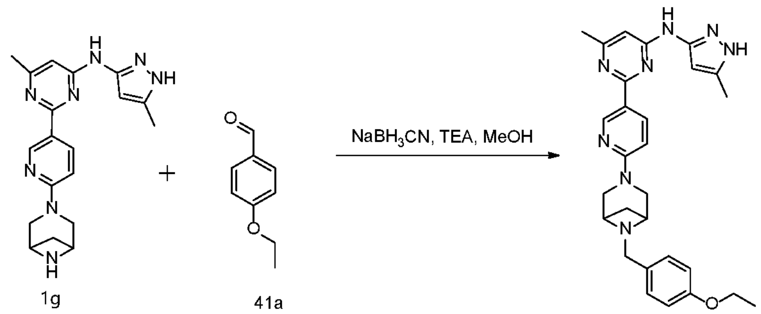


1g 41a 41

Trifluoroacetate of Compound 1g (30 mg) and Compound 41a (28.37 mg) were added into methanol (0.5 mL), and then triethylamine (6.37 mg) and sodium cyanoborohydride (19.78 mg) were successively added. The mixture was kept for reaction at room temperature for 16 h. After completion of the reaction, the reaction mixture was concentrated to dryness under reduced pressure, and separated and purified by Prep-HPLC to provide Compound 41 (15.0 mg). MS m/z (ESI): 497.3 $[M+H]^+$.

$^1H$ NMR (400 MHz, DMSO-$d_6$) δ 11.97 (br, 1H), 9.65 (s, 1H), 9.11 (d, *J* = 2.4 Hz, 1H), 8.43 (dd, *J* = 8.8, 2.4 Hz, 1H), 7.22 (d, *J*= 8.8 Hz, 2H), 6.88-6.76 (m, 4H), 6.32 (br, 1H), 3.98 (q, *J* = 6.9 Hz, 2H), 3.68 (dd, *J*= 30.7, 8.6 Hz, 4H), 3.51 (d, *J*= 30.4 Hz, 4H), 2.59-2.55 (m, 1H), 2.33 (s, 3H), 2.26 (s, 3H), 1.56 (d, *J*= 8.3 Hz, 1H), 1.31 (t, *J* = 7.0 Hz, 3H).

**Example 24: 2-(6-(6-(1-(4-methoxyphenyl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 50)**

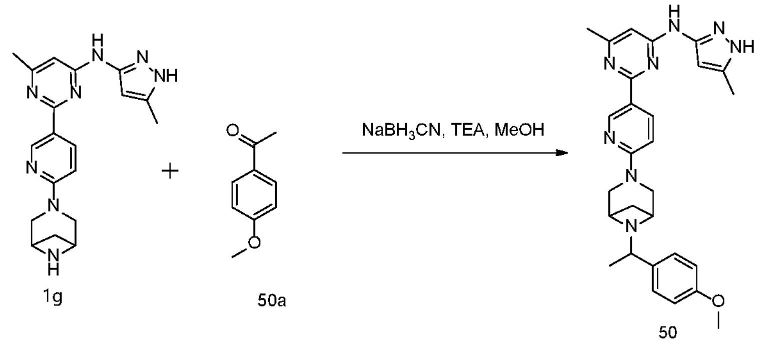


1g 50a 50

Trifluoroacetate of Compound 1g (30 mg) and Compound 50a (28.37 mg) were added into methanol (0.5 mL), and then triethylamine (6.37 mg) and sodium cyanoborohydride (19.78 mg) were successively added. The mixture was kept for reaction at room temperature for 16 h. After completion of the reaction, the reaction mixture was concentrated to dryness under reduced pressure, and separated and purified by Prep-HPLC to provide Compound 50 (15.0 mg). MS m/z (ESI): 497.3 $[M+H]^+$.

$^1$H NMR (400 MHz, DMSO-$d_6$) δ 11.97 (s, 1H), 9.63 (s, 1H), 9.11 (d, *J* = 2.1 Hz, 1H), 8.42 (dd, *J* = 8.9, 2.2 Hz, 1H), 7.25 (d, *J* = 8.6 Hz, 2H), 7.02-6.57 (m, 4H), 6.30 (br, 1H), 3.97-3.77 (m, 2H), 3.72 (s, 3H), 3.67-3.50 (m, 2H), 3.50-3.36 (m, 2H), 2.49-2.39 (m, 2H), 2.33 (s, 3H), 2.26 (s, 3H), 1.51 (d, *J*= 8.3 Hz, 1H), 1.12 (d, *J* = 6.2 Hz, 3H).

**Example 25: 2-(6-(6-(4-fluorobenzyl)-3,6-diazabicyclo[3.1.1]heptan-3-yl)pyridin-3-yl)-6-methyl-N-(5-methyl-1H-pyrazol-3-yl)pyrimidin-4-amine (Compound 83)**

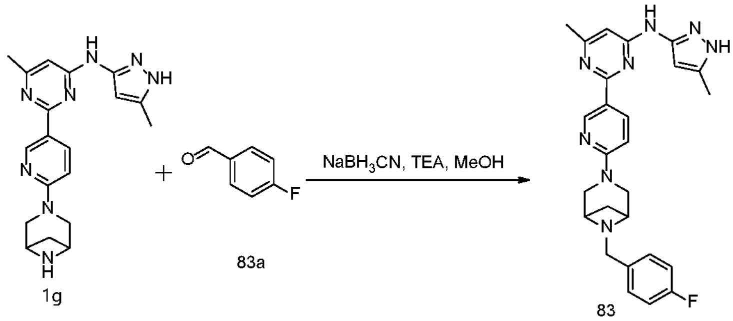


1g 83a 83

Trifluoroacetate of Compound 1g (35.0 mg) and Compound 83a (18.2 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 83 (26.0 mg). MS m/z (ESI): 471.2 $[M+H]^+$.

$^1$H NMR (400 MHz, DMSO-$d_6$) δ 11.97 (s, 1H), 9.64 (s, 1H), 9.11 (d, *J* = 2.4 Hz, 1H), 8.42 (dd, *J* = 9.2, 2.4 Hz, 1H), 7.93-7.35 (m, 2H), 7.13-7.09 (m, 2H), 6.83 (br, 1H), 6.76 (d, *J* = 9.2 Hz, 1H), 6.30 (br, 1H), 3.74-3.66 (m, 4H), 3.62-3.53 (m, 4H), 2.55-2.52 (m, 1H), 2.32 (s, 3H), 2.25 (s, 3H), 1.57 (d, *J* = 8.4 Hz, 1H).

**Example 26: 6-methyl-N-(5-methyl-1H-pyrazol-3-yl)-2-(6-(6-(4-(trifluoromethyl)benzyl)-3,6-diazabicyclo[3.1.1]heptan-3-yl)pyridin-3-yl)pyrimidin-4-amine (Compound 84)**

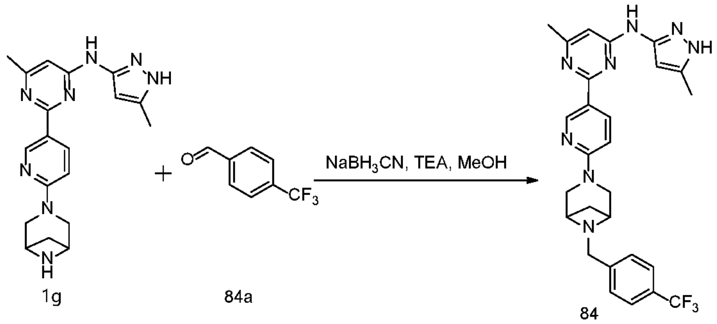


1g 84a 84

Trifluoroacetate of Compound 1g (35.0 mg) and Compound 84a (25.6 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 84 (17.0 mg). MS m/z (ESI): 521.2 $[M+H]^+$.

$^1H$ NMR (400 MHz, DMSO-$d_6$) $\delta$ 11.90 (br, 1H), 9.65 (s, 1H), 9.11 (d, $J$ = 2.4 Hz, 1H), 8.42 (dd, $J$= 8.8, 2.4 Hz, 1H), 7.66 (d, $J$= 8.0, 2H), 7.58 (d, $J$= 8.4, 2H), 6.94 (br, 1H), 6.76 (d, $J$= 9.2 Hz, 1H), 6.30 (br, 1H), 3.74-3.57 (m, 8H), 2.57-2.55 (m, 1H), 2.33 (s, 3H), 2.25 (s, 3H), 1.59 (d, $J$ = 8.4 Hz, 1H).

**Example 27: 4-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)benzonitrile (Compound 85)**

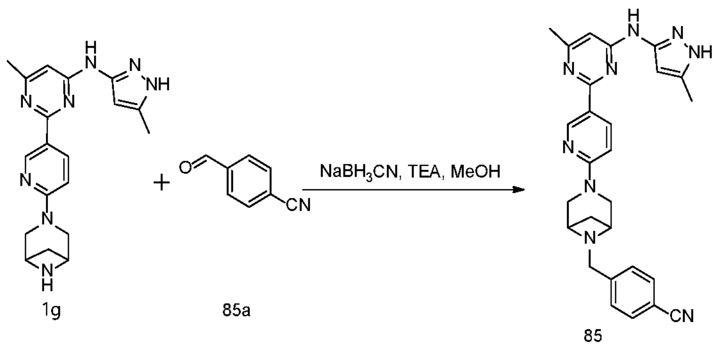


1g 85a 85

Trifluoroacetate of Compound 1g (35.0 mg) and Compound 85a (19.3 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 85 (12.0 mg). MS m/z (ESI): 478.2 $[M+H]^+$.

$^1H$ NMR (400 MHz, DMSO-$d_6$) $\delta$ 12.08 (br, 1H), 9.65 (s, 1H), 9.11 (d, $J$ = 2.4 Hz, 1H), 8.42 (dd, $J$ = 9.2, 2.4 Hz, 1H), 7.77 (d, $J$ = 8.4, 2H), 7.56 (d, $J$ = 8.0, 2H), 6.89 (br, 1H), 6.76 (d, $J$ = 8.8 Hz, 1H), 6.30 (br, 1H), 3.74-3.56 (m, 8H), 2.59-2.58 (m, 1H), 2.33 (s, 3H), 2.25 (s, 3H), 1.61 (d, $J$ = 8.4 Hz, 1H).

**Example 28: 2-(6-(6-(4-(1H-pyrazol-1-yl)benzyl)-3,6-diazabicyclo[3.1.1]heptan-3-yl) pyridin-3-yl)-6-methyl-N-(5-methyl-1H-pyrazol-3-yl)pyrimidin-4-amine (Compound 86)**

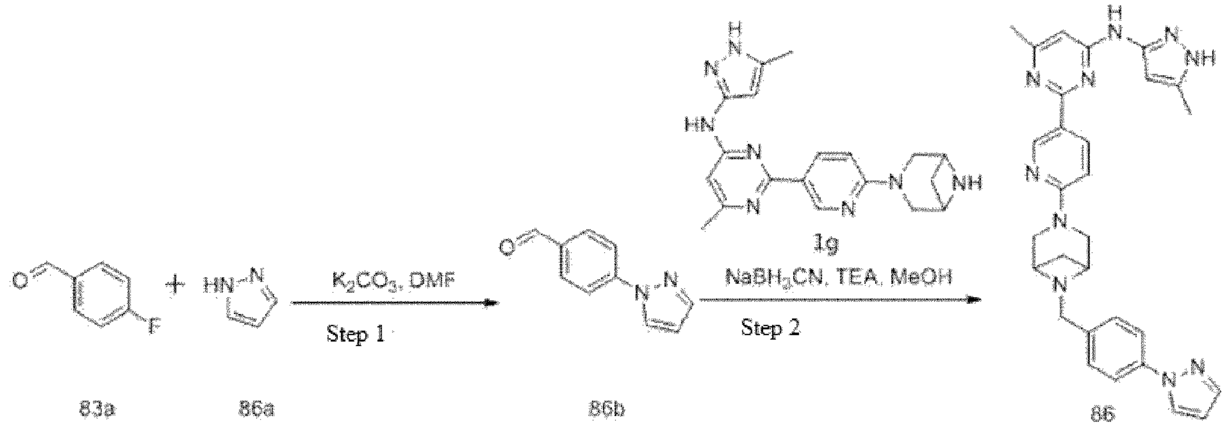


83a 86a 86b 86

**Step 1: Preparation of 4-(1H-pyrazol-1-yl)benzaldehyde (Compound 86b)**

Compound 83a (1.0 g) and Compound 86a (0.8 g) were dissolved in DMF (10.0 mL), and anhydrous potassium carbonate (2.2 g) was added. The mixture was heated to 120 °C, and kept for reaction at this temperature for 16 h until the raw materials were fully converted. After completion of the reaction, the reaction mixture was washed with saturated sodium carbonate solution, extracted with EA (30 mL x 3), dried over anhydrous sodium sulfate, filtered, and then concentrated under reduced pressure to provide Compound 86b (1.1 g). MS m/z (ESI): 173.1 $[M+H]^+$.

**Step 2: Preparation of 2-(6-(6-(4-(1H-pyrazol-1-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 86)**

Trifluoroacetate of Compound 1g (35.0 mg) and Compound 86b (25.3 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 86 (12.0 mg). MS m/z (ESI): 519.2 $[M+H]^+$.

$^1$H NMR (400 MHz, DMSO-$d_6$) 11.97 (s, 1H), 9.65 (s, 1H), 9.12 (d, *J* = 2.4 Hz, 1H), 8.46-8.42(m, 2H), 7.77-7.72 (m, 3H), 7.45 (d, *J* = 8.8 Hz, 2H), 6.89 (br, 1H), 6.77 (d, *J* = 9.2 Hz, 1H), 6.52 (t, *J* = 2.0 Hz, 1H), 6.30 (br, 1H), 3.77-3.59 (m, 8H), 2.56-2.54 (m, 1H), 2.33 (s, 3H), 2.25 (s, 3H), 1.59 (d, *J* = 8.4 Hz, 1H).

**Example 29: 2-(6-(6-(4-chlorobenzyl)-3,6-diazabicyclo[3.1.1]heptan-3-yl)pyridin-3-yl)-6-methyl-N-(5-methyl-1H-pyrazol-3-yl)pyrimidin-4-amine (Compound 82)**

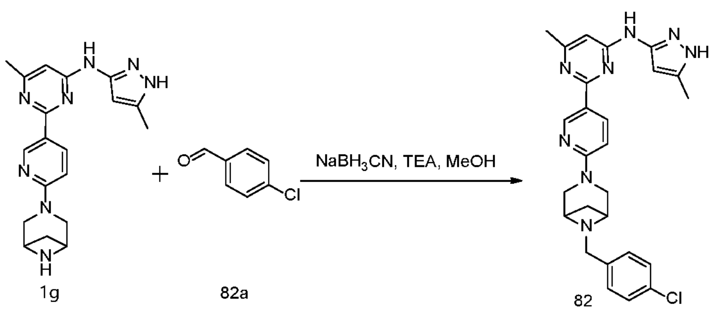


1g 82a 82

Trifluoroacetate of Compound 1g (35.0 mg) and Compound 82a (20.6 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 82 (26.0 mg). MS m/z (ESI): 487.2 $[M+H]^+$.

$^1$H NMR (400 MHz, DMSO-$d_6$) $\delta$ 12.21 (br, 1H), 9.65 (s, 1H), 9.11 (d, *J* = 2.4 Hz, 1H), 8.43 (dd, *J* = 8.8, 2.4 Hz, 1H), 7.41-7.36 (m, 4H), 6.86 (br, 1H), 6.76 (d, *J* = 9.2 Hz, 1H), 6.30 (br, 1H), 3.75-3.63 (m, 8H), 2.60-2.56 (m, 1H),

2.33 (s, 3H), 2.25 (s, 3H), 1.63 (d, *J* = 8.4 Hz, 1H).

**Example 30: 2-(6-(6-(4-(4-fluoro-1H-pyrazol-1-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 91)**

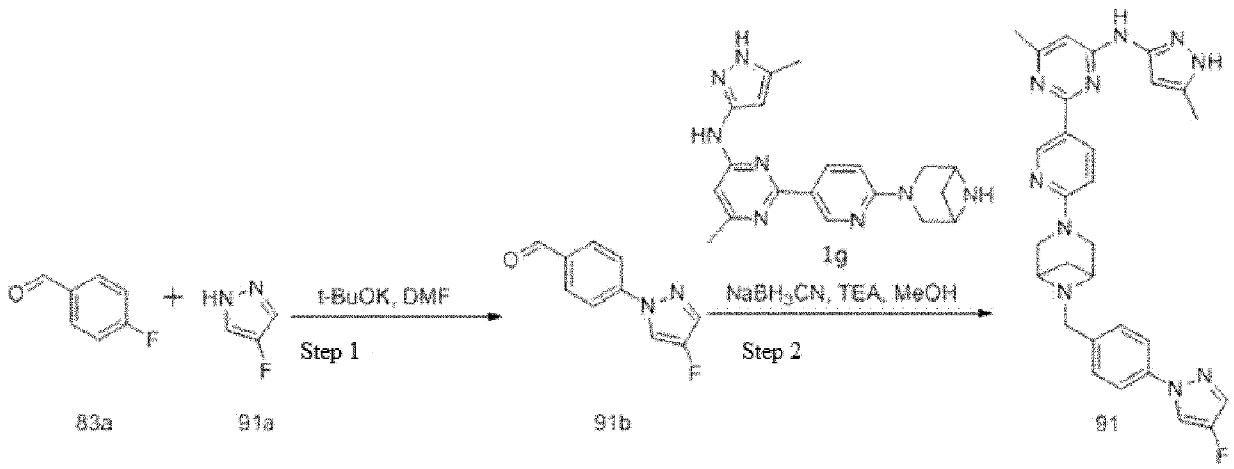


**Step 1: Preparation of 4-(4-fluoro-1H-pyrazol-1-yl)benzaldehyde (Compound 91b)**

Compound 83a (0.1 g) and Compound 91a (0.1 g) were dissolved in DMF (5.0 mL), and potassium tert-butoxide (0.3 g) was added. The mixture was heated to 120 °C, and kept for reaction at this temperature for 16 h until the raw materials were fully converted. After completion of the reaction, the reaction mixture was washed with saturated sodium carbonate solution, extracted with EA (30 mL x 3), dried over anhydrous sodium sulfate, filtered, and then concentrated under reduced pressure to provide Compound 91b (60 mg). MS m/z (ESI): 190.1 $[M+H]^+$.

**Step 2: Preparation of 2-(6-(6-(4-(4-fluoro-1H-pyrazol-1-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 91)**

Trifluoroacetate of Compound 1g (35.0 mg) and Compound 91b (27.9 mg) were added into methanol (0.5 mL), and then triethylamine (7.4 mg) and sodium cyanoborohydride (23.1 mg) were successively added. The mixture was kept for reaction at room temperature for 20 h. After completion of the reaction, the reaction mixture was concentrated to dryness under reduced pressure, and separated and purified by Prep-HPLC to provide Compound 91 (23.0 mg). MS m/z (ESI): 537.3 $[M+H]^+$.

$^1H$ NMR (400 MHz, DMSO-$d_6$) 11.97 (s, 1H), 9.63 (s, 1H), 9.12 (d, *J* = 2.4 Hz, 1H), 8.61 (d, *J* = 4.8 Hz, 1H), 8.43 (dd, *J* = 8.8, 2.0 Hz, 1H), 7.80 (d, *J* = 4.0 Hz, 1H), 7.71 (d, *J* = 8.8 Hz, 2H), 7.46 (d, *J* = 6.8 Hz, 2H), 6.89 (br, 1H), 6.77 (d, *J* = 8.8 Hz, 1H), 6.30 (br, 1H), 3.76-3.59 (m, 8H), 2.56-2.54 (m, 1H), 2.33 (s, 3H), 2.25 (s, 3H), 1.59 (d, *J* = 8.4 Hz, 1H).

**Example 31: 6-methyl-2-(6-(6-(4-(4-methyl-1H-pyrazol-1-yl)benzyl)-3,6-diazabicyclo[3.1.1]heptan-3-yl)pyridin-3-yl)-N-(5-methyl-1H-pyrazol-3-yl)pyrimidin-4-amine (Compound 88)**

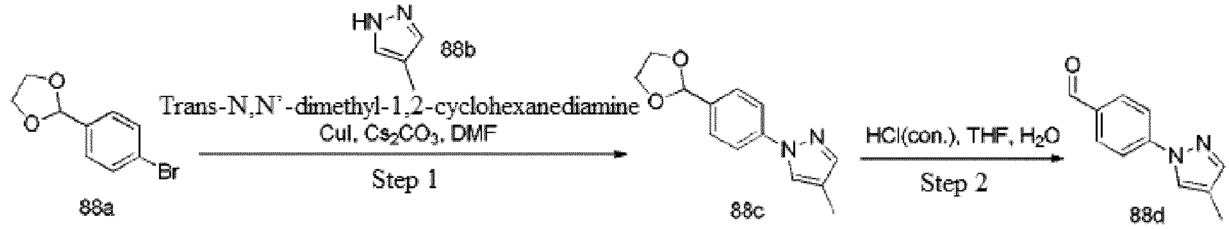


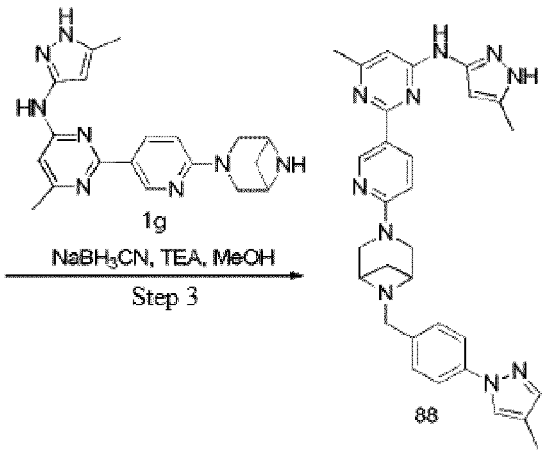


**Step 1: Preparation of 1-(4-(1,3-dioxolan-2-yl)phenyl)-4-methyl-1H-pyrazole (Compound 88c)**

Compound 88a (300 mg), Compound 88b (161.3 mg), trans-N,N'-dimethyl-1,2-cyclohexanediamine (74.5 mg), CuI (50.0 mg), and cesium carbonate (1.71 g) were added into DMF (5.0 mL). The mixture was heated to 115 °C, and kept for reaction under the protection of nitrogen at this temperature for 7 h. $H_2O$ (10 mL) was added into the reaction mixture to quench the reaction. The reaction mixture was extracted with EA (10 mL×3). The organic phases were combined, washed with water (10 mL ×3), dried over anhydrous sodium sulfate, filtered, concentrated under reduced pressure, and separated and purified by silica gel column chromatography (PE:EA=3:1) to provide Compound 88c (120 mg). MS m/z (ESI): 231.2 $[M+H]^+$.

**Step 2: Preparation of 4-(4-methyl-1H-pyrazol-1-yl)benzaldehyde (Compound 88d)**

Concentrated hydrochloric acid (1.5 mL) was added dropwise into a solution of Compound 88c (120 mg) in THF (10 mL) and $H_2O$ (8 mL). The mixture was heated to 65 °C, and kept for reaction at this temperature for 1.5 h. After completion of the reaction, the reaction mixture was cooled in an ice bath, and then saturated sodium bicarbonate solution was slowly added dropwise to adjust the pH value of the reaction mixture to about 8. A THF solvent was removed under reduced pressure, and then the mixture was extracted with DCM (10 mL x 3). The organic phases were combined, dried over anhydrous sodium sulfate, filtered, concentrated under reduced pressure, and separated and purified by silica gel column chromatography (PE:EA=5:1) to provide Compound 88d (90 mg). MS m/z (ESI): 187.1 $[M+H]^+$.

**Step 3: Preparation of 6-methyl-2-(6-(6-(4-(4-fluoro-1H-pyrazol-1-yl)benzyl)-3,6-diazabicyclo[3.1.1]heptan-3-yl)pyridin-3-yl)-N-(5-methyl-1H-pyrazol-3-yl)pyrimidin-4-amine (Compound 88)**

Trifluoroacetate of Compound 1g (35.0 mg) and Compound 88d (54.0 mg) were added into methanol (0.5 mL), and then triethylamine (9.8 mg) and sodium cyanoborohydride (30.3 mg) were successively added. The mixture was kept for reaction at room temperature for 16 h. After completion of the reaction, the reaction mixture was concentrated to dryness under reduced pressure, and separated and purified by Prep-HPLC to provide Compound 88 (10.0 mg). MS m/z (ESI): 533.3 $[M+H]^+$.

$^1H$ NMR (400 MHz, DMSO) δ 11.98 (s, 1H), 9.66 (s, 1H), 9.14 (s, 1H), 8.45 (d, *J* = 8.4 Hz, 1H), 8.23 (s, 1H), 7.71 (d, *J* = 8.0 Hz, 2H), 7.54 (s, 1H), 7.44 (d, *J* = 8.0 Hz, 2H), 6.97-6.65 (m, 2H), 6.32 (br, 1H), 3.82-3.67 (m, 4H), 3.65-3.50 (m, 4H), 2.62-2.55 (m, 1H), 2.35 (s, 3H), 2.27 (s, 3H), 2.11 (s, 3H), 1.60 (d, *J* = 8.0 Hz, 1H).

**Example 32: 2-(6-(6-((6-(3,4-dimethyl-1H-pyrazol-1H-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 121)**

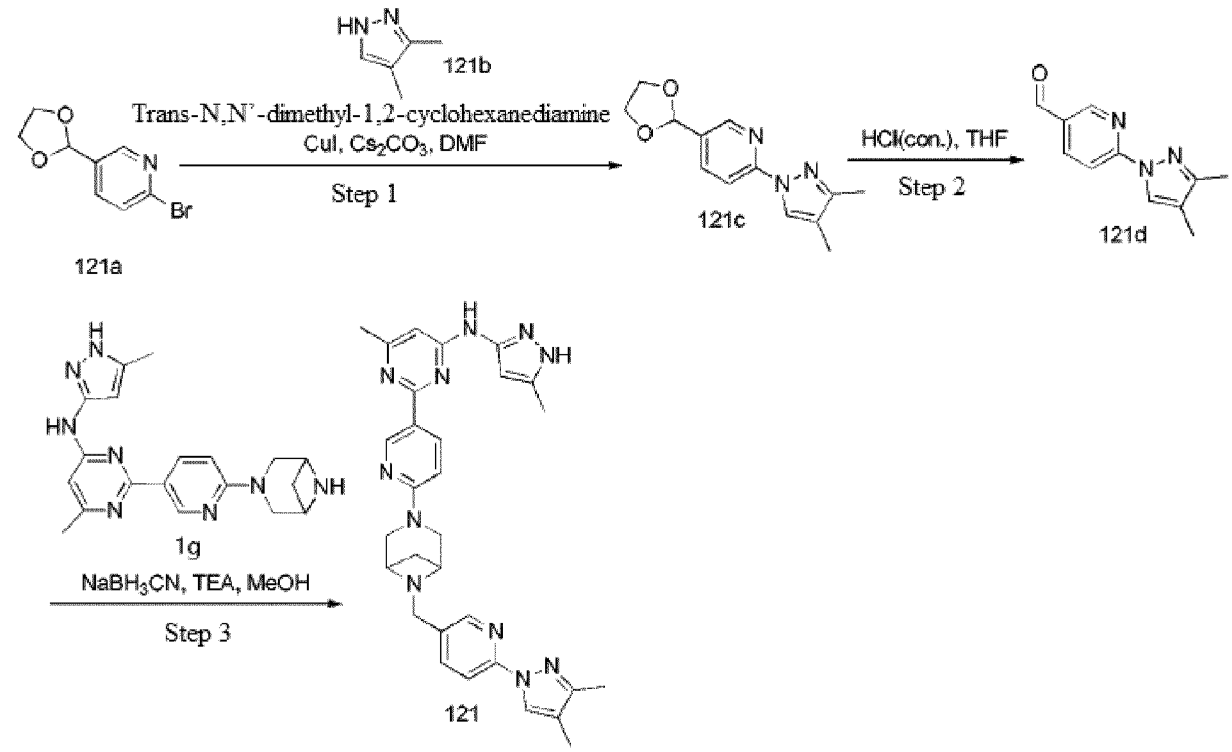


**Step 1: Preparation of 2-(3,4-dimethyl-1H-pyrazol-1-yl)-5-(1,3-dioxolan-2-yl)pyridine (Compound 121c)**

Compound 121a (300 mg), Compound 121b (188.0 mg), trans-N,N'-dimethyl-1,2-cyclohexanediamine (74.2 mg), CuI (49.7 mg), and cesium carbonate (1.70 g) were added into DMF (5.0 mL). The mixture was heated to 120 °C, and kept for reaction under the protection of nitrogen at this temperature for 5 h. $H_2O$ (10 mL) was added into the reaction mixture to quench the reaction. The reaction mixture was extracted with EA (10 mL×3). The organic phases were combined, washed with water (10 mL ×3), dried over anhydrous sodium sulfate, filtered, concentrated under reduced pressure, and separated and purified by silica gel column chromatography (PE:EA=4:1) to provide Compound 121c (305 mg). MS m/z (ESI): 246.1 $[M+H]^+$.

**Step 2: Preparation of 6-(3,4-dimethyl-1H-pyrazol-1-yl)nicotinaldehyde (Compound 121d)**

Concentrated hydrochloric acid (2.0 mL) was added dropwise into a solution of Compound 121c (305 mg) in THF (10 mL). The mixture was heated to 65 °C, and kept for reaction at this temperature for 1.5 h. After completion of the reaction, the reaction mixture was cooled in an ice bath, then adjusted to a pH value of about 8 by slowly adding potassium carbonate, and then extracted with EA (10 mL×3). The organic phases were combined, dried over anhydrous sodium sulfate, filtered, concentrated under reduced pressure, and separated and purified by silica gel column chromatography (PE:EA=4:1) to provide Compound 121d (200 mg). MS m/z (ESI): 202.2 $[M+H]^+$.

**Step 3: Preparation of 2-(6-(6-((6-(3,4-dimethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 121)**

Trifluoroacetate of Compound 1g (40.4 mg) and Compound 121d (53.4 mg) were added into methanol (0.5 mL), and then triethylamine (8.7 mg) and sodium cyanoborohydride (26.9 mg) were successively added. The mixture was kept for reaction at 20 °C for 16 h. After completion of the reaction, the reaction mixture was concentrated to dryness under reduced pressure, and separated and purified by Prep-HPLC to provide Compound 121 (3.0 mg). MS m/z (ESI): 548.3 $[M+H]^+$.

$^1H$ NMR (400 MHz, DMSO-$d_6$) δ 11.96 (s, 1H), 9.63 (s, 1H), 9.12 (d, *J* = 2.4 Hz, 1H), 8.43 (dd, *J* = 8.8, 2.4 Hz,

1H), 8.32 (d, *J* = 1.6 Hz, 1H), 8.27 (s, 1H), 7.88 (dd, *J* = 8.4, 2.4 Hz, 1H), 7.79-7.70 (m, 1H), 7.03-6.60 (m, 2H), 6.29 (br, 1H), 3.83-3.70 (m, 4H), 3.66-3.47 (m, 4H), 2.58-2.52 (m, 1H), 2.33 (s, 3H), 2.25 (s, 3H), 2.19 (s, 3H), 2.02 (s, 3H), 1.58 (d, *J* = 8.4 Hz, 1H).

**Example 33: 2-(6-(6-((5-fluoropyridin-2-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 70)**

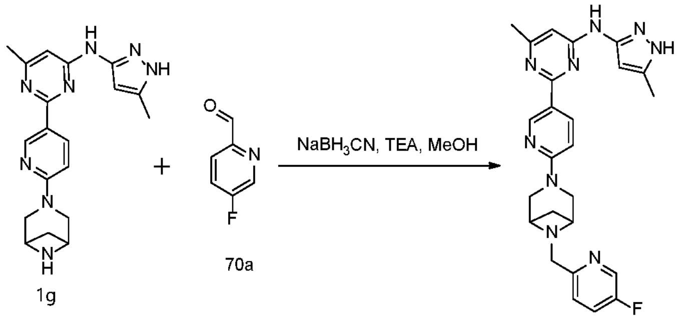


1g 70a 70

Trifluoroacetate of Compound 1g (30 mg) and Compound 70a (23.63 mg) were added into methanol (0.5 mL), and then triethylamine (6.37 mg) and sodium cyanoborohydride (19.78 mg) were successively added. The mixture was kept for reaction at room temperature for 20 h. After completion of the reaction, the reaction mixture was concentrated to dryness under reduced pressure, and separated and purified by Prep-HPLC to provide Compound 70 (12.0 mg). MS m/z (ESI): 472.3 $[M+H]^+$.

$^1$H NMR (400 MHz, DMSO-$d_6$)) δ 11.97 (br, 1H), 9.64 (s, 1H), 9.11 (d, *J* = 2.2 Hz, 1H), 8.43 (dd, *J* = 9.6, 2.7 Hz, 2H), 7.69 (td, *J* = 8.8, 3.0 Hz, 1H), 7.54 (dd, *J* = 8.7, 4.7 Hz, 1H), 6.77 (d, *J* = 9.0 Hz, 2H), 6.31 (s, 1H), 3.80 (d, *J* = 11.9 Hz, 2H), 3.72 (d, *J* = 5.9 Hz, 2H), 3.64 (s, 2H), 3.56 (d, *J* = 10.2 Hz, 2H), 2.57-2.51 (m, 1H), 2.33 (s, 3H), 2.26 (s, 3H), 1.59 (d, *J* = 8.4 Hz, 1H).

**Example 34: 2-(6-(6-((6-(3-fluoro-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 63)**

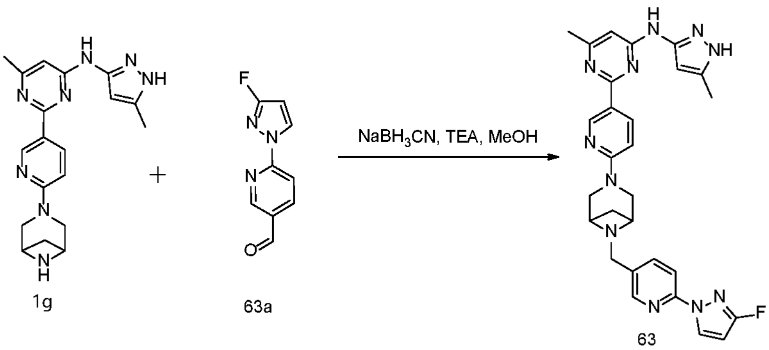


1g 63a 63

Trifluoroacetate of Compound 1g (30 mg) and Compound 63a (47.47 mg, prepared by referring to the method of preparing Compound 17a in Example 6 except that 4-fluoropyrazole was replaced with 3-fluoropyrazole) were added into methanol (0.5 mL), and then triethylamine (8.38 mg) and sodium cyanoborohydride (26.01 mg) were successively added. The mixture was kept for reaction at 20 °C for 16 h. After completion of the reaction, the reaction mixture was concentrated to dryness under reduced pressure, and separated and purified by Prep-HPLC to provide Compound 63 (10.0 mg). MS m/z (ESI): 538.2 $[M+H]^+$.

$^1$H NMR (400 MHz, DMSO-$d_6$) δ 11.97 (s, 1H), 9.65 (s, 1H), 9.12 (d, *J* = 2.1 Hz, 1H), 8.55 (m, 1H), 8.47-8.38 (m, 2H), 7.98 (d, *J* = 8.6 Hz, 1H), 7.70 (d, *J* = 8.4 Hz, 1H), 6.80 (d, *J* = 8.0 Hz, 2H), 6.56 (dd, *J* =8, 4 Hz, 1H), 6.29 (br, 1H), 3.84-3.68 (m, 4H), 3.67-3.47 (m, 4H), 2.60-2.54 (m, 1H), 2.33 (s, 3H), 2.25 (s, 3H), 1.60 (d, *J* = 8.2 Hz, 1H).

**Example 35: 2-(6-(6-((6-(4-fluoro-1H-imidazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 64)**

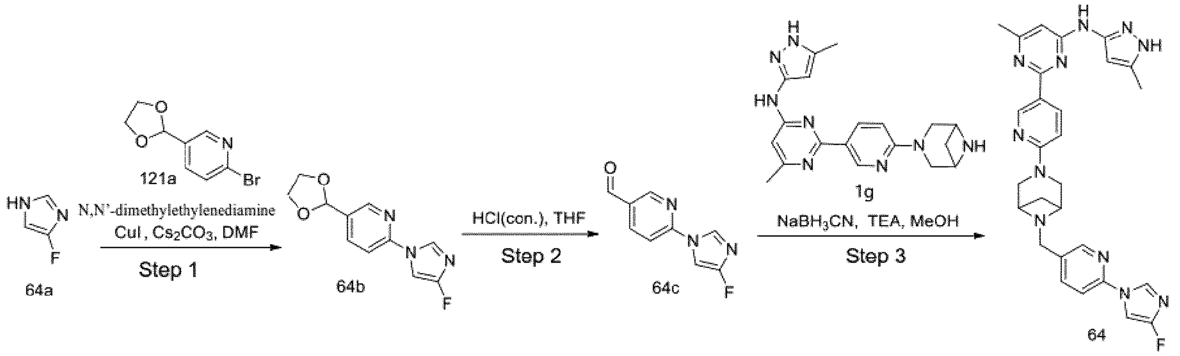


**Step 1: Preparation of 5-(1,3-dioxolan-2-yl)-2-(4-fluoro-1H-imidazol-1-yl)pyridine (Compound 64b)**

Compound 64a (230 mg), Compound 121a (86 mg), N,N'-dimethylethylenediamine (38 mg), CuI (83 mg), and cesium carbonate (425 mg) were added into DMF (1 mL). The mixture was heated to 115 °C, and kept for reaction under the protection of nitrogen at this temperature for 3 h. Water was added into the reaction mixture to quench the reaction. The reaction mixture was extracted with EA. The organic phase was washed with water, dried over anhydrous sodium sulfate, filtered, and then concentrated under reduced pressure, to provide Compound 64b (120 mg, crude), which was directly used for next step reaction without purification. MS m/z (ESI): 236.1 $[M+H]^+$.

**Step 2: Preparation of 6-(4-fluoro-1H-imidazol-1-yl)nicotinaldehyde (Compound 64c)**

Concentrated hydrochloric acid (12 N, 3.0 mL) was added dropwise into a solution of Compound 64b (120 mg) in THF (10 mL) and water (10 mL). The mixture was kept for reaction at room temperature for 18 h. After completion of the reaction, the reaction mixture was adjusted with saturated sodium bicarbonate solution to a pH value of about 8, extracted with EA, and dried over anhydrous sodium sulfate. The dried product was filtered, and then concentrated under reduced pressure, to provide Compound 64c (60 mg, crude), which was directly used for next step reaction without purification. MS m/z (ESI): 192.1 $[M+H]^+$.

**Step 3: Preparation of 2-(6-(6-((6-(4-fluoro-1H-imidazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 64)**

Trifluoroacetate of Compound 1g (30 mg) and Compound 64c (47.47 mg) were added into methanol (0.5 mL), and then triethylamine (8.38 mg) and sodium cyanoborohydride (26.01 mg) were successively added. The mixture was kept for reaction at 20 °C for 16 h. After completion of the reaction, the reaction mixture was concentrated to dryness under reduced pressure, and separated and purified by Prep-HPLC to provide Compound 64 (10.0 mg). MS m/z (ESI): 538.2 $[M+H]^+$.

$^1H$ NMR (400 MHz, DMSO-$d_6$) δ 11.97 (s, 1H), 9.64 (s, 1H), 9.12 (d, *J* = 2.2 Hz, 1H), 8.44 (dd, *J* = 9.1, 2.4 Hz, 2H), 8.28 (m, 1H), 7.99 (d, *J* = 8.4 Hz, 1H), 7.76-7.74(d, *J* = 8.0 Hz, 1H), 7.69-7.67 (dd, *J* = 8.4, 1.6 Hz, 1H), 6.86 (br, 1H), 6.77 (d, *J* = 9.2 Hz, 1H), 6.30 (br, 1H), 3.78-3.71(m, 4H), 3.67-3.51(m, 4H), 2.59-2.53 (m, 1H), 2.33 (s, 3H), 2.26 (s, 3H), 1.60 (d, *J* = 8.4 Hz, 1H).

**Example 36: 2-(6-(6-(1-(6-(4-fluoro-1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 52)**

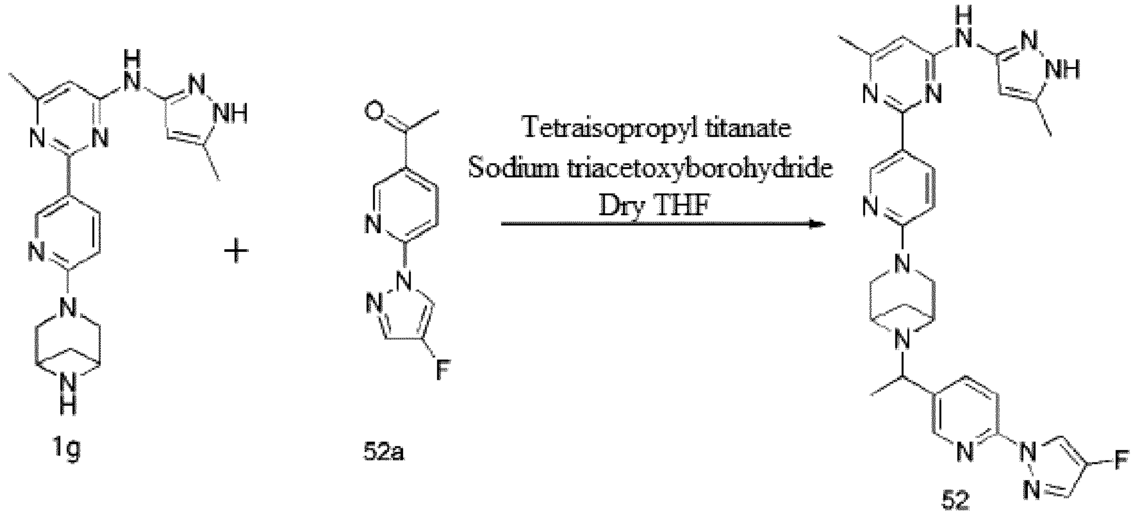


1g 52a 52

Compound 1g (320 mg), Compound 52a (181.17 mg, prepared by referring to the method of preparing Compound 17a in Example 6, except that 6-bromonicotinaldehyde was replaced with 1-(6-bromopyridin-3-yl)ethanone), and tetraisopropyl titanate (752.81 mg) were added into dry THF (25 mL), and were, after nitrogen replacement three times, stirred at 75 °C for 24 h. Then, sodium triacetoxyborohydride (935.64 mg) was added into the system portionwise, dry THF (15 mL) was supplemented, and the mixture was stirred at 75 °C for an additional 16 h. After completion of the reaction, the reaction mixture was concentrated to dryness under reduced pressure, and separated and purified by flash column chromatography (MeOH:DCM=1:9) to provide crude Compound 52, which was further separated and purified by Prep-HPLC to provide Compound 52 (90.0 mg). MS m/z (ESI): 552.3 $[M+H]^+$.

$^1H$ NMR (400 MHz, DMSO-$d_6$) δ 11.96 (s, 1H), 9.63 (s, 1H), 9.12 (d, *J* = 2.1 Hz, 1H), 8.66 (d, *J* = 4.4 Hz, 1H), 8.46-8.39 (m, 2H), 8.01 (dd, *J* = 8.5, 2.0 Hz, 1H), 7.90 (dd, *J* = 14.7, 6.2 Hz, 2H), 6.82(br, 1H), 6.75(d, *J* = 12.0 Hz, 1H), 6.27(br, 1H),3.96-3.82 (m, 2H), 3.77 (q, *J* = 6.1 Hz, 1H), 3.63 (m, 1H), 3.49-3.37 (m, 3H), 2.55-2.51 (m, 1H), 2.33 (s, 3H), 2.25 (s, 3H), 1.55 (d, *J* = 8.4 Hz, 1H), 1.23 (d, *J* = 6.2 Hz, 3H).

**Example 37: 2-(6-(6-((6-(3-cyclopropyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 120)**

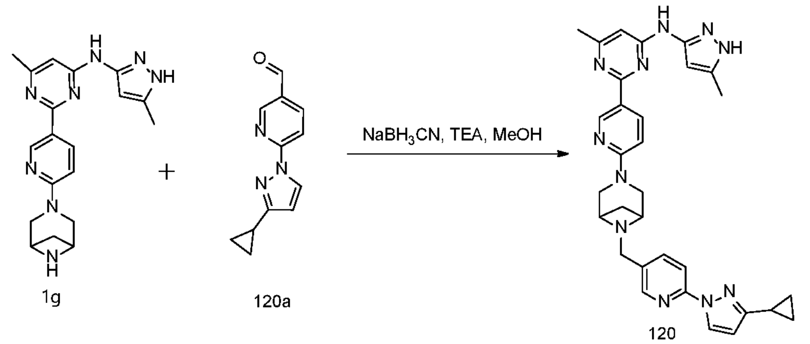


1g 120a 120

Trifluoroacetate of Compound 1g (30 mg) and Compound 120a (22.95 mg, prepared by referring to the method of preparing Compound 64c in Example 35 except that the starting material 4-fluoroimidazole was replaced with 3-cyclopropylimidazole) were added into methanol (0.5 mL), and then triethylamine (8.38 mg) and sodium cyanoborohydride (26.01 mg) were successively added. The mixture was stirred at 20 °C for 16 h. After completion of the reaction, the reaction mixture was concentrated to dryness under reduced pressure, and separated and purified by Prep-HPLC to provide Compound 120 (13.0 mg). MS m/z (ESI): 560.3 $[M+H]^+$.

$^1H$ NMR (400 MHz, DMSO-$d_6$) δ 11.97 (s, 1H), 9.64 (s, 1H), 9.12 (d, *J* = 2.2 Hz, 1H), 8.44 (dd, *J* = 8.9, 2.4 Hz, 2H), 8.35 (d, *J* = 1.3 Hz, 1H), 7.92 (dd, *J* = 8.5, 1.8 Hz, 1H), 7.78 (d, *J* = 8.4 Hz, 1H), 6.8(br, 1H), 6.78 (d, *J* = 9.0 Hz, 1H), 6.28(br, 1H), 6.26 (d, *J* = 2.5 Hz, 1H), 3.81-3.66 (m, 4H), 3.67-3.52 (m, 4H), 2.58-2.53 (m, 1H), 2.33 (s, 3H), 2.26 (s, 3H), 2.03-1.95 (m, 1H), 1.59 (d, *J* = 8.4 Hz, 1H), 0.98-0.91 (m, 2H), 0.79-0.73 (m, 2H).

**Example 38: 6-methyl-N-(5-methyl-1H-pyrazol-3-yl)-2-(6-(4-((6-methylpyridin-3-yl)oxy)piperidin-1-yl)pyridin-3-yl)pyrimidin-4-amine (Compound 119)**

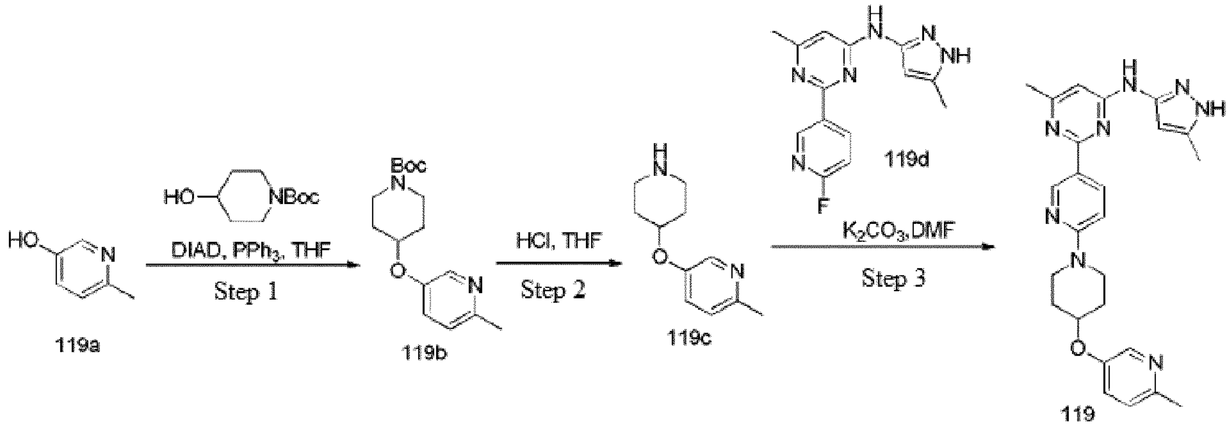


119a 119b 119c 119d 119

**Step 1: Preparation of tert-butyl 4-((6-methylpyridin-3-yl)-oxy)piperidine-1-carboxylate (Compound 119b)**

Compound 119a (100 mg), N-Boc-4-hydroxypiperidine (276.65 mg), and triphenylphosphine (480.18 mg) were added into dry THF (5 mL), and cooled to 0 °C. DIAD (370.21 mg) was added dropwise into the system, and the mixture was stirred at room temperature for 16 h. After completion of the reaction, the system was concentrated under reduced pressure, and purified by flash column chromatography (PE:EA=1:1) to provide Compound 119b (65 mg). MS m/z (ESI): 293.2 $[M+H]^+$.

**Step 2: Preparation of 2-methyl-5-(piperidin-4-yloxy)pyridine (Compound 119c)**

Compound 119b (65 mg) was added into a mixed solution of hydrogen chloride in 1,4-dioxane (4 N, 2 mL) and THF (1 mL). The mixture was stirred at room temperature for 2 h, and concentrated to dryness under reduced pressure to provide hydrochloride of Compound 119c (54 mg). MS m/z (ESI): 193.2 $[M+H]^+$.

**Step 3: Preparation of 6-methyl-N-(5-methyl-1H-pyrazol-3-yl)-2-(6-(4-((6-methylpyridin-3-yl)oxy)piperidin-1-yl)pyridin-3-yl)pyrimidin-4-amine (Compound 119)**

Hydrochloride of Compound 119c (50 mg), Compound 119d (50 mg), and potassium carbonate (73 mg) were added into DMF (2 mL), and the mixture was heated to 100 °C, and stirred at this temperature for 16 h. After completion of the reaction, the reaction mixture was cooled to room temperature, diluted with water (30 mL), and extracted with EA (30 mL×2). The organic phases were combined, dried over anhydrous sodium sulfate, filtered, and concentrated, and separated and purified by Prep-HPLC to provide Compound 119 (13 mg). MS m/z (ESI): 457.3 $[M+H]^+$.

$^1H$ NMR (400 MHz, DMSO-$d_6$) δ 11.96 (s, 1H), 9.63 (s, 1H), 9.04 (d, *J* = 2.4 Hz, 1H), 8.37 (dd, *J* = 9.0, 2.4 Hz, 1H), 8.19 (d, *J* = 2.9 Hz, 1H), 7.38 (dd, *J* = 8.5, 3.0 Hz, 1H), 7.18 (d, J = 8.5 Hz, 1H), 6.96 (d, *J* = 9.1 Hz, 1H), 6.82 (s, 1H), 6.28 (s, 1H), 4.72-4.65 (m, 1H), 4.13-4.01 (m, 2H), 3.49-3.40 (m, 2H), 2.40 (s, 3H), 2.32 (s, 3H), 2.25 (s, 3H), 2.06-1.98 (m, 2H), 1.69-1.58 (m, 2H).

**Example 39: 2-(6-(6-(4-(3-fluoro-1H-pyrazol-1-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 89)**

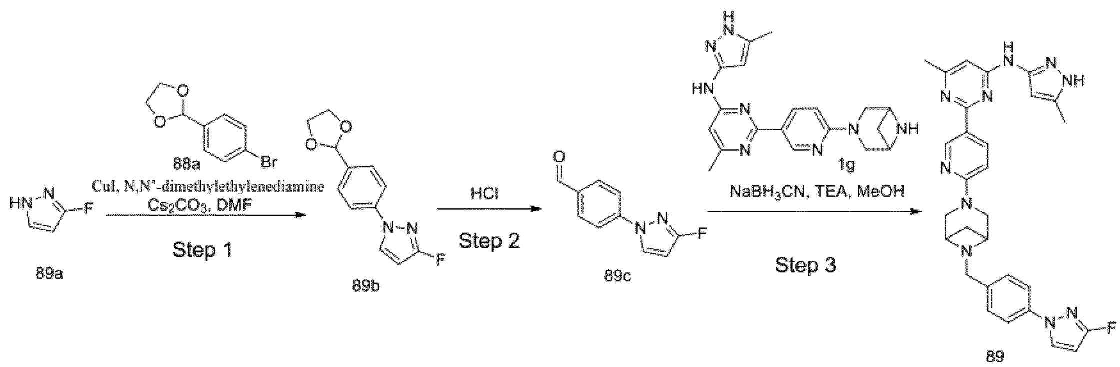


**Step 1: Preparation of 1-(4-(1,3-dioxolan-2-yl)phenyl)-3-fluoro-1H-pyrazole (Compound 89b)**

Compound 89a (80 mg), Compound 88a (212.92 mg), N,N'-dimethylethylenediamine (81.94 mg), cesium carbonate (908.55 mg), and CuI (177.02 mg) were successively added into DMF (6 mL). The mixture was heated to 110 °C, and stirred at this temperature for 12 h. The reaction mixture was cooled to room temperature, diluted with water (50 mL), and extracted with DCM (50 mL×2). The organic phases were combined, washed with water and saturated brine, dried over anhydrous sodium sulfate, filtered, concentrated under reduced pressure, and separated and purified by flash column chromatography (PE:EA=63:37), to provide Compound 89b (35 mg). MS m/z (ESI): 235.1 $[M+H]^+$.

**Step 2: Preparation of 4-(3-fluoro-1H-pyrazol-1-yl)benzaldehyde (Compound 89c)**

Compound 89b (35 mg) was added into a mixed solution of hydrogen chloride in 1,4-dioxane (4 N, 2 mL) and DCM (1 mL). The mixture was stirred at room temperature for 2 h. The reaction mixture was concentrated under reduced pressure to provide Compound 89c (28 mg). MS m/z (ESI): 191.1 $[M+H]^+$.

**Step 3: Preparation of 2-(6-(6-(4-(3-fluoro-1H-pyrazol-1-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 89)**

Compound 89c (14.37 mg), trifluoroacetate of Compound 1g (30 mg), triethylamine (6.37 mg), and sodium cyanoborohydride (19.78 mg) were successively added into methanol (0.5 mL), and stirred at room temperature for 36 h. A saturated aqueous solution of ammonium chloride (0.1 mL) was added to quench the reaction. The reaction mixture was concentrated, and pre-purified by preparative TLC (DCM:MeOH=10:1) to provide 5 mg of crude product ($R_f$=0.15-0.25), which was further separated and purified by Prep-HPLC to provide Compound 89 (4 mg). MS m/z (ESI): 537.3 $[M+H]^+$.

$^1$H NMR (400 MHz, DMSO-$d_6$) δ 11.97 (s, 1H), 9.64 (s, 1H), 9.12 (d, *J* = 2.3 Hz, 1H), 8.49-8.39 (m, 2H), 7.68 (d, *J* = 8.6 Hz, 2H), 7.46 (d, *J* = 8.5 Hz, 2H), 6.78 (d, *J* = 9.0 Hz, 2H), 6.31 (dd, *J* = 5.8, 2.6 Hz, 2H), 3.80-3.67 (m, 4H), 3.64-3.48 (m, 4H), 2.59-2.54 (m, 1H), 2.33 (s, 3H), 2.25 (s, 3H), 1.59 (d, *J* = 8.4 Hz, 1H).

**Example 40: 6'-(6-((6-(4-fluoro-1H-pyrazol-1-yl)pyridin-3-yl)methyl)-3,6-diazabicyclo[3.1.1]heptan-3-yl)-4-methyl-N-(5-methyl-1H-pyrazol-3-yl)-[2,3'-dipyridyl]-6-amine (Compound 6)**

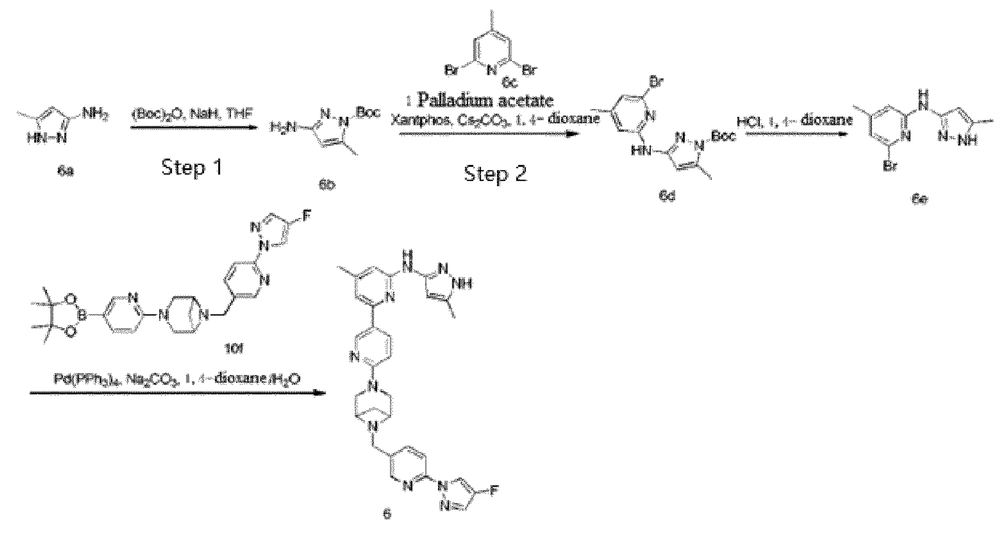


6a 6b 6c 6d 6e 10f 6

**Step 1: Preparation of tert-butyl 3-amino-5-methyl-1H-pyrazole-1-carboxylate (Compound 6b)**

Compound 6a (2.4 g) was dissolved in dry THF (50 mL), NaH (988.39 mg, purity 60%) was added portionwise, and then the mixture was stirred for 10 min. Di-tert-butyl dicarbonate (5.39 g) was added dropwise, and the mixture was stirred under the protection of nitrogen at 25 °C for 2 h. Water was added into the reaction mixture to quench the reaction. The reaction mixture was extracted with EA. The organic phase was dried over anhydrous sodium sulfate, filtered, concentrated, and separated and purified by silica gel column chromatography (PE:EA=5:1) to provide Compound 6b (3.95 g).

**Step 2: Preparation of tert-butyl 3-((6-bromo-4-methylpyridin-2-yl)amino)-5-methyl-1H-pyrazole-1-carboxylate (Compound 6d)**

Compound 6c (1.0 g), Compound 6b (864.65 mg), palladium acetate (89.47 mg), Xantphos (461.20 mg), and cesium carbonate (2.60 g) were successively added into 1,4-dioxane (10 mL), and stirred under the protection of nitrogen at 95 °C for 2 h. LC-MS showed that the raw materials were fully converted into the target product. The reaction mixture was cooled to room temperature, filtered, and concentrated, and separated and purified by silica gel column chromatography (PE:EA=3:1) to provide Compound 6d (415 mg). MS m/z (ESI): 367 $[M+H]^+$.

**Step 3: Preparation of 6-bromo-4-methyl-N-(5-methyl-1H-pyrazol-3-yl)pyridin-2-amine (Compound 6e)**

Compound 6d (300 mg) was added into a solution of hydrogen chloride in 1,4-dioxane (4 N, 2 mL). The mixture was stirred at 25 °C for 1 h, and directly concentrated to dryness. The crude product was dissolved in methanol (5 mL). Then, triethylamine (1 mL) was added, and the mixture was stirred at room temperature for 15 min. The reaction mixture was concentrated, and purified by flash silica gel column chromatography (DCM:MeOH=97:3) to provide Compound 6e (145 mg). MS m/z (ESI): 267 $[M+H]^+$.

**Step 4: Preparation of 6'-(6-((6-(4-fluoro-1H-pyrazol-1-yl)pyridin-3-yl)methyl)-3,6-diazabicyclo[3.1.1]heptan-3-yl)-4-methyl-N-(5-methyl-1H-pyrazol-3-yl)-[2,3'-dipyridyl]-6-amine (Compound 6)**

Compound 6e (30 mg), Compound 10f (85 mg), $Na_2CO_3$ (27.85 mg), $Pd(PPh_3)_4$ (12.98 mg), $H_2O$ (1 mL), and 1,4-dioxane (5 mL) were successively added into a reaction flask, and were, after nitrogen replacement for three times, stirred at 95 °C for 5 h. After completion of the reaction, the reaction mixture was diluted with water, and extracted with EA. The organic layer was collected, washed with water and saturated brine, dried over anhydrous sodium sulfate, filtered under suction, concentrated, pre-purified by preparative TLC (DCM:MeOH=9:1), and then separated and purified by Prep-HPLC, to provide Compound 6 (6 mg). MS m/z (ESI): 537.3 $[M+H]^+$.

$^1H$ NMR (400 MHz, DMSO-$d_6$) δ 11.63 (s, 1H), 9.02 (s, 1H), 8.86 (d, *J* = 2.3 Hz, 1H), 8.67 (dd, *J* = 4.5, 0.6 Hz, 1H), 8.41 (d, *J* = 1.7 Hz, 1H), 8.21 (dd, *J* = 8.9, 2.4 Hz, 1H), 7.98 (dd, *J* = 8.5, 2.2 Hz, 1H), 7.92 (d, *J* = 4.3 Hz, 1H), 7.86 (d, *J* = 8.4 Hz, 1H), 7.03 (s, 1H), 6.88 (s, 1H), 6.77 (d, *J* = 9.0 Hz, 1H), 6.22 (s, 1H), 3.81-3.69 (m, 4H),3.66-3.53 (m, 4H), 2.58-2.53 (m, 1H), 2.26 (s, 3H), 2.22 (s, 3H), 1.59 (d, *J* = 8.4 Hz, 1H).

**Example 41: 2-(6-(6-((6'-methoxy-[2,3'-dipyridyl]-5-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 7)**

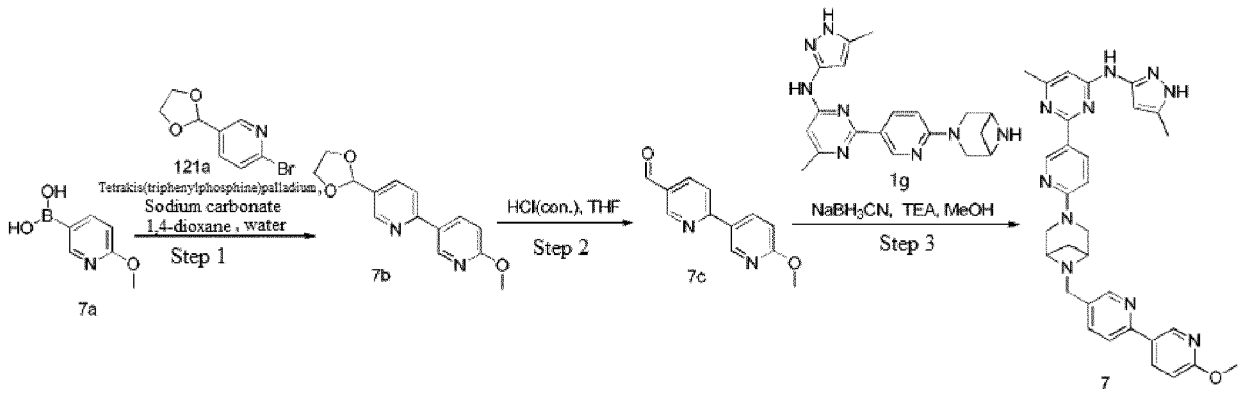


**Step 1: Preparation of 5-(1,3-dioxolan-2-yl)-6'-methoxy-2,3'-dipyridine (Compound 7b)**

Compound 7a (499 mg), Compound 121a (500 mg), $Na_2CO_3$ (691 mg), $Pd(PPh_3)_4$ (126 mg), $H_2O$ (3 mL), and 1,4-dioxane (17 mL) were successively added into a reaction flask, and were, after nitrogen replacement for three times, stirred at 95 °C for 5 h. After completion of the reaction, the reaction mixture was concentrated to dryness, and separated and purified by silica gel column chromatography (PE:EA=1:1) to provide Compound 7b (350 mg).

**Step 2: Preparation of 6'-methoxy-[2,3'-dipyridyl]-5-carbaldehyde (Compound 7c)**

Concentrated hydrochloric acid (12 N, 3.0 mL) was added dropwise into a solution of Compound 7b (200 mg) in THF (11 mL) and water (9 mL). The mixture was kept for reaction at room temperature for 18 h. After completion of the reaction, the reaction mixture was adjusted with a potassium carbonate solution to a pH value of about 10, and then extracted with EA. The organic phase was dried over anhydrous sodium sulfate, filtered, concentrated under reduced pressure, and separated and purified by silica gel column chromatography (PE:EA=2:1) to provide Compound 7c (60 mg). MS m/z (ESI): 215.1 $[M+H]^+$.

**Step 3: Preparation of 2-(6-(6-((6'-methoxy-[2,3'-dipyridyl]-5-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 7)**

Trifluoroacetate of Compound 1g (50 mg) and Compound 7c (67.44 mg) were added into methanol (0.5 mL), and then triethylamine (10.62 mg) and sodium cyanoborohydride (32.97 mg) were successively added. The mixture was kept for reaction at room temperature for 20 h. After completion of the reaction, the reaction mixture was concentrated to dryness under reduced pressure, and separated and purified by Prep-HPLC to provide Compound 7 (10.0 mg). MS m/z (ESI): 561.3 $[M+H]^+$.

$^1H$ NMR (400 MHz, DMSO-$d_6$) $\delta$ 11.98 (br, 1H), 9.65 (s, 1H), 9.13 (d, $J$ = 2.2 Hz, 1H), 8.86 (d, $J$ = 2.2 Hz, 1H), 8.60 (d, $J$ = 1.5 Hz, 1H), 8.44 (dd, $J$ = 8.9, 2.3 Hz, 1H), 8.36 (dd, $J$ = 8.7, 2.5 Hz, 1H), 7.90 (d, $J$ = 8.1 Hz, 1H), 7.83 (dd, $J$ = 8.2, 2.1 Hz, 1H), 6.85 (dd, $J$ = 56.9, 8.8 Hz, 3H), 6.33 (s, 1H), 3.91 (s, 3H), 3.75 (dd, $J$ = 20.2, 8.9 Hz, 4H),3.65-3.49 (m, 4H), 2.59-2.53 (m, 1H), 2.33 (s, 3H), 2.26 (s, 3H), 1.60 (d, $J$ = 8.4 Hz, 1H).

**Example 42: 2-(6-(6-((5'-fluoro-2'-methyl-[2,3'-dipyridyl]-5-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 8)**

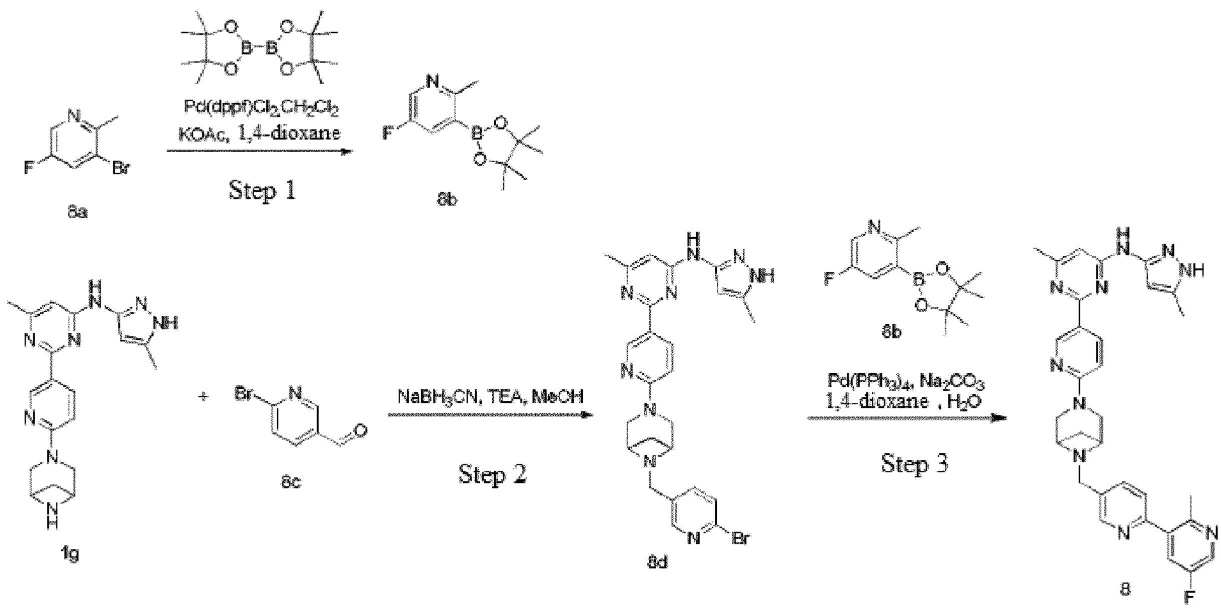


**Step 1: Preparation of 5-fluoro-2-methylpyridine-3-boronic acid pinacol ester (Compound 8b)**

Bis(pinacolato)diboron (1.20 g), Compound 8a (300 mg), Pd(dppf)$Cl_2$ DCM (128.93 mg), potassium acetate (464.85 mg), and dry 1,4-dioxane (10 mL) were successively added into a reaction flask. The mixture was heated to 100 °C under the protection of nitrogen, and stirred at this temperature for 4 h. After completion of the reaction, the mixture was filtered. The filtrate was diluted with water, and extracted with EA. The organic phase was washed with water twice, dried over anhydrous sodium sulfate, and filtered. The filtrate was concentrated, separated and purified by flash silica gel column chromatography (PE:EA=1:1) to provide Compound 8b (300 mg). MS m/z (ESI): 238.2 $[M+H]^+$.

**Step 2: Preparation of 2-(6-(6-((6-bromopyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 8d)**

Trifluoroacetate of Compound 1g (200 mg) and Compound 8c (195.20 mg) were added into methanol (10 mL), and triethylamine (42.48 mg) was added while stirring at room temperature. After stirring for 30 min, sodium cyanoborohydride (131.89 mg) was added, and the mixture was stirred at 20 °C for 16 h. LC-MS showed that there was an obvious product peak. The mixture was separated and purified by flash silica gel column chromatography (DCM:MeOH=10:1) to provide Compound 8d (220 mg). MS m/z (ESI): 532.1$[M+H]^+$.

**Step 3: Preparation of 2-(6-(6-((5'-fluoro-2'-methyl-[2,3'-dipyridyl]-5-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 8)**

Compound 8b (222.63 mg), Compound 8d (100 mg), $Na_2CO_3$ (69.87 mg), $Pd(PPh_3)_4$ (32.55 mg), water (1 mL), and 1,4-dioxane (5 mL) were successively added into a reaction flask, and were stirred under the protection of nitrogen at 95 °C for 5 h. After completion of the reaction, the reaction mixture was rotarily evaporated, pre-purified by preparative TLC (DCM:MeOH=9:1), and then separated and purified by Prep-HPLC, to provide Compound 8 (25 mg). MS m/z (ESI): 563.3 $[M+H]^+$.

$^1H$ NMR (400 MHz, DMSO-$d_6$) $\delta$ 11.97 (s, 1H), 9.66 (s, 1H), 9.13 (d, $J$ = 2.2 Hz, 1H), 8.66 (d, $J$ = 1.5 Hz, 1H), 8.51 (d, $J$ = 2.9 Hz, 1H), 8.44 (dd, $J$ = 8.9, 2.3 Hz, 1H), 7.90 (dd, $J$ = 8.1, 2.1 Hz, 1H), 7.76 (dd, $J$ = 9.5, 2.8 Hz, 1H), 7.62 (d, $J$ = 8.0 Hz, 1H), 6.81 (br, 1H), 6.79 (d, $J$ = 9.0 Hz, 1H), 6.32 (br, 1H), 3.85-3.71(m, 4H), 3.70-3.51 (m, 4H), 2.61-2.53 (m, 1H), 2.51 (s, 3H), 2.33 (s, 3H), 2.26 (s, 3H), 1.61 (d, $J$ = 8.4 Hz, 1H).

**Example 43: 6-methyl-2-(6-(6-((6-(5-methyl-1H-pyrazol-1-yl)pyridin-3-yl)methyl)-3,6-diazabicyclo[3.1.1]heptan-3-yl)pyridin-3-yl)-N-(5-methyl-1H-pyrazol-3-yl)pyrimidin-4-amine (Compound 9)**

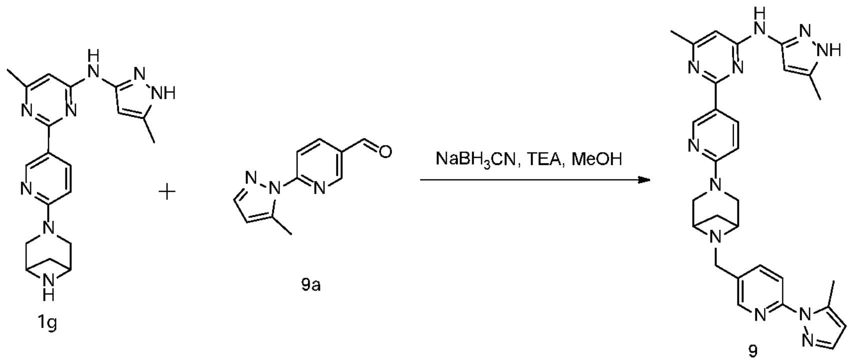


Trifluoroacetate of Compound 1g (30 mg) and Compound 9a (17.68 mg, prepared by referring to the method of preparing Compound 64c in Example 35 except that the starting material 4-fluoroimidazole was replaced with 5-methylimidazole) were added into methanol (0.5 mL), and then triethylamine (8.38 mg) was added. After stirring for 30 min, sodium cyanoborohydride (19.78 mg) was added, and the mixture was stirred at 20 °C for 16 h. LC-MS showed that the raw materials were fully converted, and there was an obvious product peak. The mixture was separated and purified by Prep-HPLC to provide Compound 9 (7 mg). MS m/z (ESI): 534.3 $[M+H]^+$.

$^1H$ NMR (400 MHz, DMSO-$d_6$) $\delta$ 11.99 (s, 1H), 9.67 (s, 1H), 9.14 (d, *J* = 2.1 Hz, 1H), 8.49 (d, *J* = 2.4 Hz, 1H), 8.45 (dd, *J* = 9.2, 2.4 Hz, 1H), 8.38 (d, *J* = 1.6 Hz, 1H), 7.94 (dd, *J* = 8.5, 2.1 Hz, 1H), 7.81 (d, *J* = 8.4 Hz, 1H), 6.83(br, 1H), 6.79 (d, *J* = 9.2 Hz, 1H), 6.38 (d, *J* = 2.4 Hz, 1H), 6.33 (br, 1H), 3.85-3.71 (m, 4H), 3.69-3.51 (m, 4H), 2.61-2.55 (m, 1H), 2.35 (s, 3H), 2.30 (s, 3H), 2.27 (s, 3H), 1.61 (d, *J* = 8.4 Hz, 1H).

**Example 44: N-(5-cyclopropyl-1H-pyrazol-3-yl)-2-(6-(6-((6-(4-fluoro-1H-pyrazol-1-yl)pyridin-3-yl)methyl)-3,6-diazabicyclo [3.1.1] heptan-3-yl)pyridin-3-yl)-6-methylpyrimidin-4-amine (Compound 10)**

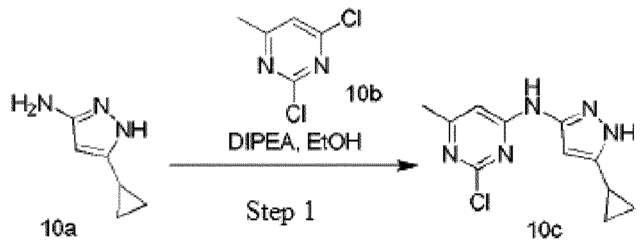


10a 10c

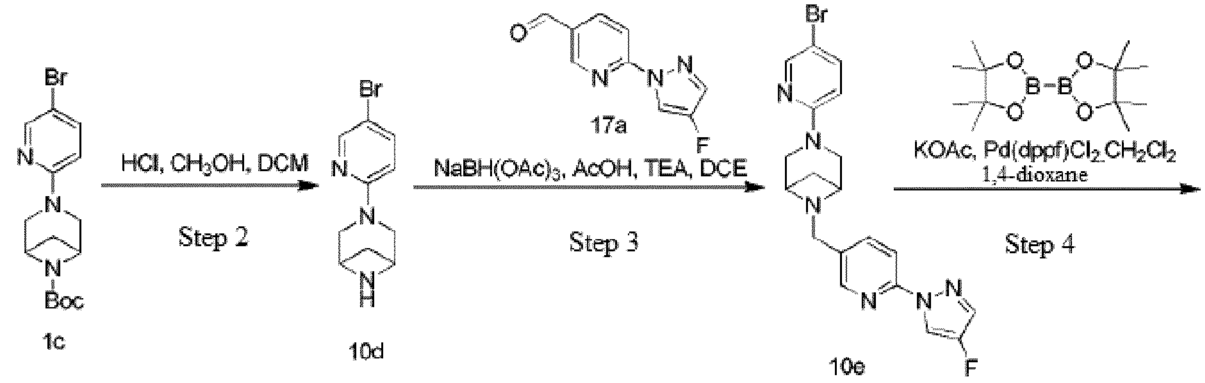


1c 10d 10e

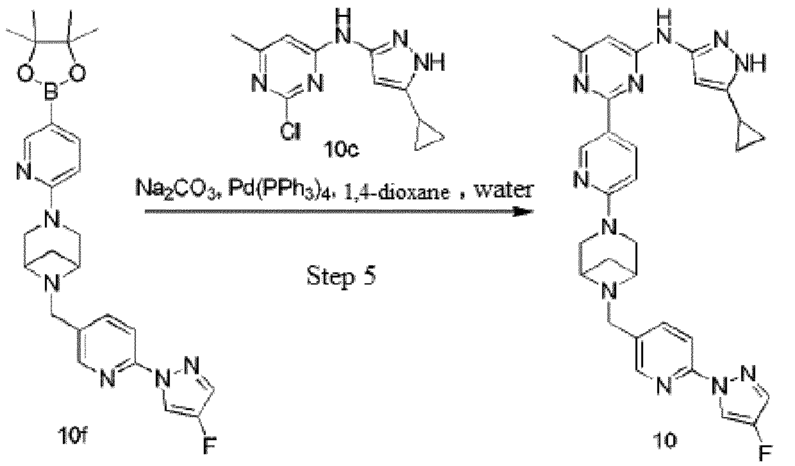


10f 10

**Step 1: Preparation of 2-chloro-N-(5-cyclopropyl-1H-pyrazol-3-yl)-6-methylpyrimidin-4-amine (Compound 10c)**

Compound 10b (1.0 g) and Compound 10a (755.53 mg) were dissolved in ethanol (20.00 mL), and then N,N-diisopropylethylamine (1590.00 mg) was added. The mixture was heated to 70 °C and stirred under the protection of nitrogen at this temperature for 48 h. The reaction mixture was concentrated under reduced pressure, and diluted with EA (300.00 mL). The organic phase was washed with water for three times, further washed with a saturated aqueous solution of sodium chloride, dried over anhydrous sodium sulfate, filtered, concentrated, and separated and purified by silica gel column chromatography (DCM:MeOH=9:1) to provide Compound 10c (820.00 mg). MS m/z (ESI): 250.1 $[M+H]^+$.

**Step 2: Preparation of 3-(5-bromopyridin-2-yl)-3,6-diazabicyclo[3.1.1]heptane (Compound 10d)**

Compound 1c (1180.00 mg) was dissolved in a mixed solvent of methanol (5.00 mL) and DCM (10.00 mL), and then a solution of hydrogen chloride in 1,4-dioxane solution(4 N, 6.00 mL) was slowly added dropwise in an ice bath. The mixture was stirred at 25 °C for 16 h. The reaction mixture was concentrated under reduced pressure to provide hydrochloride of Compound 10d (1040.00 mg). MS m/z (ESI): 254.0 $[M+H]^+$.

**Step 3: Preparation of 3-(5-bromopyridin-2-yl)-6-((6-(4-fluoro-1H-pyrazol-1-yl)pyridin-3-yl)methyl)-3,6-diazabicyclo[3.1.1]heptane (Compound 10e)**

Hydrochloride of Compound 10d (300.00 mg) and Compound 17a (297.04 mg) were dissolved in 1,2-dichloroethane (10.00 mL), and triethylamine (104.82 mg) was slowly added dropwise. After stirring for 10 min, acetic acid (31.10 mg) was added dropwise, and the mixture was stirred for an additional 30 min. Then, sodium triacetoxyborohydride (878.21 mg) was added, and the mixture was stirred at 25 °C for 20 h. A saturated aqueous solution of ammonium chloride was added into the reaction mixture to quench the reaction. The reaction mixture was diluted with water, and extracted with DCM. The organic layer was dried over anhydrous sodium sulfate, filtered, concentrated, and separated and purified by silica gel column chromatography (PE:EA=1:1) to provide Compound 10e (363.00 mg). MS m/z (ESI): 429.1 $[M+H]^+$.

**Step 4: Preparation of 6-((-6-(4-fluoro-1H-pyrazol-1-yl)pyridin-3-yl)methyl)-3-(5-(4,4,5,5-tetramethy1-1,3,2-dioxaborolan-2-yl)pyridin-2-yl)-3,6-diazabicyclo[3.1.1]heptane (Compound 10f)**

Compound 10e (187.00 mg) and bis(pinacolato)diboron (221.23 mg) were dissolved in 1,4-dioxane (15.00 mL), and then potassium acetate (106.88 mg) and $Pd(dppf)Cl_2$·DCM (35.57 mg) were added. The mixture was heated to 95 °C under the protection of nitrogen, and stirred at this temperature for 5 h. The reaction mixture was diluted with EA (100.00 mL), and washed with water three times. The organic phase was dried over anhydrous sodium sulfate, filtered, concentrated, and separated and purified by silica gel column chromatography (PE:EA=3:1) to provide Compound 10f (100.00 mg). MS m/z (ESI): 477.3 $[M+H]^+$.

**Step 5: Preparation of N-(5-cyclopropyl-1H-pyrazol-3-yl)-2-(6-(6-((6-(4-fluoro-1H-pyrazol-1-yl)pyridin-3-yl)methyl)-3,6-diazabicyclo [3.1.1] heptan-3-yl)pyridin-3-yl)-6-methylpyrimidin-4-amine (Compound 10)**

Compound 10c (26.21 mg) and Compound 10f (50.00 mg) were dissolved in 1,4-dioxane (4.00 mL), and then $Pd(PPh_3)_4$ (18.19 mg) and an aqueous solution of sodium carbonate (33.38 mg dissolved in 1.00 mL of water) were successively added. The mixture was heated to 95 °C and stirred under the protection of nitrogen at this temperature for 12 h. After completion of the reaction, the reaction mixture was diluted with EA (20.00 mL), and washed with water for three times. The organic phase was dried over anhydrous sodium sulfate, filtered, and then concentrated, to provide a crude product. The crude product was separated and purified by Prep-HPLC to provide Compound 10 (9.00 mg). MS m/z (ESI): 564.3 $[M+H]^+$.

$^1H$ NMR (400 MHz, DMSO-$d_6$) δ 12.02 (s, 1H), 9.64 (s, 1H), 9.11 (d, *J* = 2.4 Hz, 1H), 8.67 (dd, *J* = 4.8, 0.8 Hz, 1H), 8.42 (dd, *J* = 9.2, 2.0 Hz, 2H), 7.99 (dd, *J* = 8.4, 2.0 Hz, 1H), 7.92 (dd, *J* = 4.4, 0.8 Hz, 1H), 7.87 (d, *J* = 8.4 Hz, 1H), 6.78 (d, *J* = 9.2 Hz, 2H), 6.18 (br, 1H), 3.79-3.71 (m, 4H), 3.64-3.53 (m, 4H), 2.56-2.51 (m, 1H), 2.33 (s, 3H), 1.95-1.89 (m, 1H), 1.60 (d, *J* = 8.4 Hz, 1H), 0.97-0.92 (m, 2H), 0.75-0.69 (m, 2H).

**Example 45: 2-(6-(6-((6-(3,5-dimethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 11)**

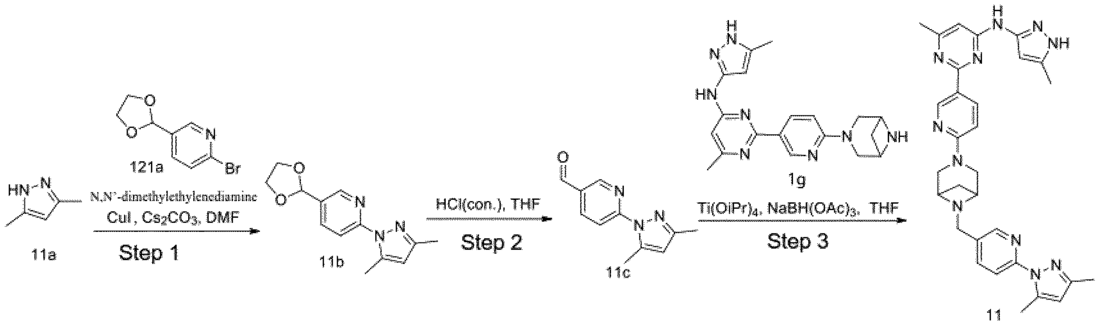


**Step 1: Preparation of 2-(3,5-dimethyl-1H-pyrazol-1-yl)-5-(1,3-dioxolan-2-yl)pyridine (Compound 11b)**

Compound 11a (1.0 g), Compound 121a (1.74 g), N,N'-dimethylethylenediamine (664.83 mg), CuI (1.44 g), and cesium carbonate (7.37 g) were added into DMF (30 mL). The mixture was heated to 98 °C, and kept for reaction under the protection of nitrogen at this temperature for 16 h. Water was added into the reaction mixture to quench the reaction. The reaction mixture was extracted with EA. The organic phases were combined, washed with saturated brine, dried over anhydrous sodium sulfate, filtered, and concentrated under reduced pressure, and separated and purified by silica gel column chromatography (PE:EA=3:1) to provide Compound 11b (0.69 g). MS m/z (ESI): 246.2 $[M+H]^+$.

**Step 2: Preparation of 6-(3,5-dimethyl-1H-pyrazol-1-yl)nicotinaldehyde (Compound 11c)**

Hydrochloric acid (2 N, 3.0 mL) was added dropwise into a solution of Compound 11b (250 mg) in THF (6 mL). The mixture was kept for reaction at room temperature for 3 h. After completion of the reaction, the reaction mixture was cooled in an ice bath, adjusted to a pH value of about 8 by slowly adding potassium carbonate, and then extracted with EA (10 mL x 3). The organic phases were combined, dried over anhydrous sodium sulfate, filtered, concentrated under reduced pressure, and separated and purified by silica gel column chromatography (PE:EA=3:1) to provide Compound 11c (150 mg).

**Step 3: Preparation of 2-(6-(6-((6-(3,5-dimethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 11)**

Compound 1g (30 mg) and Compound 11c (25 mg) were added into methanol (1 mL), and then acetic acid (5 mg) was added. The mixture was stirred at room temperature for 0.5 h. Sodium cyanoborohydride (15.6 mg) was added, and the mixture was kept for reaction at room temperature for 20 h. After completion of the reaction, the reaction mixture was concentrated to dryness under reduced pressure, and pre-purified by preparative TLC (DCM:MeOH=96:4) to provide a crude product, which was further separated and purified by Prep-HPLC to provide Compound 11 (7 mg). MS m/z (ESI): 548.3 $[M+H]^+$.

$^1H$ NMR (400 MHz, DMSO-$d_6$)) $\delta$ 11.97 (s, 1H), 9.65 (s, 1H), 9.12 (d, $J$ = 2.1 Hz, 1H), 8.44 (dd, $J$ = 8.9, 2.2 Hz, 1H), 8.37 (d, $J$ = 1.8 Hz, 1H), 7.91 (dd, $J$ = 8.5, 2.2 Hz, 1H), 7.74 (d, $J$ = 8.4 Hz, 1H), 6.96-6.67 (m, 2H), 6.31 (s, 1H), 6.09 (s, 1H), 3.85-3.68 (m, 4H), 3.66-3.49 (m, 4H), 2.56 (s, 3H), 2.54 (s, 1H), 2.33 (s, 3H), 2.26 (s, 3H), 2.19 (s, 3H), 1.60 (d, $J$ = 8.5 Hz, 1H).

**Example 46: 2-(6-(6-((6-(5-isopropyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 12)**

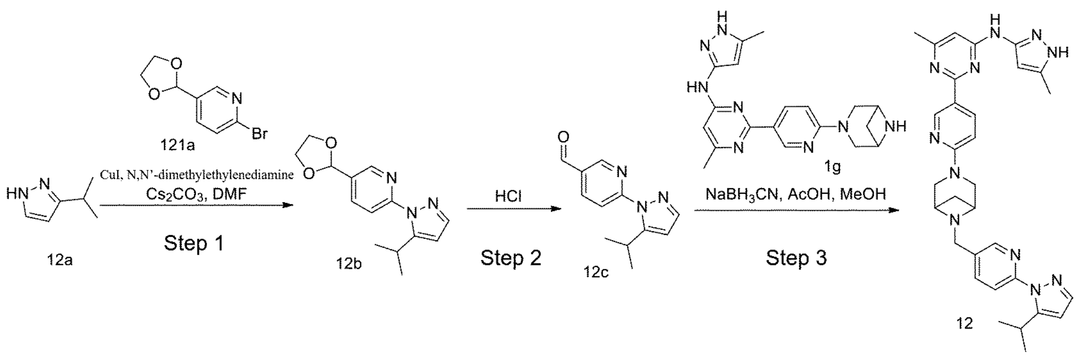


**Step 1: Preparation of 5-(1,3-dioxolan-2-yl)-2-(5-isopropyl-1H-pyrazol-1-yl)pyridine (Compound 12b)**

Compound 12a (210.68 mg), Compound 121a (400 mg), N,N'-dimethylethylenediamine (153.27 mg), cesium carbonate (1.13 g), and CuI (331.13 mg) were successively added into DMF (10 mL). The mixture was heated to 110 °C, and stirred at this temperature for 3 h. The reaction mixture was cooled to room temperature, diluted with water (30 mL), and extracted with DCM (50 mL × 2). The organic phases were combined, washed with water and saturated brine, dried over anhydrous sodium sulfate, filtered, concentrated under reduced pressure, and separated and purified by flash silica gel column chromatography (PE:EA=55:45), to provide Compound 12b (300 mg). MS m/z (ESI): 260.2 $[M+H]^+$.

**Step 2: Preparation of 6-(5-isopropyl-1H-pyrazol-1-yl)nicotinaldehyde (Compound 12c)**

Compound 12b (300.00 mg) was added into a mixed solution of hydrogen chloride (4 N, 6 mL) in 1,4-dioxane and DCM (4 mL). The mixture was stirred at room temperature for 2 h. The reaction mixture was concentrated under reduced pressure, and purified by flash column chromatography (PE:EA=80:20) to provide Compound 12c (120 mg). MS m/z (ESI): 216.2 $[M+H]^+$.

**Step 3: Preparation of 2-(6-(6-((6-(5-isopropyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 12)**

Compound 12c (26.73 mg), Compound 1g (30 mg), and sodium cyanoborohydride (26.01 mg) were successively added into a mixed solvent of methanol (1 mL) and acetic acid (0.1 mL). The mixture was heated to 40 °C, and stirred at this temperature for 16 h. A saturated aqueous solution of ammonium chloride (0.1 mL) was added to quench the reaction. The reaction mixture was concentrated, and separated and purified by Prep-HPLC to provide trifluoroacetate of Compound 12 (15 mg), which was further separated by MPLC to provide Compound 12 (8 mg) in a free state. MS m/z (ESI): 562.3 $[M+H]^+$.

MPLC conditions:

Instrument model: Biotage Isolera Prime 2.3.1, chromatographic column: Agela Technologies C18 spherical 20-35 um 100A, 12 g; chromatographic column temperature: 25 °C; flow rate: 15.0 mL/min; detection wavelength: 254 nm; eluent gradient: (0 min: 0% A, 100% B; 3.0 min: 0% A, 100% B; 20 min: 80% A, 20% B); mobile phase A: 100% acetonitrile; mobile phase B: 0.5% aqueous solution of ammonium bicarbonate; compound collection time: 10.4 min-11.8 min.

$^1$H NMR (400 MHz, DMSO-$d_6$) $\delta$ 11.97 (s, 1H), 9.65 (s, 1H), 9.16-9.09 (m, 1H), 8.49-8.40 (m, 2H), 8.36 (d, $J$ = 1.9 Hz, 1H), 7.93 (dd, $J$ = 8.5, 2.3 Hz, 1H), 7.81 (dd, $J$ = 8.4, 0.7 Hz, 1H), 6.78 (d, $J$ = 9.0 Hz, 2H), 6.42 (d, $J$ = 2.5 Hz, 1H), 6.33 (s, 1H), 3.83-3.68 (m, 4H), 3.66-3.50 (m, 4H), 2.99 (p, $J$ = 6.9 Hz, 1H), 2.59-2.53 (m, 1H), 2.33 (s, 3H), 2.26 (s, 3H), 1.60 (d, $J$ = 8.4 Hz, 1H), 1.27 (s, 3H), 1.25 (s, 3H).

**Example 47: 2-(6-(6-(4-(5,6-dihydrocyclopenteno[c]pyrazol-2(4H)-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 25)**

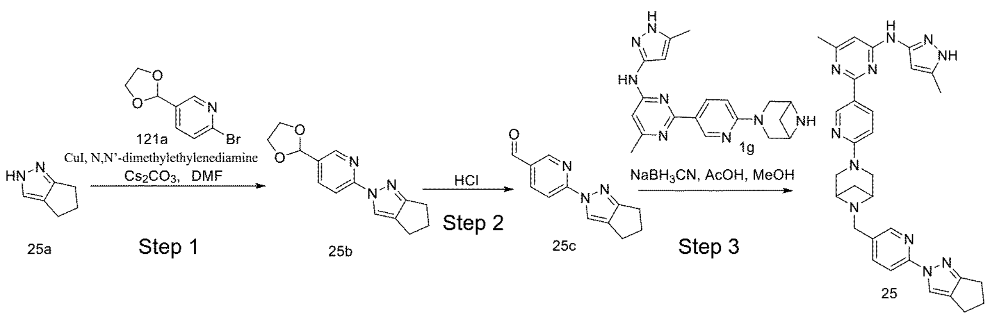


**Step 1: Preparation of 2-(5-(1,3-dioxolan-2-yl)pyridin-2-yl)-2,4,5,6-tetrahydrocyclopeneno[c]pyrazole (Compound 25b)**

Compound 25a (206.83 mg), Compound 121a (400 mg), N,N'-dimethylethylenediamine (153.27 mg), cesium carbonate (1.13 g), and CuI (331.13 mg) were successively added into DMF (10 mL). The mixture was heated to 110 °C, and stirred at this temperature for 12 h. The reaction mixture was cooled to room temperature, diluted with water (50 mL), and extracted with DCM (50 mL×2). The organic phases were combined, washed with water and saturated brine, dried over anhydrous sodium sulfate, filtered, and then concentrated under reduced pressure, to provide Compound 25b (525 mg). MS (ESI, m/z): 258.2 $[M+H]^+$.

**Step 2: Preparation of 6-(5,6-dihydrocyclopeneno[c]pyrazol-2(4H)-yl)nicotinaldehyde (Compound 25c)**

Compound 25b (315 mg) was added into a mixed solution of hydrogen chloride (4 N, 10 mL) 1,4-dioxane solution and DCM (10 mL). The mixture was stirred at room temperature for 2 h. The reaction mixture was concentrated under reduced pressure, and purified by flash column chromatography (PE:EA=80:20) to provide Compound 25c (200 mg). MS m/z (ESI): 214.2 $[M+H]^+$.

**Step 3: Preparation of 2-(6-(6-(4-(5,6-dihydrocyclopeneno[c]pyrazol-2(4H)-yl)benzyl)-3,6-diazabicyclo[3.1.1]heptan-3-yl)pyridin-3-yl)-6-methyl-N-(5-methyl-1H-pyrazol-3-yl)pyrimidin-4-amine (Compound 25)**

Compound 25c (29.42 mg), Compound 1g (50 mg), and sodium cyanoborohydride (43.35 mg) were successively added into a mixed solvent of methanol (0.5 mL) and acetic acid (0.05 mL). The mixture was heated to 40 °C, and stirred at this temperature for 16 h. A saturated aqueous solution of ammonium chloride (0.1 mL) was added to quench the reaction. The reaction mixture was concentrated, and separated and purified by Prep-HPLC to provide trifluoroacetate of Compound 25 (25 mg), which was further separated by MPLC to provide Compound 25 (15 mg) in a free state. MS m/z (ESI): 560.3 $[M+H]^+$.

MPLC conditions:

Instrument model: Biotage Isolera Prime 2.3.1, chromatographic column: Agela Technologies C18 spherical 20-35 um 100A, 12 g; chromatographic column temperature: 25 °C; flow rate: 15.0 mL/min; detection wavelength: 254 nm; eluent gradient: (0 min: 0% A, 100% B; 3.0 min: 0% A, 100% B; 20 min: 80% A, 20% B); mobile phase A: 100% acetonitrile; mobile phase B: 0.5% aqueous solution of ammonium bicarbonate; compound collection time: 11.4 min-12.8 min.

$^1H$ NMR (400 MHz, DMSO-$d_6$) δ 11.97 (s, 1H), 9.65 (s, 1H), 9.15-9.10 (m, 1H), 8.44 (dd, *J* = 8.9, 2.3 Hz, 1H), 8.35-8.30 (m, 1H), 8.19 (d, *J* = 1.2 Hz, 1H), 7.89 (dd, *J* = 8.5, 2.3 Hz, 1H), 7.76 (dd, *J* = 8.4, 0.7 Hz, 1H), 6.78 (d, *J* = 9.0 Hz, 2H), 6.31 (s, 1H), 3.82-3.69 (m, 4H), 3.66-3.50 (m, 4H), 2.74-2.62 (m, 4H), 2.57-2.54 (m, 2H), 2.38 (q, *J* = 7.4 Hz, 2H), 2.33 (s, 3H), 2.26 (s, 3H), 1.59 (d, *J* = 8.4 Hz, 1H).

**Example 48: (6-(4-fluoro-1H-pyrazol-1-yl)pyridin-3-yl)(3-(5-(4-methyl-6-((5-methyl-1H-pyrazol-3-yl)-amino)pyrimidin-2-yl)pyridin-2-yl)-3,6-diazabicyclo [3.1.1] heptan-6-yl)methanone (Compound 26)**

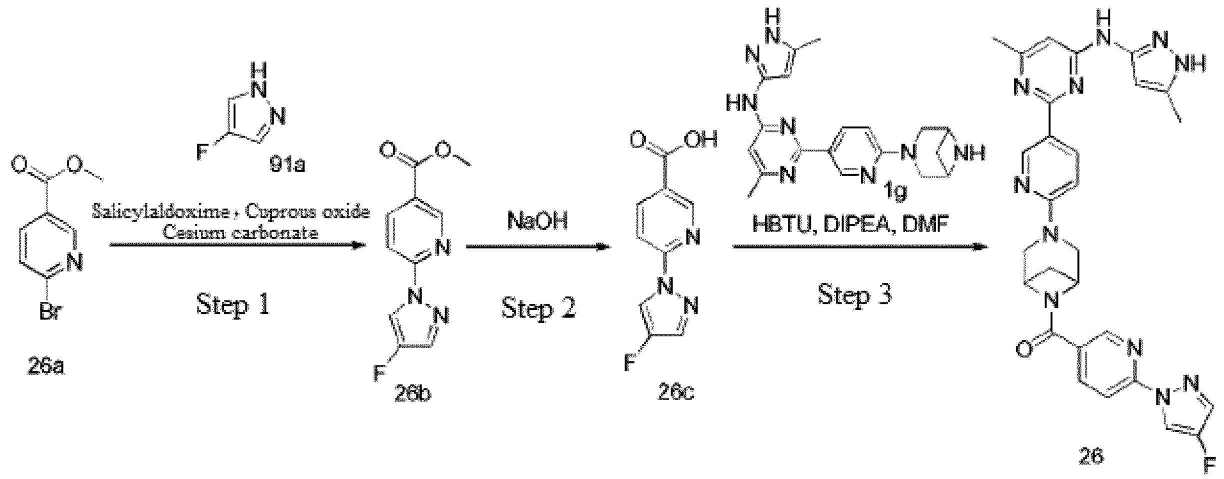


**Step 1: Preparation of methyl 6-(4-fluoro-1H-pyrazol-1-yl)nicotinate (Compound 26b)**

Compound 26a (1 g) and Compound 91a (478.08 mg) were added into acetonitrile (20 mL), and then salicylaldoxime (129.96 mg), cesium carbonate (3.77 g), and cuprous oxide (132.47 mg) were successively added. The mixture was heated to 85 °C, and stirred under the protection of nitrogen at this temperature for 16 h. After completion of the reaction, diluted hydrochloric acid was added into the reaction mixture to adjust the pH to about 5. Silica gel was added for blending with samples, which were separated and purified by silica gel column chromatography (DCM:MeOH=20:1) to provide Compound 26b (602 mg). MS m/z (ESI): 221.9 $[M+H]^+$.

**Step 2: Preparation of 6-(4-fluoro-1H-pyrazol-1-yl)nicotinic acid (Compound 26c)**

Compound 26b (580 mg) was added into THF (10 mL) and $H_2O$ (5 mL), and then NaOH (314.66 mg) was added. The mixture was stirred at 25 °C for 14 h. After completion of the reaction, diluted hydrochloric acid was added into the reaction mixture to adjust the pH to about 5. The reaction mixture was extracted with EA (80 mL x 3). The organic

phases were combined, washed with saturated brine, dried over anhydrous sodium sulfate, filtered, and then concentrated, to provide Compound 26c (312 mg). MS m/z (ESI): 208.1 [M+H]$^+$.

**Step 3: (6-(4-fluoro-1H-pyrazol-1-yl)pyridin-3-yl)(3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methanone (Compound 26)**

Compound 26c (20 mg) was added into DMF (5 mL), and then HBTU (29.41 mg), DIPEA (37.43), and trifluoroacetate of Compound 1g (50.60 mg) were successively added. The mixture was stirred at 25 °C for 1 h. Water (25 mL) was added into the reaction mixture to quench the reaction. The reaction mixture was extracted with EA (80 mL×3). The organic phases were combined, washed with saturated brine, dried over anhydrous sodium sulfate, filtered, concentrated, and separated and purified by Prep-HPLC, to provide Compound 26 (25 mg). MS m/z (ESI): 551.8 [M+H]$^+$.

$^1$H NMR (400 MHz, DMSO) δ 11.97 (s, 1H), 9.65 (s, 1H), 9.04 (s, 1H), 8.77-8.73 (m, 2H), 8.38 (dd, *J*= 8.8, 2.0 Hz, 1H), 8.25 (dd, *J*= 8.4, 2.0 Hz, 1H), 8.03 (d, *J*= 4.4 Hz, 1H), 7.97 (d, *J*= 8.8 Hz, 1H), 7.05-6.47 (m, 2H), 6.27 (br, 1H), 4.96 (s, 1H), 4.67 (s, 1H), 4.17 (d, *J*= 9.6 Hz, 1H), 3.80-3.65 (m, 2H), 3.63-3.50 (m, 1H), 2.92-2.82 (m, 1H), 2.31 (s, 3H), 2.24 (s, 3H), 1.72 (d, *J* = 8.4 Hz, 1H).

**Example 49: 2-(6-(6-((6-(5-cyclobutoxy-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazo1-3-yl)pyrimidin-4-amine (Compound 27)**

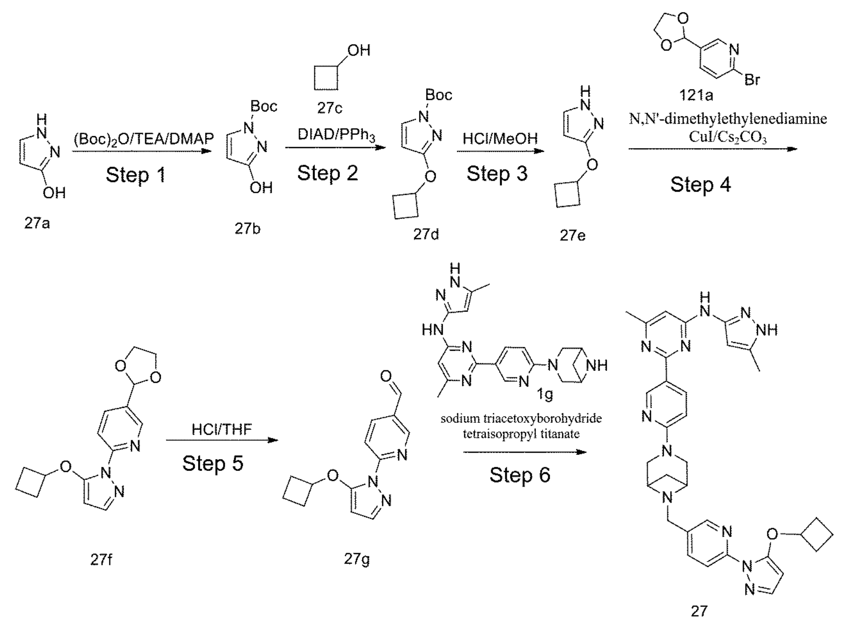


**Step 1: Preparation of tert-butyl 3-hydroxyl-1H-pyrazole-1-carboxylate (Compound 27b)**

Compound 27a (500 mg), $Boc_2O$ (1.30 g), TEA (1.81 g), and DMAP (145.31 mg) were successively added into a reaction flask, and then THF (20 mL) was added. The mixture was stirred at room temperature for 16 h, such that the yellow turbid reaction mixture gradually became clear. After completion of the reaction, the mixture was directly concentrated under reduced pressure, and separated and purified by flash silica gel column chromatography (DCM:MeOH=20:1) to provide Compound 27b (425 mg).

**Step 2: Preparation of tert-butyl 3-cyclobutoxy-1H-pyrazole-1-carboxylate (Compound 27d)**

Compound 27b (360 mg), Compound 27c (215.71 mg), and $PPh_3$ (776.71 mg) were dissolved in toluene (10 mL), and cooled in an ice water bath. DIAD (628.74 mg) was added, and the mixture was heated under the protection of nitrogen 头 110 °C and reacted for 6 h. After completion of the reaction, the reaction mixture was cooled to room temperature, diluted with water (30 mL), and extracted with EA. The organic phases were combined, washed with saturated brine, dried over anhydrous sodium sulfate, filtered, concentrated to dryness under reduced pressure, and

separated and purified by flash silica gel column chromatography (EA:PE=1:20), to provide Compound 27d (374 mg).

**Step 3: Preparation of 3-cyclobutoxy-1H-pyrazole (Compound 27e)**

Compound 27d (374 mg) was dissolved in methanol (3 mL), and then a solution of hydrogen chloride in 1,4-dioxane (4 N, 3 mL) was added. The reaction mixture was kept for reaction under the protection of nitrogen at room temperature. After completion of the reaction, the reaction mixture was concentrated to dryness under reduced pressure to provide hydrochloride of Compound 27e (280 mg). MS m/z (ESI): 139.1 $[M+H]^+$.

**Step 4: Preparation of 2-(5-cyclobutoxy-1H-pyrazol-1-yl)-5-(1,3-dioxolan-2-yl)pyridine (Compound 27f)**

Compound 121a (315 mg), hydrochloride of Compound 27e (239 mg), $Cs_2CO_3$ (675.94 mg), and DMF (10 mL) were added into a reaction flask, and fully stirred, and then N,N'-dimethylethylenediamine (48.77 mg) and CuI (52.68 mg, 273.84 μmol) were added. The mixture was heated to 100 °C, and kept for reaction under the protection of nitrogen at this temperature for 14 h. After completion of the reaction, the reaction mixture was cooled to room temperature, diluted with water (30 mL), and extracted with EA (40 mL×3). The organic phases were combined, washed with saturated brine, dried over anhydrous sodium sulfate, filtered, concentrated to dryness under reduced pressure, and separated and purified by flash silica gel column chromatography (EA:PE=1:1), to provide Compound 27f (300 mg). MS m/z (ESI): 287.9 $[M+H]^+$.

**Step 5: Preparation of 6-(5-cyclobutoxy-1H-pyrazol-1-yl)nicotinaldehyde (Compound 27g)**

Compound 27f (300 mg) was dissolved in THF (5 mL), and then hydrochloric acid (2 N, 5 mL) was added into the solution. The mixture was kept for reaction at room temperature for 8 h. After completion of the reaction, the reaction mixture was diluted with water (20 mL), adjusted with a saturated aqueous solution of $NaHCO_3$ to a pH from 7 to 8, and extracted with EA (40 mL×3). The organic phases were combined, washed with saturated brine, dried over anhydrous sodium sulfate, filtered, concentrated to dryness under reduced pressure, and separated and purified by flash silica gel column chromatography (EA:PE=1:15), to provide Compound 27g (140 mg). MS m/z (ESI): 243.9 $[M+H]^+$.

**Step 6: Preparation of 2-(6-(6-((6-(5-cyclobutoxy-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 27)**

Compound 1g (65 mg), Compound 27g (50.66 mg), and tetraisopropyl titanate (195.65 mg) were dissolved in dry THF (3 mL), and the mixture was heated to 75 °C, and kept for reaction at this temperature for 1 h. Sodium triacetoxyborohydride (182.37 mg) was added into a reaction flask, and the mixture was kept for reaction at 75 °C for 18 h. After completion of the reaction, the reaction mixture was cooled to room temperature, diluted with water (30 mL), and extracted with EA (20 mL×3). The organic phases were combined, washed with saturated brine, dried over anhydrous sodium sulfate, filtered, concentrated to dryness under reduced pressure, and purified by Prep-HPLC, to provide Compound 27 (20 mg). MS m/z (ESI): 589.9 $[M+H]^+$.

$^1H$ NMR (400 MHz, DMSO-$d_6$) δ 11.98 (s, 1H), 9.66 (s, 1H), 9.13 (d, *J*= 2.4 Hz, 1H), 8.44 (dd, *J* = 8.8, 2.4 Hz, 1H), 8.40 (d, *J* = 2.4 Hz, 1H), 8.33 (d, *J* = 2.0 Hz, 1H), 7.91 (dd, *J* = 8.4, 2.4 Hz, 1H), 7.66 (d, *J* = 8.4 Hz, 1H), 7.12-6.69 (m, 2H), 6.49-6.14 (m, 1H), 6.02 (d, *J* = 2.8 Hz, 1H), 4.90 (p, *J*= 7.6 Hz, 1H), 3.83-3.73 (m, 2H), 3.73-3.66 (m, 2H), 3.65-3.47 (m, 4H), 2.60-2.53 (m, 1H), 2.46-2.38 (m, 2H), 2.33 (s, 3H), 2.26 (s, 3H), 2.13-2.03 (m, 2H), 1.82-1.73 (m, 1H), 1.67-1.56 (m, 2H).

**Example 50: 2-(6-(6-((6-(4-chloro-3-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine and 2-(6-(6-((6-(4-chloro-5-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 28/Compound 28')**

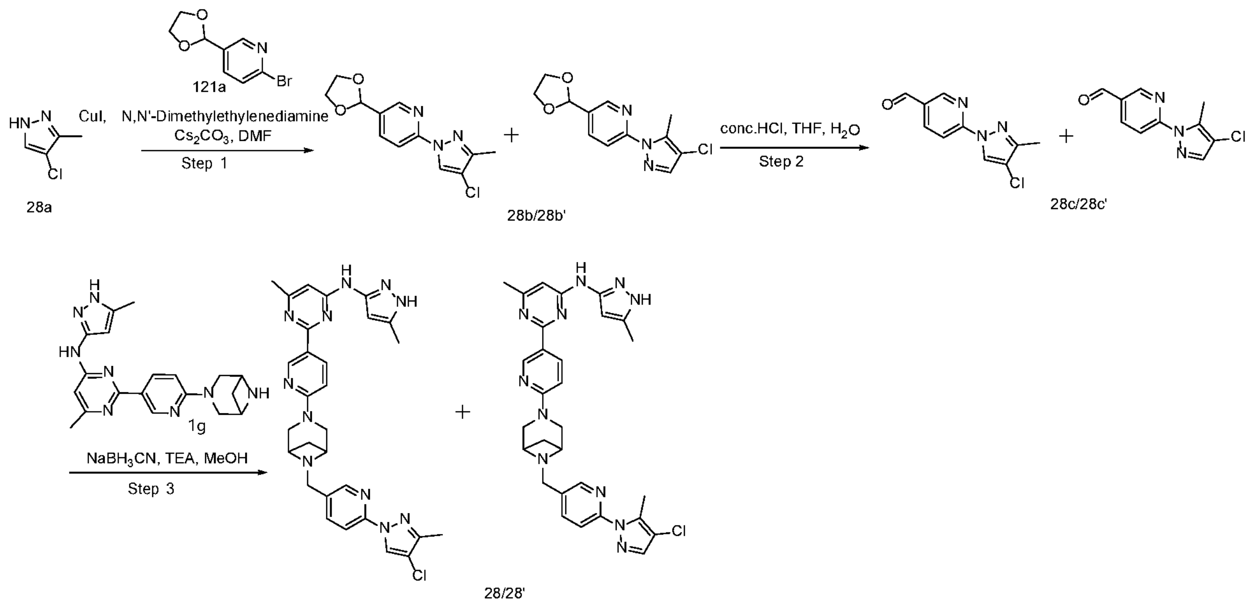


**Step 1: Preparation of 2-(4-chloro-3-methyl-1H-pyrazol-1-yl)-5-(1,3-dioxolan-2-yl)pyridine and 2-(4-chloro-5-methyl-1H-pyrazol-1-yl)-5-(1,3-dioxolan-2-yl)pyridine (Compound 28b/Compound 28b')**

Compound 121a (202.0 mg), Compound 28a (102.3 mg), CuI (167.2 mg), N,N'-dimethylethylenediamine (77.3 mg), and $Cs_2CO_3$ (856.1 mg) were dissolved in DMF (5.0 mL) under the protection of nitrogen. The mixture was heated to 120 °C, and kept for reaction at this temperature until the raw materials were fully converted. After completion of the reaction, the reaction mixture was washed with a saturated aqueous solution of sodium carbonate, and extracted with EA (30 mL x 3). The organic phases were combined, dried over anhydrous sodium sulfate, filtered, concentrated, and separated and purified by preparative TLC, to provide a mixture of Compound 28b and Compound 28b' (230.0 mg). MS m/z (ESI): 266.0 $[M+H]^+$.

**Step 2: Preparation of 6-(4-chloro-3-methyl-1H-pyrazol-1-yl)nicotinaldehyde and 6-(4-chloro-5-methyl-1H-pyrazol-1-yl)nicotinaldehyde (Compound 28c/Compound 28c')**

A mixture (230.0 mg) of Compound 28b and Compound 28b' was added into a mixed solvent of concentrated hydrochloric acid (3 mL), THF (10 mL), and water (10 mL), and the mixture was kept for reaction at 25 °C, until the raw materials were fully converted. The reaction mixture was concentrated to dryness under reduced pressure to provide a mixture (39.0 mg) of Compound 28c and Compound 28c'. MS m/z (ESI): 222.1 $[M+H]^+$.

**Step 3: Preparation of 2-(6-(6-((6-(4-chloro-3-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine and 2-(6-(6-((6-(4-chloro-5-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 28/Compound 28')**

A mixture (29.0 mg) of Compound 28c and Compound 28c', and trifluoroacetate of Compound 1g (30.0 mg) were added into methanol (0.5 mL), and then triethylamine (6.4 mg) and sodium cyanoborohydride (19.8 mg) were successively added. The mixture was kept for reaction at room temperature until the raw materials were fully converted. After completion of the reaction, the reaction mixture was concentrated to dryness under reduced pressure, and separated and purified by Prep-HPLC to provide Compound 28 (2.0 mg, collection time 5.6-6.0 min), MS m/z (ESI): 568.2 $[M+H]^+$, and Compound 28' (1.0 mg, collection time 5.0-5.4 min), MS m/z (ESI): 568.2 $[M+H]^+$.

$^1H$ NMR (400 MHz, DMSO-$d_6$) δ 11.98 (s, 1H), 9.64 (s, 1H), 9.13 (d, *J*= 2.0 Hz, 1H), 8.69 (s, 1H), 8.45 (d, *J*= 8.0 Hz, 1H), 8.40 (s, 1H), 7.98 (d, *J*= 8.8 Hz, 1H), 7.82 (d, *J*= 8.4 Hz, 1H), 6.89 (d, *J*= 8.8 Hz, 1H), 6.76-6.67 (m, 1H), 6.38-6.23 (m, 1H), 3.80-3.55 (m, 8H), 2.62-2.58 (m, 1H), 2.34 (s, 3H), 2.28 (s, 3H), 2.27 (s, 3H), 1.61 (d, *J*= 8.0 Hz, 1H) (Compound 28).

$^1H$ NMR (400 MHz, DMSO-$d_6$) δ 12.03 (s, 1H), 9.68 (s, 1H), 9.18 (d, *J*= 2.4 Hz, 1H), 8.51 (d, *J* = 2.4 Hz, 1H), 8.48 (d, *J* = 2.4 Hz, 1H), 8.05 (dd, *J* = 8.4, 2.4 Hz, 1H), 7.90 (s, 1H), 7.83 (d, *J* = 8.4 Hz, 1H), 6.85 (d, *J*= 9.2 Hz, 1H), 6.82-6.69 (m, 1H), 6.43-6.29 (m, 1H), 3.86-3.56 (m, 8H), 2.63 (s, 3H), 2.62-2.58 (m, 1H), 2.39 (s, 3H), 2.31 (s, 3H), 1.66

(d, $J$ = 8.4 Hz, 1H) (Compound 28').

**Example 51: 2-(6-(4-((5-fluoropyridin-3-yl)oxy)piperidin-1-yl)pyridin-3-yl)-6-methyl-N-(5-methyl-1H-pyrazol-3-yl)pyrimidin-4-amine (Compound 29)**

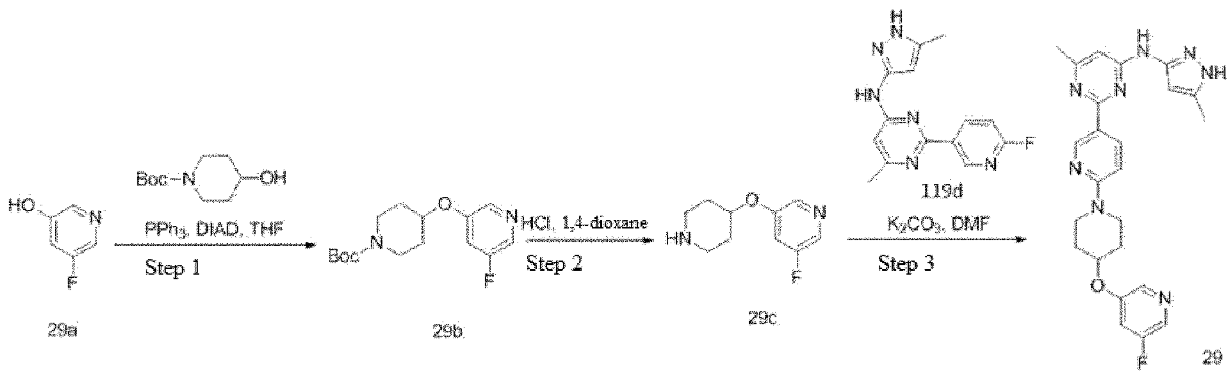


Step 1: **Preparation of tert-butyl 4-((5-fluoropyridin-3-yl)-oxy)piperidine-1-carboxylate (Compound 29b)**

Under the protection of nitrogen, Compound 29a (425.6 mg), N-Boc-4-hydroxypiperidine (505.0 mg), and $PPh_3$ (1.3 g) were dissolved in dry THF (5.0 mL), and cooled to 0 °C. DIAD (1.0 g) was added dropwise. Then, the mixture was slowly warmed to 25 °C, and kept for reaction at this temperature, until the raw materials were fully converted. After completion of the reaction, the reaction mixture was concentrated to dryness under reduced pressure, and separated and purified by column chromatography to provide Compound 29b (736.2 mg). MS m/z (ESI): 297.1 $[M+H]^+$.

**Step 2: Preparation of hydrochloride of 3-fluoro-5-(piperidin-4-yloxy)pyridine (Compound 29c)**

Compound 29b (555.5 mg) was added into a solution of hydrogen chloride in 1,4-dioxane (4 N, 20 mL). The mixture was kept for reaction at 25 °C, until the raw materials were fully converted. The reaction mixture was concentrated to dryness under reduced pressure to provide hydrochloride of Compound 29c (390.0 mg). MS m/z (ESI): 197.1 $[M+H]^+$.

**Step 3: Preparation of 2-(6-(4-((5-fluoropyridin-3-yl)oxy)piperidin-1-yl)pyridin-3-yl)-6-methyl-N-(5-methyl-1H-pyrazol-3-yl)pyrimidin-4-amine (Compound 29)**

Hydrochloride of Compound 29c (86.0 mg), Compound 119d (55.5 mg), and $K_2CO_3$ (122.7 mg) were added into DMF (5 mL), heated to 125 °C, and kept for reaction at this temperature until the raw materials were fully converted. After completion of the reaction, the reaction mixture was washed with a saturated aqueous solution of sodium carbonate, and extracted with EA (10 mL x 3). The organic phases were combined, dried over anhydrous sodium sulfate, filtered, concentrated, and separated and purified by Prep-HPLC, to provide Compound 29 (17.0 mg). MS m/z (ESI): 460.9 $[M+H]^+$.

$^1H$ NMR (400 MHz, DMSO-$d_6$) δ 12.41 (s, 1H), 11.12 (s, 1H), 8.97 (d, $J$ = 2.4 Hz, 1H), 8.31 (dd, $J$= 9.2, 2.4 Hz, 1H), 8.25 (t, $J$ = 1.6 Hz, 1H), 8.20 (d, $J$= 2.4 Hz, 1H), 7.59 (dt, $J$= 11.6, 2.4 Hz, 1H), 7.14 (d, $J$= 9.2 Hz, 1H), 6.90-6.80 (m, 2H), 4.87-4.81 (m, 1H), 4.19-4.13 (m , 2H), 3.85-3.73 (m, 2H), 2.47 (s, 3H), 2.28 (s, 3H), 2.11-2.05 (m, 2H), 1.72-1.63 (m, 2H).

**Example 52: 2-(6-(6-((*R*)-1-(6-(4-fluoro-1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine and 2-(6-(6-((*S*)-1-(6-(4-fluoro-1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 52-1/Compound 52-2)**

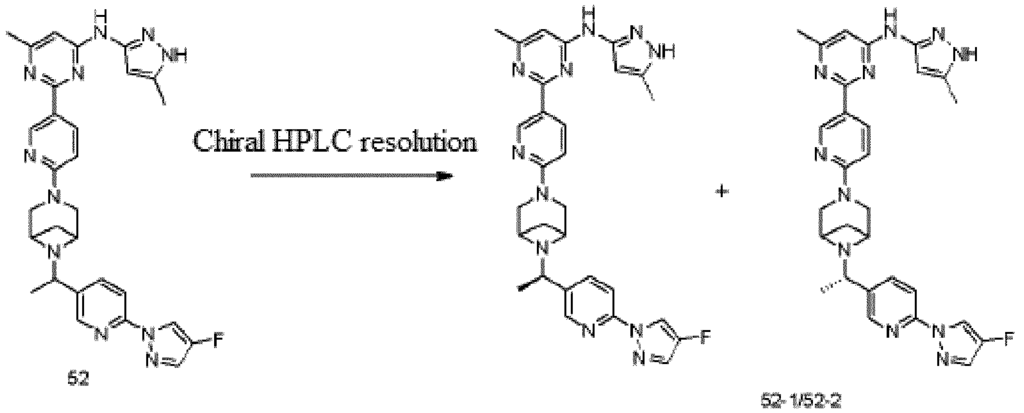


52

52-1/52-2

Compound 52 (100 mg) was resolved by chiral Prep-HPLC to provide Compound 52-1 (retention time 6.788 min) and Compound 52-2 (retention time 10.115 min). The two compounds were distinguished and defined based on the retention time of chiral resolution. Thus, Compound 52-1 (14 mg, ee: 98.85%), MS m/z (ESI): 552.3 $[M+H]^+$; and Compound 52-2 (20 mg, ee: 99.22%), MS m/z (ESI): 552.3 $[M+H]^+$ were given.

Chiral HPLC resolution conditions:

Instrument model: Shimadzu LC-20AD; chromatographic column: CHIRALPAK IE (IE00CD-RH008), 0.46 cm I.D. × 15 cm L; chromatographic column temperature: 35 °C; flow rate: 1.0 mL/min; detection wavelength: 254 nm; mobile phase: MeOH:CAN:DEA=80:20:0.1 (V/V/V); appearance time of Compound 52-1: 6.788 min, appearance time of Compound 52-2: 10.115 min.

$^1$H NMR (400 MHz, DMSO-$d_6$) δ 11.97 (s, 1H), 9.65 (s, 1H), 9.11 (d, *J*= 2.0 Hz, 1H), 8.66 (d, *J* = 4.6 Hz, 1H), 8.43 (d, *J* = 2 Hz, 1H), 8.41 (d, *J* = 2.4 Hz, 1H), 8.01 (dd, *J* = 8.5, 1.9 Hz, 1H), 7.90 (dd, *J* = 16.8, 6.4 Hz, 2H), 6.82(br, 1H), 6.75(d, *J* = 8.8 Hz, 1H), 6.29(br, 1H), 3.95-3.81 (m, 2H), 3.77 (q, *J* = 6.1 Hz, 1H), 3.63 (s, 1H), 3.53-3.38 (m, 3H), 2.55-2.53 (m, 1H), 2.32 (s, 3H), 2.25 (s, 3H), 1.55 (d, *J*= 8.4 Hz, 1H), 1.22 (d, *J*= 6.2 Hz, 3H). (Compound 52-1)

$^1$H NMR (400 MHz, DMSO-$d_6$) δ 11.97 (s, 1H), 9.65 (s, 1H), 9.11 (d, *J*= 2.0 Hz, 1H), 8.66 (d, *J* = 4.6 Hz, 1H), 8.43 (d, *J* = 2 Hz, 1H), 8.41 (d, *J* = 2.4 Hz, 1H), 8.00 (dd, *J* = 8.5, 1.9 Hz, 1H), 7.89 (dd, *J* = 16.8, 6.4 Hz, 2H), 6.82(br, 1H), 6.75(d, *J* = 8.8 Hz, 1H), 6.27(br, 1H), 3.95-3.81 (m, 2H), 3.76 (q, *J*= 6.1 Hz, 1H), 3.62 (s, 1H), 3.53-3.38 (m, 3H), 2.55-2.51 (m, 1H), 2.32 (s, 3H), 2.25 (s, 3H), 1.54 (d, *J*= 8.4 Hz, 1H), 1.21 (d, *J*= 6.2 Hz, 3H). (Compound 52-2)

**Example 53: 2-(6-(6-((6-(5-cyclopropoxy-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 30)**

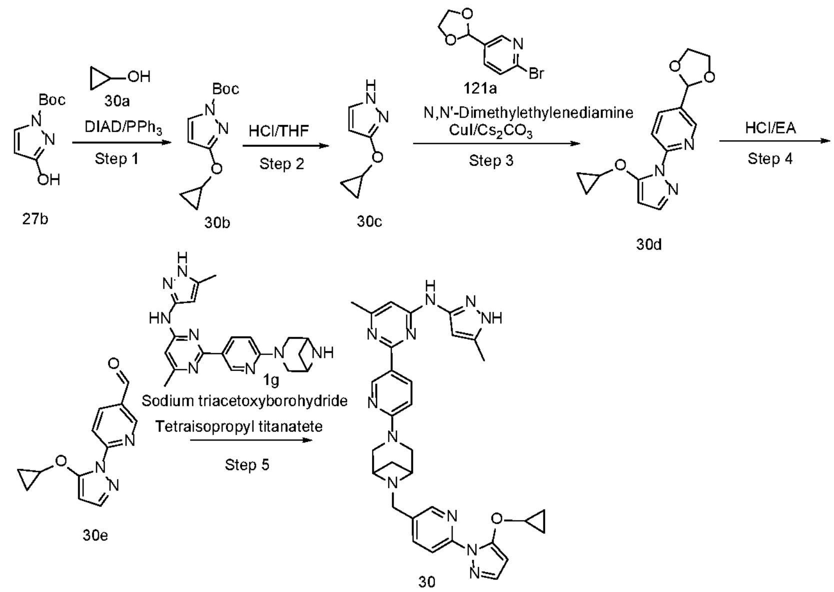


**Step 1: Preparation of tert-butyl 3-cyclopropoxy-1H-pyrazole-1-carboxylate (Compound 30b)**

Compound 27b (300 mg), Compound 30a (141.89 mg), and triphenylphosphine (640.09 mg) were added into toluene (10 mL), and cooled to 0 °C. DIAD (493.51 mg) was added dropwise. The mixture was heated to 110 °C, and kept for reaction at this temperature for 6 h. The reaction mixture was cooled to room temperature, concentrated under reduced pressure, and purified by flash silica gel column chromatography (PE:EA=23:77) to provide Compound 30b (60 mg).

**Step 2: Preparation of 3-cyclopropoxy-1H-pyrazole (Compound 30c)**

Compound 30b (102 mg) was added into a mixed solution of hydrogen chloride (4 N, 2 mL) in 1,4-dioxane and THF (2 mL). The mixture was stirred at room temperature for 16 h. The reaction mixture was concentrated under reduced pressure to provide hydrochloride of Compound 30c (73 mg). MS m/z (ESI): 125.1 $[M+H]^+$.

**Step 3: Preparation of 2-(5-cyclopropoxy-1H-pyrazol-1-yl)-5-(1,3-dioxolan-2-yl)pyridine (Compound 30d)**

Hydrochloride of Compound 30c (69.81 mg), Compound 121a (100 mg), N,N'-dimethylethylenediamine (38.32 mg), cesium carbonate (424.87 mg), and CuI (82.78 mg) were successively added into DMF (10 mL). The mixture was heated to 110 °C, and stirred at this temperature for 3 h. The reaction mixture was cooled to room temperature, diluted with water (30 mL), and extracted with DCM (50 mL × 2). The organic phases were combined, washed with water and saturated brine, dried over anhydrous sodium sulfate, filtered, concentrated under reduced pressure, and separated and purified by Prep-HPLC, to provide Compound 30d (80 mg). MS m/z (ESI): 273.9 $[M+H]^+$.

**Step 4: Preparation of 6-(5-cyclopropoxy-1H-pyrazol-1-yl)nicotinaldehyde (Compound 30e)**

Compound 30d (80 mg) was added into a mixed solution of hydrogen chloride (4 N, 3 mL) in 1,4-dioxane and EA (2 mL). The mixture was stirred at room temperature for 2 h. The reaction mixture was concentrated under reduced pressure, diluted with DCM (30 mL), washed with a saturated aqueous solution of sodium bicarbonate and saturated brine, dried over anhydrous sodium sulfate, filtered, and then concentrated under reduced pressure, to provide Compound 30e (55 mg). MS m/z (ESI): 229.9 $[M+H]^+$.

**Step 5: Preparation of 2-(6-(6-((6-(5-cyclopropoxy-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 30)**

Dry THF (2 mL) was added into Compound 30e (40 mg), Compound 1g (40 mg), and tetraisopropyl titanate (26.01 mg) present in a 5 mL reaction flask. The mixture was heated to 75 °C, and stirred at this temperature for 16 h. Then, sodium triacetoxyborohydride (26.01 mg) was added, and the mixture was stirred at 75 °C for 8 h. The reaction mixture was cooled to room temperature, and a saturated aqueous solution of ammonium chloride (0.1 mL) was added

to quench the reaction. The reaction mixture was concentrated, and pre-purified by preparative TLC (DCM:MeOH=10:1) to provide 25 mg of crude product ($R_f$=0.35-0.45), which was further separated and purified by Prep-HPLC to provide Compound 30 (10 mg). MS m/z (ESI): 575.9 $[M+H]^+$.

$^1H$ NMR (400 MHz, DMSO-$d_6$) δ 11.95 (s, 1H), 9.62 (s, 1H), 9.12 (d, *J* = 2.3 Hz, 1H), 8.46-8.41 (m, 2H), 8.34 (d, *J* = 2.2 Hz, 1H), 7.92 (dd, *J* = 8.4, 2.2 Hz, 1H), 7.67 (d, *J* = 8.4 Hz, 1H), 6.78 (d, *J*= 9.0 Hz, 2H), 6.29 (s, 1H), 6.17 (d, *J*= 2.7 Hz, 1H), 4.10 (tt, *J* = 6.0, 3.2 Hz, 1H), 3.84-3.68 (m, 4H), 3.66-3.48 (m, 4H), 2.60-2.54 (m, 1H), 2.33 (s, 3H), 2.26 (s, 3H), 1.59 (d, *J*= 8.4 Hz, 1H), 0.79-0.68 (m, 4H).

**Example 54: 2-(6-(6-((6-(3-(fluoromethyl)-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-**yl)pyrimidin-4-amine (Compound 31)

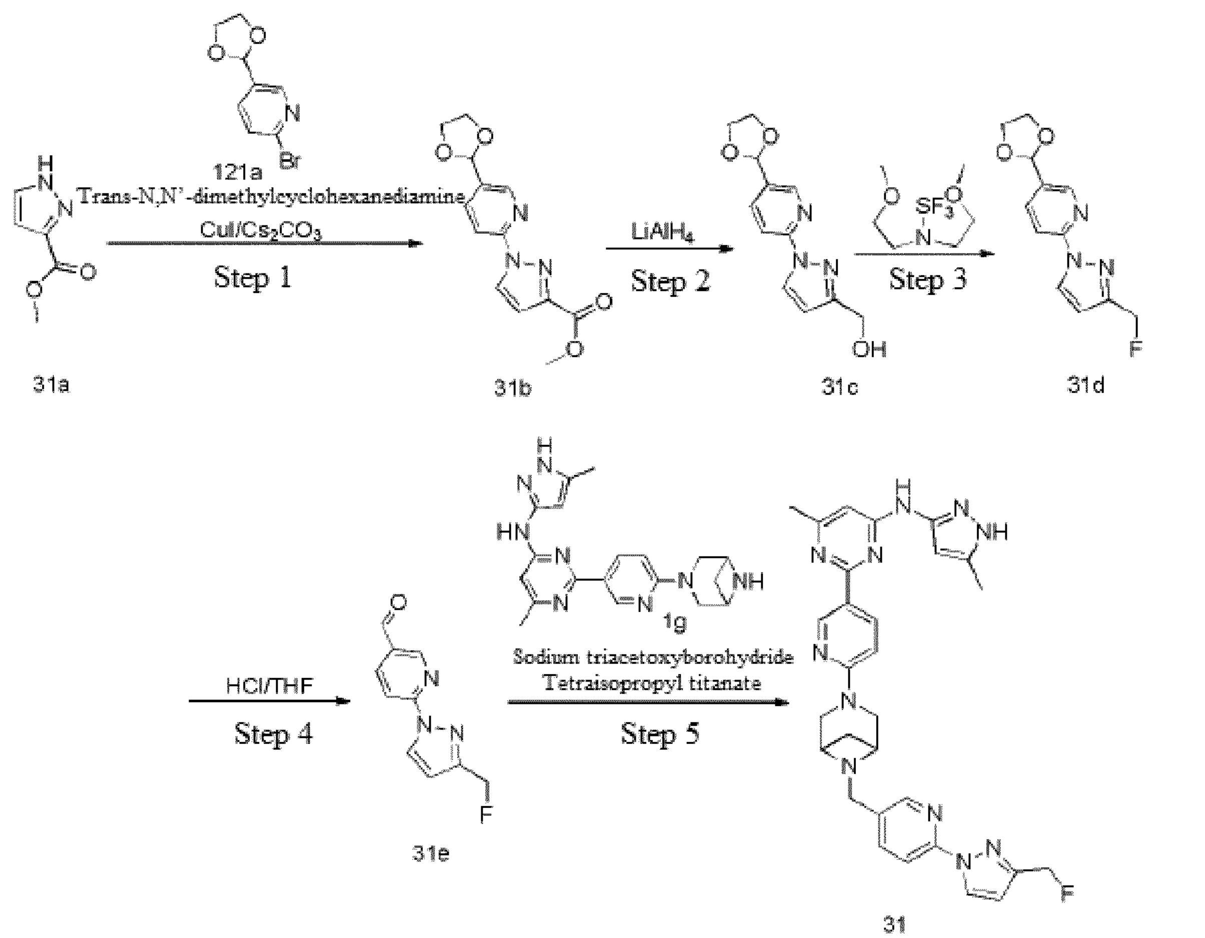


**Step 1: Preparation of methyl 1-(5-(1,3-dioxolan-2-yl)pyridin-2-yl)-1H-pyrazole-3-carboxylate (Compound 31b)**

Compound 121a (2.0 g), Compound 31a (1.12 g), $Cs_2CO_3$ (5.72 g), trans-N,N'-dimethylcyclohexanediamine (504.72 mg), CuI (337.89 mg), and DMF (10 mL) were added into a reaction flask. The mixture was heated to 90 °C, and kept for reaction under the protection of nitrogen at this temperature for 2 h. After completion of the reaction, the reaction mixture was cooled to room temperature, diluted with water (20 mL), and extracted with EA (30 mL×3). The organic phases were combined, washed with water, dried over anhydrous sodium sulfate, filtered, concentrated to dryness under reduced pressure, and separated and purified by flash silica gel column chromatography (EA:PE=20:80), to provide Compound 31b (2.2 g). MS m/z (ESI): 276.0 $[M+H]^+$.

**Step 2: Preparation of (1-(5-(1,3-dioxolan-2-yl)pyridin-2-yl)-1H-pyrazol-3-yl)methanol (Compound 31c)**

Compound 31b (2.2 g) was dissolved in THF (20 mL), and cooled to -20 °C. $LiAlH_4$ (464.31 mg) was slowly added into the reaction mixture portionwise, and the mixture was kept for reaction at this temperature for 15 min. After

completion of the reaction, EA (1 mL) was slowly added dropwise to consume excess $LiAlH_4$. Then, water (1 mL) was added dropwise to quench the reaction. The mixture was diluted with water (20 mL), and extracted with EA (30 mL×3). The organic phases were combined, dried over anhydrous sodium sulfate, filtered, concentrated to dryness under reduced pressure, and separated and purified by flash silica gel column chromatography (EA:PE=50:50), to provide Compound 31c (1.24 g). MS m/z (ESI): 248.0 $[M+H]^+$.

**Step 3: Preparation of 5-(1,3-dioxolan-2-yl)-2-(3-(fluoromethyl)-1H-pyrazol-1-yl)pyridine (Compound 31d)**

Under the protection of nitrogen, dry DCM (20 mL) was cooled to -40 °C, and then bis(2-methoxyethyl)aminosulfur trifluoride (2.86 g) was slowly added dropwise. A solution of Compound 31c (0.8 g) in DCM (20 mL) was added dropwise into the reaction mixture. Then, the reaction mixture was slowly warmed to 25 °C, and kept for reaction at this temperature for 20 h. After completion of the reaction, the reaction mixture was poured into a saturated aqueous solution of sodium bicarbonate, and was, After completion of the release of bubbles, extracted with DCM (20 mL x 3). The organic phases were combined, dried over anhydrous sodium sulfate, filtered, concentrated to dryness under reduced pressure, and separated and purified by flash silica gel column chromatography (EA:PE=20:80), to provide Compound 31d (200 mg). MS m/z (ESI): 249.9 $[M+H]^+$.

**Step 4: Preparation of 6-(3-(fluoromethyl)-1H-pyrazol-1-yl)nicotinaldehyde (Compound 31e)**

Compound 31d (200 mg) was dissolved in THF (5 mL), and then hydrochloric acid (2 N, 2.3 mL) was added into the solution. The mixture was kept for reaction at room temperature for 16 h. After completion of the reaction, the reaction mixture was diluted with water (20 mL), adjusted with a saturated aqueous solution of $NaHCO_3$ to a pH from 7 to 8, directly concentrated, and separated and purified by flash silica gel column chromatography (EA:PE=15:85) to provide Compound 31e (110 mg). MS m/z (ESI): 206.0 $[M+H]^+$.

**Step 5: Preparation of 2-(6-(6-((6-(3-(fluoromethyl)-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 31)**

Compound 1g (87 mg), Compound 31e (110 mg), and tetraisopropyl titanate (278.46 mg) were dissolved in dry THF (10 mL), heated to 75 °C, and kept for reaction at this temperature for 8 h. The reaction mixture was cooled to 25 °C. Sodium triacetoxyborohydride (259.57 mg) was added into a reaction flask, and the mixture was kept for reaction at 25 °C for 12 h. Water (1 mL) was added dropwise to quench the reaction. The mixture was concentrated, pre-purified by flash silica gel column chromatography (DCM:MeOH=90:10), and then separated and purified by Prep-HPLC to provide Compound 31 (25 mg). MS m/z (ESI): 551.8 $[M+H]^+$.

$^1H$ NMR (400 MHz, DMSO-$d_6$) $\delta$ 9.63 (s, 1H), 9.13 (d, *J*= 2.0 Hz, 1H), 8.62 (d, *J*= 2.4 Hz, 1H), 8.49-8.39 (m, 2H), 8.15 (s, 1H), 7.99 (dd, *J*= 8.4, 1.6 Hz, 1H), 7.90-7.84 (m, 1H), 7.07-6.74 (m, 2H), 6.70 (s, 1H), 6.30 (br, 1H), 5.53 (s, 1H), 5.41 (s, 1H), 3.81-3.73 (m, 4H), 3.67-3.58 (m, 4H), 2.63-2.53 (m, 1H), 2.34 (s, 3H), 2.26 (s, 3H), 1.61 (d, *J*= 8.4 Hz, 1H).

**Example 55: 2-(6-(6-((6-(5-ethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 46)**

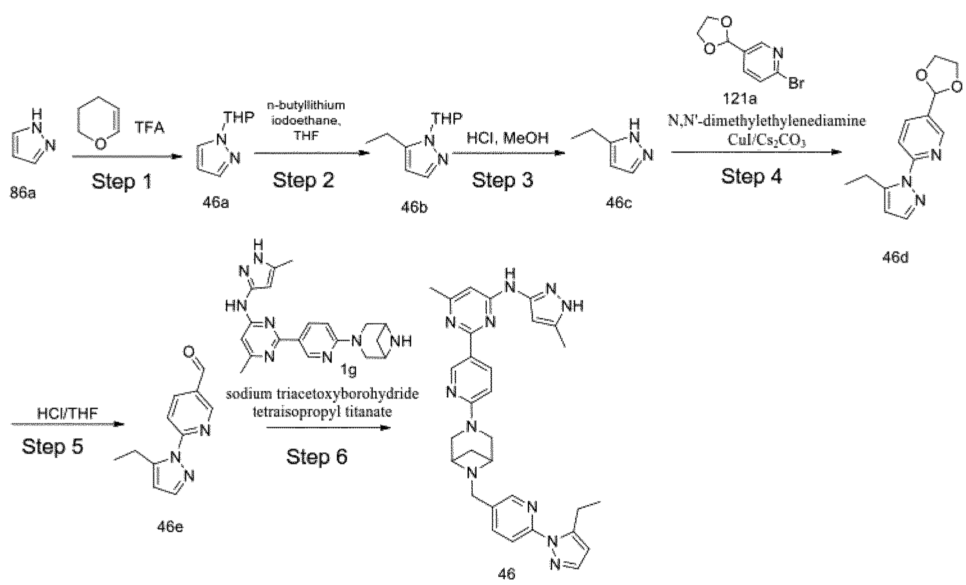


86a, 46a, 46b, 46c, 46d, 46e, 46

**Step 1: Preparation of 1-(tetrahydro-2H-pyran-2-yl)-1H-pyrazole (Compound 46a)**

Compound 86a (2 g) was added into 3,4-dihydropyran (6 mL), and then TFA (334.97 mg) was added. The mixture was heated to 100°C, and stirred at this temperature for 16 h. Water (100 mL) was added into the reaction mixture to quench the reaction. The reaction mixture was extracted with EA (50 mL × 3). The organic phases were combined, washed with saturated brine, dried over anhydrous sodium sulfate, filtered, concentrated, and separated and purified by silica gel column chromatography (PE:EA=60:1-10:1), to provide Compound 46a (2.4 g). MS m/z (ESI): 153.2 $[M+H]^+$.

**Step 2: Preparation of 5-ethyl-1-(tetrahydro-2H-pyran-2-yl)-1H-pyrazole (Compound 46b)**

Compound 46a (2 g) was added into dry THF (30 mL), and then n-butyllithium (2.5 M, 6.31 mL) was added at -78 °C. The mixture was stirred for 1 h. Then, iodoethane (3.07 g) was slowly added at -78°C. The mixture was slowly warmed to room temperature, and stirred at this temperature for 5 h. Methanol (50 mL) was added into the reaction mixture to quench the reaction. Silica gel was directly blended with samples, which were separated and purified by column chromatography (PE:EA=40:1-5:1) to provide Compound 46b (721 mg). MS m/z (ESI): 181.0 $[M+H]^+$.

**Step 3: Preparation of 5-ethyl-1H-pyrazole (Compound 46c)**

Compound 46b (721 mg) was added into MeOH (7 mL), and then a solution of hydrogen chloride in 1,4-dioxane(4 N, 1 mL) was added. The mixture was stirred at 25 °C for 1 h. A saturated aqueous solution of sodium bicarbonate (50 mL) was added into the reaction mixture to quench the reaction. The reaction mixture was extracted with EA (80 mL×3). The organic phases were combined, washed with saturated brine, dried over anhydrous sodium sulfate, and filtered. Then, the organic phases were spin-dried, to provide Compound 46c (185 mg). MS m/z (ESI): 97.1 $[M+H]^+$.

**Step 4: Preparation of 5-(1,3-dioxolan-2-yl)-2-(5-ethyl-1H-pyrazol-1-yl)pyridine (Compound 46d)**

Compound 121a (401 mg) and Compound 46c (184.31 mg) were added into DMF (15 mL), and then N,N-dimethylethylenediamine (153.39 mg), cesium carbonate (1.7 g), and CuI (331.96 mg) were successively added. The mixture was heated to 110 °C, and stirred under the protection of nitrogen at this temperature for 10 h. Water (100 mL) was added into the reaction mixture to quench the reaction. The reaction mixture was extracted with EA (50 mL × 3). The organic phases were combined, washed with saturated brine, dried over anhydrous sodium sulfate, filtered, concentrated, and separated and purified by silica gel column chromatography (PE:EA=40:1-3:1), to provide Compound 46d (199 mg). MS m/z (ESI): 245.9 $[M+H]^+$.

**Step 5: Preparation of 6-(5-ethyl-1H-pyrazol-1-yl)nicotinaldehyde (Compound 46e)**

Compound 46d (200 mg) was added into THF (4 mL) and $H_2O$ (2 mL), and then a solution of hydrogen chloridein 1,4-dioxane (4 N, 1 mL) was added. The mixture was stirred at 25 °C for 5 h. A saturated aqueous solution of sodium bicarbonate (50 mL) was added into the reaction mixture to quench the reaction. The reaction mixture was extracted with EA (50 mL×3). The organic phases were combined, washed with saturated brine, dried over anhydrous sodium sulfate, filtered, concentrated, and separated and purified by silica gel column chromatography (PE:EA=40:1-3:1), to provide Compound 46c (91 mg). MS m/z (ESI): 202.1 $[M+H]^+$.

**Step 6: Preparation of 2-(6-(6-((6-(5-ethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 46)**

Compound 1g (40.93 mg), Compound 46e (25 mg), and tetraisopropyl titanate (128.41 mg) were added into dry THF (10 mL), and were, after nitrogen replacement three times, stirred at 75 °C for 10 h. Then, sodium triacetoxyborohydride (119.69 mg) was added portionwise, and the mixture was stirred at 75 °C for an additional 6 h. After completion of the reaction, the reaction mixture was concentrated to dryness under reduced pressure, and separated and purified by Prep-HPLC to provide Compound 46 (8 mg). MS m/z (ESI): 547.9 $[M+H]^+$.

$^1H$ NMR (400 MHz, DMSO-$d_6$) δ 11.98 (s, 1H), 9.65 (s, 1H), 9.12 (d, *J* = 2.0 Hz, 1H), 8.47 (d, *J* = 2.4 Hz, 1H), 8.44 (dd, *J* = 9.20, 2.4 Hz, 1H), 8.36 (d, *J* = 1.6 Hz, 1H), 7.93 (dd, *J* = 8.4, 2.0 Hz, 1H), 7.81 (d, *J* = 8.4 Hz, 1H), 7.20-6.69 (m, 2H), 6.40 (d, *J* = 2.8 Hz, 1H), 6.32 (s, 1H), 3.83-3.68 (m, 4H), 3.66-3.53 (m, 4H), 2.66 (q, *J*= 7.6 Hz, 2H), 2.61-2.53 (m, 1H), 2.33 (s, 3H), 2.25 (s, 3H), 1.60 (d, *J*= 8.4 Hz, 1H), 1.23 (t, *J*= 7.6 Hz, 3H).

**Example 56: 2-(6-(6-((6-(4-fluoro-5-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 122)**

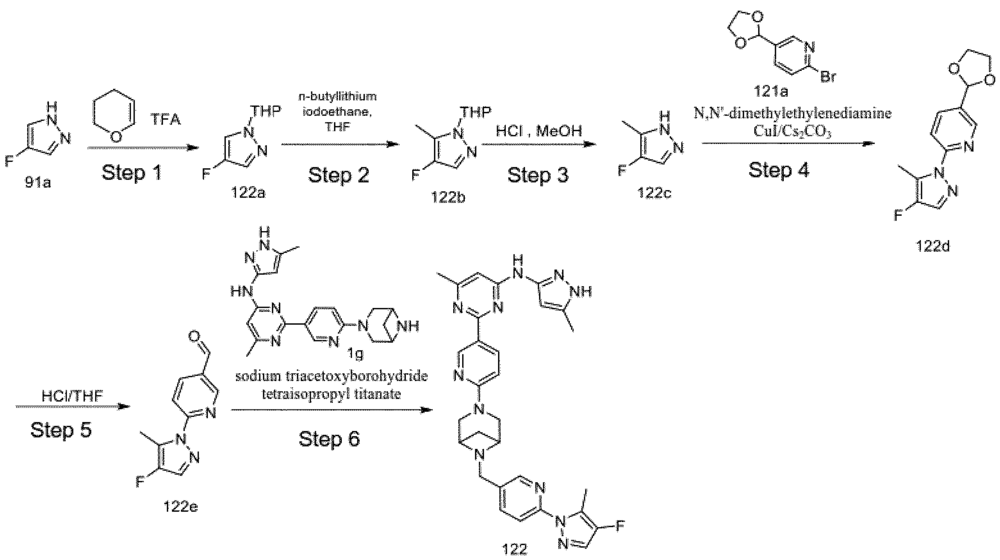


**Step 1: Preparation of 4-fluoro-1-(tetrahydro-2H-pyran-2-yl)-1H-pyrazole (Compound 122a)**

Compound 91a (500 mg) was added into 3,4-dihydropyran (2.5 mL), and then TFA (129.96 mg) was added. The mixture was heated to 100 °C, and stirred at this temperature for 16 h. Water (100 mL) was added into the reaction mixture to quench the reaction. The reaction mixture was extracted with EA (50 mL × 3). The organic phases were combined, washed with saturated brine, dried over anhydrous sodium sulfate, filtered, concentrated, and separated and purified by silica gel column chromatography (PE:EA=50:1-10:1), to provide Compound 122a (901 mg). MS m/z (ESI): 171.1 $[M+H]^+$.

**Step 2: Preparation of 4-fluoro-5-methyl-1-(tetrahydro-2H-pyran-2-yl)-1H-pyrazole (Compound 122b)**

Compound 122a (900 mg) was added into dry THF (10 mL), and then n-butyllithium (2.5 M, 2.33 mL) was

added at -78 °C. The mixture was stirred for 1 h. Then, iodomethane (1.13 g) was slowly added at -78°C. The mixture was slowly warmed to room temperature, and stirred at this temperature for 3 h. Methanol was added into the reaction mixture to quench the reaction. The reaction mixture was extracted with EA (80 ×3). The organic phases were combined, washed with saturated brine, dried over anhydrous sodium sulfate, filtered, concentrated, and separated and purified by silica gel column chromatography (DCM:MeOH=60:1-10:1), to provide Compound 122b (845 mg) as light yellow liquid. MS m/z (ESI): 185.0 $[M+H]^+$.

**Step 3: Preparation of 4-fluoro-5-methyl-1H-pyrazole (Compound 122c)**

Compound 122b (800 mg) was added into MeOH (7 mL), and then a solution of hydrogen chloride (4 N, 7 mL) in 1,4-dioxane was added. The mixture was stirred at 25 °C for 1 h. A saturated aqueous solution of sodium bicarbonate (50 mL) was added into the reaction mixture to quench the reaction. The reaction mixture was extracted with EA (80 mL×3). The organic phases were combined, washed with saturated brine, dried over anhydrous sodium sulfate, filtered, and then concentrated, to provide Compound 122c (300 mg). MS m/z (ESI): 101.2 $[M+H]^+$.

**Step 4: Preparation of 5-(1,3-dioxolan-2-yl)-2-(4-fluoro-5-methyl-1H-pyrazol-1-yl)pyridine (Compound 122d)**

Compound 121a (300 mg) and Compound 122c (143.58 mg) were added into DMF (15 mL), and then N,N-dimethylethylenediamine (114.75 mg), cesium carbonate (1.27 g), and CuI (248.35 mg) were successively added. The mixture was heated to 110 °C, and stirred under the protection of nitrogen at this temperature for 10 h. Water (100 mL) was added into the reaction mixture to quench the reaction. The reaction mixture was extracted with EA (50 mL × 3). The organic phases were combined, washed with saturated brine, dried over anhydrous sodium sulfate, filtered, concentrated, and separated and purified by silica gel column chromatography (PE:EA=50:1-10:1), to provide Compound 122d (254 mg). MS m/z (ESI): 249.9 $[M+H]^+$.

**Step 5: Preparation of 6-(4-(fluoro-5-methyl)-1H-pyrazol-1-yl)nicotinaldehyde (Compound 122e)**

Compound 122d (240 mg) was added into THF (5 mL) and $H_2O$ (2 mL), and then a solution of hydrogen chloride (4 N, 991.74 uL) in 1,4-dioxane was added. The mixture was stirred at 25 °C for 5 h. A saturated aqueous solution of sodium bicarbonate (50 mL) was added into the reaction mixture to quench the reaction. The reaction mixture was extracted with EA (50 mL×3). The organic phases were combined, washed with saturated brine, dried over anhydrous sodium sulfate, filtered, and then concentrated, to provide Compound 122e (55 mg). MS m/z (ESI): 205.9 $[M+H]^+$.

**Step 6: Preparation of 2-(6-(6-((6-(4-fluoro-5-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 122)**

Compound 1g (101 mg), Compound 122e (51.98 mg), and tetraisopropyl titanate (288.01 mg) were added in dry THF (25 mL), and were, after nitrogen replacement three times, stirred at 75 °C for 10 h. Then, sodium triacetoxyborohydride (268.46 mg) was added portionwise, and the mixture was stirred at 75 °C for an additional 6 h. After completion of the reaction, the reaction mixture was concentrated to dryness under reduced pressure, and separated and purified by Prep-HPLC to provide Compound 122 (22 mg). MS m/z (ESI): 551.8 $[M+H]^+$.

$^1H$ NMR (400 MHz, DMSO-$d_6$) δ 11.99 (s, 1H), 9.65 (s, 1H), 9.13 (d, *J*= 4.0 Hz, 1H), 8.57 (d, *J* = 4.8 Hz, 1H), 8.44 (dd, *J* = 8.8, 2.0 Hz, 1H), 8.37 (s, 1H), 7.94 (dd, *J* = 8.4, 1.6 Hz, 1H), 7.80 (d, *J* = 8.4 Hz, 1H), 6.97-6.74 (m, 2H), 6.31 (s, 1H), 3.81-3.69 (m, 4H), 3.66-3.52 (m, 4H), 2.59-2.53 (m, 1H), 2.33 (s, 3H), 2.28 (s, 3H), 2.24 (s, 3H), 1.59 (d, *J* = 8.4 Hz, 1H).

**Example 57: 2-(6-(6-((6-(1H-pyrrol-2-yl)pyridin-3-yl)methyl)-3,6-diazabicyclo [3.1.1] heptan-3-yl)pyridin-3-yl)-6-methyl-N-(5-methyl-1H-pyrazol-3-yl)pyrimidin-4-amine (Compound 123)**

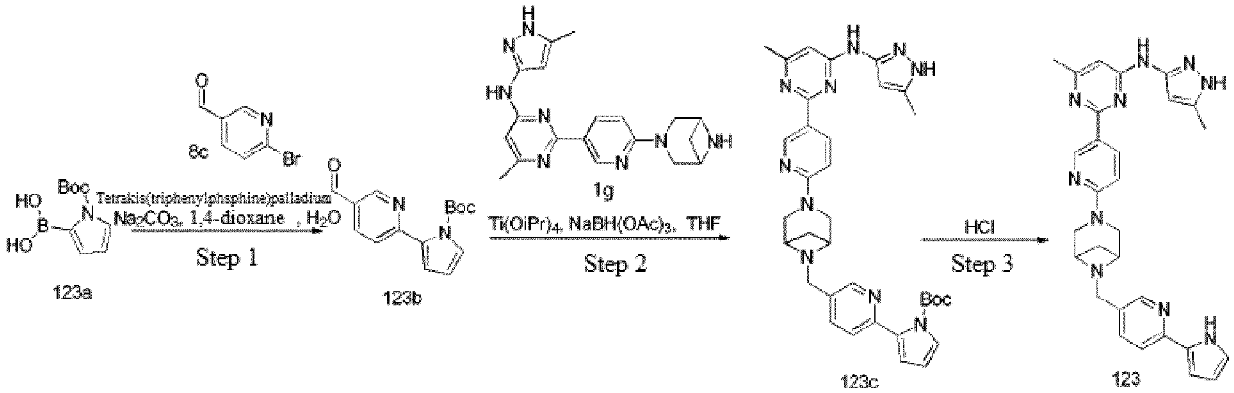


**Step 1: Preparation of tert-butyl 2-(5-formylpyridin-2-yl)-1H-pyrrole-1-carboxylate (Compound 123b)**

Compound 123a (1.13 g), Compound 8c (1.0 g), tetrakis(triphenylphosphine)palladium (310.62 mg), and sodium carbonate (1.71 g) were added into 1,4-dioxane (60 mL) and water (15 mL). The mixture was heated to 95 °C, and kept for reaction under the protection of nitrogen at this temperature for 14 h. After completion of the reaction, the mixture was concentrated to remove a part of organic solvent, and extracted with EA. The organic phases were combined, washed with saturated brine, dried over anhydrous sodium sulfate, filtered, concentrated under reduced pressure, and separated and purified by silica gel column chromatography (PE:EA=5:1), to provide Compound 123b (1.25 g).

**Step 2: Preparation of tert-butyl 2-(5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)pyridin-2-yl)-1H-pyrrole-1-carboxylate (Compound 123c)**

Compound 1g (50 mg), Compound 123b (37.57 mg), and tetraisoproppyl titanate (156.84 mg) were added into dry THF (5 mL), and the mixture was stirred at 72 °C for 18 h. Then, sodium triacetoxyborohydride (146.19 mg) was added, and the mixture was kept for reaction at 72 °C for 4 h. After completion of the reaction, the mixture was purified directly by silica gel column chromatography (DCM:MeOH=93:7) to provide Compound 123c (40 mg). MS m/z (ESI): 618.9 $[M+H]^+$.

**Step 3: Preparation of 2-(6-(6-((6-(1H-pyrrol-2-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 123)**

A solution of hydrogen chloride in 1,4-dioxane (4 N, 2.0 mL) was added dropwise into a solution of Compound 123c (40 mg) in methanol (2 mL). The mixture was kept for reaction at room temperature for 2 h. After completion of the reaction, the reaction mixture was concentrated to dryness. The crude product was dissolved in methanol, excess potassium carbonate was added, and the mixture was stirred at room temperature for 0.5 h to free hydrochloride. The mixture was filtered, concentrated under reduced pressure, and separated and purified by Prep-HPLC to provide Compound 123 (22 mg). MS m/z (ESI): 518.9 $[M+H]^+$.

$^1H$ NMR (400 MHz, DMSO-$d_6$) δ 11.98 (s, 1H), 11.43 (s, 1H), 9.66 (s, 1H), 9.12 (d, *J* = 2.1 Hz, 1H), 8.49-8.35 (m, 2H), 7.70 (d, *J*= 7.9 Hz, 1H), 7.61 (d, *J*= 8.2 Hz, 1H), 6.93-6.59 (m, 4H), 6.31 (s, 1H), 6.12 (dd, *J*= 5.7, 2.5 Hz, 1H), 3.85-3.68 (m, 4H), 3.65-3.49 (m, 4H), 2.59-2.54 (m, 1H), 2.33 (s, 3H), 2.26 (s, 3H), 1.59 (d, *J*= 8.2 Hz, 1H).

**Example 58: 2-(6-(6-((6-(3-cyclopropyl-4-fluoro-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 124)**

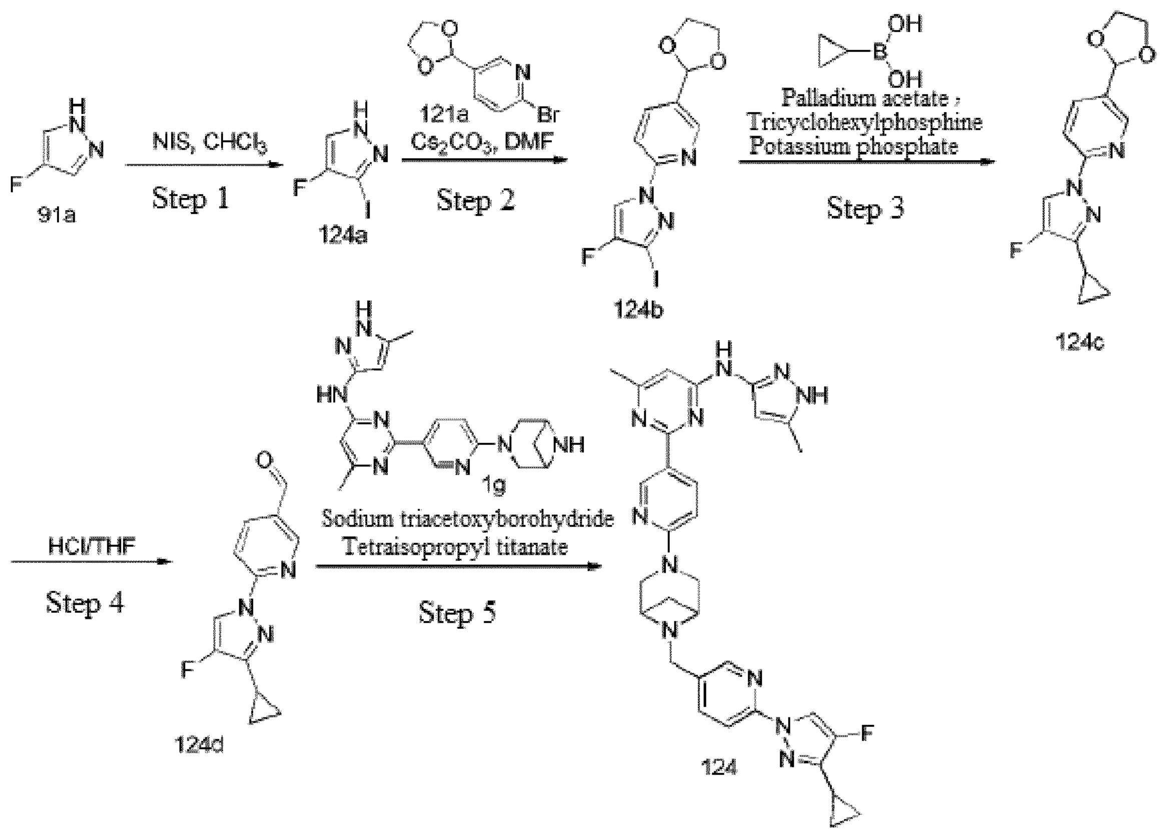


**Step 1: Preparation of 4-fluoro-3-iodo-1H-pyrazole (Compound 124a)**

4-fluoropyrazole (1.5 g), NIS (4.31 g), and chloroform (30 mL) were successively added into a reaction flask, and the mixture was stirred under the protection of nitrogen at 80 °C for 7 h. After completion of the reaction, the reaction mixture was cooled to room temperature. Silica gel was directly blended with samples, which were separated and purified by flash silica gel column chromatography (PE:EA=4:1) to provide Compound 124a (1.2 g). MS m/z (ESI): 213 $[M+H]^+$.

**Step 2: Preparation of 5-(1,3-dioxolan-2-yl)-2-(4-fluoro-3-iodo-1H-pyrazol-1-yl)pyridine (Compound 124b)**

Compound 121a (576.33 mg), Compound 124a (354 mg), cesium carbonate (1.63 g), and DMF (15 mL) were successively added into a reaction flask, and the mixture was stirred at 90 °C for 12 h. After completion of the reaction, the reaction mixture was cooled to room temperature, diluted with water (20 mL), and extracted with EA. The organic phase was washed with water for three times, dried over anhydrous sodium sulfate, filtered, concentrated, and separated and purified through a $C_{18}$ column (acetonitrile:0.05% aqueous solution of ammonium bicarbonate=68:32), to provide Compound 124b (300 mg). MS m/z (ESI): 362 $[M+H]^+$.

**Step 3: Preparation of 2-(3-cyclopropyl-4-fluoro-1H-pyrazol-1-yl)-5-(1,3-dioxolan-2-yl)pyridine (Compound 124c)**

Cyclopropylboronic acid (89.20 mg), Compound 124b (125 mg), palladium acetate (15.54 mg), potassium phosphate (257.17 mg), tricyclohexylphosphine (9.71 mg), toluene (5 mL), and water (1 mL) were successively added into a reaction flask, and the mixture was stirred under the protection of nitrogen at 90 °C for 12 h. After completion of the reaction, the reaction mixture was cooled to room temperature. Silica gel was directly blended with samples, which were separated and purified by silica gel column chromatography (PE:EA=3:1) to provide Compound 124c (70 mg). MS m/z (ESI): 276 $[M+H]^+$.

**Step 4: Preparation of 6-(3-cyclopropyl-4-fluoro-lH-pyrazol-1-yl)nicotinaldehyde (Compound 124d)**

Compound 124c (70 mg) was dissolved in a mixed solution of THF (8 mL) and water (8 mL), and then concentrated hydrochloric acid (5 mL, 37%) was added dropwise. The mixture was stirred at 25 °C for 18 h. After completion of the reaction, the reaction mixture was spin-dried to remove a part of solvent, adjusted with a saturated aqueous

solution of sodium bicarbonate to a pH of about 9, and then extracted with EA. The organic phase was dried over anhydrous sodium sulfate, filtered, concentrated, and separated and purified by flash silica gel column chromatography (PE:EA=3:1) to provide Compound 124d (18 mg). MS m/z (ESI): 232 $[M+H]^+$.

**Step 5: Preparation of 2-(6-(6-((6-(3-cyclopropyl-4-fluoro-1H-pyrazol-l-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 124)**

Compound 124d (18 mg), Compound 1g (31.04 mg), isopropyl titanate (88.50 mg), and dry THF (5 mL) were successively added into a reaction flask, and the mixture was stirred under the protection of nitrogen at 75 °C for 18 h. Then, sodium triacetoxyborohydride (82.49 mg) was added portionwise, and the mixture was stirred at 75 °C for an additional 6 h. After completion of the reaction, the reaction mixture was spin-dried to remove a part of solvent, adjusted with a saturated sodium bicarbonate solution to a pH of about 9, and then extracted with EA. The organic phase was dried over anhydrous sodium sulfate, filtered, concentrated, pre-purified by preparative TLC (DCM:MeOH=9:1), and then separated and purified by Prep-HPLC, to provide Compound 124 (15 mg). MS m/z (ESI): 577.9 $[M+H]^+$.

$^1$H NMR (400 MHz, DMSO-$d_6$) $\delta$ 11.98 (s, 1H), 9.65 (s, 1H), 9.12 (d, *J*= 2.1 Hz, 1H), 8.55 (d, *J* = 4.6 Hz, 1H), 8.44 (dd, *J* = 8.9, 2.2 Hz, 1H), 8.35 (d, *J* = 1.5 Hz, 1H), 7.93 (dd, *J* = 8.5, 2.0 Hz, 1H), 7.76 (d, *J* = 8.5 Hz, 1H), 6.82 (br, 1H), 6.79 (d, *J* = 12Hz, 1H), 6.30 (br, 1H),3.84-3.75 (m, 4H), 3.73-3.49 (m, 4H), 2.59-2.53 (m, 1H), 2.33 (s, 3H), 2.26 (s, 3H), 2.02-1.93 (m, 1H), 1.59 (d, *J*= 8.4 Hz, 1H), 1.03-0.95 (m, 2H), 0.94-0.86 (m, 2H).

**Example 59: 2-(6-(6-((*R*)-1-(6-(1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine and 2-(6-(6-((*S*)-1-(6-(1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 125-1/Compound 125-2)**

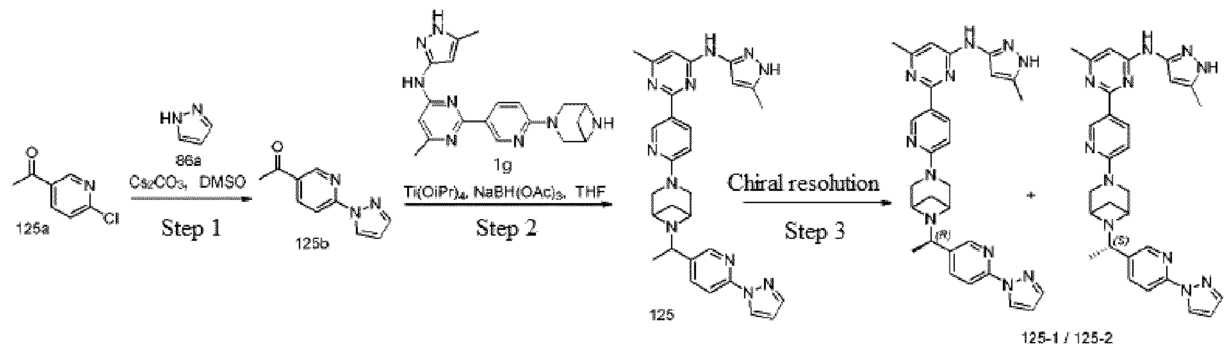


**Step 1: Preparation of 1-(6-(1H-pyrazol-1-yl)pyridin-3-yl)ethanone (Compound 125b)**

Compound 86a (328.18 mg), Compound 125a (500 mg), and cesium carbonate (1.57 g) were successively added into DMSO (5 mL). The mixture was heated to 100 °C, and stirred at this temperature for 2 h. The reaction mixture was cooled to room temperature, diluted with water, and extracted with EA. The organic phase was washed with water and saturated brine, dried over anhydrous sodium sulfate, filtered, concentrated under reduced pressure, and purified by flash silica gel column chromatography (PE:EA=50:50), to provide Compound 125b (364 mg). MS m/z (ESI): 188.1 $[M+H]^+$.

**Step 2: Preparation of 2-(6-(6-(1-(6-(1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 125)**

Dry THF (20 mL) was added into Compound 125b (309.90 mg), Compound 1g (400 mg), and tetraisopropyl titanate (1.25 g) present in a 250 mL reaction flask. The mixture was heated to 75 °C, and stirred at this temperature for 16 h. Then, sodium triacetoxyborohydride (1.17 g) was added, and the mixture was stirred at 75 °C for 2 h. The reaction mixture was cooled to room temperature, and a saturated aqueous solution of ammonium chloride (3 mL) was added to quench the reaction. The reaction mixture was concentrated, and separated and purified by flash silica gel column chromatography (DCM:MeOH=9:1) to provide Compound 125 (270 mg). MS m/z (ESI): 533.9 $[M+H]^+$.

**Step 3: Preparation of 2-(6-(6-((*R*)-1-(6-(1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine and 2-(6-(6-((*S*)-1-(6-(1H-pyrazol-1-yl)pyridin-3-yl)ethyl)-3,6-diazabicyclo[3.1.1]heptan-3-yl)pyridin-3-yl)-6-methyl-N-(5-methyl-1H-pyrazol-3-yl)pyrimidin-4-amine (Compound 125-1/Compound 125-2)**

Compound 125 (250 mg) was resolved by chiral Prep-HPLC to provide Compound 125-1 (retention time 7.647 min) and Compound 125-2 (retention time 11.638 min). The two compounds were distinguished and defined based on the retention time of chiral resolution, and were not identified for stereostructures. Thus, Compound 125-1 (101 mg, ee: 100 %), MS m/z (ESI): 533.9 $[M+H]^+$; Compound 125-2 (100 mg, ee: 99.93 %), MS m/z (ESI): 533.9 $[M+H]^+$ were given.

Chiral HPLC resolution conditions:

Instrument model: Shimadzu LC-20AD; chromatographic column: CHIRALPAK IE-3 (IE30CD-UL006), 0.46 cm I.D. × 15 cm L; chromatographic column temperature: 25 °C; flow rate: 1.0 mL/min; detection wavelength: 254 nm; mobile phase: MeOH:ACN:DEA=70:30:0.1 (V/V/V); appearance time of Compound 125-1: 7.647 min, appearance time of Compound 125-2: 11.638 min.

$^1$H NMR (400 MHz, DMSO-$d_6$) δ 11.98 (s, 1H), 9.66 (s, 1H), 9.12 (d, *J*= 2.3 Hz, 1H), 8.59 (s, 1H), 8.47-8.39 (m, 2H), 7.99 (d, *J*= 6.4 Hz, 1H), 7.89 (d, *J*= 7.4 Hz, 1H), 7.81 (s, 1H), 6.75 (d, *J* = 9.0 Hz, 2H), 6.56 (s, 1H), 6.31 (s, 1H), 3.96-3.83 (m, 2H), 3.75 (d, *J*= 3.3 Hz, 1H), 3.63 (s, 1H), 3.54-3.36 (m, 3H), 2.57-2.53 (m, 1H), 2.33 (s, 3H), 2.25 (s, 3H), 1.54 (d, *J*= 6.9 Hz, 1H), 1.22 (d, *J*= 2.5 Hz, 3H). (Compound 125-1)

$^1$H NMR (400 MHz, DMSO-$d_6$) δ 11.98 (s, 1H), 9.66 (s, 1H), 9.12 (d, *J*= 2.3 Hz, 1H), 8.59 (d, *J* = 2.6 Hz, 1H), 8.47-8.39 (m, 2H), 8.00 (dd, *J* = 8.5, 2.2 Hz, 1H), 7.90 (d, *J* = 8.4 Hz, 1H), 7.81 (d, *J*= 1.6 Hz, 1H), 6.75 (d, *J* = 9.0 Hz, 2H), 6.57 (t, *J*= 2.1 Hz, 1H), 6.31 (s, 1H), 3.96-3.83 (m, 2H), 3.76 (q, *J* = 6.2 Hz, 1H), 3.63 (s, 1H), 3.54-3.36 (m, 3H), 2.57-2.53 (m, 1H), 2.33 (s, 3H), 2.25 (s, 3H), 1.55 (d, *J*= 8.4 Hz, 1H), 1.23 (d, *J*= 6.1 Hz, 3H). (Compound 125-2)

**Example 60: 2-(6-(6-((6-(4-fluoromethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazo1-3-yl)pyrimidin-4-amine (Compound 126) and (1-(5-((3-(5-(4-methyl-6-((5-methyl-1H-pyrazol-3-yl)amino)pyrimidin-2-yl)pyridin-2-yl)-3,6-diazabicyclo [3.1.1] heptan-6-yl)methyl)pyridin-2-yl)-1H-pyrazol-4-yl)methanol (Compound 127)**

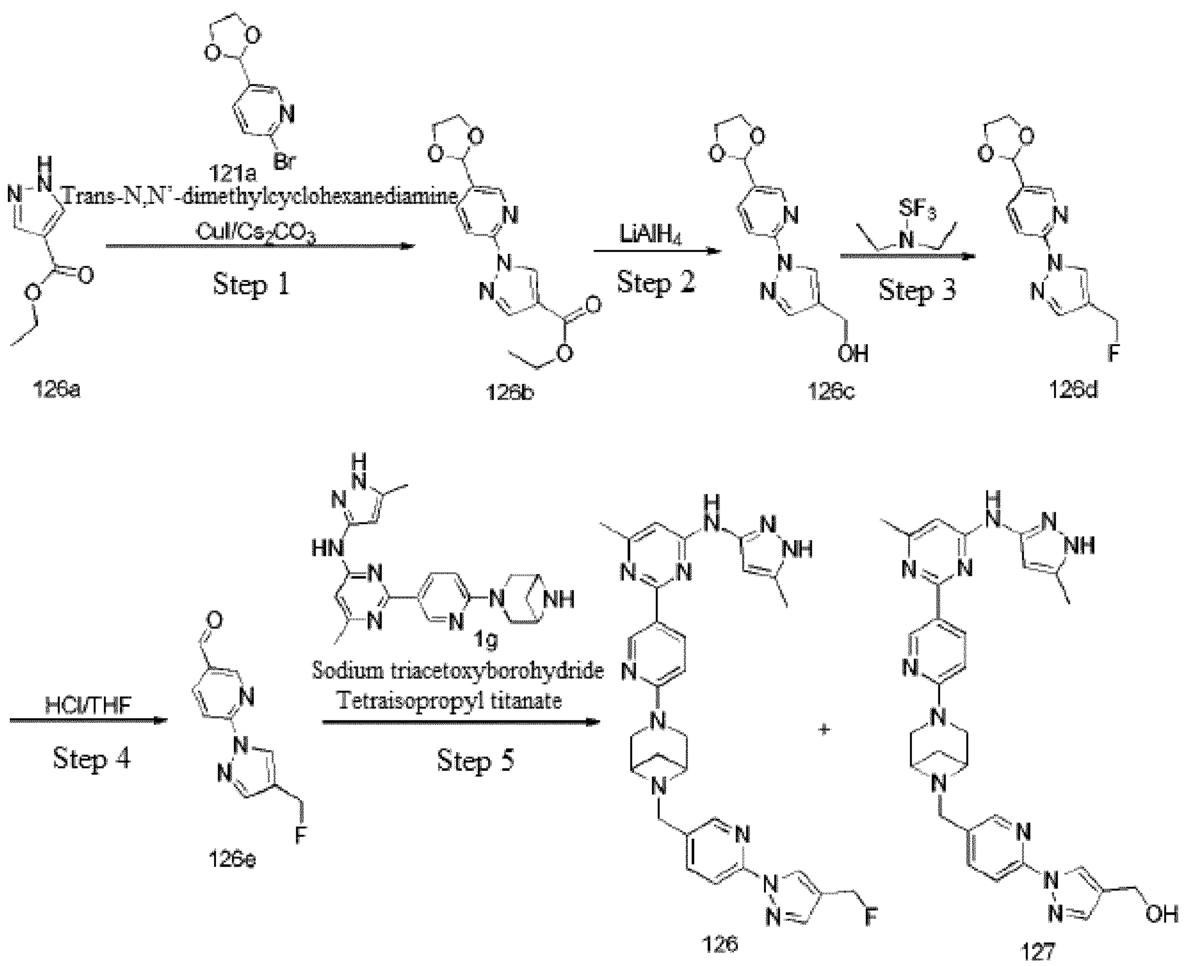


**Step 1: Preparation of ethyl 1-(5-(1,3-dioxolan-2-yl)pyridin-2-yl)-1H-pyrazole-4-carboxylate (Compound 126b)**

Compound 121a (3.0 g), Compound 126a (1.86 g), $Cs_2CO_3$ (8.67 g), trans-N,N'-dimethylcyclohexanediamine (757.08 mg), CuI (506.84 mg), and DMF (15 mL) were added into a reaction flask. The mixture was heated to 90 °C, and kept for reaction under the protection of nitrogen at this temperature for 2 h. After completion of the reaction, the reaction mixture was cooled to room temperature, diluted with water (30 mL), and extracted with EA (30 mL×3). The organic phases were combined, washed with water, dried over anhydrous sodium sulfate, filtered, concentrated to dryness under reduced pressure, and separated and purified by flash silica gel column chromatography (EA:PE=20:80), to provide Compound 126b (3.31 g). MS m/z (ESI): 290.0 $[M+H]^+$.

**Step 2: Preparation of (1-(5-(1,3-dioxolan-2-yl)pyridin-2-yl)-1H-pyrazol-4-yl)methanol (Compound 126c)**

Compound 126b (3.31 g) was dissolved in THF (30 mL), and cooled to -20 °C. $LiAlH_4$ (651.40 mg, 16.99 mmol) was slowly added into the reaction mixture portionwise, and the reaction mixture was kept for reaction at this temperature for 15 min. After completion of the reaction, EA (2 mL) was slowly added dropwise to consume excess $LiAlH_4$. Then, water (1 mL) was added dropwise to quench the reaction. The mixture was diluted with water (20 mL), and extracted with EA (30 mL×3). The organic phases were combined, dried over anhydrous sodium sulfate, filtered, concentrated to dryness under reduced pressure, and separated and purified by flash silica gel column chromatography (EA:PE=50:50), to provide Compound 126c (2.30 g). MS m/z (ESI): 248.0 $[M+H]^+$.

**Step 3: Preparation of 5-(1,3-dioxolan-2-yl)-2-(4-(fluoromethyl)-1H-pyrazol-1-yl)pyridine (Compound 126d)**

Under the protection of nitrogen, dry DCM (30 mL) was cooled to -40 °C, and then diethylaminosulfur trifluoride (6.06 g) was slowly added dropwise. A solution of Compound 126c (2.30 g) in DCM(30 mL) was added dropwise into the reaction mixture. Then, the reaction mixture was slowly warmed to 25 °C, and kept for reaction at this temperature for 20 h. After completion of the reaction, the reaction mixture was poured into a saturated aqueous solution of sodium

bicarbonate, and was, After completion of the release of bubbles, extracted with DCM (30 mL x 3). The organic phases were combined, dried over anhydrous sodium sulfate, filtered, concentrated to dryness under reduced pressure, and separated and purified by flash silica gel column chromatography (EA:PE=20:80), to provide Compound 126d (684 mg). MS m/z (ESI): 249.9 $[M+H]^+$.

**Step 4: Preparation of 6-(4-(fluoromethyl)-1H-pyrazol-1-yl)nicotinaldehyde (Compound 126e)**

Compound 126d (684 mg) was dissolved in THF (10 mL), and then hydrochloric acid (1 N, 5 mL) was added into the solution. The mixture was kept for reaction at 25 °C for 16 h. After completion of the reaction, the reaction mixture was diluted with water (20 mL), adjusted with a saturated aqueous solution of $NaHCO_3$ to a pH from 7 to 8. The mixture was directly concentrated to remove THF, and then extracted with DCM (30 mL x 3). The organic phases were combined, dried over anhydrous sodium sulfate, filtered under suction, and concentrated, to provide Compound 126e (460 mg). MS m/z (ESI): 206.0 $[M+H]^+$.

**Step 5: Preparation of 2-(6-(6-((6-(4-fluoromethyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 126) and (1-(5-((3-(5-(4-methyl-6-((5-methyl-1H-pyrazol-3-yl)amino)pyrimidin-2-yl)pyridin-2-yl)-3,6-diazabicyclo [3.1.1] heptan-6-yl)methyl)pyridin-2-yl)-1H-pyrazol-4-yl)methanol (Compound 127)**

Compound 1g (200 mg), Compound 126e (182 mg), and tetraisopropyl titanate (640 mg) were dissolved in dry THF (40 mL), and the mixture was heated to 75 °C, and kept for reaction at this temperature for 8 h. The reaction mixture was cooled to 25 °C. Sodium triacetoxyborohydride (597 mg) was added into a reaction flask, and the mixture was kept for reaction at 25 °C for 12 h. Water (1 mL) was added dropwise to quench the reaction. The reaction mixture was concentrated, and separated and pre-purified by flash silica gel column chromatography (DCM:MeOH=90:10) to provide a crude product (a mixture of Compound 126 and Compound 127), which was further separated and purified by Prep-HPLC to provide Compound 126 (21 mg), MS m/z (ESI): 552.3 $[M+H]^+$; Compound 127 (19 mg), MS m/z (ESI): 550.3 $[M+H]^+$.

$^1H$ NMR (400 MHz, DMSO-$d_6$) $\delta$ 12.00 (br, 1H), 9.65 (s, 1H), 9.13 (d, *J*= 2.2 Hz, 1H), 8.77 (d, *J* = 3.2 Hz, 1H), 8.44 (dd, *J* = 8.8, 2.2 Hz, 2H), 7.99 (dd, *J* = 8.4, 2.0 Hz, 1H), 7.94 (s, 1H), 7.91-7.96 (m, 1H), 6.95-6.69 (m, 2H), 6.30 (br, 1H), 5.46 (s, 1H), 5.34 (s, 1H), 3.82-3.70 (m, 4H), 3.68-3.52 (m, 4H), 2.59-2.54 (m, 1H), 2.33 (s, 3H), 2.26 (s, 3H), 1.60 (d, *J* = 8.4 Hz, 1H). (Compound 126)

$^1H$ NMR (400 MHz, DMSO-$d_6$) $\delta$ 12.04 (br, 1H), 9.66 (s, 1H), 9.13 (d, *J* = 2.2 Hz, 1H), 8.52-8.35 (m, 3H), 7.95 (dd, *J* = 8.4, 1.6 Hz, 1H), 7.88-7.81 (m, 1H), 7.73 (s, 1H), 6.99-6.64 (m, 2H), 6.31 (br, 1H), 5.05 (br, 1H), 4.45 (s, 2H), 3.93-3.70 (m, 4H), 3.67-3.51 (m, 4H), 2.59-2.53 (m, 1H), 2.33 (s, 3H), 2.26 (s, 3H), 1.60 (d, *J*= 8.4 Hz, 1H). (Compound 127)

**Example 61: 6-methyl-N-(5-methyl-1H-pyrazol-3-yl)-2-(6-(6-((6-(1-methyl-1H-pyrrol-2-yl)pyridin-3-yl)methyl)-3,6-diazabicyclo[3.1.1]heptan-3-yl)pyridin-3-yl)pyrimidin-4-amine (Compound 128)**

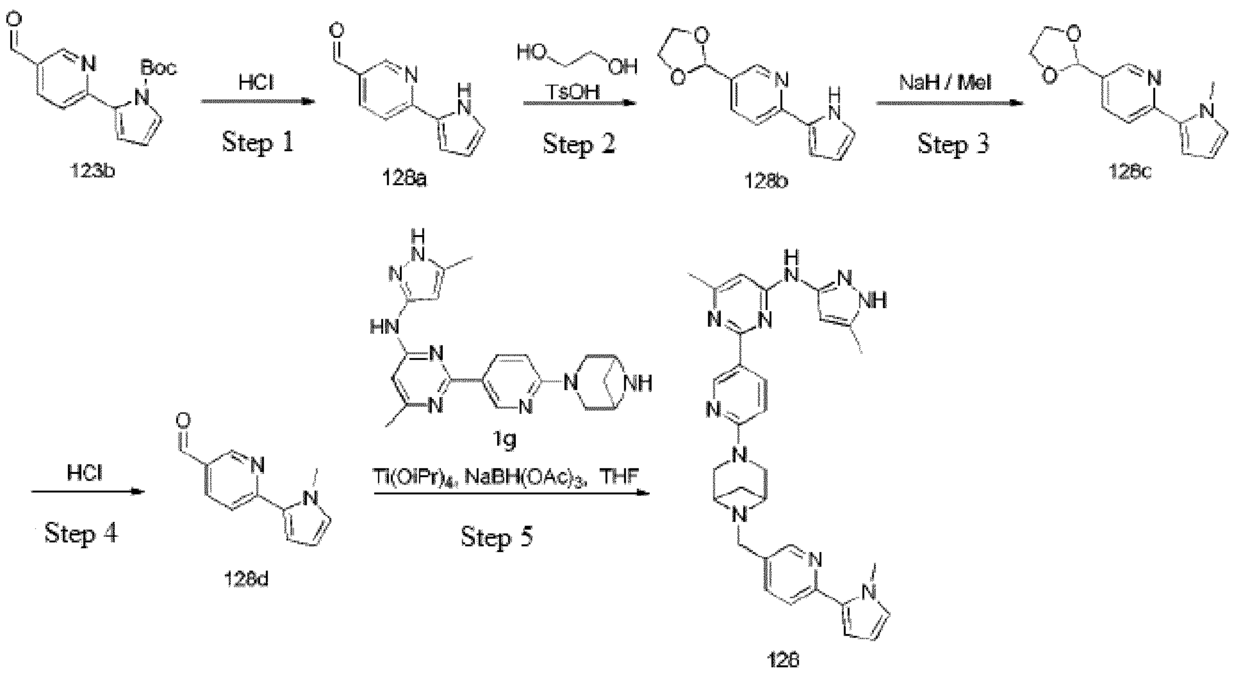


123b 128a 128b 128c 1g 128d 128

**Step 1: Preparation of 6-(1H-pyrrol-2-yl)nicotinaldehyde (Compound 128a)**

A solution of hydrogen chloride in 1,4-dioxane (4 N, 6.0 mL) was added dropwise into a solution of Compound 123b (500 mg) in methanol (10 mL). The mixture was kept for reaction at room temperature for 2 h. After completion of the reaction, the reaction mixture was concentrated to dryness. The crude product was dissolved in methanol, excess potassium carbonate was added, and the mixture was stirred at room temperature for 0.5 h to free hydrochloride. The mixture was filtered, concentrated under reduced pressure, and separated and purified by silica gel column chromatography (PE:EA=4:1) to provide Compound 128a (250 mg). MS m/z (ESI): 173.0 $[M+H]^+$.

**Step 2: Preparation of 5-(1,3-dioxolan-2-yl)-2-(1H-pyrrol-2-yl)pyridine (Compound 128b)**

Compound 128a (250 mg), ethylene glycol (180 mg) and p-methylphenylsulfonic acid (27.6 mg) were added into toluene (15 mL), and the mixture was refluxed to divert water for 18 h. After completion of the reaction, the reaction mixture was diluted with EA, and washed with a saturated aqueous solution of sodium bicarbonate. The organic phases were dried over anhydrous sodium sulfate, filtered, and then concentrated under reduced pressure, to provide Compound 128b (290 mg). MS m/z (ESI): 217.0 $[M+H]^+$.

**Step 3: Preparation of 5-(1,3-dioxolan-2-yl)-2-(1-methyl-1H-pyrazol-2-yl)pyridine (Compound 128c)**

Compound 128b (250 mg) was dissolved in dry DMF (5 mL), NaH (138.7 mg, purity 60%) was added under the protection of nitrogen at 0 °C, and then the mixture was stirred for 15 min. Iodomethane (820 mg) was added, and the mixture was kept for reaction at room temperature for 5 h. After completion of the reaction, the reaction mixture was poured into saturated brine, extracted with EA, dried over anhydrous sodium sulfate, filtered, concentrated under reduced pressure, and separated and purified by silica gel column chromatography (PE:EA=5:1), to provide Compound 128c (125 mg). MS m/z (ESI): 231.0 $[M+H]^+$.

**Step 4: Preparation of 6-(1-methyl-1H-pyrrol-2-yl)nicotinaldehyde (Compound 128d)**

Diluted hydrochloric acid (2 N, 2.0 mL) was added dropwise into a solution of Compound 128c (110 mg) in THF (4.0 mL). The mixture was kept for reaction at room temperature for 3 h. After completion of the reaction, the reaction mixture was concentrated to dryness. The crude product was dissolved in methanol, excess potassium carbonate was added, and the mixture was stirred at room temperature for 0.5 h to free hydrochloride. The mixture was filtered, concentrated under reduced pressure, and separated and purified by silica gel column chromatography (PE:EA=3:1) to provide Compound 128d (75 mg).

**Step 5: Preparation of 6-methyl-N-(5-methyl-1H-pyrazol-3-yl)-2-(6-(6-((6-(1-methyl-1H-pyrrol-2-yl)pyridin-3-yl)methyl)-3,6-diazabicyclo [3.1.1] heptan-3-yl)pyridin-3-yl)pyrimidin-4-amine (Compound 128)**

Compound 1g (40 mg), Compound 128d (25 mg), and tetraisoproppyl titanate (125 mg) were added into dry THF (5 mL), and the mixture was stirred at 72 °C for 10 h. Then, sodium triacetoxyborohydride (117 mg) was added, and the mixture was kept for reaction at 72 °C for 6 h. After completion of the reaction, the mixture was pre-purified directly by silica gel column chromatography (DCM:MeOH=93:7), and then separated and purified by Prep-HPLC to provide Compound 128 (34 mg). MS m/z (ESI): 532.9 $[M+H]^+$.

$^1$H NMR (400 MHz, $CD_3OD$) δ 9.14 (d, *J*= 2.1 Hz, 1H), 8.59-8.43 (m, 2H), 7.78 (dd, *J*= 8.2, 2.2 Hz, 1H), 7.55 (d, *J* = 8.2 Hz, 1H), 6.85 (d, *J* = 9.0 Hz, 1H), 6.81-6.65 (m, 2H), 6.50 (dd, *J* = 3.7, 1.8 Hz, 1H), 6.35 (s, 1H), 6.10 (dd, *J* = 3.7, 2.7 Hz, 1H), 4.02-3.77 (m, 7H), 3.77-3.59 (m, 4H), 2.73 (d, *J*= 6.3 Hz, 1H), 2.41 (s, 3H), 2.32 (s, 3H), 1.71 (d, *J*= 8.8 Hz, 1H).

**Example 62: 6-methyl-N-(5-methyl-1H-pyrazol-3-yl)-2-(6-(6-((6-(thiazol-4-yl)pyridin-3-yl)methyl)-3,6-diazabicyclo[3.1.1]heptan-3-yl)pyridin-3-yl)pyrimidin-4-amine (Compound 129)**

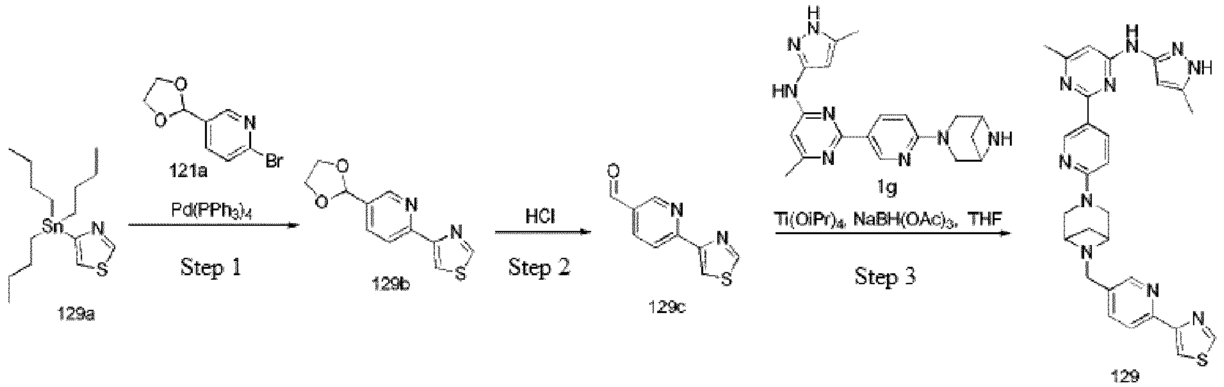


**Step 1: Preparation of 4-(5-(1,3-dioxolan-2-yl)pyridin-2-yl)thiazole (Compound 129b)**

Compound 129a (325 mg), Compound 121a (200 mg), and tetrakis(triphenylphosphine)palladium (50 mg) were added into toluene (10 mL), and the mixture was kept at 120 °C for 6 h. After completion of the reaction, the reaction mixture was concentrated to dryness, and separated and purified by silica gel column chromatography (PE:EA=2:1) to provide Compound 129b (115 mg). MS m/z (ESI): 235.1 $[M+H]^+$.

**Step 2: Preparation of 6-(thiazol-4-yl)nicotinaldehyde (Compound 129c)**

Diluted hydrochloric acid (2.0 mL, 3 N) was added dropwise into a solution of Compound 129b (115 mg) in THF (3.0 mL). The mixture was kept for reaction at room temperature for 15 h. After completion of the reaction, the reaction mixture was adjusted to a pH of about 10 by slowly adding a saturated sodium bicarbonate solution dropwise, and then extracted with EA. The organic phase was dried over anhydrous sodium sulfate, filtered, and then concentrated, to provide Compound 129c (83 mg), which was directly used for next step reaction without purification. MS m/z (ESI): 191.1 $[M+H]^+$.

**Step 3: Preparation of 6-methyl-N-(5-methyl-1H-pyrazol-3-yl)-2-(6-(6-((6-(thiazol-4-yl)pyridin-3-yl)methyl)-3,6-diazabicyclo[3.1.1]heptan-3-yl)pyridin-3-yl)pyrimidin-4-amine (Compound 129)**

Compound 1g (50 mg), Compound 129c (26.2 mg), and tetraisopropyl titanate (156.8 mg) were added into dry THF (5 mL), and the mixture stirred at 72 °C for 10 h. Then, sodium triacetoxyborohydride (146.2 mg) was added, and the mixture was kept for reaction at 72 °C for 6 h. After completion of the reaction, the mixture was crudely purified directly by silica gel column chromatography (DCM:MeOH=8:1), and then separated and purified by Prep-HPLC to provide Compound 129 (51 mg). MS m/z (ESI): 537.3 $[M+H]^+$.

$^1$H NMR (400 MHz, DMSO-$d_6$) δ 11.98 (s, 1H), 9.66 (s, 1H), 9.22 (d, *J*= 2.1 Hz, 1H), 9.13 (d, *J* = 2.1 Hz, 1H), 8.58 (d, *J* = 1.6 Hz, 1H), 8.44 (dd, *J* = 8.9, 2.3 Hz, 1H), 8.29 (d, *J* = 2.0 Hz, 1H), 8.06 (d, *J* = 8.0 Hz, 1H), 7.88 (dd, *J* =

8.1, 2.2 Hz, 1H), 7.01 - 6.64 (m, 2H), 6.33 (s, 1H), 3.87-3.49 (m, 8H), 2.61-2.54 (m, 1H), 2.33 (s, 3H), 2.26 (s, 3H), 1.60 (d, *J* = 8.4 Hz, 1H).

**Example 63: 2-(6-(6-([[2,2'-dipyridyl]-5-ylmethyl)-3,6-diazabicyclo[3.1.1]heptan-3-yl)pyridin-3-yl)-6-methyl-N-(5-methyl-1H-pyrazol-3-yl)pyrimidin-4-amine (Compound 130)**

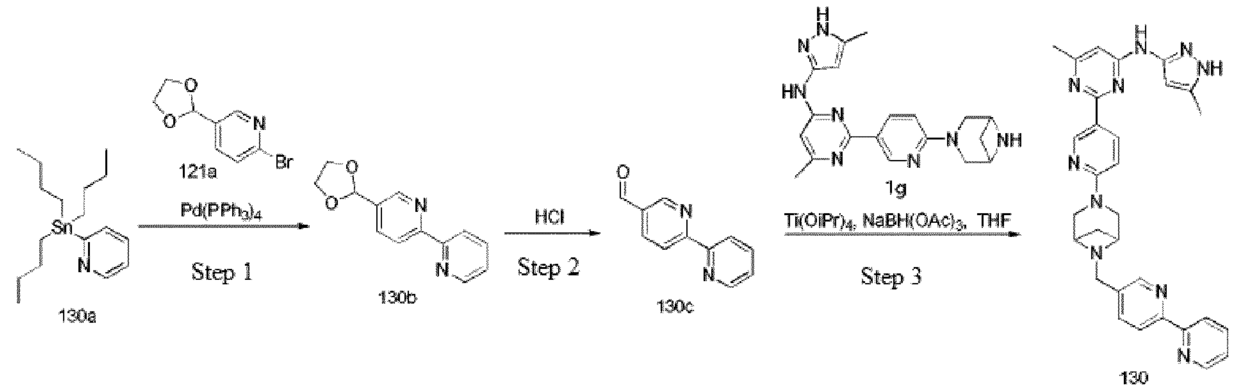


**Step 1: Preparation of 5-(1,3-dioxolan-2-yl)-2,2'-dipyridine (Compound 130b)**

Compound 130a (320 mg), Compound 121a (200 mg), and tetrakis(triphenylphosphine)palladium (50 mg) were added into toluene (10 mL), and the mixture was kept at 120 °C for microwave reaction for 4 h. After completion of the reaction, the reaction mixture was concentrated to dryness, and separated and purified by silica gel column chromatography (PE:EA=2:1) to provide Compound 130b (100 mg). MS m/z (ESI): 229.1 $[M+H]^+$.

**Step 2: Preparation of [2,2'-dipyridyl]-5-carbaldehyde (Compound 130c)**

Diluted hydrochloric acid (5.0 mL, 3 N) was added dropwise into a solution of Compound 130b (100 mg) in THF (5.0 mL). The mixture was kept for reaction at room temperature for 15 h. After completion of the reaction, the reaction mixture was adjusted to a pH of about 10 by slowly adding a saturated sodium bicarbonate solution dropwise, and then extracted with EA. The organic phase was dried over anhydrous sodium sulfate, filtered, and then concentrated, to provide Compound 130c (45 mg), which was directly used for next step reaction without purification. MS m/z (ESI): 185.2 $[M+H]^+$.

**Step 3: Preparation of 2-(6-(6-([[2,2'-dipyridyl]-5-ylmethyl)-3,6-diazabicyclo[3.1.1]heptan-3-yl)pyridin-3-yl)-6-methyl-N-(5-methyl-1H-pyrazol-3-yl)pyrimidin-4-amine (Compound 130)**

Compound 1g (50 mg), Compound 130c (25.4 mg), and tetraisopropyl titanate (156.8 mg) were added into dry THF (5 mL), and the mixture was stirred at 72 °C for 10 h. Then, sodium triacetoxyborohydride (146.2 mg) was added, and the mixture was kept for reaction at 72 °C for 6 h. After completion of the reaction, the mixture was crudely purified directly by silica gel column chromatography (DCM:MeOH=10:1), and then separated and purified by Prep-HPLC to provide Compound 130 (40 mg). MS m/z (ESI): 531.3 $[M+H]^+$.

$^1H$ NMR (400 MHz, DMSO-$d_6$) δ 11.97 (s, 1H), 9.65 (s, 1H), 9.13 (d, *J*= 2.2 Hz, 1H), 8.75-8.57 (m, 2H), 8.44 (dd, *J* = 8.9, 2.3 Hz, 1H), 8.35 (dd, *J* = 9.7, 8.2 Hz, 2H), 7.93 (ddd, *J* = 8.3, 5.9, 1.9 Hz, 2H), 7.44 (ddd, *J*= 7.5, 4.8, 1.1 Hz, 1H), 7.08 - 6.57 (m, 2H), 6.31 (s, 1H), 3.82-3.57 (m, 8H), 2.63 - 2.56 (m, 1H), 2.33 (s, 3H), 2.26 (s, 3H), 1.61 (d, *J*= 8.4 Hz, 1H).

**Example 64: 2-(6-(6-((6-(5-fluoro-1H-pyrrol-2-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 131)**

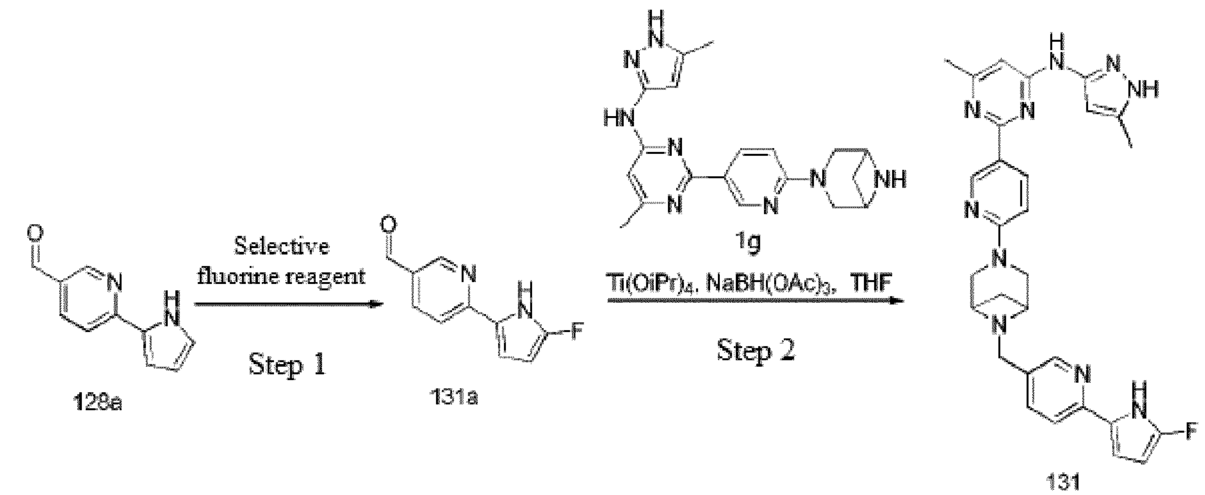


128a 131a 1g 131

**Step 1: Preparation of 6-(5-fluoro-1H-pyrrol-2-yl)nicotinaldehyde (Compound 131a)**

Compound 128a (200 mg) and 1-chloromethyl-4-fluoro-1,4-diazabicyclo[2.2.2]octane bis(tetrafluoroborate) (432 mg, selective fluorine reagent) were added into acetonitrile (15 mL), and kept at 70 °C for microwave reaction for 10 min. After completion of the reaction, the reaction mixture was concentrated to dryness, and separated and purified by silica gel column chromatography (PE:EA=5:1) to provide Compound 131a (60 mg). MS m/z (ESI): 191.1 $[M+H]^+$.

**Step 2: Preparation of 2-(6-(6-((6-(5-fluoro-1H-pyrrol-2-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 131)**

Compound 1g (50 mg), Compound 131a (26.2 mg), and tetraisopropyl titanate (156.8 mg) were added into dry THF (5 mL), and the mixture was stirred at 72 °C for 10 h. Then, sodium triacetoxyborohydride (146.2 mg) was added, and the mixture was kept for reaction at 72 °C for 6 h. After completion of the reaction, the mixture was crudely purified directly by silica gel column chromatography (DCM:MeOH=8:1), and then separated and purified by Prep-HPLC to provide Compound 131 (9 mg). MS m/z (ESI): 537.3 $[M+H]^+$.

$^1$H NMR (400 MHz, DMSO-$d_6$) δ 12.06 (s, 1H), 11.98 (s, 1H), 9.65 (s, 1H), 9.12 (d, *J* = 2.1 Hz, 1H), 8.43 (dd, *J* = 8.9, 2.3 Hz, 1H), 8.39 (d, *J* = 1.3 Hz, 1H), 7.74 - 7.65 (m, 1H), 7.57 (d, *J* = 8.2 Hz, 1H), 6.92 - 6.72 (m, 2H), 6.57 (t, *J* = 4.2 Hz, 1H), 6.31 (s, 1H), 5.57 (t, *J* = 3.8 Hz, 1H), 3.76 (d, *J* = 11.2 Hz, 2H), 3.70 (d, *J* = 5.7 Hz, 2H), 3.63 - 3.52 (m, 4H), 2.60-2.55 (m, 1H), 2.33 (s, 3H), 2.26 (s, 3H), 1.59 (d, *J*= 8.2 Hz, 1H).

**Example 65: 2-(6-(6-((6-(1H-pyrazol-5-yl)pyridin-3-yl)methyl)-3,6-diazabicyclo[3.1.1] heptan-3-yl)pyridin-3-yl)-6-methyl-N-(5-methyl-1H-pyrazol-3-yl)pyrimidin-4-amine (Compound 132)**

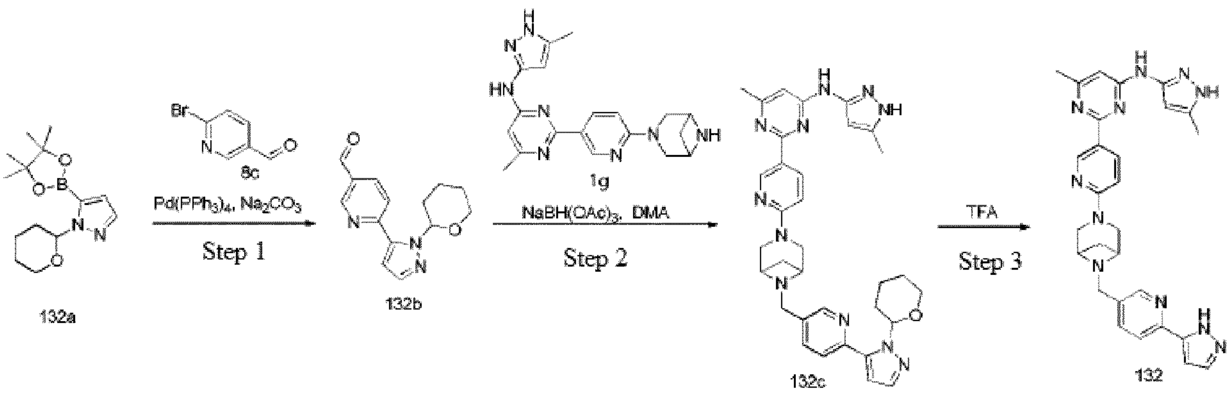


132a 8c 132b 1g 132c 132

**Step 1: Preparation of 6-(1-(tetrahydro-2H-pyran-2-yl)-1H-pyrazol-5-yl)nicotinaldehyde (Compound 132b)**

Compound 132a (2.24 g), Compound 8c (1 g), tetrakis(triphenylphosphine)palladium (310.6 mg), and 1,4-dioxane (20 mL) were successively added into a reaction flask, and then a solution of sodium carbonate (1.71 g) in water (5 mL) was added. The mixture was stirred under the protection of nitrogen at 95 °C for 5 h. After completion of the reaction, the reaction mixture was diluted with water, and extracted with EA (20 mL×3). The organic phases were combined, successively washed with water and saturated brine once, dried over anhydrous sodium sulfate, filtered, concentrated, and separated and purified by flash column chromatography (DCM:MeOH=96:4), to provide Compound 132b (1.14 g).

**Step 2: Preparation of 6-methyl-N-(5-methyl-1H-pyrazol-3-yl)-2-(6-((6-(1-(tetrahydro-2H-pyran-2-yl)-1H-pyrazol-5-yl)pyridin-3-yl)methyl)-3,6-diazabicyclo[3.1.1]heptan-3-yl)pyridin-3-yl)pyrimidin-4-amine (Compound 132c)**

Compound 1g (500 mg) and Compound 132b (477.8 mg) were dissolved in DMA (10 mL), and the mixture was stirred at 25 °C for reaction for 2 h. Then, sodium triacetoxyborohydride (1.17 g) was added, and the mixture was stirred at 25 °C for reaction for an additional 16 h. After completion of the reaction, water was added to quench the reaction, and the mixture was extracted with EA. The organic phases were washed with saturated brine, dried over anhydrous sodium sulfate, filtered, and then concentrated, to provide Compound 132c (697 mg). MS m/z (ESI): 604.3 $[M+H]^+$.

**Step 3: Preparation of 2-(6-(6-((6-(1H-pyrazol-5-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 132)**

Compound 132c (697 mg) was dissolved in MeOH (15 mL), and then TFA (0.86 mL) was added dropwise. The mixture was kept for reaction at 25 °C for 16 h. After completion of the reaction, a saturated sodium bicarbonate solution was added to quench the reaction, and the pH of solution was adjusted to a about 9. The solution was concentrated to remove methanol, diluted with water, and extracted with dichloromethane. The organic phase was dried over anhydrous sodium sulfate, filtered, concentrated, and separated and purified by Prep-HPLC, to provide Compound 132 (279 mg). MS m/z (ESI): 521.3 $[M+H]^+$.

$^1$H NMR (400 MHz, DMSO-$d_6$) δ 13.48 (s, 0.3H, tautomer 1), 13.01 (s, 0.7H, tautomer 2), 11.98 (s, 1H), 9.66 (s, 1H), 9.13 (d, *J* = 2.2 Hz, 1H), 8.52 (s, 1H), 8.44 (dd, *J* = 8.9, 2.3 Hz, 1H), 8.01-7.64 (m, 3H), 7.01 - 6.59 (m, 3H),6.32 (br, 1H), 3.85-3.68(m, 4H), 3.68 - 3.45 (m, 4H), 2.60 - 2.53 (m, 1H), 2.33 (s, 3H), 2.26 (s, 3H), 1.60 (d, *J*= 8.4 Hz, 1H).

**Example 66: 6-methyl-N-(5-methyl-1H-pyrazol-3-yl)-2-(6-(6-((6-(5-methylfuran-2-yl)pyridin-3-yl)methyl)-3,6-diazabicyclo [3.1.1] heptan-3-yl)pyridin-3-yl)pyrimidin-4-amine (Compound 133)**

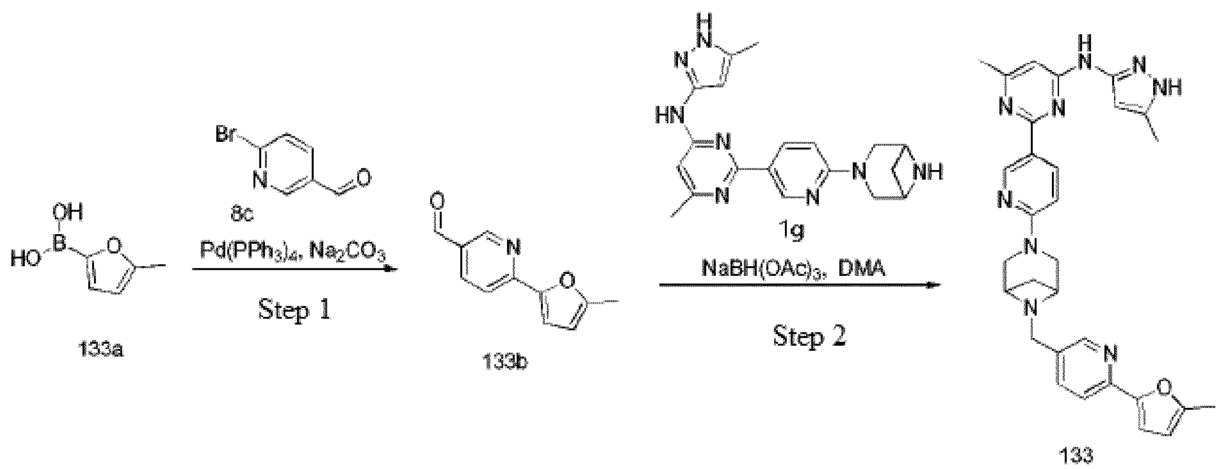


**Step 1: Preparation of 6-(5-methylfuran-2-yl)nicotinaldehyde (Compound 133b)**

Compound 133a (203.1 mg), Compound 8c (200 mg), $Na_2CO_3$ (341.9 mg), tetrakis(triphenylphosphine)palladium (62.1 mg), water (2.5 mL), and 1,4-dioxane (10 mL) were successively added into a reaction flask, and the mixture was stirred at 95 °C for 2 h. After completion of the reaction, the reaction mixture was cooled to room temperature, and

separated and purified directly by silica gel column chromatography (PE:EA=87:13) to provide Compound 133b (124 mg). MS m/z (ESI): 188.1 $[M+H]^+$.

**Step 2: Preparation of 6-methyl-N-(5-methyl-1H-pyrazol-3-yl)-2-(6-(6-((6-(5-methylfuran-2-yl)pyridin-3-yl)methyl)-3,6-diazabicyclo[3.1.1]heptan-3-yl)pyridin-3-yl)pyrimidin-4-amine (Compound 133)**

Compound 133b (23.2 mg), Compound 1g (30 mg), and DMA(1 mL) were successively added into a reaction flask, and the mixture was stirred at 25 °C for 2 h. Then, sodium triacetoxyborohydride (70.2 mg) was added, and the mixture was stirred at 25 °C for reaction for an additional 16 h. After completion of the reaction, the reaction mixture was separated and purified by Prep-HPLC to provide Compound 133 (9 mg). MS m/z (ESI): 534.3 $[M+H]^+$.

$^1$H NMR (400 MHz, DMSO-$d_6$) δ 11.98 (s, 1H), 9.66 (s, 1H), 9.12 (d, *J*= 2.2 Hz, 1H), 8.48 (d, *J*= 1.5 Hz, 1H), 8.44 (dd, *J* = 8.9, 2.3 Hz, 1H), 7.78 (dd, *J* = 8.2, 2.1 Hz, 1H), 7.60 (d, *J* = 8.1 Hz, 1H), 6.96 (d, *J* = 4.5 Hz, 1H), 6.84(br, 1H), 6.78 (d, *J* = 9.0 Hz, 1H), 6.31(br, 1H), 6.25 (dd, *J* = 3.2, 1.0 Hz, 1H), 3.82-3.68 (m, 4H), 3.68-3.45 (m, 4H), 2.60 - 2.53 (m, 1H), 2.36 (s, 3H), 2.33 (s, 3H), 2.26 (s, 3H), 1.59 (d, *J*= 8.4 Hz, 1H).

**Example 67: 6-methyl-N-(5-methyl-1H-pyrazol-3-yl)-2-(6-(6-((6-(oxazol-2-yl)pyridin-3-yl)methyl)-3,6-diazabicyclo[3.1.1]heptan-3-yl)pyridin-3-yl)pyrimidin-4-amine (Compound 134)**

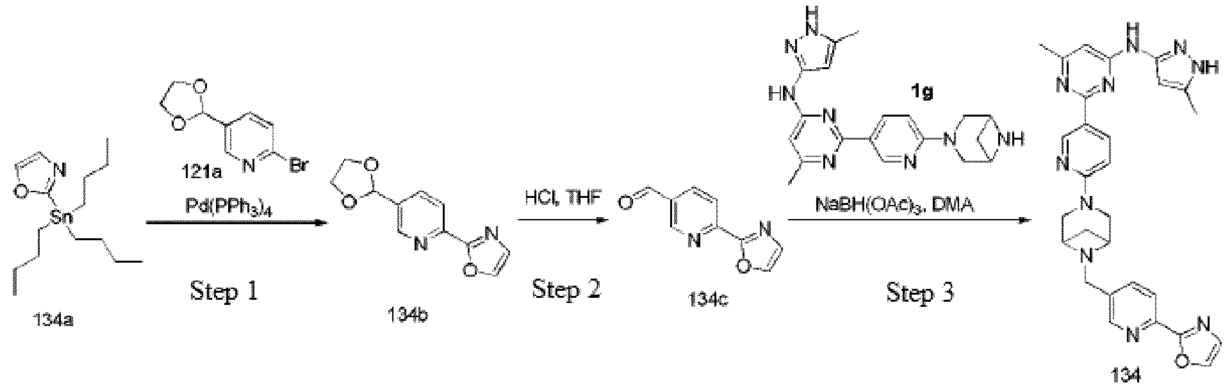


**Step 1: Preparation of 2-(5-(1,3-dioxolan-2-yl)pyridin-2-yl)oxazole (Compound 134b)**

Compound 121a (300.0 mg) and Compound 134a (467.0 mg) were dissolved in toluene (15.0 mL), and then tetrakis(triphenylphosphine)palladium (75.3 mg) was added. The mixture was stirred under the protection of nitrogen at 120 °C for 12 h. The reaction mixture was concentrated under reduced pressure, and then diluted with EA (200.0 mL). The organic phase was washed with water for 3 times, further washed with a saturated sodium chloride solution, dried over anhydrous sodium sulfate, filtered, concentrated, and separated and purified by silica gel column chromatography (PE:EA=7:3), to provide Compound 134b (78.0 mg). MS m/z (ESI): 219.1 $[M+1]^+$.

**Step 2: Preparation of 6-(oxazol-2-yl)nicotinaldehyde (Compound 134c)**

Compound 134b (78.0 mg) was dissolved in a mixed solvent of THF (2.0 mL) and water (2.0 mL), and then concentrated hydrochloric acid (1.0 mL, 12 N) was slowly added dropwise. The mixture was stirred at 25 °C for 8 h. The reaction mixture was adjusted with an aqueous solution of potassium carbonate to an alkaline pH, and extracted with EA (100.0 mL). The organic phase was washed with water for 3 times, further washed with a saturated sodium chloride solution, dried over anhydrous sodium sulfate, filtered, concentrated, and separated and purified by silica gel column chromatography (DCM:MeOH=9:1), to provide Compound 134c (51.0 mg). MS m/z (ESI): 175.1 $[M+1]^+$.

**Step 3: Preparation of 6-methyl-N-(5-methyl-1H-pyrazol-3-yl)-2-(6-(6-((6-(oxazol-2-yl)pyridin-3-yl)methyl)-3,6-diazabicyclo[3.1.1]heptan-3-yl)pyridin-3-yl)pyrimidin-4-amine (Compound 134)**

Compound 134c (28.8 mg) and Compound 1g (30.0 mg) were dissolved in N,N-dimethylacetamide (1.0 mL), and the mixture was stirred at 25 °C for 1 h. Then, sodium triacetoxyborohydride (70.2 mg) was added into the reaction system, and the mixture was stirred at 25 °C for 12 h. The reaction mixture was diluted with EA (20 mL), washed with

water for 3 times, further washed with a saturated sodium chloride solution, dried over anhydrous sodium sulfate, filtered, and concentrated to provide a crude product, which was separated and purified by Prep-HPLC, to provide Compound 134 (17.0 mg). MS m/z (ESI): 521.3 $[M+1]^+$.

$^1$H NMR (400 MHz, DMSO-$d_6$) δ 12.00 (s, 1H), 9.68 (s, 1H), 9.14 (d, *J*= 2.4 Hz, 1H), 8.66 (s, 1H), 8.45 (dd, *J*= 9.2, 2.4 Hz, 1H), 8.29 (s, 1H), 8.06 (d, *J*= 8.4 Hz, 1H), 7.95 (d, *J*= 8.0 Hz, 1H), 7.45 (s, 1H), 6.79 (br, 1H), 6.78 (d, *J* = 8.8 Hz, 1H), 6.32 (br, 1H), 3.79-3.75 (m, 4H), 3.67-3.60 (m, 4H), 2.58-2.56 (m, 1H), 2.34 (s, 3H), 2.26 (s, 3H), 1.61 (d, J = 8.0 Hz, 1H).

**Example 68: 2-(6-(6-((6-(1-isopropyl-1H-pyrazo|-4-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 135)**

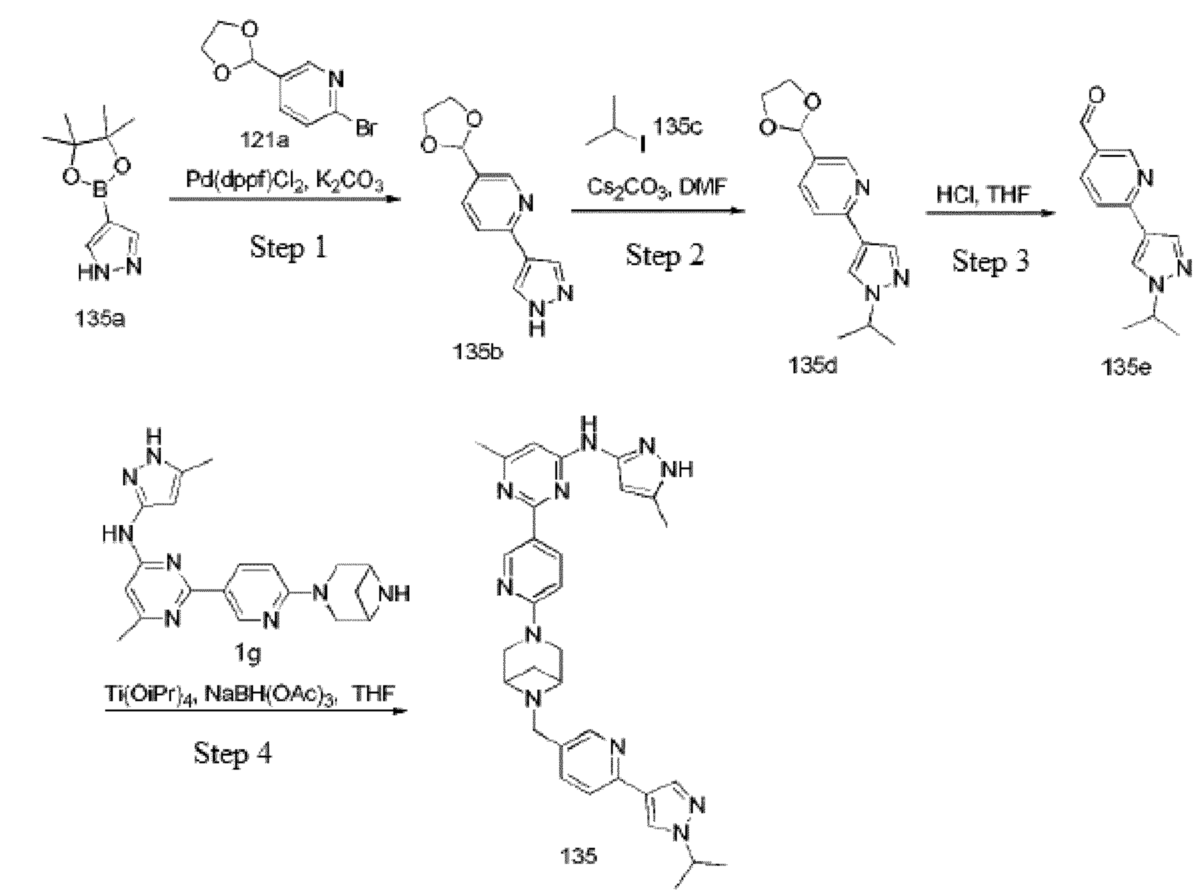


**Step** 1: **Preparation of 5-(1,3-dioxolan-2-yl)-2-(1H-pyrazol-4-yl)pyridine (Compound 135b)**

Compound 121a (1.0 g) and Compound 135a (1.0 g) were dissolved in 1,4-dioxane (30 mL) and $H_2O$ (6 mL), and then Pd(dppf)$Cl_2$ (130 mg) and $K_2CO_3$ (1.5 g) were successively added. The mixture was stirred under the protection of nitrogen at 95 °C for 2 h. After completion of the reaction, the mixture was cooled in an ice water bath, and filtered through Celite. The filtrate was concentrated to dryness, and separated and purified by MPLC to provide Compound 135b (452 mg). MS m/z (ESI): 218.2 $[M+H]^+$.

MPLC conditions:

Instrument model: Biotage Isolera Prime 2.3.1; chromatographic column: Agela Technologies C18 spherical 20-35 um 100A, 120 g; chromatographic column temperature: 25°C; flow rate: 30.0 mL/min; detection wavelength: 254 nm; eluent gradient: (0 min: 20% A, 80% B; 3.0 min: 20% A, 80% B; 25min: 90% A, 10% B); mobile phase A: acetonitrile, mobile phase B: 0.05% aqueous solution of TFA.

**Step 2: Preparation of 5-(1,3-dioxolan-2-yl)-2-(1-isopropyl-1H-pyrazol-4-yl)pyridine (Compound 135d)**

Compound 135b (100 mg) and $Cs_2CO_3$ (374.9 mg) were added into dry DMF (10 mL), and then Compound 135c (195.6 mg) was added. The mixture was stirred at 80 °C for 12 h. After completion of the reaction, water (100 mL) was added into the reaction mixture to quench the reaction. The reaction mixture was extracted with EA. The organic phase was washed with saturated brine, dried over anhydrous sodium sulfate, filtered, concentrated, and separated and purified by silica gel column chromatography (PE:EA=1:1), to provide Compound 135d (52 mg). MS m/z (ESI): 260.0 $[M+H]^+$.

**Step 3: Preparation of 6-(1-isopropyl-1H-pyrazol-4-yl)nicotinaldehyde (Compound 135e)**

Compound 135d (240 mg) was added into THF (4 mL) and $H_2O$ (2 mL), and then a solution of HCl in 1,4-dioxane (4 N, 1 mL) was added. The mixture was stirred at 25 °C for 5 h. After completion of the reaction, a saturated aqueous solution of sodium bicarbonate (50 mL) was added into the reaction mixture to quench the reaction. The reaction mixture was extracted with EA (50 mL×3). The organic phases were combined, washed with saturated brine, dried over anhydrous sodium sulfate, filtered, and then concentrated to dryness, to provide Compound 135e (40 mg). MS m/z (ESI): 216.1 [M+H].

**Step 4: Preparation of 2-(6-(6-((6-(1-isopropy|-1H-pyrazo|-4-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 135)**

Compound 1g (40 mg), Compound 135e (26.1 mg), and tetraisoproppyl titanate (31.4 mg) were added into dry THF (25 mL), and the mixture was stirred under the protection of nitrogen at 75 °C for 10 h. Then, sodium triacetoxyborohydride (23.4 mg) was added into the reaction system, and the mixture was stirred at 75 °C for an additional 6 h. After completion of the reaction, the reaction mixture was concentrated to dryness under reduced pressure, and separated and purified by Prep-HPLC to provide Compound 135 (2.1 mg). MS m/z (ESI): 562.1 $[M+H]^+$.

$^1H$ NMR (400 MHz, DMSO-$d_6$) δ 11.98 (s, 1H), 9.67 (s, 1H), 9.12 (d, *J* = 2.0 Hz, 1H), 8.56-8.39 (m, 2H), 8.32 (s, 1H), 7.97 (s, 1H), 7.71 (dd, *J* = 8.0, 2.0 Hz, 1H), 7.59 (d, *J* = 8.0 Hz, 1H), 7.05-6.65 (m, 2H), 6.33 (s, 1H), 4.64-4.36 (m, 1H), 3.94-3.63 (m, 4H), 3.64-3.45 (m, 4H), 2.62-2.50 (m, 1H), 2.31 (s, 3H), 2.29 (s, 3H), 1.56 (d, *J*= 8.0 Hz, 1H), 1.45 (d, *J* = 6.4 Hz, 6H).

**Example 69: 2-(1-(5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)pyridin-2-yl)-1H-pyrazol-5-yl)propan-2-ol (Compound 136)**

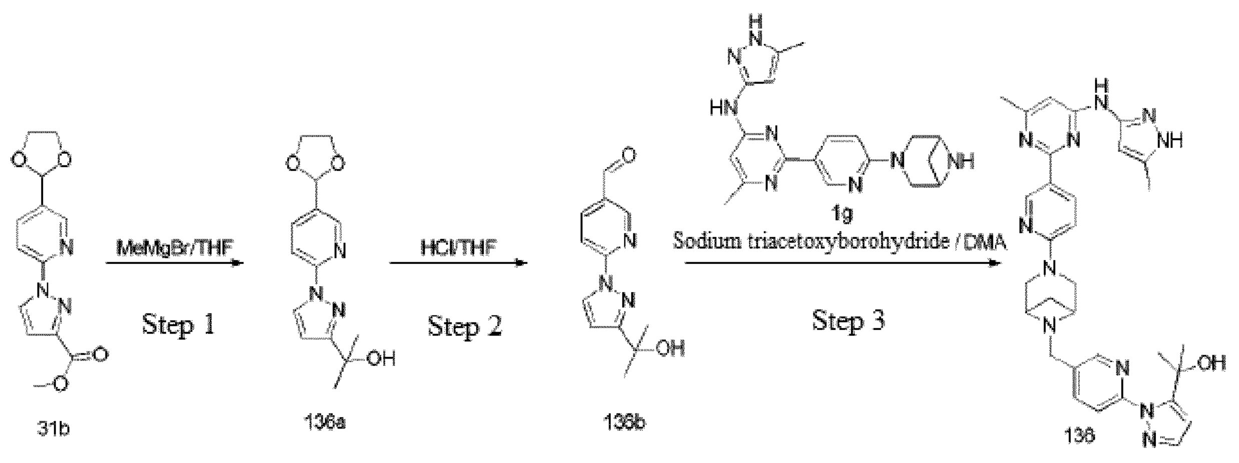


**Step 1: Preparation of 2-(1-(5-(1,3-dioxolan-2-yl)pyridin-2-yl)-1H-pyrazol-3-yl)propan-2-ol (Compound 136a)**

Compound 31b (200 mg) was added into dry THF (10 mL), and cooled in a dry ice-ethanol bath for 15 min. Then, a solution of methylmagnesium bromide in diethyl ether (3N, 0.65 mL) was slowly added dropwise, the mixture was kept for reaction at the temperature for 15 min, then warmed to room temperature, and kept for reaction at the temperature for an additional 4 h. A saturated aqueous solution of ammonium chloride (1 mL) was added into the reaction

mixture to quench the reaction. Then, the reaction mixture was diluted with water (30 mL), and extracted with EA (30 mL x 3). The organic phases were combined, washed with saturated brine, dried over anhydrous sodium sulfate, filtered, and then concentrated, to provide Compound 136a (200 mg). MS m/z (ESI): 276.1 $[M+H]^+$.

**Step 2: Preparation of 6-(3-(2-hydroxypropan-2-yl)-1H-pyrazol-1-yl)nicotinaldehyde (Compound 136b)**

Compound 136a (200 mg) was added into THF (5 mL), and then hydrochloric acid (2 N, 4.5 mL) was added. The mixture was stirred at 25°C for 12 h. The reaction mixture was adjusted with a saturated aqueous solution of sodium bicarbonate to a pH from 7 to 8, and extracted with EA (30 mL×3). The organic phases were combined, washed with saturated brine, dried over anhydrous sodium sulfate, filtered, and then concentrated, to provide Compound 136b (165 mg). MS m/z (ESI): 232.1 $[M+H]^+$.

**Step 3: Preparation of 2-(1-(5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)pyridin-2-yl)-1H-pyrazol-5-yl)propan-2-ol (Compound 136)**

Compound 136b (50.5 mg) and Compound 1g (30 mg) were added into DMA (3 mL), and the mixture was stirred under the protection of nitrogen at room temperature for 1 h. Then, sodium triacetoxyborohydride (101 mg) was added, and the mixture was kept at room temperature overnight. After completion of the reaction, water (60 mL) was added into the reaction mixture to quench the reaction. The reaction mixture was extracted with EA (30 mL × 3). The organic phases were combined, washed with saturated brine, dried over anhydrous sodium sulfate, filtered, concentrated, and separated and purified by Pre-HPLC, to provide Compound 136 (12 mg). MS m/z (ESI): 578.3 $[M+H]^+$.

$^1$H NMR (400 MHz, DMSO-$d_6$) $\delta$ 12.14 (br, 1H), 9.67 (s, 1H), 9.12 (d, *J*= 2.0 Hz, 1H), 8.50-8.41 (m, 2H), 8.41-8.35 (m, 1H), 7.99-7.92 (m, 1H), 7.82 (d, *J* = 8.4 Hz, 1H), 6.79 (d, *J*= 9.2 Hz, 2H), 6.52 (d, *J* = 2.4 Hz, 1H), 6.31 (br, 1H), 5.10 (s, 1H), 3.82-3.70 (m, 4H), 3.68-3.55 (m, 4H), 2.61-2.54 (m, 1H), 2.33 (s, 3H), 2.26 (s, 3H), 1.60 (d, *J*= 8.4 Hz, 1H), 1.49 (s, 6H).

**Example 70: (1-(5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)pyridin-2-yl)-1H-pyrazol-3-yl)methanol (Compound 137)**

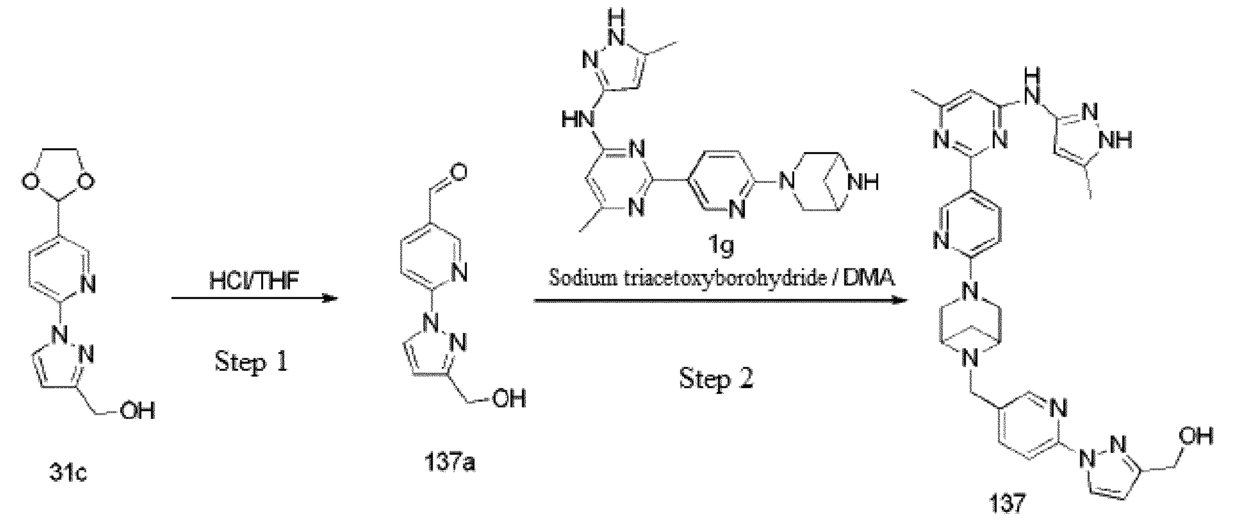


**Step 1: Preparation of 6-(3-(hydroxymethyl)-1H-pyrazol-1-yl)nicotinaldehyde (Compound 137a)**

Compound 31c (189 mg) was added into THF (5 mL), and then diluted hydrochloric acid (2 N, 5 mL) was added. The mixture was stirred at 25 °C for 2 h. The reaction mixture was adjusted with a saturated aqueous solution of sodium bicarbonate to a pH from 7 to 8, and extracted with EA (30 mL×3). The organic phases were combined, washed with saturated brine, dried over anhydrous sodium sulfate, filtered, and then concentrated, to provide Compound 137a (157 mg). MS m/z (ESI): 204.1 $[M+H]^+$.

**Step 2: Preparation of (1-(5-((3-(5-(4-methyl-6-((5-methyl-1H-pyrazol-3-yl)-amino)pyrimidin-2-yl)pyridin-2-yl)-3,6-diazabicyclo[3.1.1]heptan-6-yl)methyl)pyridin-2-yl)-1H-pyrazol-3-yl)methanol (Compound 137)**

Compound 137a (50.5 mg) and Compound 1g (50 mg) were added into DMA (2 mL), and the mixture was stirred under the protection of nitrogen at room temperature for 1 h. Then, sodium triacetoxyborohydride (159 mg) was added, and the mixture was kept at room temperature overnight. After completion of the reaction, water (60 mL) was added into the reaction mixture to quench the reaction. The reaction mixture was extracted with EA (30 mL × 3). The organic phases were combined, washed with saturated brine, dried over anhydrous sodium sulfate, filtered, concentrated, and separated and purified by Pre-HPLC, to provide Compound 137 (10 mg). MS m/z (ESI): 550.3 $[M+H]^+$.

$^1H$ NMR (400 MHz, DMSO-$d_6$) δ 12.00 (s, 1H), 9.68 (s, 1H), 9.13 (d, *J*= 2.0 Hz, 1H), 8.52 (d, *J* = 2.4 Hz, 1H), 8.44 (dd, *J* = 8.8, 2.4 Hz, 1H), 8.38 (d, *J* = 2.4 Hz, 1H), 7.94 (dd, *J* = 8.4, 2.0 Hz, 1H), 7.82 (d, *J*= 8.4 Hz, 1H), 7.06-6.65 (m, 2H), 6.51 (d, *J*= 2.4 Hz, 1H), 6.29 (br, 1H), 5.26 (t, *J* = 6.0 Hz, 1H), 4.53 (d, *J* = 5.6 Hz, 2H), 3.81-3.69 (m, 4H), 3.67-3.49 (m, 4H), 2.59-2.53 (m, 1H), 2.34 (s, 3H), 2.26 (s, 3H), 1.59 (d, *J*= 8.4 Hz, 1H).

**Example 71: 2-(6-(6-((6-(4-fluoro-3-methyl-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 138)**

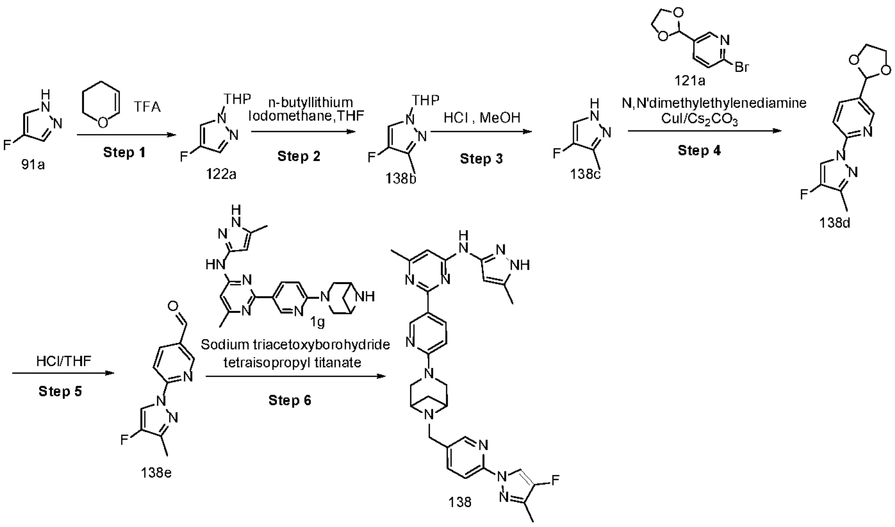


Compound 138 was prepared by referring to the synthesis method in Example 56. MS m/z (ESI): 551.8 $[M+H]^+$.

**Separation method:**

Prep-HPLC purification of the compounds in Examples 1 to 51, 53 to 58, and 60 to 71 were all carried out using Aglient 1260, Waters 2489, or GeLai 3500 HPLC at a column temperature of 25 °C, at a detection wavelength of 214 nm, 254 nm, or 280 nm, and with additional separation conditions as shown in the table below:

| Examples | Compounds | Separation column model | Mobile phase and gradient | Flow rate (mL/min) |
|---|---|---|---|---|
| 1 | 1 | Waters SunFire Prep $C_{18}$ OBD (19 mm×150 mm×5.0 μm) | A: MeCN; B: 0.05% aqueous solution of formic acid Gradient: 0 min 10% A, 90% B 4 min 10% A, 90% B 6 min 23.4% A, 76.6% B | 28.0 |
| 2 | 2 | Waters SunFire Prep $C_{18}$ OBD (19 mm×150 mm×5.0 μm) | A: MeCN; B: 0.05% aqueous solution of formic acid Gradient: 0 min 10% A, 90% B 2 min 10% A, 90% B 5 min 20.7% A, 79.3% B | 28.0 |
| 3 | 3 | Waters SunFire Prep $C_{18}$ OBD (19 mm×150 mm×5.0 μm) | A: MeCN; B: 0.05% aqueous solution of formic acid Gradient: 0 min 10% A, 90% B 2 min 10% A, 90% B 16 min 60% A, 40% B | 28.0 |

(continued)

| Examples | Compounds | Separation column model | Mobile phase and gradient | Flow rate (mL/min) |
|---|---|---|---|---|
| 4 | 4 | Waters Xbridge Prep $C_{18}$ OBD (19 mm×150 mm×5.0 μm) | A: MeCN; B: 0.05% aqueous solution of ammonium formate Gradient: 0 min 70% A, 30% B 4 min 70% A, 30% B 16 min 10% A, 90% B | 24.0 |
| 5 | 18 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 7.0 min: 20% A, 80% B | 24.0 |
| 6 | 17 | Waters Xbridge Prep $C_{18}$ OBD (19 mm×150 mm×5.0 μm) | A: 100% acetonitrile; B: 0.05% aqueous solution of ammonium formate Gradient: 0 min: 30% A, 70% B 4.0 min: 30%A, 70%B 16 min: 90% A, 10% B | 26.0 |
| 7 | 16 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 4 min: 30% A, 70% B 16 min: 90% A, 10% B | 26.0 |
| 8 | 15 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 2 min: 10% A, 90% B 16 min: 70% A, 30% B | 28.0 |
| 9 | 49 | Waters Xbridge Prep $C_{18}$ OBD (19 mm×150 mm×5.0 μm) | A: 100% acetonitrile; B: 0.05% aqueous solution of formic acid Gradient: 0 min: 10% A, 90% B 2 min: 10% A, 90% B 16 min: 90% A, 10% B | 28.0 |
| 10 | 23 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 60% A, 40% B | 24.0 |
| 11 | 21 | Waters SunFire Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 28.0 |
| 12 | 96 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 13 | 110 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 10 min: 36% A, 64% B | 28.0 |
| 14 | 111 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 10 min: 32% A, 68% B | 28.0 |
| 15 | 80 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 10% A, 90% B 2 min: 10% A, 90% B 16 min: 90% A, 10% B | 24.0 |
| 16 | 117 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 20% A, 80% B 3 min: 20% A, 80% B 16 min: 47% A, 53% B | 28.0 |

(continued)

| Examples | Compounds | Separation column model | Mobile phase and gradient | Flow rate (mL/min) |
|---|---|---|---|---|
| 17 | 118 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 20% A, 80% B 3 min: 20% A, 80% B 16 min: 47% A, 53% B | 28.0 |
| 18 | 62 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 19 | 60 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 4 min: 30% A, 70% B 16 min: 90% A, 10% B | 26.0 |
| 20 | 98 | Waters SunFire Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 24.0 |
| 21 | 69 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 60% A, 40% B | 28.0 |
| 22 | 67 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 24.0 |
| 23 | 41 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 4 min: 30% A, 70% B 16 min: 90% A, 10% B | 26.0 |
| 24 | 50 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 25 | 83 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 26 | 84 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 90% A, 10% B | 28.0 |
| 27 | 85 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 90% A, 10% B | 28.0 |
| 28 | 86 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 70% A, 30% B | 26.0 |
| 29 | 82 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 90% A, 10% B | 28.0 |
| 30 | 91 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 70% A, 30% B | 28.0 |

(continued)

| Examples | Compounds | Separation column model | Mobile phase and gradient | Flow rate (mL/min) |
|---|---|---|---|---|
| 31 | 88 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 32 | 121 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 33 | 70 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 24.0 |
| 34 | 63 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 35 | 64 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 2 min: 30% A, 70% B 16 min: 90% A, 10% B | 24.0 |
| 36 | 52 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 37 | 120 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 38 | 119 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 39 | 89 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 40 | 6 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 41 | 7 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 42 | 8 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 43 | 9 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 24.0 |
| 44 | 10 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 45 | 11 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 18 min: 60% A, 40% B | 24.0 |

(continued)

| Examples | Compounds | Separation column model | Mobile phase and gradient | Flow rate (mL/min) |
|---|---|---|---|---|
| 46 | 12 | Waters SunFire Prep $C_{18}$ OBD (19 mm×150 mm×5.0 μm) | A: MeCN; B: 0.05% aqueous solution of TFA Gradient: 0 min: 10% A, 90% B 15.0 min: 60% A, 40% B | 24.0 |
| 47 | 25 | Waters SunFire Prep $C_{18}$ OBD (19 mm×150 mm×5.0 μm) | A: MeCN; B: 0.05% aqueous solution of TFA Gradient: 0 min: 10% A, 90% B 8.0 min: 70% A, 30% B | 24.0 |
| 48 | 26 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 30.0 |
| 49 | 27 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 24.0 |
| 50 | 28/28' | Waters SunFire Prep $C_{18}$ OBD (19 mm×150 mm×5.0 μm) | A: MeCN; B: 0.05% aqueous solution of TFA Gradient: 0 min: 10% A, 90% B 16.0 min: 90% A, 10% B | 28.0 |
| 51 | 29 | Waters SunFire Prep $C_{18}$ OBD (19 mm×150 mm×5.0 μm) | A: MeCN; B: 0.05% aqueous solution of TFA Gradient: 0 min: 10% A, 90% B 16.0 min: 70% A, 30% B | 26.0 |
| 53 | 30 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 26.0 |
| 54 | 31 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 70% A, 30% B | 28.0 |
| 55 | 46 | Waters SunFire Prep $C_{18}$ OBD (19 mm×150 mm×5.0 μm) | A: MeCN; B: 0.05% aqueous solution of formic acid Gradient: 0 min: 10% A, 90% B 16.0 min: 90% A, 10% B | 30.0 |
| 56 | 122 | Waters SunFire Prep $C_{18}$ OBD (19 mm×150 mm×5.0 μm) | A: MeCN; B: 0.05% aqueous solution of TFA Gradient: 0 min: 10% A, 90% B 16.0 min: 90% A, 10% B | 28.0 |
| 57 | 123 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 58 | 124 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 70% A, 30% B | 30.0 |
| 60 | 126/127 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 90% A, 10% B | 28.0 |
| 61 | 128 | Waters SunFire Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 80% A, 20% B | 30.0 |
| 62 | 129 | Waters SunFire Prep $C_{18}$ OBD (19 mm×150 mm×5.0 μm) | A: 100% acetonitrile; B: 0.05% aqueous solution of ammonium bicarbonate Gradient: 0 min: 20% A, 80% B 16 min: 80% A, 20% B | 28.0 |

(continued)

| Examples | Compounds | Separation column model | Mobile phase and gradient | Flow rate (mL/min) |
|---|---|---|---|---|
| 63 | 130 | Waters SunFire Prep $C_{18}$ OBD (19 mm×150 mm×5.0 μm) | A: 100% acetonitrile; B: 0.05% aqueous solution of ammonium bicarbonate Gradient: 0 min: 20% A, 80% B 16 min: 80% A, 20% B | 28.0 |
| 64 | 131 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 90% A, 10% B | 28.0 |
| 65 | 132 | GeLai $C_{18}$ ODS(45 mm×450 mm×8 μm) | A: MeCN; B: 0.05% aqueous solution of ammonium bicarbonate Gradient: 0 min 25% A, 75% B 5 min 25% A, 75% B 50 min 70% A, 30% B | 70 |
| 66 | 133 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 30% A, 70% B 16 min: 80% A, 20% B | 28.0 |
| 67 | 134 | Waters Xbridge Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 28.0 |
| 68 | 135 | Waters SunFire Prep $C_{18}$ OBD (19 mm×150 mm×5.0 μm) | A: 100% acetonitrile; B: 0.05% aqueous solution of ammonium bicarbonate Gradient: 0 min: 10% A, 90% B 16 min: 90% A, 10% B | 28.0 |
| 69 | 136 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 60% A, 40% B | 30.0 |
| 70 | 137 | Waters SunFire Prep $C_{18}$ OBD (19 mm×150 mm×5.0 μm) | A: 100% acetonitrile; B: 0.05% aqueous solution of formic acid Gradient: 0 min: 10% A, 90% B 16 min: 90% A, 10% B | 28.0 |
| 71 | 138 | Waters SunFire Prep $C_{18}$ OBD (19 mm×150 mm×5.0 μm) | A: MeCN; B: 0.05% aqueous solution of TFA Gradient: 0 min: 10% A, 90% B 16.0 min: 90% A, 10% B | 28.0 |

The following intermediate compounds in the examples were purified using GeLai 3500 HPLC at a column temperature of 25 °C, at a detection wavelength of 214 nm, 254 nm, or 280 nm, and with additional separation conditions as shown in the table below:

| Compounds | Separation column model | Mobile phase and gradient | Flow rate (mL/min) |
|---|---|---|---|
| 1g | GeLai $C_{18}$ ODS(45 mm×450 mm×10 μm) | A: MeCN; B: 0.05% aqueous solution of TFA Gradient: 0 min 8% A, 92% B 10 min 8% A, 92% B 50 min 50% A, 50% B | 70 |
| 49d | GeLai $C_{18}$ ODS(45 mm×450 mm×8 μm) | A: MeCN; B: 0.05% aqueous solution of TFA Gradient: 0 min 8% A, 92% B 5 min 8% A, 92% B 50 min 50% A, 50% B | 240 |

## Biological Evaluation

### Experimental Example 1: RET Inhibition Experiment

Experimental method: According to the instructions of the HTRF KinEASE-TK kit (Cisbio), the compounds of the present disclosure were tested for their inhibitory effects on the activity of wild-type RET enzyme, mutant RET enzyme

(RET-V804M, RET-V804L, and RET-M918T) and fusion-type RET enzyme (RET-CCDC6). After pre-incubation of different RET enzymes and different concentrations of the test compounds at room temperature for 30 min, a substrate and adenosine triphosphate (ATP) were added to initiate the reaction. After incubation at room temperature for 40 min, TK antibody-cryptate and streptavidin-XL665 were added, and the test was performed after incubation at room temperature for 45 min. With the solvent group (DMSO) as the negative control and the buffer group (without RET enzyme) as the blank control, the relative inhibitory activity percentages (i.e., inhibition rates) of different concentrations of the compounds were computed as per the following formula:

Relative inhibitory activity percentage=1-(compound group of different concentrations-blank control)/(negative control-blank control)*100%

The relative inhibitory activity percentages of different concentrations of the compounds were plotted with respect to the compound concentrations, and the curve was fitted according to a four-parameter model to compute the $IC_{50}$ value as per the following formula:

$$y=min+(max-min)/(1+(x/IC_{50})^{(-Hillslope)})$$

where y is the relative inhibitory activity percentage, max is the maximum value of the fitted curve, min is the minimum value of the fitted curve, x is the logarithmic concentration of the compound, and Hillslope is the slope of the curve.

**Experimental Example 2: VEGFR2 Inhibition Experiment**

Experimental method: According to the instructions of the HTRF KinEASE-TK kit (Cisbio), the compounds of the present disclosure were tested for their inhibitory effects on the VEGFR2 enzyme activity. After pre-incubation of the VEGFR2 enzyme and different concentrations of test compounds at room temperature for 30 min, a substrate and adenosine triphosphate (ATP) were added to initiate the reaction. After incubation at room temperature for 40 min, TK antibody-cryptate and streptavidin-XL665 were added, and the test was performed after incubation at room temperature for 45 min. With the solvent group (DMSO) as the negative control and the buffer group (without VEGFR2 enzyme) as the blank control, the relative inhibitory activity percentages (i.e., inhibition rates) of different concentrations of the compounds were computed as per the following formula:

Relative inhibitory activity percentage=1-(compound group of different concentrations-blank control)/(negative control-blank control)*100%

The relative inhibitory activity percentages of different concentrations of the compounds were plottedwith respect to the compound concentrations, and the curve was fitted according to a four-parameter model to compute the $IC_{50}$ value as per the following formula:

$$y=min+(max-min)/(1+(x/IC_{50})^{(-Hillslope)})$$

where y is the relative inhibitory activity percentage, max is the maximum value of the fitted curve, min is the minimum value of the fitted curve, x is the logarithmic concentration of the compound, and Hillslope is the slope of the curve.

Experimental Results:

The experimental results are shown in Tables 1 to 4.

**Table 1** Inhibition rates of the compounds of the present disclosure at a concentration of 100 nM on the mutant RET enzyme activity

| Compound No. | Inhibition rate on RET-V804M | Inhibition rate on RET-M918T |
| --- | --- | --- |
| 1 | 86% | 33% |

(continued)

| Compound No. | Inhibition rate on RET-V804M | Inhibition rate on RET-M918T |
|---|---|---|
| 2 | 93% | 51% |
| 3 | 86% | 34% |
| 4 | N/A | 69% |
| **Note:** N/A means "not tested". | | |

As can be seen from Table 1, the compounds of the present disclosure have a significant inhibitory effect on the mutant RET enzyme.

**Table 2** Inhibition rate of the compound of the present disclosure on the enzyme RET-V804M

| Compound No. | Inhibition rate on RET-V804M | Compound No. | Inhibition rate on RET-V804M |
|---|---|---|---|
| 6 (10 nM) | 83% | 84 (100 nM) | 72% |
| 7 (10 nM) | 46% | 85 (100 nM) | 77% |
| 8 (10 nM) | 46% | 86 (10 nM) | 64% |
| 15 (100 nM) | 91% | 88 (10 nM) | 62% |
| 16 (100 nM) | 92% | 89 (10 nM) | 70% |
| 18 (100 nM) | 70% | 91 (10 nM) | 70% |
| 21 (100 nM) | 49% | 96 (100 nM) | 84% |
| 23 (10 nM) | 47% | 98 (100 nM) | 63% |
| 26 (10 nM) | 83% | 110 (100 nM) | 62% |
| 27 (10 nM) | 87% | 111 (100 nM) | 46% |
| 28' (10 nM) | 34% | 117 (100 nM) | 76% |
| 29 (10 nM) | 52% | 118 (100 nM) | 74% |
| 30 (10 nM) | 88% | 125-2 (10 nM) | 82% |
| 31 (10 nM) | 81% | 126 (100 nM) | 82% |
| 41 (10 nM) | 64% | 127 (100 nM) | 84% |
| 46 (10 nM) | 92% | 128 (10 nM) | 48% |
| 49 (100 nM) | 75% | 129 (10 nM) | 84% |
| 50 (100 nM) | 50% | 130 (10 nM) | 44% |
| 63 (10 nM) | 65% | 131 (10 nM) | 72% |
| 64 (10 nM) | 79% | 132 (10 nM) | 84% |
| 67 (100 nM) | 82% | 133 (10 nM) | 82% |
| 69 (10 nM) | 56% | 134 (10 nM) | 50% |
| 80 (10 nM) | 51% | 135 (10 nM) | 65% |
| 82 (10 nM) | 43% | 137 (10 nM) | 93% |
| 83 (10 nM) | 56% | - | - |

As can be seen from Table 2, the compounds of the present disclosure have a significant inhibitory effect on the enzyme RET-V804M.

**Table 3-1** The $IC_{50}$ (nM) of the compound of the present disclosure for inhibiting the enzyme RET-WT

| Compound No. | The $IC_{50}$ (nM) for inhibiting RET-WT |
|---|---|
| 17 | 1.32±0.31 |
| 60 | 2.70±0.69 |
| 120 | 7.33±1.19 |
| 122 | 2.35±0.24 |

**Table 3-2** The $IC_{50}$ (nM) of the compound of the present disclosure for inhibiting the enzyme RET-CCDC6

| Compound No. | The $IC_{50}$ (nM) for inhibiting RET-CCDC6 |
|---|---|
| 17 | 2.50±0.55 |
| 60 | 3.99±0.79 |
| 69 | 10.97±9.65 |
| 120 | 18.66±7.27 |
| 122 | 2.91±0.41 |

**Table 3-3** The $IC_{50}$ (nM) of the compound of the present disclosure for inhibiting the enzyme RET-V804L

| Compound No. | The $IC_{50}$ (nM) for inhibiting RET-V804L |
|---|---|
| 17 | 6.54±2.43 |
| 60 | 5.07±0.40 |
| 120 | 10.09±1.05 |
| 122 | 4.79±1.68 |

**Table 3-4** The $IC_{50}$ (nM) of the compound of the present disclosure for inhibiting the enzyme RET-V804M

| Compound No. | The $IC_{50}$ (nM) for inhibiting V804M |
|---|---|
| 9 | 5.43±0.91 |
| 10 | 3.97±0.30 |
| 11 | 7.22±0.58 |
| 12 | 6.94±0.78 |
| 17 | 1.03±0.47 |
| 25 | 6.29±0.90 |
| 28 | 10.41±1.38 |
| 52-2 | 12.95±2.34 |
| 60 | 3.77±0.52 |
| 62 | 4.53±1.23 |
| 70 | 41.36±3.73 |
| 119 | 14.21±1.27 |
| 120 | 9.12±1.85 |
| 121 | 6.22±1.39 |
| 122 | 3.33±0.75 |

(continued)

| Compound No. | The $IC_{50}$ (nM) for inhibiting V804M |
|---|---|
| 123 | 1.88±0.11 |
| 124 | 10.68±0.98 |
| 136 | 2.35±0.13 |

**Table 3-5** The $IC_{50}$ (nM) of the compound of the present disclosure for inhibiting the enzyme RET-M918T

| Compound No. | The $IC_{50}$ (nM) for inhibiting the enzyme RET-M918T |
|---|---|
| 17 | 1.47±0.29 |
| 60 | 1.29±0.27 |
| 69 | 8.60±1.46 |
| 120 | 15.81±3.65 |
| 122 | 4.03±0.66 |

As can be seen from Tables 3-1 to 3-5, the compounds of the present disclosure have a significant inhibitory effect on any one of the enzymes RET-CCDC6, RET-M918T, RET-V804M, RET-V804L, and RET-WT.

**Table 4** Inhibition rate of the compounds of the present disclosure on VEGFR2

| Compound No. | Inhibition rate on VEGFR2 | Compound No. | Inhibition rate on VEGFR2 |
|---|---|---|---|
| 1 (100 nM) | 38% | 70 (100 nM) | 14% |
| 2 (100 nM) | 29% | 80 (100 nM) | -10% |
| 3 (100 nM) | 14% | 82 (100 nM) | 15% |
| 4 (1000 nM) | 52% | 83 (100 nM) | 18% |
| 6 (100 nM) | -11% | 86 (100 nM) | 52% |
| 7 (100 nM) | 16% | 88 (100 nM) | 32% |
| 8 (300 nM) | 5% | 89 (100 nM) | 16% |
| 9 (300 nM) | 69% | 91 (100 nM) | 26% |
| 10 (300 nM) | 69% | 96 (100 nM) | 4% |
| 12 (300 nM) | 62% | 119 (100 nM) | -7% |
| 15 (100 nM) | 29% | 120 (100 nM) | 28% |
| 23 (100 nM) | -10% | 121 (100nM) | 37% |
| 25 (300 nM) | 53% | 122 (300 nM) | 67% |
| 26 (300 nM) | 64% | 123 (30 nM) | 56% |
| 27 (30 nM) | 23% | 124 (30 nM) | 41% |
| 28 (300 nM) | 23% | 125 (300 nM) | 44% |
| 29 (300 nM) | 47% | 125-2 (300 nM) | 63% |
| 30 (30 nM) | 51% | 128 (100 nM) | 20% |
| 31 (30 nM) | 17% | 129 (30 nM) | 52% |
| 41 (100 nM) | 22% | 130 (100 nM) | 63% |
| 46 (300 nM) | 79% | 131 (30 nM) | 50% |
| 52 (100 nM) | -5% | 132 (30 nM) | 31% |

(continued)

| Compound No. | Inhibition rate on VEGFR2 | Compound No. | Inhibition rate on VEGFR2 |
|---|---|---|---|
| 52-2 (300 nM) | 12% | 133 (50 nM) | 28% |
| 60 (100 nM) | 65% | 134 (100 nM) | 11% |
| 62 (100 nM) | 62% | 135 (100 nM) | 34% |
| 63 (100 nM) | 38% | 136 (30 nM) | 18% |
| 64 (100nM) | 38% | 137 (30 nM) | 26% |
| 69 (100 nM) | 2% | - | - |

In addition, tests showed that the $IC_{50}$ of Compound 11 for inhibiting VEGFR2 is 158.02 ± 25.08 nM, and the $IC_{50}$ of Compound 17 for inhibiting VEGFR2 is 62.97±11.77 nM. It was shown by the above results in combination with the inhibition rate data in Table 4 that the compounds of the present disclosure have weak inhibition on VEGFR2, and have better selective inhibitory effect on RET enzyme than on VEGFR2.

**Experimental Example 3: Pharmacokinetics and Tissue Distribution of Compounds in Rats**

By intragastric administration (PO) of BLU-667 (prepared according to Example 5 of WO2017/079140A1) and Compound 17 to male SD rats respectively, the plasma concentrations and the tissue concentrations of BLU-667 and Compound 17 in brains, lungs and thyroid of rats were determined to investigate the pharmacokinetic characteristics. The dosage of administration by PO was 5 mg/kg, and the solvent was 0.5% MC (methyl cellulose). With administration by PO, blood samples were collected at different time points (0 h before administration, and 0.25 h, 0.5 h, 1 h, 2 h, 4 h, 6 h, 8 h, and 24 h after administration). The blood samples were anticoagulated with dipotassium edetate, and centrifuged to provide plasma samples, which were stored at -80 °C. Rats were sacrificed by exsanguination from abdominal aorta at 0.5 h, 2 h, 8 h, and 24 h after PO administration. Brain, lung and thyroid were collected, washed, and homogenized with normal saline at a certain ratio, to provide tissue samples which were stored at -80 °C. The plasma samples and tissue samples were processed with precipitated protein and then analyzed by LC-MS/MS. The pharmacokinetic parameters were computed using WinNonlin 6.3 software and using a non-compartmental model. The results are shown in Table 5.

**Table 5** Pharmacokinetic Parameters of Compounds Administered by PO in Plasma and Tissues of Rats

| | **Compounds** | **BLU-667** | | | | **17** | | | |
|---|---|---|---|---|---|---|---|---|---|
| | **Samples** | **Plasma** | **Brain** | **Lung** | **Thyroid** | **Plasma** | **Brain** | **Lung** | **Thyroid** |
| | **Dosage, mg/kg** | 5 | 5 | 5 | 5 | 5 | 5 | 5 | 5 |
| | **AUC $AUC_{last}$, h*ng/mL** | 8650 | 157 | 25120 | 6351 | 6794 | 1011 | 79643 | 11416 |
| | **Peak concentration $C_{max}$, ng/mL** | 1490 | 30.8 | 3566 | 888 | 772 | 153 | 9620 | 1874 |
| | **Time to peak concentration $T_{max}$, h** | 1.00 | 0.50 | 0.50 | 0.50 | 2.00 | 2.00 | 2.00 | 8.49 |
| | **T/P Ratio of $AUC_{last}$ in tissue to plasma** | 1 | 0.018 | 2.90 | 0.73 | 1 | 0.15 | 11.70 | 1.68 |

The data in Table 5 shows that after intragastric administration of 5 mg/kg of BLU-667 and Compound 17 to SD rats, the exposed quantity of Compound 17 in each target organ tissue, such as brain, lung, and thyroid, was better than the exposed quantity of BLU-667.

**Experimental Example 4: Efficacy Test of Compound in Mice**

**Experimental purpose:** To evaluate the in vivo efficacy of Compound 17 and BLU-667 in a Balb/c-nu mouse

model with a subcutaneous xenograft tumor of human medullary thyroid carcinoma TT cells.

**Drug preparation:** Compound 17 was dissolved in 0.1 M aqueous solution of $H_3PO_4$, and BLU-667 was dissolved in 0.1 M aqueous solution of HOAc, to prepare clear solutions (pH: about 4.0). An aqueous solution of $H_3PO_4$ at a pH of about 4.0 was used for the solvent control group. The mode of administration was PO, BID.

**Tumor measurement:** The tumor diameter was measured with a vernier caliper twice a week. The computing formula of the tumor volume is: $V=0.5\times a\times b^2$, where a represents a long diameter of the tumor, and b represents a short diameter of the tumor. Evaluation of tumor growth inhibition rate (TGI (%)) for tumor inhibition efficacy of the compounds: TGI (%)=[(1-(mean tumor volume of a treatment group at the end of drug administration-mean tumor volume of the treatment group at the commencement of drug administration))/(mean tumor volume of a solvent control group at the end of treatment-mean tumor volume of the solvent control group at the commencement of treatment)] $\times$ 100%. The results are shown in FIG. 1.

**Statistical analysis:** Prism Graphpad5.0 software was used for statistical analysis based on the relative tumor volume at the end of the experiment. The comparison between multiple groups was analyzed by two-way ANOVA, and $p<0.05$ was considered as a significant difference.

**Experimental results:** In the TT nude mouse xenograft model of human medullary thyroid carcinoma, Compound 17 has a significant dose-dependent anti-tumor effect at a dose of 5 mg/kg. The anti-tumor effect of Compound 17 at a dose of 5 mg/kg (T/C=17.44%, TGI=131.36%, $p<0.05$) is better than that of the BLU-667 group at a dose of 5 mg/kg (T/C=33.62 %, TGI=88.82%, $p<0.05$). Relative tumor growth rate T/C (%)= $T_{RTV}/C_{RTV}\times100\%$, where $T_{RTV}$ is the mean relative tumor volume of the test compound group, $C_{RTV}$ is the relative tumor volume of the solvent control group; and the relative tumor volume RTV = $V_t/V_0$, where $V_0$ is the mean tumor volume at the commencement of the administration, and Vt is the mean tumor volume measured on day t after the administration.

The above examples do not limit the solution of the present disclosure in any way. In addition to those described herein, various modifications of the present disclosure will be apparent to those skilled in the art based on the foregoing description. Such modifications are also intended to fall within the scope of the appended claims. The references cited in the present disclosure (including all patents, patent applications, journal articles, books, and any other publications) are incorporated herein by reference in their entirety.

## Claims

1. A compound, a stereoisomer, tautomer, or mixture thereof, a N-oxide thereof, a pharmaceutically acceptable salt, eutecticum, polymorph, or solvate thereof, or a stable isotope derivative, metabolite, or prodrug thereof, wherein the compound has a structure of formula I:

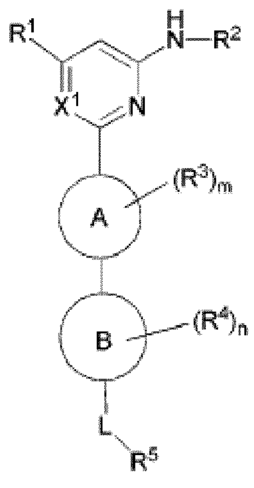

Formula I

wherein:

ring A is selected from $C_{6-10}$ aromatic ring and 5-6-membered heteroaromatic ring;
ring B is selected from $C_{3-8}$ cycloalkyl and 4-11-membered heterocyclyl;
$X^1$ is selected from CH and N;
$R^1$ is selected from the group consisting of H, halogen, hydroxy, cyano, $C_{1-6}$ alkyl, $C_{1-6}$ heteroalkyl (e.g., $C_{1-6}$

alkoxy), $C_{3-8}$ cycloalkyl, 4-10-membered heterocyclyl, and $-NR^{20a}R^{20b}$, and the alkyl, heteroalkyl (e.g., alkoxy), cycloalkyl, and heterocyclyl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, and $C_{1-4}$ heteroalkyl (e.g., $C_{1-4}$ alkoxy);

$R^2$ is selected from the group consisting of $C_{1-6}$ alkyl, $C_{1-6}$ heteroalkyl, $C_{3-8}$ cycloalkyl, 4-10-membered heterocyclyl, 5-10-membered heteroaryl, and $-C(=O)R^{21}$, and the alkyl, heteroalkyl, cycloalkyl, heterocyclyl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;

$R^3$ and $R^4$ are absent or are, at each occurrence, each independently selected from the group consisting of hydroxy, halogen, CN, $C_{1-6}$ alkyl, $C_{1-6}$ heteroalkyl (e.g., $C_{1-6}$ alkoxy), and $C_{3-6}$ cycloalkyl, the alkyl, heteroalkyl (for example, alkoxy), and cycloalkyl are each optionally substituted with one or more substituents selected from the group consisting of: halogen, CN, $C_{1-4}$ alkyl, $C_{1-4}$ haloalkyl, $C_{1-4}$ alkoxy, and $C_{1-4}$ haloalkoxy; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl;

L is selected from the group consisting of -O-, -S-, -S(O)-, $-S(O)_2-$, $-N=CR^{21}-$, $-N(R^{23a})-C(O)-$, $C_{1-6}$ alkylene, $C_{1-6}$ heteroalkylene, $C_{2-6}$ alkenylene, $C_{2-6}$ alkynylene,

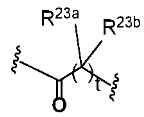 , 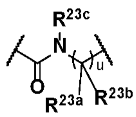 ,

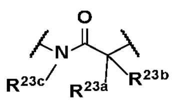 , 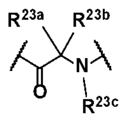 , 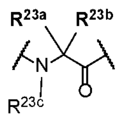 , 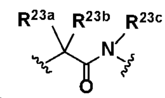 , 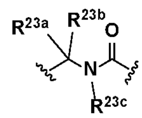 , 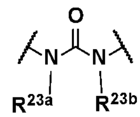 , 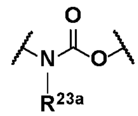 , 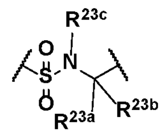 ,

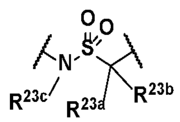 , 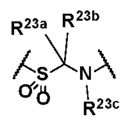 , 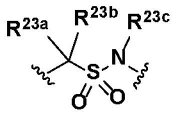 , 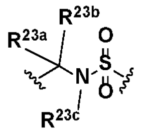 , 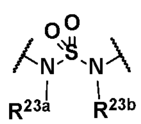 , 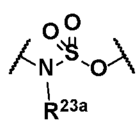 and 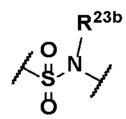 ,

the alkylene, heteroalkylene, alkenylene, and alkynylene are each optionally substituted with one or more substituents selected from the group consisting of: hydroxy, halogen, CN, $NO_2$, $C_{1-6}$ alkyl, $C_{1-6}$ haloalkyl, $C_{1-6}$ hydroxyalkyl, $C_{1-6}$ haloalkoxy, $C_{1-6}$ heteroalkyl (e.g., $C_{1-6}$ alkoxy), and $C_{3-8}$ cycloalkyl; or L is $-N(R^{23a})-$;

$R^5$ is selected from the group consisting of hydroxy, halogen, CN, $NO_2$, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-OR^{21}$, $-SR^{21}$, - $S(=O)R^{22}$, $-S(=O)_2R^{22}$, $-S(=O)NR^{20a}R^{20b}$, $-S(=O)_2NR^{20a}R^{20b}$, $-NR^{20a}S(=O)R^{20b}$, $-NR^{20a}S(=O)_2R^{20b}$, $-C(=O)R^{21}$, $-C(=O)NR^{23a}R^{23b}$, $-NR^{23a}C(=O)R^{23b}$, $-OC(=O)NR^{23a}R^{23b}$, and $-NR^{24a}C(=O)NR^{25a}R^{25b}$, and the alkyl, heteroalkyl (e.g., alkoxy), alkenyl, alkynyl, cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, - $OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$, - $NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$ and - $NR^{34a}C(=O)NR^{35a}R^{35b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl;

$R^{20a}$, $R^{20b}$, $R^{23a}$, $R^{23b}$, $R^{23c}$, $R^{24a}$, $R^{25a}$, and $R^{25b}$ are each independently selected from the group consisting of H, OH, $C_{1-6}$ alkyl, $C_{1-6}$ alkoxy, and $C_{3-8}$ cycloalkyl; or $R^{20a}$ and $R^{20b}$, $R^{23a}$ and $R^{23b}$, or $R^{25a}$ and $R^{25b}$ form, together with an atom to which they are attached, a 3-8-membered cycloalkyl or heterocyclyl, and the alkyl,

alkoxy, cycloalkyl, and heterocyclyl are each optionally substituted with one or more substituents selected from the group consisting of: OH, CN, halogen, $NO_2$, $C_{1-4}$ alkyl, $C_{1-4}$ alkoxy, $C_{1-4}$ hydroxyalkyl, $C_{1-4}$ haloalkyl, and $C_{1-4}$ haloalkoxy;

$R^{30a}$, $R^{30b}$, $R^{33a}$, $R^{33b}$, $R^{34a}$, $R^{35a}$, and $R^{35b}$ are each independently selected from the group consisting of H, $C_{1-6}$ alkyl, $C_{1-6}$ haloalkyl, $C_{1-6}$ hydroxyalkyl, $C_{1-6}$ alkoxy, and $C_{1-6}$ haloalkoxy;

$R^{21}$, $R^{22}$, $R^{31}$, and $R^{32}$ are each independently selected from the group consisting of $C_{1-6}$ alkyl, $C_{1-6}$ alkoxy, $C_{3-8}$ cycloalkyl, 4-10-membered heterocyclyl, $C_{6-12}$ aryl, and 5-10-membered heteroaryl, and the alkyl, alkoxy, cycloalkyl, heterocyclyl, aryl, and heteroaryl are each optionally substituted with one or more substituents selected from the group consisting of: OH, halogen, CN, $C_{1-4}$ alkyl, $C_{1-4}$ alkoxy, $C_{1-4}$ haloalkyl, $C_{1-4}$ haloalkoxy, $C_{3-6}$ cycloalkyl, and 4-10-membered heterocyclyl;

m is 0, 1, 2, 3, or 4, and is preferably 0;

n is 0, 1, 2, 3, or 4, and is preferably 0, 1, or 2;

t is 0, 1, 2, 3 or 4, and is preferably 0 or 1; and

u is 0, 1, 2, 3, or 4, and is preferably 0 or 1;

provided that when ring B is a piperazine ring and $X^1$ is CH, $R^2$ is not 4-$CF_3$-pyridin-2-yl or 4-CN-pyridin-2-yl.

2. The compound according to claim 1, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, wherein:

the ring A is a benzene ring or a 5-6-membered heteroaromatic ring;

preferably, the ring A is a benzene ring, a thiazole ring, a pyridine ring, a pyrazine ring, or a pyrimidine ring;

more preferably, the ring A is

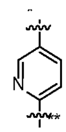, 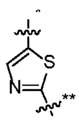, or 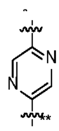,

is linked to the ring where $X^1$ is located through a position marked with *, and is linked to the ring B through a position marked with **;

and/or

the ring B is $C_{3-6}$ cycloalkyl or 5-7-membered heterocyclyl;

preferably, the ring B is a piperidine ring, a piperazine ring, an azacycloheptane bridged ring, or a diazacycloheptane bridged ring;

more preferably, the ring B is

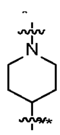, 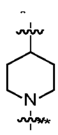, 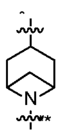, 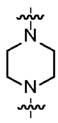, 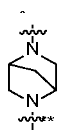, 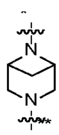, or 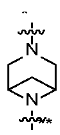,

is linked to the ring A through the position marked with *, and is linked to L through the position marked with **;

and/or

$X^1$ is CH or N, and is preferably N.

3. The compound according to claim 1 or 2, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, wherein:

$R^1$ is selected from the group consisting of H, halogen, hydroxy, cyano, $C_{1-4}$ alkyl, $C_{1-4}$ heteroalkyl (e.g., $C_{1-4}$ alkoxy), $C_{3-6}$ cycloalkyl, and 4-10-membered heterocyclyl, and the alkyl, heteroalkyl (for example, alkoxy), cycloalkyl, and heterocyclyl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, and $C_{1-4}$ heteroalkyl (e.g., $C_{1-4}$ alkoxy);

preferably, $R^1$ is selected from the group consisting of $C_{1-4}$ alkyl, 5-membered nitrogen-containing heterocyclyl,

and $C_{1-4}$ heteroalkyl (e.g., $C_{1-4}$ alkoxy), and the alkyl, heterocyclyl, and heteroalkyl (e.g., alkoxy) are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, and $C_{1-3}$ heteroalkyl (e.g., $C_{1-3}$ alkoxy);
more preferably, $R^1$ is selected from the group consisting of $C_{1-3}$ alkyl (e.g., methyl), pyrrolidinyl (e.g., pyrrolidin-1-yl), and $C_{1-3}$ alkoxy (e.g., ethoxy);
and/or
$R^2$ is selected from the group consisting of $C_{1-4}$ alkyl, $C_{1-4}$ heteroalkyl, $C_{3-6}$ cycloalkyl, 4-6-membered heterocyclyl, 5-6-membered heteroaryl, and -C(=O)$R^{21}$, and the alkyl, heteroalkyl, cycloalkyl, heterocyclyl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;
preferably, $R^2$ is selected from the group consisting of $C_{1-3}$ alkyl, 5-6-membered heteroaryl, and -C(=O)$CH_3$, and the alkyl and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl, and $C_{3-6}$ cycloalkyl;
more preferably, $R^2$ is selected from the group consisting of $C_{1-3}$ alkyl (e.g., methyl), - C(=O)$CH_3$, thienyl, pyrrolyl, pyrazolyl, imidazolyl, thiazolyl, thiadiazolyl, isothiazolyl, oxazolyl, oxadiazolyl, isoxazolyl, and pyridyl, and the alkyl, thienyl, pyrrolyl, pyrazolyl, imidazolyl, thiazolyl, thiadiazolyl, isothiazolyl, oxazolyl, oxadiazolyl, isoxazolyl, and pyridyl are each optionally substituted with one or more substituents selected from the group consisting of: hydroxy, halogen, CN, $C_{1-3}$ alkyl (e.g., methyl), $C_{1-3}$ haloalkyl, $C_{1-3}$ haloalkoxy, $C_{1-3}$ heteroalkyl (e.g., $C_{1-3}$ alkoxy), and $C_{3-6}$ cycloalkyl; and
further preferably, $R^2$ is a methyl-substituted pyrazolyl (e.g., 5-methyl-1H-pyrazol-3-yl or 1-methyl-1H-pyrazol-4-yl), a cyclopropyl-substituted pyrazolyl (e.g., 5-cyclopropyl-1H-pyrazol-3-yl), or -C(O)$CH_3$.

**4.** The compound according to any one of claims 1 to 3, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, wherein:

$R^3$ and $R^4$ are absent or are, at each occurrence, independently selected from the group consisting of hydroxy, halogen, CN, $C_{1-4}$ alkyl, and $C_{1-4}$ alkoxy, the alkyl and alkoxy are each optionally substituted with one or more substituents selected from the group consisting of: halogen, CN, $C_{1-4}$ alkyl, $C_{1-4}$ haloalkyl, $C_{1-4}$ alkoxy, and $C_{1-4}$ haloalkoxy; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl;
preferably, $R^3$ and $R^4$ are absent or are, at each occurrence, independently selected from the group consisting of hydroxy, halogen, CN, $C_{1-3}$ alkyl, $C_{1-3}$ alkoxy, the alkyl and alkoxy are each optionally substituted with one or more substituents selected from the group consisting of: halogen, CN, and $C_{1-3}$ alkyl; when m is greater than 1, two $R^3$ optionally form, together with an atom to which they are attached, a $C_{3-6}$ cycloalkyl or a 4-10-membered heterocyclyl; and/or when n is greater than 1, two $R^4$ optionally form, together with an atom to which they are attached, a $C_{3-6}$ cycloalkyl or a 4-10-membered heterocyclyl;
more preferably, $R^3$ and $R^4$ are absent or are, at each occurrence, independently selected from the group consisting of: F, Cl, CN, OH, $C_{1-3}$ alkyl, and $C_{1-3}$ alkoxy; and
further preferably, $R^3$ and $R^4$ are absent.

**5.** The compound according to any one of claims 1 to 4, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, wherein:

L is selected from the group consisting of -O-, -S-, -C(O)-, -N($R^{23a}$)-C(O)-, -C(O)-N($R^{23c}$)-, $C_{1-4}$ alkylene, $C_{1-4}$ heteroalkylene,

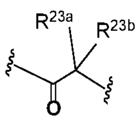 , 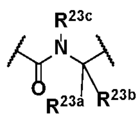 , 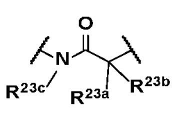 , 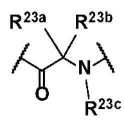 , 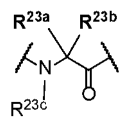 ,

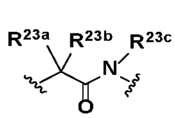, and 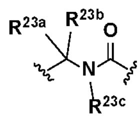,

and the alkylene and heteroalkylene are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), and $C_{3-6}$ cycloalkyl;
preferably, L is selected from the group consisting of -O-, -C(O)-, -NHC(O)-, -C(O)NH-, $C_{1-3}$ alkylene, $C_{1-3}$ heteroalkylene,

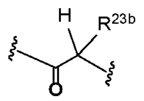, 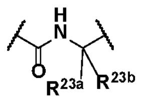, 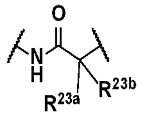, 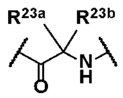, 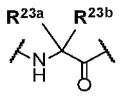,

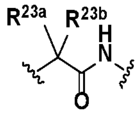, and 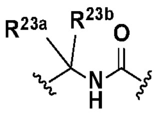,

and the alkylene and heteroalkylene are each optionally substituted with one or more substituents selected from the group of: hydroxy, halogen, CN, $NO_2$, $C_{1-3}$ alkyl, $C_{1-3}$ haloalkyl, $C_{1-3}$ hydroxyalkyl, $C_{1-3}$ haloalkoxy, $C_{1-3}$ heteroalkyl(e.g., $C_{1-3}$ alkoxy), and $C_{3-6}$ cycloalkyl, wherein $R^{23a}$ and $R^{23b}$ are preferably H or $C_{1-3}$ alkyl;
more preferably, L is selected from the group consisting of -O-, -C(O)-, -NHC(O)-, -C(O)NH- , $C_{1-3}$ alkylene,

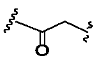 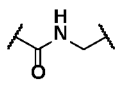, 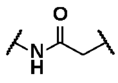, 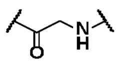, 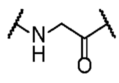, 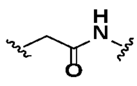, and 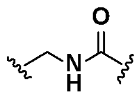,

and the alkylene is optionally substituted with one or more substituents selected from the group consisting of: hydroxy, halogen, CN, $C_{1-3}$ alkyl, and $C_{1-3}$ haloalkyl; and
further preferably, L is $-CH_2-$, $-CH(CH_3)-$, -O-, -C(O)-,

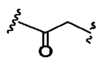,

-C(O)NH-, or

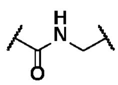.

**6.** The compound according to any one of claims 1 to 5, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof, wherein:

$R^5$ is selected from the group consisting of hydroxy, halogen, CN, $NO_2$, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{20a}R^{20b}$, $-OR^{21}$, $-SR^{21}$, - $S(=O)R^{22}$, $-S(=O)_2R^{22}$, $-S(=O)NR^{20a}R^{20b}$, $-S(=O)_2NR^{20a}R^{20b}$, $-NR^{20a}S(=O)R^{20b}$, $-NR^{20a}S(=O)_2R^{20b}$, $-C(=O)R^{21}$, $-C(=O)NR^{23a}R^{23b}$, $-NR2^{3a}C(=O)R^{23b}$, $-OC(=O)NR^{23a}R^{23b}$, and $-NR^{24a}C(=O)NR^{25a}R^{25b}$, and the alkyl, heteroalkyl (e.g., alkoxy), alkenyl, alkynyl, cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$ and $-NR^{34a}C(=O)NR^{35a}R^{35b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl;

preferably, $R^5$ is selected from the group consisting of $C_{3-6}$ cycloalkyl, 4-10-membered heterocyclyl, $C_{6-12}$ aryl, and 5-10-membered heteroaryl, and the cycloalkyl, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$ and $-NR^{34a}C(=O)NR^{35a}R^{35b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl;

more preferably, $R^5$ is selected from the group consisting of $C_{6-10}$ aryl and 5-6-membered heteroaryl, and the aryl and heretoaryl are each optionally substituted with one or more substituents selected from the group consisting of: hydroxy, halogen, CN, $NO_2$, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-C(=O)R^{31}$, $-C(=O)NR^{33a}R^{33b}$, and $-NR^{33a}C(=O)R^{33b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-6-membered heterocyclyl;

further preferably, $R^5$ is selected from phenyl and 5-6-membered heteroaryl (e.g., pyridyl, pyrimidyl, pyrazinyl, pyridazinyl, pyrazolyl, oxazolyl, imidazolyl, or thiazolyl), and the phenyl and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$alkoxy), $C_{3-6}$ cycloalkyl, $C_{3-6}$ cycloalkoxy, 4- 6-membered heterocyclyl, 5-8-membered heteroaryl (e.g., pyridyl, pyrrolyl, pyrazolyl, furyl, oxazolyl, imidazolyl, thiazolyl, or cyclopentyl-pyrazolyl), $-NR^{30a}R^{30b}$, $-OR^{31}$, $-C(=O)R^{31}$, $-C(=O)NR^{33a}R^{33b}$, and $-NR^{33a}C(=O)R^{33b}$, wherein the cycloalkyl, cycloalkoxy, heterocyclyl, and heteroaryl are each optionally substituted with one or more substituents selected from the group consisting of: hydroxy, halogen, CN, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$alkoxy), $C_{3-6}$ cycloalkyl, $C_{3-6}$ cycloalkoxy, and 4-6-membered heterocyclyl;

further more preferably, $R^5$ is selected from the group consisting of phenyl, pyridyl, pyrazolyl, and thiazolyl, and the phenyl, pyridyl, pyrazolyl, and thiazolyl are each optionally substituted with one or more substituents selected from the group consisting of: halogen, CN, $C_{1-3}$ alkyl, $C_{1-3}$ haloalkyl, $C_{1-3}$ hydroxyalkyl, $C_{1-3}$ haloalkoxy, $C_{1-3}$ alkoxy, $C_{3-6}$ cycloalkyl, $C_{3-6}$ cycloalkoxy, 4-6-membered heterocyclyl, 5-8-membered heteroaryl (e.g., pyridyl, pyrrolyl, pyrazolyl, furyl, oxazolyl, imidazolyl, thiazolyl, or cyclopentyl-pyrazolyl), $-NR^{30a}R^{30b}$, and $-OR^{31}$, wherein the heterocyclyl and heteroaryl are each optionally substituted with one or more substituents selected from the group consisting of: halogen, CN, $C_{1-3}$ alkyl, $C_{1-3}$ haloalkyl, $C_{1-3}$ hydroxyalkyl, $C_{1-3}$ haloalkoxy, $C_{1-3}$ alkoxy, $C_{3-6}$ cycloalkyl, $C_{3-6}$ cycloalkoxy, and 4-6-membered heterocyclyl; and

most preferably, $R^5$ is phenyl, pyridyl, pyrazolyl, or thiazolyl that is optionally substituted with one or more substituents selected from the group consisting of halogen (e.g., fluoro or chloro), CN, $C_{1-3}$ alkyl (e.g., methyl or ethyl), $C_{1-3}$ haloalkyl (e.g., trifluoromethyl), $C_{1-3}$ alkoxy (e.g., methoxy or ethoxy), $C_{3-6}$ cycloalkyl (e.g., cyclopropyl), $C_{3-6}$ cycloalkoxy (e.g., cyclopropoxy), and 5-6-membered heteroaryl (e.g., pyridyl, pyrrolyl, pyrazolyl, furyl, oxazolyl, imidazolyl, or thiazolyl), wherein the 5-6-membered heteroaryl is optionally further substituted with one or more substituents selected from the group consisting of halogen (e.g., fluoro or chloro), $C_{1-3}$ alkyl (e.g., methyl, ethyl, or isopropyl), $C_{1-3}$ haloalkyl (e.g., fluoromethyl), $C_{1-3}$ hydroxyalkyl (e.g., hydroxymethyl or hydroxypropyl), $C_{1-3}$ alkoxy (e.g., methoxy), $C_{3-6}$ cycloalkyl (e.g., cyclopropyl), and $C_{3-6}$ cycloalkoxy (e.g., cyclopropoxy or cyclobutoxy).

**7.** The compound according to any one of claims 1 to 6, the stereoisomer, tautomer, or mixture thereof, the N-oxide

thereof, the pharmaceutically acceptable salt, eutecticum, polymorph, or solvate thereof, or the stable isotope derivative, metabolite, or prodrug thereof, wherein:

$R^{20a}$, $R^{20b}$, $R^{23a}$, $R^{23b}$, $R^{23c}$, $R^{24a}$, $R^{25a}$, and $R^{25b}$ are each independently selected from the group consisting of H, $C_{1-4}$ alkyl, $C_{1-4}$ alkoxy, and $C_{3-8}$ cycloalkyl; or $R^{20a}$ and $R^{20b}$, $R^{23a}$ and $R^{23b}$, or $R^{25a}$ and $R^{25b}$ form, together with an atom to which they are attached, a 3-8-membered cycloalkyl or heterocyclyl, and the alkyl, alkoxy, cycloalkyl, and heterocyclyl are each optionally substituted with one or more substituents selected from the group consisting of: OH, CN, halogen, $NO_2$, $C_{1-4}$ alkyl, $C_{1-4}$ alkoxy, $C_{1-4}$ hydroxyalkyl, $C_{1-4}$ haloalkyl, and $C_{1-4}$ haloalkoxy;

preferably, $R^{20a}$, $R^{20b}$, $R^{23a}$, $R^{23b}$, $R^{23c}$, $R^{24a}$, $R^{25a}$, and $R^{25b}$ are each independently H, $C_{1-4}$ alkyl, or $C_{1-4}$ alkoxy;

In particular, $R^{23a}$ and $R^{23b}$ are each independently selected from the group consisting of H, $C_{1-3}$ alkyl, $C_{1-3}$ alkoxy, and $C_{3-6}$ cycloalkyl; or $R^{23a}$ and $R^{23b}$ form, together with a C atom to which they are attached, a $C_{3-6}$ cycloalkyl or heterocyclyl, and the alkyl, alkoxy, cycloalkyl, and heterocyclyl are each optionally substituted with one or more substituents selected from the group consisting of: halogen, $C_{1-3}$ alkyl, $C_{1-3}$ alkoxy, $C_{1-3}$ hydroxyalkyl, $C_{1-3}$ haloalkyl, and $C_{1-3}$ haloalkoxy;

and/or

$R^{21}$, $R^{22}$, $R^{31}$, and $R^{32}$ are each independently selected from the group consisting of $C_{1-4}$ alkyl, $C_{1-4}$ alkoxy, $C_{3-8}$ cycloalkyl, and 4-10-membered heterocyclyl, and the alkyl, alkoxy, cycloalkyl, and heterocyclyl are each optionally substituted with one or more substituents selected from the group consisting of: OH, halogen, CN, $C_{1-4}$ alkyl, $C_{1-4}$ alkoxy, $C_{1-4}$ haloalkyl, $C_{1-4}$ haloalkoxy, $C_{3-6}$ cycloalkyl, and 4-10-membered heterocyclyl;

preferably, $R^{21}$, $R^{22}$, $R^{31}$, and $R^{32}$ are each independently selected from $C_{1-4}$ alkyl;

and/or

$R^{30a}$, $R^{30b}$, $R^{33a}$, $R^{33b}$, $R^{34a}$, $R^{35a}$, and $R^{35b}$ are each independently selected from the group consisting of H, $C_{1-4}$ alkyl, $C_{1-4}$ haloalkyl, $C_{1-4}$ hydroxyalkyl, $C_{1-4}$ alkoxy, and $C_{1-4}$ haloalkoxy; and

preferably, $R^{30a}$, $R^{30b}$, $R^{33a}$, $R^{33b}$, $R^{34a}$, $R^{35a}$, and $R^{35b}$ are each independently selected from H and $C_{1-4}$ alkyl.

**8.** The compound according to claim 1, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof,

wherein the compound has a structure shown in one of formula I-A to formula I-G:

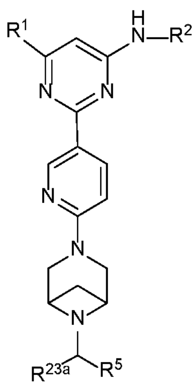

I-A ;

wherein:

$R^5$ is selected from the group consisting of $C_{6-12}$ aryl and 5-10-membered heteroaryl, wherein (1) the $C_{6-12}$ aryl is optionally substituted with one or more substituents selected from the group consisting of: $C_{3-6}$ cycloalkoxy, $C_{6-12}$ aryl, 5-10-membered heteroaryl, $-S(=O)R^{32}$, $-S(=O)_2R^{32}$, - $S(=O)NR^{30a}R^{30b}$, $-S(=O)2NR^{30a}R^{30b}$, $-NR^{30a}S(=O)R^{30b}$, $-NR^{30a}S(=O)_2R^{30b}$, $-C(=O)R^{31}$, - $C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, wherein the cycloalkoxy, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl, and (2) the 5-10-membered heteroaryl is optionally substituted with one or more substituents selected from the group consisting of: hydroxy, halogen, CN, $NO_2$, $C_{1-4}$ alkyl, $C_{1-4}$ haloalkyl, $C_{1-4}$

hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$ and $-NR^{34a}C(=O)NR^{35a}R^{35b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, $C_{3-6}$ cycloalkoxy, and 4-10-membered heterocyclyl; and

$R^1$, $R^2$, $R^{23a}$, $R^{30a}$, $R^{30b}$, $R^{31}$, $R^{32}$, $R^{33a}$, $R^{33b}$, $R^{34a}$, $R^{35a}$, and $R^{35b}$ are as defined in any one of claims 1 to 7, and $R^{23a}$ is preferably H or $C_{1-3}$ alkyl;

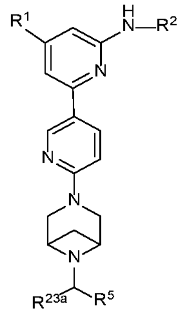

I-B

wherein:

$R^1$, $R^2$, $R^5$, and $R^{23a}$ are as defined in any one of claims 1 to 7, and $R^{23a}$ is preferably H or $C_{1-3}$ alkyl;

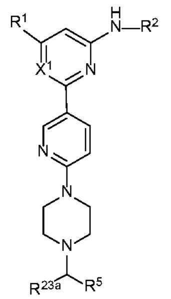

I-C

wherein:

when $X^1$ is CH, $R^1$, $R^2$, $R^5$, and $R^{23a}$ are as defined in any one of claims 1 to 7, $R^{23a}$ is preferably H or $C_{1-3}$ alkyl; and when $X^1$ is N, $R^1$, $R^2$, $R^5$, and $R^{23a}$ are as defined in the above formula I-A;

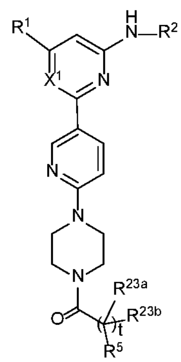

I-D

wherein:

$R^1$, $R^2$, $R^{23a}$, $R^{23b}$, and t are as defined in any one of claims 1 to 7;
when $X^1$ is CH, $R^5$ is as defined in any one of claims 1 to 7; and
when $X^1$ is N, $R^5$ is $C_{6-12}$ aryl or 5-10-membered heteroaryl, wherein

(i) when t is 0, the $C_{6-12}$ aryl and 5-10-membered heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, - $S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, - $C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$cycloalkyl, and 4-10-membered heterocyclyl,

(ii) when t is 1, (1) the $C_{6-12}$ aryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, - $NR^{30a}S(=O)R^{30b}$, $-NR^{30a}S(=O)_2R^{30b}$ $-C(=O)R^{31}$, $-C(=O)NR^{33a}R^{33b}$ $-NR^{33a}C(=O)R^{33b}$, - $OC(=O)NR^{33a}R^{33b}$, and $-NR^{34a}C(=O)NR^{35a}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4- 10-membered heterocyclyl, and (2) the 5-10-membered heteroaryl is optionally substituted with one or more substituents selected from the group consisting of: $NO_2$, $C_{2-6}$ alkenyl, $C_{2-6}$ alkynyl, $C_{3-6}$ cycloalkoxy, $C_{6-12}$ aryl, 5-10-membered heteroaryl, $-NR^{30a}R^{30b}$, $-OR^{31}$, $-SR^{31}$, $-S(=O)R^{32}$, - $S(=O)_2R^{32}$, $-S(=O)NR^{30a}R^{30b}$, $-S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, $-NR^{33a}C(=O)R^{33b}$, $-OC(=O)NR^{33a}R^{33b}$, and $-NR^{34a}C(=O)NR^{35a}R^{35b}$, wherein the aryl and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and

$R^{30a}$, $R^{30b}$, $R^{31}$, $R^{32}$, $R^{33a}$, $R^{33b}$, $R^{34a}$, $R^{35a}$, and $R^{35b}$ are as defined in any one of claims 1 to 7;

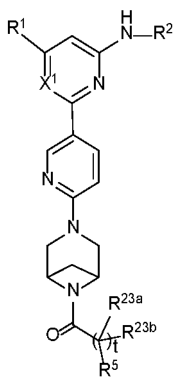

I-E

wherein:
$R^1$, $R^2$, $R^5$, $R^{23a}$, $R^{23b}$, $X^1$, and t are as defined in the above formula I-D;

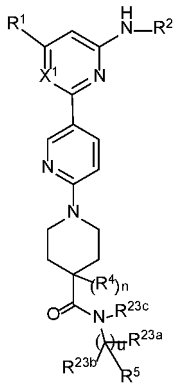

I-F

wherein:

$R^1$, $R^2$, $R^5$, $R^{23a}$, $R^{23b}$, and $X^1$ are as defined in the above formula I-D;
$R^4$ is as defined in any one of claims 1 to 7, and is preferably $C_{1-3}$ alkyl or $C_{1-3}$ alkoxy;
$R^{23c}$ is H, $C_{1-3}$ alkyl, or $C_{1-3}$ alkoxy, and the alkyl and alkoxy are each optionally substituted with one or more substituents selected from the group consisting of: OH, CN, halogen, $C_{1-4}$ alkoxy, and $C_{1-4}$ hydroxyalkyl;
u is 0 or 1; and
n is 0 or 1;

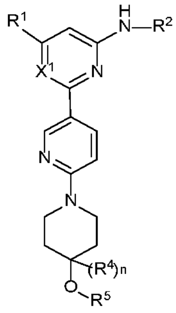

I-G

wherein:

$X^1$ is CH orN;
$R^1$, $R^2$, and $R^4$ are as defined in any one of claims 1 to 7, and $R^4$ is preferably $C_{1-3}$ alkyl or $C_{1-3}$ alkoxy;
n is 0 or 1;
$R^5$ is selected from $C_{6-12}$ aryl and 5-10-membered heteroaryl, wherein (1) the $C_{6-12}$ aryl is optionally substituted with one or more substituents selected from the group consisting of: $C_{3-6}$ cycloalkoxy, $C_{6-12}$ aryl, 5-10-membered heteroaryl, $-S(=O)R^{32}$, $-S(=O)_2R^{32}$, $-S(=O)NR^{30a}R^{30b}$, - $S(=O)_2NR^{30a}R^{30b}$, $-NR^{30a}S(=O)R^{30b}$, $-NR^{30a}S(=O)_2R^{30b}$, $-C(=O)R^{31}$, $-C(=O)NR^{33a}R^{33b}$, - $NR^{33a}C(=O)R^{33b}$, $-OC(=O)NR^{33a}R^{33b}$, and $-NR^{34a}C(=O)NR^{35a}R^{35b}$, wherein the cycloalkoxy, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and (2) the 5-10-membered heteroaryl is optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-10-membered heterocyclyl, $C_{6-12}$ aryl, 5-10-membered heteroaryl, $-NR^{30a}R^{30b}$, $-OR^{31}$, $-SR^{31}$, $-S(=O)R^{32}$, $-S(=O)_2R^{32}$, $-S(=O)NR^{30a}R^{30b}$, $-S(=O)_2NR^{30a}R^{30b}$, - $NR^{30a}S(=O)R^{30b}$, $-NR^{30a}S(=O)_2R^{30b}$, $-C(=O)R^{31}$, $-C(=O)NR^{33a}R^{33b}$ $-NR^{33a}C(=O)R^{33b}$, - $OC(=O)NR^{33a}R^{33b}$, and $-NR^{34a}C(=O)NR^{35a}R3^{5b}$, wherein the cycloalkyl, cycloalkoxy, heterocyclyl, aryl, and heteroaryl are each optionally substituted with one or more substituents selected from the group consisting of: hydroxy, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl, and 4-10-membered heterocyclyl; and

$R^{30a}$, $R^{30b}$, $R^{31}$, $R^{32}$, $R^{33a}$, $R^{33b}$, $R^{34a}$, $R^{35a}$, and $R^{35b}$ are as defined in any one of claims 1 to 7.

**9.** The compound according to claim 1, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof,
wherein the compound is selected from:

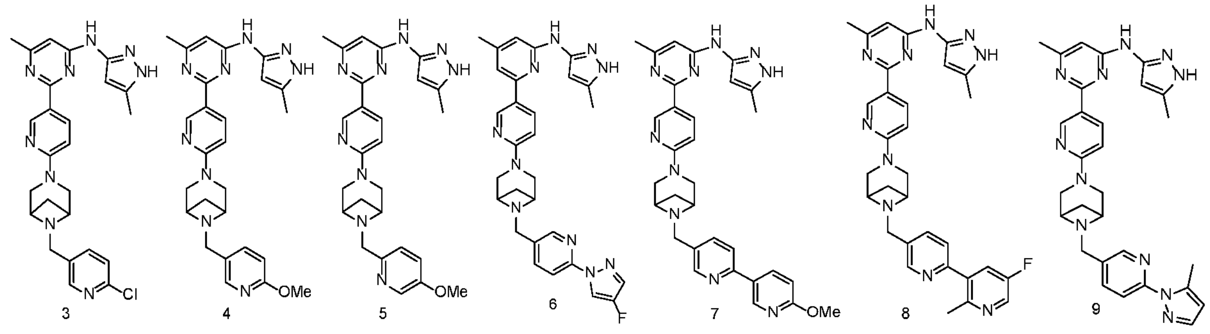

3 4 5 6 7 8 9

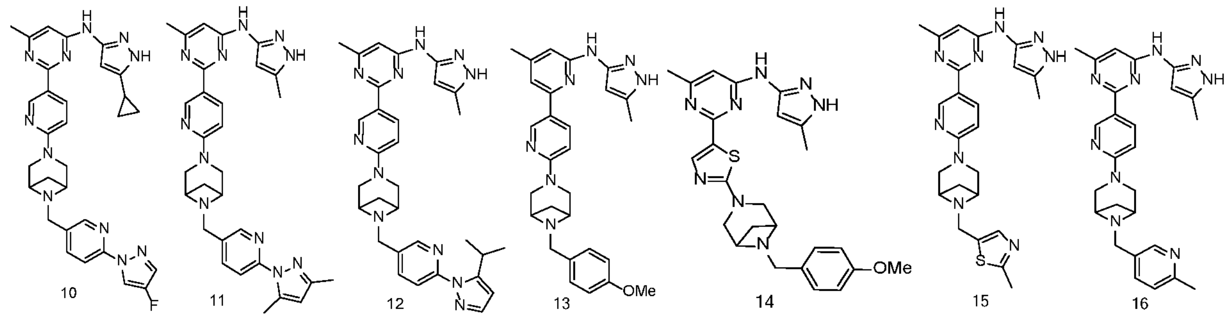

10 11 12 13 14 15 16

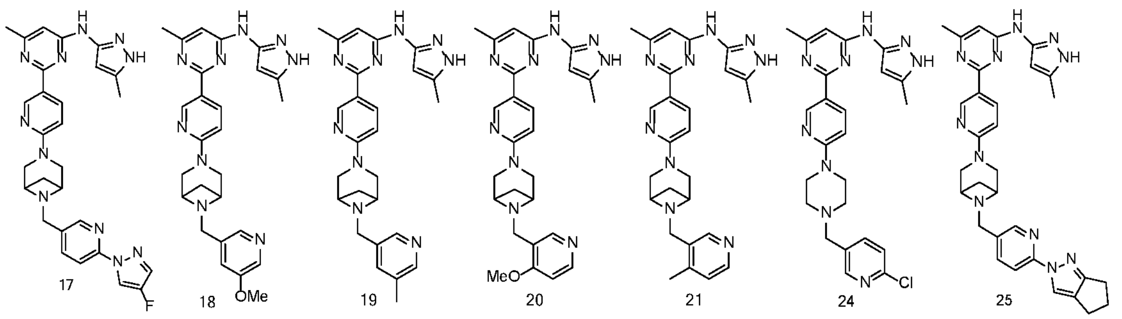

17 18 19 20 21 24 25

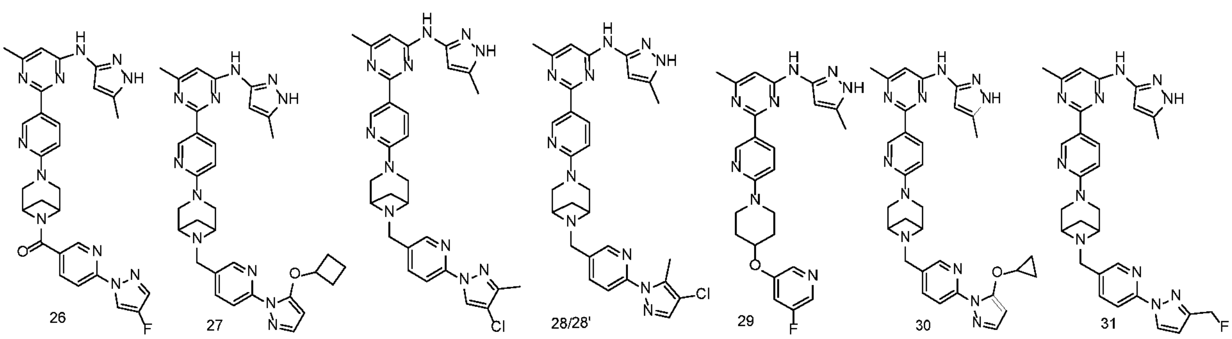

26 27 28/28' 29 30 31

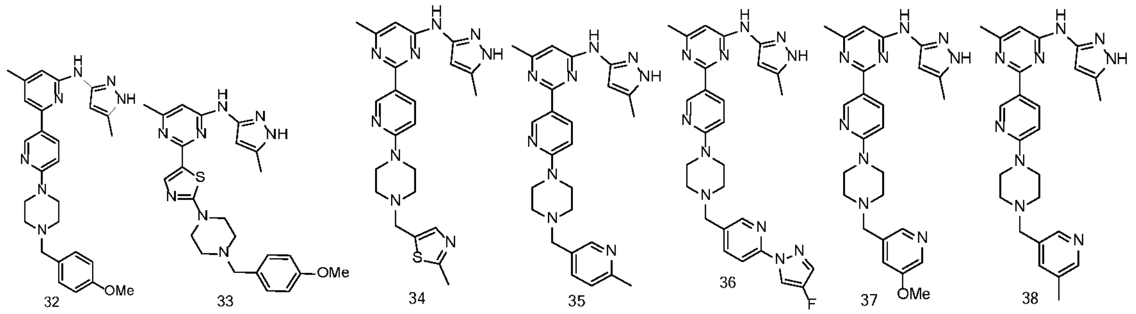

32 33 34 35 36 37 38

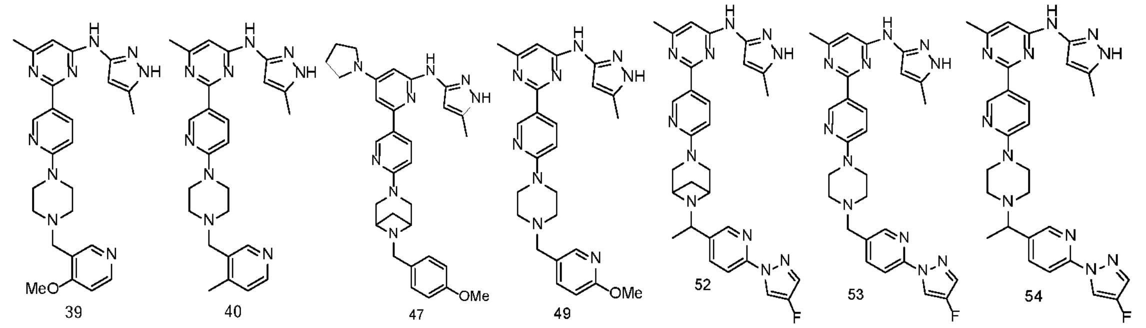

39 40 47 49 52 53 54

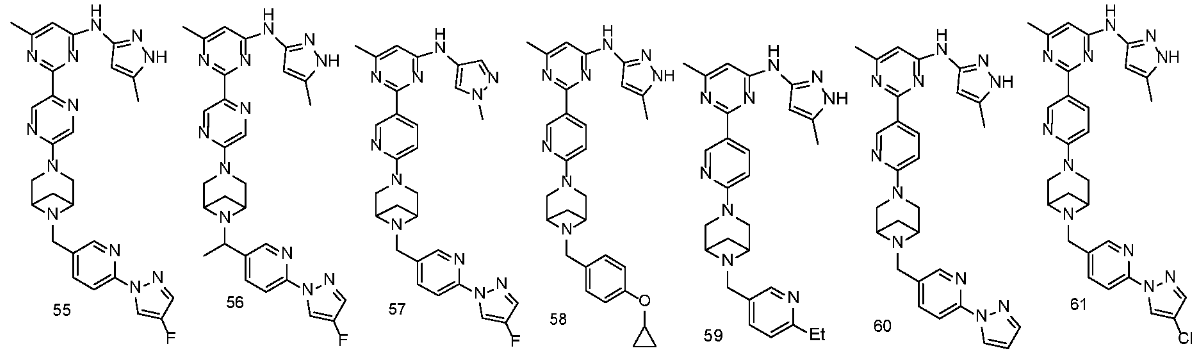

55 56 57 58 59 60 61

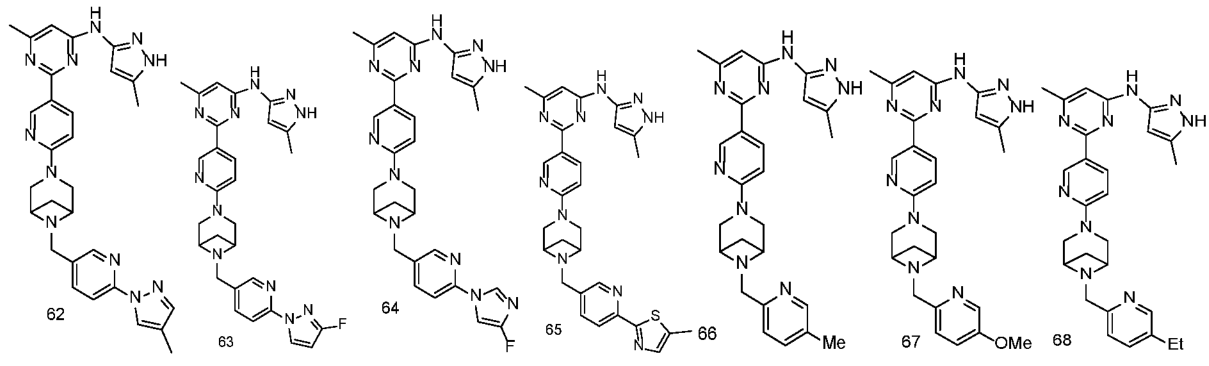

62 63 64 65 66 67 68

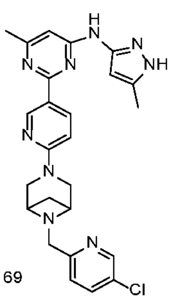
69
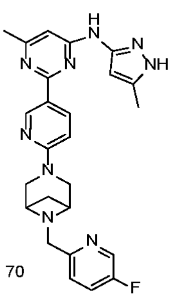
70
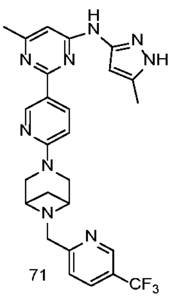
71
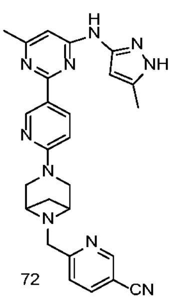
72
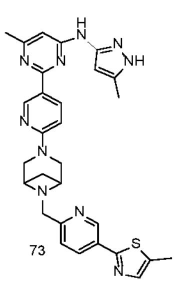
73
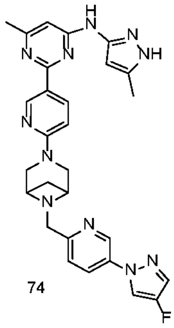
74
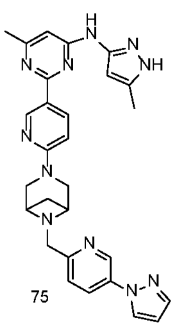
75

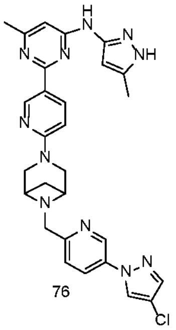
76
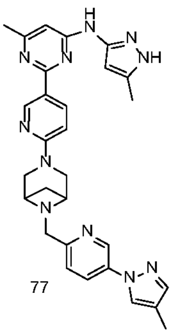
77
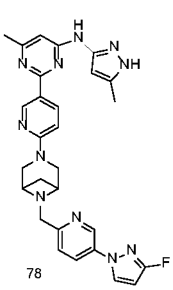
78
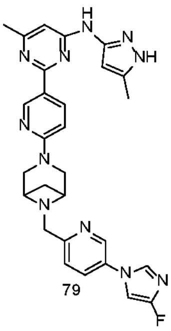
79
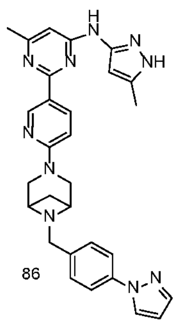
86
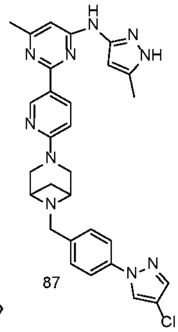
87
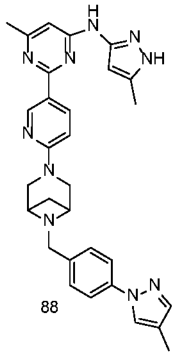
88

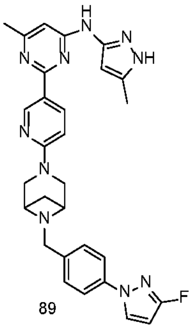
89
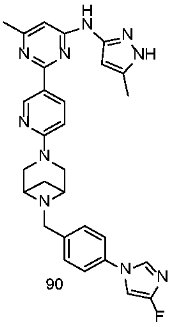
90
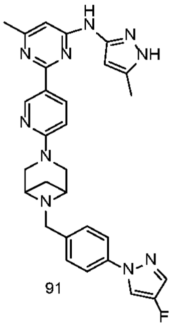
91
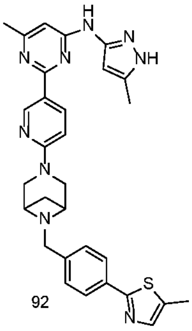
92
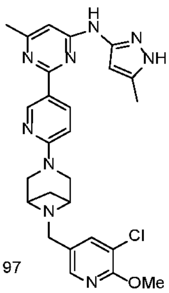
97
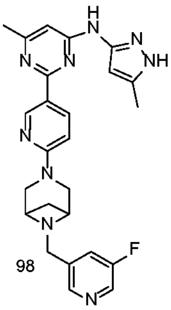
98
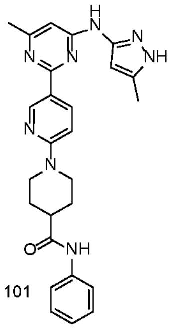
101

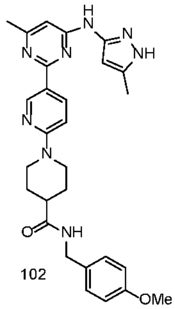
102
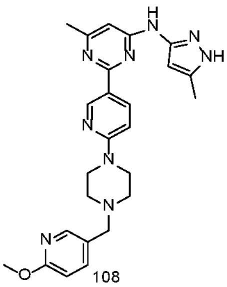
108
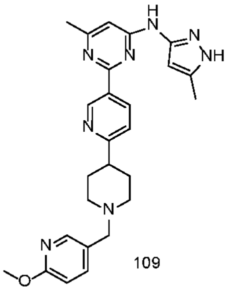
109
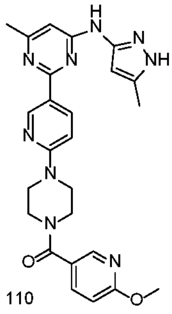
110
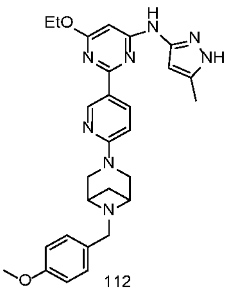
112
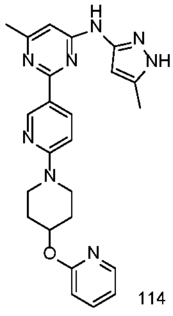
114

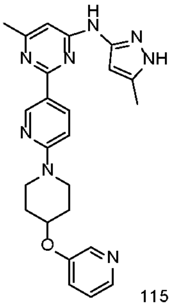 115 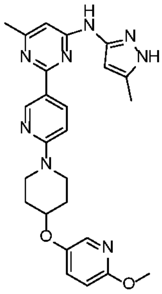 116 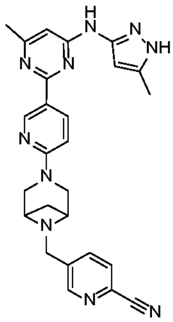 117 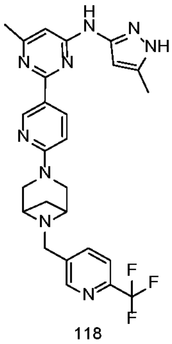 118 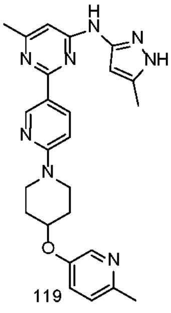 119 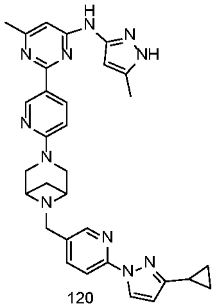 120

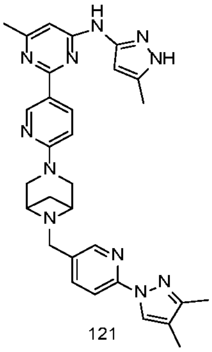 121 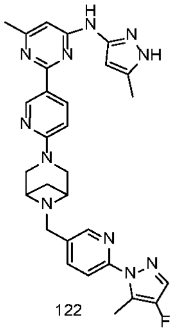 122 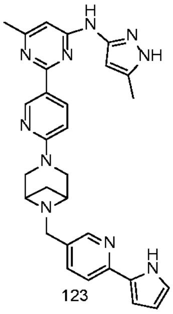 123 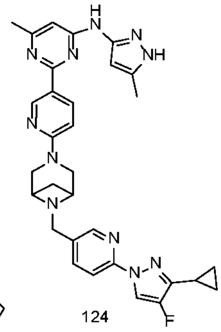 124 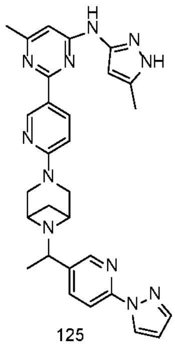 125 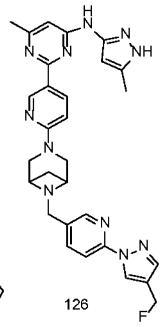 126

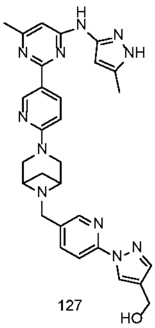 127 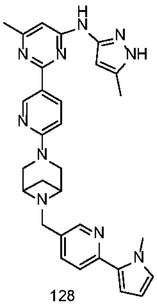 128 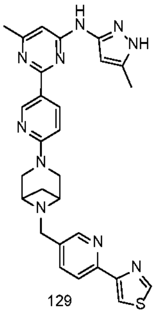 129 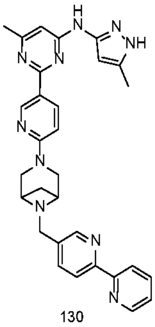 130 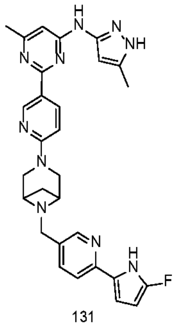 131 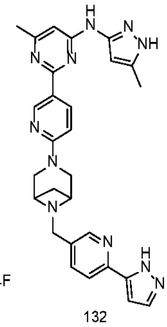 132 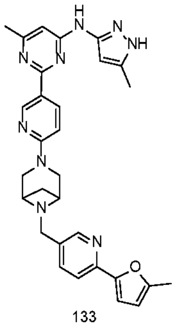 133

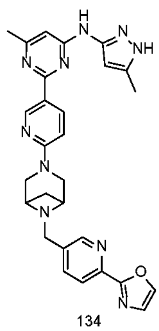 134 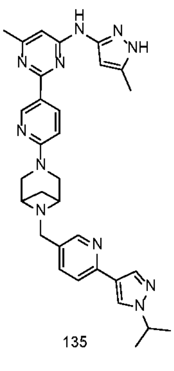 135 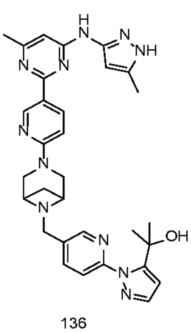 136 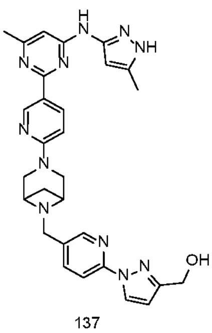 137 and 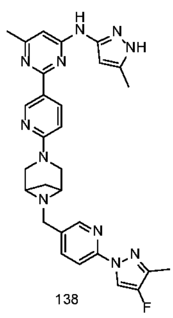 138 .

**10.** A method for preparing the compound according to claim 8, wherein the method comprises following steps:

Route A

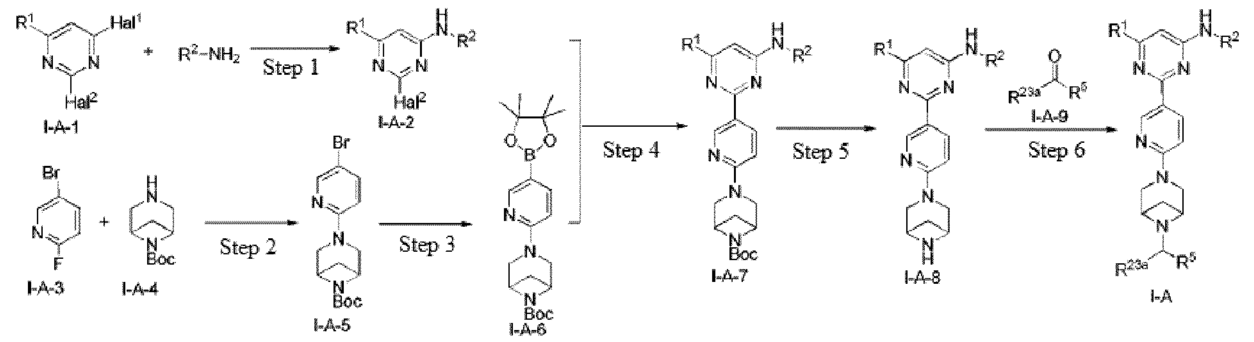


wherein:

Hal[1] and Hal[2] are each independently F, Cl, Br, or I; and preferably, Hal[1] is F, Cl, Br, or I, and Hal[2] is Cl, Br, or I;
$R^1$ is selected from the group consisting of H, cyano, $C_{1-6}$ alkyl, $C_{1-6}$ heteroalkyl (e.g., $C_{1-6}$ alkoxy), $C_{3-8}$ cycloalkyl, 4-6-membered heterocyclyl, and $-NR^{20a}R^{20b}$, and the alkyl, heteroalkyl (e.g., alkoxy), cycloalkyl, and heterocyclyl are each optionally substituted with one or more substituents selected from the group consisting of: halogen, CN, $C_{1-4}$ alkyl, $C_{1-4}$ haloalkyl, $C_{1-4}$ haloalkoxy, and $C_{1-4}$ heteroalkyl (e.g., $C_{1-4}$ alkoxy);
$R^2$ is selected from the group consisting of $_{1-6}$ alkyl, $C_{1-6}$ heteroalkyl, $C_{3-8}$ cycloalkyl, 4-6-membered heterocyclyl, and 5-6-membered heteroaryl, and the alkyl, heteroalkyl, cycloalkyl, heterocyclyl, and heteroaryl are each optionally substituted with one or more substituents selected from the group consisting of: hydroxy, halogen, CN, $C_{1-4}$ alkyl, $C_{1-4}$ haloalkyl, $C_{1-4}$ hydroxyalkyl, $C_{1-4}$ haloalkoxy, $C_{1-4}$ heteroalkyl, and $C_{3-6}$ cycloalkyl;
$R^{23a}$ is selected from the group consisting of H, $C_{1-6}$ alkyl, $C_{1-6}$ alkoxy, and $C_{3-8}$ cycloalkyl, and the alkyl, alkoxy, and cycloalkyl are each optionally substituted with one or more substituents selected from the group consisting of: OH, CN, halogen, $C_{1-4}$ alkyl, $C_{1-4}$ alkoxy, $C_{1-4}$ hydroxyalkyl, $C_{1-4}$ haloalkyl, and $C_{1-4}$haloalkoxy; and
$R^{20a}$, $R^{20b}$, and $R^5$ are as defined in formula I-A of claim 8;
Step 1: reacting compound I-A-1 with $R^2$-$NH_2$ in the presence of a base to generate compound I-A-2;
Step 2: reacting compound I-A-3 with compound I-A-4 in the presence of a base to generate compound I-A-5;
Step 3: reacting the compound I-A-5 with a boron-containing reagent to generate compound I-A-6;
Step 4: reacting the compound I-A-2 with the compound I-A-6 to generate compound I-A-7;
Step 5: deprotecting the compound I-A-7 under an acidic condition to generate compound I-A-8; and
Step 6: reacting the compound I-A-8 with compound I-A-9 to generate compound I-A;
or,
the method comprises following steps:

Route B

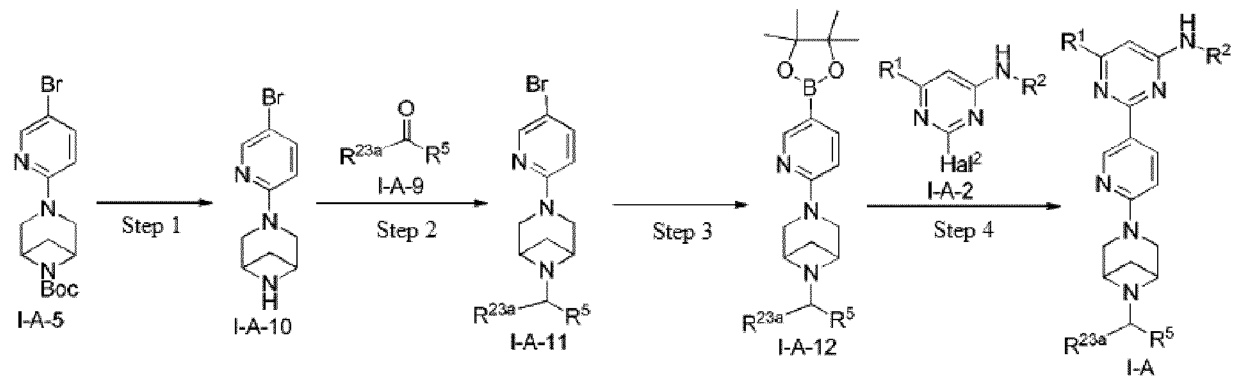


wherein:

each group is as defined in the above Route A;

Step 1: deprotecting the compound I-A-5 under an acidic condition to generate compound I-A-10;
Step 2: reacting the compound I-A-10 with the compound I-A-9 to generate compound I-A-11;
Step 3: reacting the compound I-A-11 with a boron-containing reagent to generate compound I-A-12; and
Step 4: reacting the compound I-A-12 with the compound I-A-2 to generate the compound I-A;
or,

the method comprises following steps:

Route C

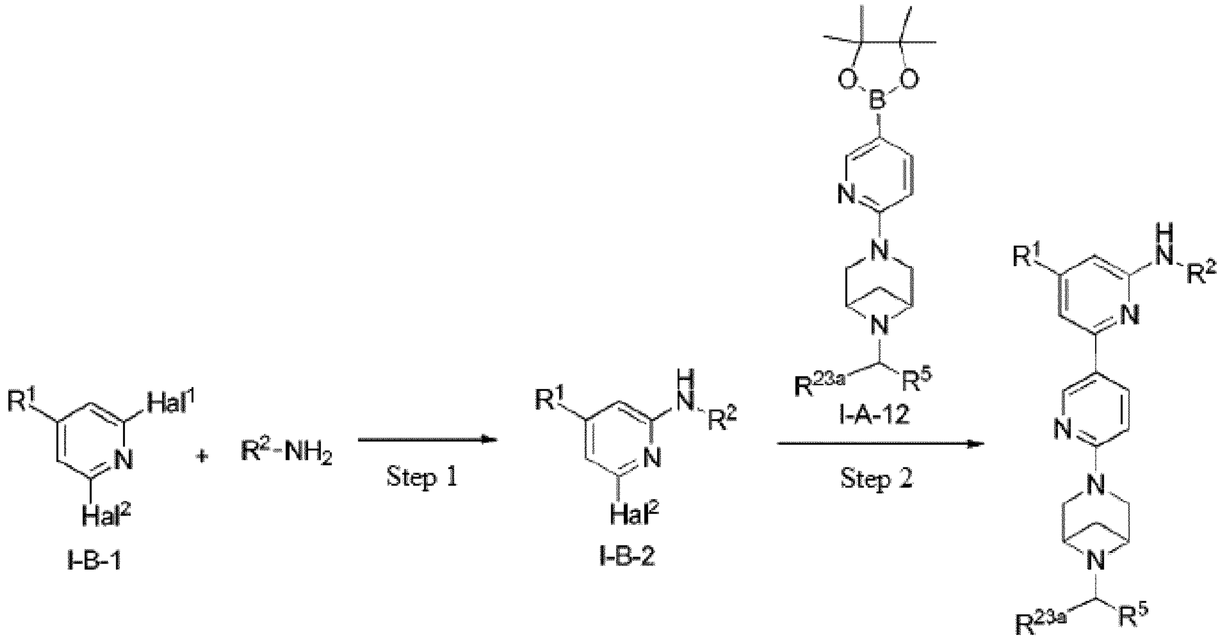

I-B-1 I-B-2 I-A-12 I-B

wherein:

each group is as defined in the above Route A;
Step 1: reacting compound I-B-1 with $R^2$-$NH_2$ in the presence of a base to generate compound I-B-2; and
Step 2: reacting the compound I-B-2 with the compound I-A-12 to generate compound I-B;
or,

the method comprises following steps:

## Route D

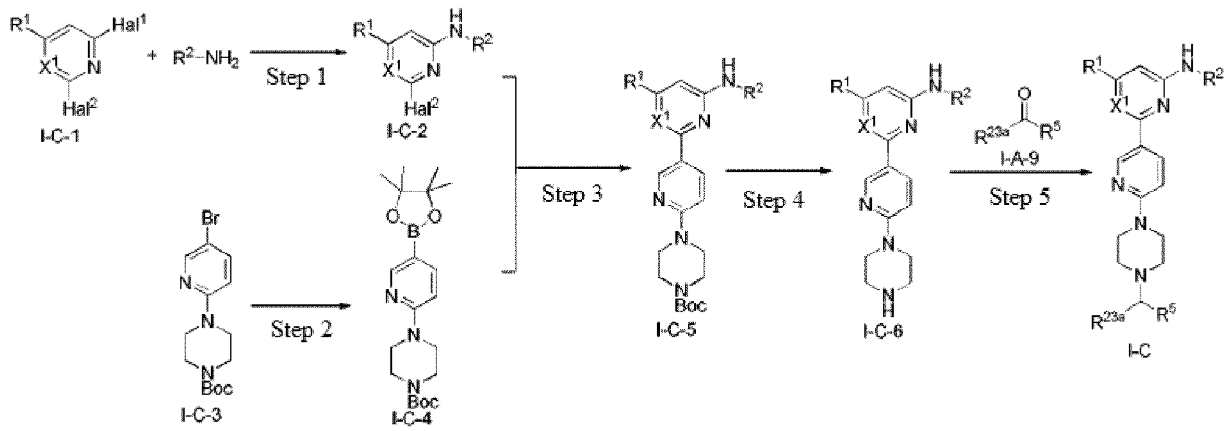


wherein:

each group is as defined in the above Route A; and $X^1$ is selected from CH and N;
Step 1: reacting compound I-C-1 with $R^2$-$NH_2$ in the presence of a base to generate compound I-C-2;
Step 2: reacting compound I-C-3 with a boron-containing reagent to generate compound I-C-4;
Step 3: reacting the compound I-C-2 with the compound I-C-4 to generate compound I-C-5;
Step 4: deprotecting the compound I-C-5 under an acidic condition to generate compound I-C-6; and
Step 5: reacting the compound I-C-6 with the compound I-A-9 to generate compound I-C;
or,

the method comprises following steps:

## Route E

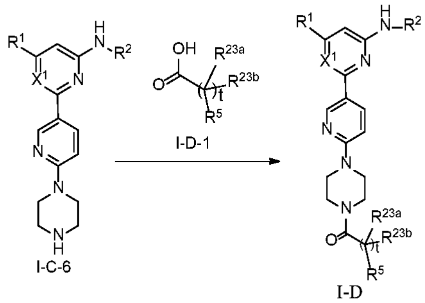


wherein:

$R^1$ and $R^2$ are as defined in the Route A;
$R^5$ is as defined in formula I-D of claim 8;
$R^{23a}$ and $R^{23b}$ are each independently selected from the group consisting of H, $C_{1-6}$ alkyl, $C_{1-6}$ alkoxy, and $C_{3-8}$ cycloalkyl; or $R^{23a}$ and $R^{23b}$ form, together with a C atom to which they are attached, a 3-8-membered cycloalkyl or heterocyclyl, and the alkyl, alkoxy, cycloalkyl, and heterocyclyl are each optionally substituted with one or more substituents selected from the group consisting of: CN, halogen, $C_{1-4}$ alkyl, $C_{1-4}$ alkoxy, $C_{1-4}$ hydroxyalkyl, $C_{1-4}$ haloalkyl, and $C_{1-4}$ haloalkoxy;
$X^1$ is selected from CH and N; and
t is 0 or 1;
or,

the method comprises following steps:

Route F

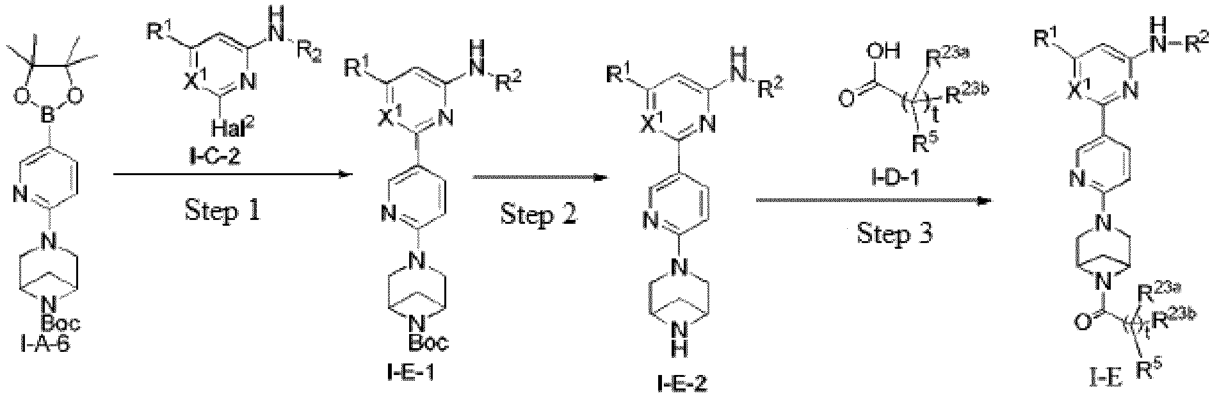


wherein:

$R^1$, $R^2$, $R^5$, $R^{23a}$, $R^{23b}$, and t are as defined in the Route E;
$X^1$ is selected from CH and N; and
$Hal^2$ is F, Cl, Br, or I; and preferably, $Hal^2$ is Cl, Br, or I;
Step 1: reacting the compound I-C-2 with the compound I-A-6 to generate compound I-E-1;
Step 2: deprotecting the compound I-E-1 under an acidic condition to generate compound I-E-2; and
Step 3: reacting the compound I-E-2 with the compound I-D-1 through a condensation reaction to generate compound I-E;
or,

the method comprises following steps:

Route G

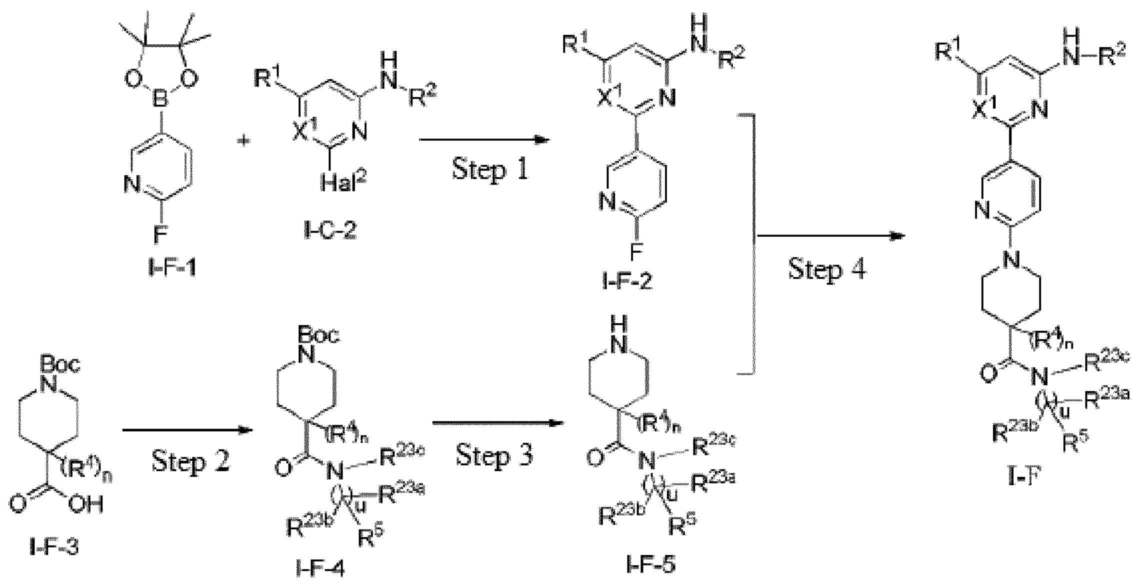


wherein:

$R^1$, $R^2$, $R^{23a}$, $R^{23b}$, and $R^5$ are as defined in the Route E;
$X^1$ is selected from CH and N;

$R^4$ is absent or is selected from the group consisting of hydroxy, CN,$C_{1-6}$ alkyl, $C_{1-6}$ haloalkyl, and $C_{1-6}$ heteroalkyl (e.g., $C_{1-6}$ alkoxy);

$R^{23c}$ is H, $C_{1-3}$ alkyl, or $C_{1-3}$ alkoxy, and the alkyl and alkoxy are each optionally substituted with one or more substituents selected from the group consisting of: OH, CN, halogen, $C_{1-4}$ alkoxy, and $C_{1-4}$ hydroxyalkyl;

$Hal^2$ is F, Cl, Br, or I; and preferably, $Hal^2$ is Cl, Br, or I;

u is 0 or 1; and

n is 0 or 1;

Step 1: reacting the compound I-C-2 with compound I-F-1 to generate compound I-F-2;

Step 2: reacting compound I-F-3 with an amine to generate compound I-F-4;

Step 3: deprotecting the compound I-F-4 under an acidic condition to generate compound IF-5; and

Step 4: reacting the compound I-F-2 with the compound I-F-5 in the presence of a base to generate compound I-F;

or,

the method comprises following steps:

Route H

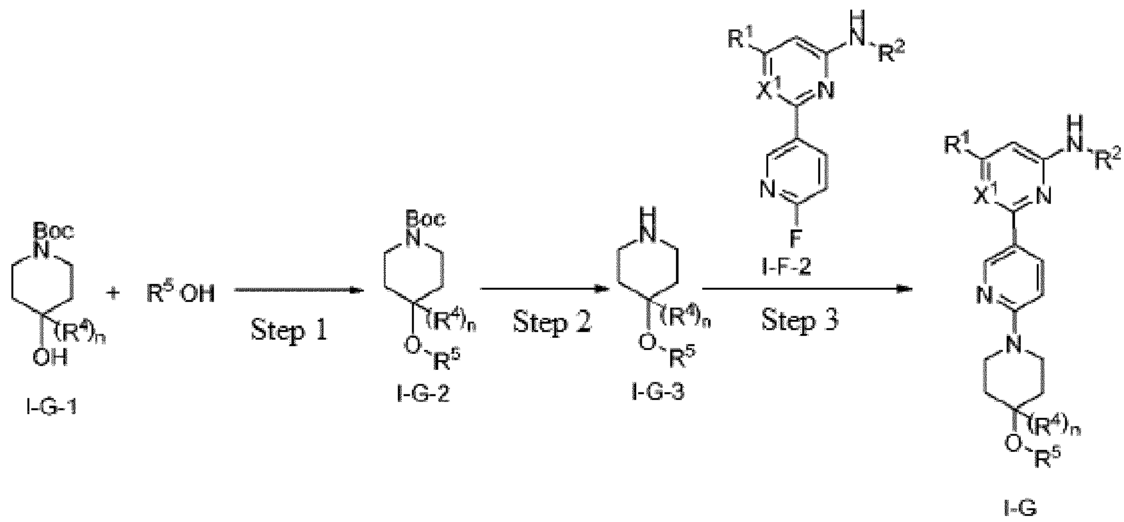


wherein:

$R^1$ and $R^2$ are as defined in the Route A;

$X^1$ is selected from CH and N;

$R^4$ is selected from the group consisting of H, $C_{1-6}$ alkyl, $C_{1-6}$ haloalkyl, and $C_{1-6}$ heteroalkyl;

$R^5$ is as defined in the above formula I-G; and

n is 0 or 1;

Step 1: reacting compound I-G-1 with $R^5$-OH to generate compound I-G-2;

Step 2: deprotecting the compound I-G-2 under an acidic condition to generate compound I-G-3; and

Step 3: reacting the compound I-F-2 with the compound I-G-3 through a nucleophilic substitution reaction in the presence of a base to generate compound I-G.

**11.** A pharmaceutical composition, comprising a prophylactically or therapeutically effective amount of the compound according to any one of claims 1 to 9, the stereoisomer, tautomer, or mixture thereof, the N-oxide thereof, the pharmaceutically acceptable salt, eutecticum, polymorph, or solvate thereof, or the stable isotope derivative, metabolite, or prodrug thereof;

optionally, the pharmaceutical composition further comprises one or more pharmaceutically acceptable carriers.

**12.** Use of the compound according to any one of claims 1 to 9, the stereoisomer, tautomer, or mixture thereof, the N-oxide thereof, the pharmaceutically acceptable salt, eutecticum, polymorph, or solvate thereof, or the stable isotope

derivative, metabolite, or prodrug thereof, or the pharmaceutical composition according to claim 11 in the preparation of a drug for preventing or treating a disease or condition associated with RET activity;

wherein, preferably the disease or condition associated with RET activity is a cancer or tumor, or an irritable bowel syndrome;
and the cancer or tumor further preferably is lung cancer (such as non-small cell lung cancer), breast cancer, head and neck cancer, rectal cancer, liver cancer, lymphoma, thyroid cancer (such as medullary thyroid cancer or papillary thyroid cancer), colon cancer, multiple myeloma, melanoma, glioma, brain tumor, or sarcoma.

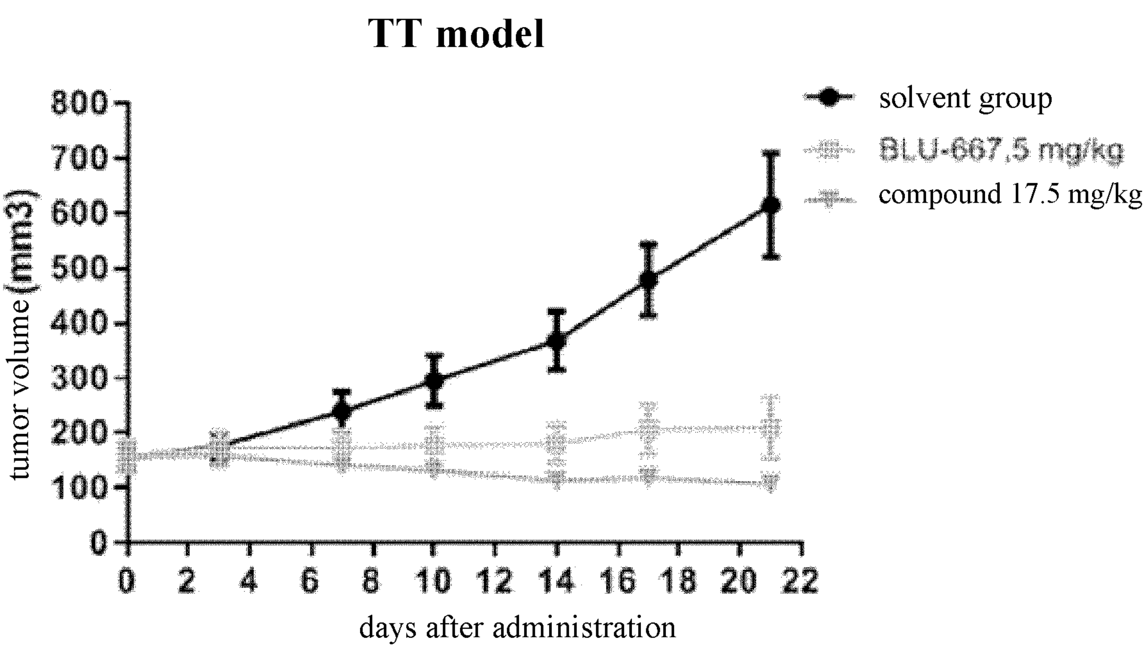
TT model
800
700
600
500
400
300
200
100
0
tumor volume (mm3)
0 2 4 6 8 10 12 14 16 18 20 22
days after administration
solvent group
BLU-667,5 mg/kg
compound 17.5 mg/kg

**FIG. 1**

# INTERNATIONAL SEARCH REPORT

International application No.
PCT/CN2020/074696

**A. CLASSIFICATION OF SUBJECT MATTER**

C07D 487/08 (2006.01) i; C07D 471/08 (2006.01) i; C07D 401/14 (2006.01) i; C07D 413/14 (2006.01) i; C07D 417/14 (2006.01) i; A61P 35/00 (2006.01) i; A61K 31/506 (2006.01) i; A61K 31/504 (2006.01) i; A61K 31/444 (2006.01) i; A61K 31/496 (2006.01) i

According to International Patent Classification (IPC) or to both national classification and IPC

B. FIELDS SEARCHED

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNABS; CNTXT; CJFD; VEN; USTXT; EPTXT; WOTXT; STN-REGISTRY; STN-CAPLUS; CNKI: 科伦博泰, 陈忠辉, 段霜霜, 李桂英, 韩润丰, 孙启正, 景连栋, 韩晓军, 田强, 宋宏梅, 薛彤彤, 王晶翼, 蛋白激酶, 吡啶, 哌啶, 哌嗪, 联吡啶, 嘧啶, 氮杂环庚烷, 癌症, 肿瘤, RET, rearranged during transfection, protein kinase, pyridine, pyridyl, piperidin+, piperazin+, bipyridin+, pyrimidin+, azacycloheptane, cancer, tumor, tumour, 结构式 (I), structural formula (I)

C. DOCUMENTS CONSIDERED TO BE RELEVANT

| Category* | Citation of document, with indication, where appropriate, of the relevant passages | Relevant to claim No. |
|---|---|---|
| X | CN 103298788 A (NIPPON SHINYAKU CO., LTD.), 11 September 2013 (11.09.2013), description, paragraphs [0026]-[0061], [0261]-[0289], [0293]-[0295], [0299]-[0854], [0890]-[0899], [0985]-[0987], [1041]-[1056], [1113]-[1130] and [1134]-[1139] | 1-12 |
| X | WO 2012154518 A1 (MERCK SHARP & DOHME CORP. et al.), 15 November 2012 (15.11.2012), description, page 20, paragraph 2 to page 24, paragraph 1, pages 61 and 65, tables, page 90, lines 21-25 and page 91, lines 10-14 | 1-9, 11, 12 |
| X | CN 101166734 A (JAPAN TOBACCO INC.), 23 April 2008 (23.04.2008), description, page 9, last paragraph to page 10, paragraph 2, page 128, paragraph 2 to page 155, paragraph 2, page 156, table 1-1 to page 309, table 1-153 | 1-9, 11, 12 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 24 March 2020 | 29 April 2020 |

| Name and mailing address of the ISA | Authorized officer |
|---|---|
| State Intellectual Property Office of the P. R. China<br>No. 6, Xitucheng Road, Jimenqiao<br>Haidian District, Beijing 100088, China<br>Facsimile No. (86-10) 62019451 | WU, Shanshan<br>Telephone No. (86-512) 88996534 |

Form PCT/ISA/210 (second sheet) (January 2015)

# INTERNATIONAL SEARCH REPORT

International application No.
PCT/CN2020/074696

C (Continuation). DOCUMENTS CONSIDERED TO BE RELEVANT

| Category* | Citation of document, with indication, where appropriate, of the relevant passages | Relevant to claim No. |
|---|---|---|
| X | LI, Bingke et al., In Silico Prediction of Spleen Tyrosine Kinase Inhibitors Using Machine Learning Approaches and an Optimized Molecular Descriptor Subset Generated by Recursive Feature Elimination Method, Computers in Biology and Medicine, 43(4), 01 May 2013 (01.05.2013), ISSN: 0010-4825, abstract, and supporting information, tables S1 and S2 | 1-7, 11, 12 |
| X | "RN1349252-44-3" REGISTRY enter STN, 05 December 2011 (05.12.2011) | 1-7 |
| X | "RN1349069-14-2" REGISTRY enter STN, 05 December 2011 (05.12.2011) | 1-7 |
| X | "RN909344-08-7" REGISTRY enter STN, 02 October 2006 (02.10.2006) | 1-7 |
| X | "RN909344-04-3" REGISTRY enter STN, 02 October 2006 (02.10.2006) | 1-7 |
| X | "RN909344-03-2" REGISTRY enter STN, 02 October 2006 (02.10.2006) | 1-7 |
| X | "RN909343-99-3" REGISTRY enter STN, 02 October 2006 (02.10.2006) | 1-7 |
| X | "RN1374201-58-7" REGISTRY enter STN, 22 May 2012 (22.05.2012) | 1-7 |

Form PCT/ISA/210 (continuation of second sheet) (January 2015)

**INTERNATIONAL SEARCH REPORT**
Information on patent family members

International application No.
PCT/CN2020/074696

| Patent Documents referred in the Report | Publication Date | Patent Family | Publication Date |
|---|---|---|---|
| CN 103298788 A | 11 September 2013 | BR 112013010552 A2 | 02 August 2016 |
| | | US 2013225548 A1 | 29 August 2013 |
| | | WO 2012057262 A1 | 03 May 2012 |
| | | KR 20130130729 A | 02 December 2013 |
| | | MX 2013004407 A | 31 May 2013 |
| | | EP 2634176 A1 | 04 September 2013 |
| | | RU 2013124395 A | 10 December 2014 |
| | | IN 201303942 P4 | 09 January 2015 |
| | | EP 2634176 A4 | 16 April 2014 |
| | | AU 2011321348 A1 | 09 May 2013 |
| | | CA 2815170 A1 | 03 May 2012 |
| WO 2012154518 A1 | 15 November 2012 | US 2014249130 A1 | 04 September 2014 |
| | | EP 2706852 B1 | 22 August 2018 |
| | | TW 201249829 A | 16 December 2012 |
| | | US 9145391 B2 | 29 September 2015 |
| | | EP 2706852 A4 | 05 November 2014 |
| | | EP 2706852 A1 | 19 March 2014 |
| CN 101166734 A | 23 April 2008 | EP 1854793 A4 | 26 January 2011 |
| | | BR 200607062 A2 | 04 August 2009 |
| | | IN 200703771 P4 | 23 November 2007 |
| | | AU 2006219231 B2 | 14 January 2010 |
| | | RU 2363699 C2 | 10 August 2009 |
| | | JP 4741043 B2 | 03 August 2011 |
| | | EP 1854793 A1 | 14 November 2007 |
| | | US 7803801 B2 | 28 September 2010 |
| | | SG 135392 A1 | 29 October 2007 |
| | | MX 2007010459 A | 01 October 2007 |
| | | KR 917511 B1 | 16 September 2009 |
| | | AU 2006219231 A1 | 20 September 2007 |
| | | US 2006205731 A1 | 14 September 2006 |
| | | SG 135392 B | 30 June 2009 |
| | | WO 2006093247 A1 | 08 September 2006 |
| | | NZ 561000 A | 29 January 2010 |
| | | KR 20070099047 A | 08 October 2007 |
| | | ZA 200707227 A | 26 August 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017079140 A1


### Non-patent literature cited in the description


- *Nature Reviews Cancer,* 2014, vol. 14 (3), 173-86
- **STAHL ; WERMUTH.** Handbook of Pharmaceutical Salts: Properties, Selection, and Use. Wiley-VCH, 2002
- **T. L. GILCHRIST.** Comprehensive Organic Synthesis. Academic Press, vol. 7, 748-750
- **G. W. H. CHEESEMAN ; E. S. G. WERSTIUK.** Advances in Heterocyclic Chemistry. Academic Press, vol. 22, 390-392
- **T. HIGUCHI ; V. STELLA.** Pro-drugs as Novel Delivery Systems. *ACS Symposium Series,* vol. 14
- **H. BUNDGAARD.** Design of Prodrugs. Elsevier, 1985
- **T. W. GREENE ; P. G. M. WUTS.** Protective Groups in Organic Synthesis. John Wiley & Sons, 1991
- Remington's Pharmaceutical Sciences. 1990